# EUROPEAN PATENT SPECIFICATION

(11) **EP 3 623 369 B1**
(45) Date of publication and mention of the grant of the patent: **25.10.2023**
(21) Application number: 18193921.6
(22) Date of filing: 12.09.2018
(51) Int. Cl.: C07D 413/14, C07D 417/14, C07D 471/04, C07D 487/04, C07D 487/08, C07D 487/10, C07D 498/04, C07D 498/08, C07D 513/04, A61P 37/02, A61P 37/00

(54) **NOVEL MORPHOLINYL AMINE COMPOUNDS FOR THE TREATMENT OF AUTOIMMUNE DISEASE**
NEUARTIGE MORPHOLINYLAMINVERBINDUNGEN ZUR BEHANDLUNG VON AUTOIMMUNERKRANKUNGEN
NOUVEAUX COMPOSÉS DE MORPHOLINYLE AMINE POUR LE TRAITEMENT DES MALADIES AUTO-IMMUNES

(43) Date of publication of application: 18.03.2020
(73) Proprietor: F. Hoffmann-La Roche AG, 4070 Basel (CH)
(72) Inventor: DEY, Fabian, 4070 Basel (CH); HU, Taishan, Shanghai, 201203 (CN); LIU, Haixia, Shanghai, 201203 (CN); SHEN, Hong, Shanghai, 201203 (CN); ZHANG, Weixing, Shanghai, 201203 (CN); ZHANG, Zhiwei, Shanghai, 201203 (CN); ZHU, Wei, Shanghai, 201203 (CN); ZHU, Ge, Shanghai, 201203 (CN)
(74) Representative: Bernard, Guillaume

(56) References cited:
- US-A1- 2015 105 370
- US-A1- 2017 174 653

## Description

The present invention relates to organic compounds useful for therapy and/or prophylaxis in a mammal, and in particular to antagonist of TLR7 and/or TLR8 and/or TLR9 useful for treating systemic lupus erythematosus or lupus nephritis.

### FIELD OF THE INVENTION

Autoimmune connective tissue disease (CTD) include prototypical autoimmune syndromes such as Systemic Lupus Erythematosus (SLE), primary Sjögren's syndrome (pSjS), mixed connective tissue disease (MCTD), Dermatomyositis/Polymyositis (DM/PM), Rheumatoid Arthritis (RA), and systemic sclerosis (SSc). With the exception of RA, no really effective and safe therapies are available to patients. SLE represents the prototypical CTD with a prevalence of 20-150 per 100,000 and causes broad inflammation and tissue damage in distinct organs, from commonly observed symptoms in the skin and joints to renal, lung, or heart failure. Traditionally, SLE has been treated with nonspecific anti-inflammatory or immunosuppressive drugs. However, long term usage of immunosuppressive drug, e.g. corticosteroids is only partially effective, and is associated with undesirable toxicity and side effects. Belimumab is the only FDA-approved drug for lupus in the last 50 years, despite its modest and delayed efficacy in only a fraction of SLE patients (Navarra, S. V. et al Lancet 2011, 377, 721.). Other biologics, such as anti-CD20 mAbs, mAbs against or soluble receptors of specific cytokines, have failed in most clinical studies. Thus, novel therapies are required that provide sustained improvement in a greater proportion of patient groups and are safer for chronic use in many autoimmune as well as auto-inflammation diseases.

Toll Like Receptors (TLR) are an important family of pattern recognition receptors (PRR) which can initiate broad immune responses in a wide variety of immune cells. As natural host defense sensors, endosomal TLRs 7, 8 and 9 recognize nucleic acids derived from viruses, bacteria; specifically, TLR7/8 and TLR9 recognize single-stranded RNA (ssRNA) and single-stranded CpG-DNA, respectively. However, aberrant nucleic acid sensing of TRL7/8/9 is considered as a key node in a broad of autoimmune and auto-inflammatory diseases (Krieg, A. M. et al. Immunol. Rev. 2007, 220, 251. Jiménez-Dalmaroni, M. J. et al Autoimmun Rev. 2016, 15, 1. Chen, J. Q., et al. Clinical Reviews in Allergy & Immunology 2016, 50, 1.) Therefore, TLR7/8/9 represents a new therapeutic target for autoimmune and auto-inflammatory diseases, for which no effective steroid-free and non-cytotoxic oral drugs exist, and inhibition of these pathways from the very upstream may deliver satisfying therapeutic effects. From a safety perspective, because there are multiple nucleic acid sensing pathways (e.g. other TLRs, cGAS/STING), such redundancy should still allow responses to infection in the presence of TLR7/8/9 inhibition. US2015/105370 relates to selectively substituted quinoline compounds that act as antagonists or inhibitors for Toll-like receptors 7 and/or 8, and their use in pharmaceutical compositions effective for treatment of systemic lupus erythematosus (SLE) and lupus nephritis. US2017/174653 relates to compounds and pharmaceutically acceptable compositions thereof, useful as TLR7/8 antagonists.

As such, we proposed and invented oral compounds that target and suppress TLR7/8/9 for the treatment of autoimmune and auto-inflammatory diseases.

### SUMMARY OF THE INVENTION

The present invention relates to novel compounds of formula (I) or (Ia), 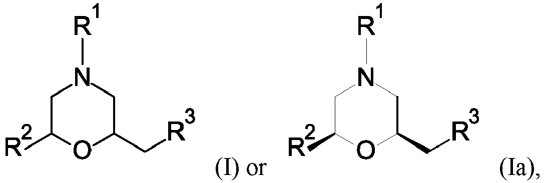 wherein
R¹ is 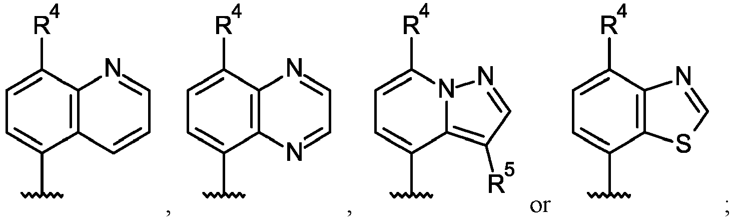 wherein R⁴ is cyano or halogen; R⁵ is H or halogen;
R² is C₁₋₆alkyl;
R³ is
   1,3-dihydropyrrolo[3,4-c]pyridinyl substituted by piperazinyl;
   3,4-dihydro-1H-2,7-naphthyridinyl substituted by piperazinyl;
   3,4-dihydro-1H-isoquinolinyl substituted by one or two substituents independently selected from hydroxy, piperazinyl and C₁₋₆alkylpiperazinyl;
   3,4-dihydro-2H-quinolinyl substituted by piperazinyl;
   5,7-dihydropyrrolo[3,4-b]pyridinyl substituted by piperazinyl;
   5,7-dihydropyrrolo[3,4-d]pyrimidinyl substituted by piperazinyl or C₁₋₆alkylpiperazinyl;
   6,7-dihydro-4H-thiazolo[5,4-c]pyridinyl substituted by C₁₋₆alkylpiperazinyl;
   7,8-dihydro-5H-1,6-naphthyridinyl substituted by piperazinyl;
   isoindolinyl substituted by one or two substituents independently selected from ((C₁₋₆alkyl)zamino)C₁₋₆alkoxy; (C₁₋₆alkoxyC₁₋₆alkyl)piperazinyl; (C₁₋₆alkyl)₂piperazinyl; (C₁₋₆alkyl)₂pyridinyl; (hydroxyC₁₋₆alkyl)piperazinyl; 1,3,4,6,7,8,9,9a-octahydropyrido[1,2-a]pyrazinyl; 1,6-diazaspiro[3.3]heptanyl; 2,6-diazaspiro[3.3]heptanyl; 3,4,6,7,9,9a-hexahydro-1H-pyrazino[2,1-c][1,4]oxazinyl; 3,8-diazabicyclo[3.2.1]octanyl; 3-oxa-7,9-diazabicyclo[3.3.1]nonanyl; 4,7-diazaspiro[2.5]octanyl; aminoazetidinyl; aminoC₁₋₆alkylamino; C₁₋₆alkoxycarbonylpiperazinyl; C₁₋₆alkyl; C₁₋₆alkylimidazolyl; C₁₋₆alkylpiperazinyl; C₃₋₇cycloalkylpiperazinyl; carboxypiperazinyl; haloC₁₋₆alkylpiperazinyl; halogen; morpholinylC₁₋₆alkyl; oxopiperazinyl; piperazinyl; piperidinyl; piperidinylC₁₋₆alkyl; pyrrolidinyl and pyrrolidinyloxy; or
   -NR^{6a}R^{6b}; wherein
      R^{6a} is H or C₁₋₆alkyl;
      R^{6b} is phenylC₁₋₆alkyl substituted by piperazinyl;
or a pharmaceutically acceptable salt, enantiomer or diastereomer thereof.

Another object of the present invention is related to novel compounds of formula (I) or (Ia), their manufacture, medicaments based on a compound in accordance with the invention and their production as well as the use of compounds of formula (I) or (Ia) as TLR7 and/or TLR8 and/or TLR9 antagonist, and for the treatment or prophylaxis of systemic lupus erythematosus or lupus nephritis. The compounds of formula (I) or (Ia) show superior TLR7 and/or TLR8 and/or TLR9 antagonism activity. In addition, the compounds of formula (I) or (Ia) also show good cytotoxicity, solubility, human microsome stability and SDPK profiles, as well as low CYP inhibition.

### DETAILED DESCRIPTION OF THE INVENTION

### DEFINITIONS

The term "C₁₋₆alkyl" denotes a saturated, linear or branched chain alkyl group containing 1 to 6, particularly 1 to 4 carbon atoms, for example methyl, ethyl, n-propyl, isopropyl, n-butyl, isobutyl, and *tert*-butyl. Particular "C₁₋₆alkyl" groups are methyl, ethyl and n-propyl.

The term "halogen" and "halo" are used interchangeably herein and denote fluoro, chloro, bromo, or iodo.

The term "haloC₁₋₆alkyl" denotes an alkyl group wherein at least one of the hydrogen atoms of the alkyl group has been replaced by same or different halogen atoms, particularly fluoro atoms. Examples of haloC₁₋₆alkyl include monofluoro-, difluoro-or trifluoro-methyl, - ethyl or -propyl, for example 3,3,3-trifluoropropyl, 2-fluoroethyl, 2,2,2-trifluoroethyl, fluoromethyl, difluoromethyl, trifluoromethyl and trifluoroethyl.

The term "heteroaryl" denotes a monovalent aromatic heterocyclic mono- or bicyclic ring system of 5 to 12 ring atoms, comprising 1, 2, 3 or 4 heteroatoms selected from N, O and S, the remaining ring atoms being carbon. Examples of heteroaryl moieties include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, or quinoxalinyl.

The term "C₃₋₇cycloalkyl" denotes a saturated monocyclic or bicyclic carbon ring containing from 3 to 7 carbon atoms, particularly from 3 to 6 carbon atoms, for example, cyclopropyl, cyclobutyl, cyclopentyl, cyclohexyl, cycloheptyl and bicyclo[1.1.1]pentanyl. Particular "C₃₋₇cycloalkyl" group is cyclopropyl.

The term "heterocyclyl" denotes a monovalent saturated or partly unsaturated mono- or bicyclic ring system of 3 to 12 ring atoms, comprising 1, 2, or 3 ring heteroatoms selected from N, O and S, the remaining ring atoms being carbon. In particular embodiments, heterocyclyl is a monovalent saturated monocyclic ring system of 4 to 10 ring atoms, comprising 1, 2, or 3 ring heteroatoms selected from N, O and S, the remaining ring atoms being carbon. Heterocyclyl can be fully or partially saturated. Examples for monocyclic saturated heterocyclyl are aziridinyl, oxiranyl, azetidinyl, oxetanyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for partially saturated monocyclic heterocyclyl are dihydrofuryl, imidazolinyl, dihydro-oxazolyl, tetrahydropyridinyl, and dihydropyranyl. Examples for bicyclic heterocyclyl are1,3-dihydropyrrolo[3,4-c]pyridinyl; 2,3,3a,7a-tetrahydro-1H-indenyl; 3,4-dihydro-1H-isoquinolinyl; 3,4-dihydro-1H-2,7-naphthyridinyl; 3,4-dihydro-2H-quinolinyl; 5,7-dihydropyrrolo[3,4-b]pyridinyl; 5,7-dihydropyrrolo[3,4-d]pyrimidinyl; 6,7-dihydro-4H-thiazolo[5,4-c]pyridinyl; 7,8-dihydro-5H-1,6-naphthyridinyl; isoindolinyl; azabicyclo[3.2.1]octanyl; azabicyclo[3.3.1]nonanyl; azaspiro[3.3]heptanyl; oxaazabicyclo[3.3.1]nonanyl; oxadiazaspiro[4.5]decanyl; diazabicyclo[2.2.2]octanyl; diazabicyclo[3.2.1]octanyl; diazabicyclo[4.2.0]octanyl; diazaspiro[2.5]octanyl; diazaspiro[3.3]heptanyl; diazaspiro[3.4]octanyl; diazaspiro[3.5]nonanyl; diazaspiro[3.6]decanyl; diazaspiro[4.4]nonanyl; diazaspiro[4.5]decanyl; diazaspiro[5.5]undecanyl; oxadiazabicyclo[3.3.1]nonanyl; oxadiazaspiro[5.5]undecanyl; and oxodiazaspiro[4.4]nonanyl. Monocyclic or bicyclic heterocyclyl can be further substituted by halogen, hydroxy, hydroxyC₁₋₆alkyl, amino, aminoC₁₋₆alkyl, aminoC₁₋₆alkylcarbonyl, carboxy, C₁₋₆alkoxycarbonyl, C₁₋₆alkylcarbonylamino, (C₁₋₆alkyl)₂amino, carbamoyl, C₁₋₆alkyl, haloC₁₋₆alkyl, phenyl, phenylC₁₋₆alkyl, or heterocyclyl.

The term "heterocyclyloxy" denotes heterocyclyl-O-.

The term "enantiomer" denotes two stereoisomers of a compound which are non-superimposable mirror images of one another.

The term "diastereomer" denotes a stereoisomer with two or more centers of chirality and whose molecules are not mirror images of one another. Diastereomers have different physical properties, e.g. melting points, boiling points, spectral properties, and reactivities.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, A-ethylpiperidine, and polyamine resins.

The term "A pharmaceutically active metabolite" denotes a pharmacologically active product produced through metabolism in the body of a specified compound or salt thereof. After entry into the body, most drugs are substrates for chemical reactions that may change their physical properties and biologic effects. These metabolic conversions, which usually affect the polarity of the compounds of the invention, alter the way in which drugs are distributed in and excreted from the body. However, in some cases, metabolism of a drug is required for therapeutic effect.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

### ANTAGONIST OF TLR7 AND/OR TLR8 AND/OR TLR9

The present invention relates to a compound of formula (I), 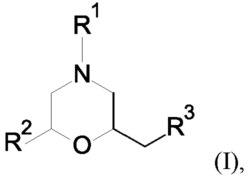 wherein
R¹ is 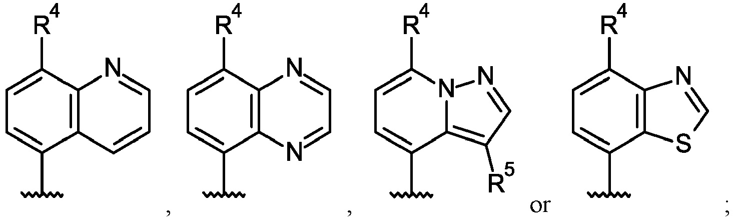 wherein R⁴ is cyano or halogen; R⁵ is H or halogen;
R² is C₁₋₆alkyl;
R³ is
   1,3-dihydropyrrolo[3,4-c]pyridinyl substituted by piperazinyl;
   3,4-dihydro-1H-2,7-naphthyridinyl substituted by piperazinyl;
   3,4-dihydro-1H-isoquinolinyl substituted by one or two substituents independently selected from hydroxy, piperazinyl and C₁₋₆alkylpiperazinyl;
   3,4-dihydro-2H-quinolinyl substituted by piperazinyl;
   5,7-dihydropyrrolo[3,4-b]pyridinyl substituted by piperazinyl;
   5,7-dihydropyrrolo[3,4-d]pyrimidinyl substituted by piperazinyl or C₁₋₆alkylpiperazinyl;
   6,7-dihydro-4H-thiazolo[5,4-c]pyridinyl substituted by C₁₋₆alkylpiperazinyl;
   7,8-dihydro-SH-1,6-naphthyridinyl substituted by piperazinyl;
   isoindolinyl substituted by one or two substituents independently selected from ((C₁₋₆alkyl)₂amino)C₁₋₆alkoxy; (C₁₋₆alkoxyC₁₋₆alkyl)piperazinyl; (C₁₋₆alkyl)₂piperazinyl; (C₁₋₆alkyl)₂pyridinyl; (hydroxyC₁₋₆alkyl)piperazinyl; 1,3,4,6,7,8,9,9a-octahydropyrido[1,2-a]pyrazinyl; 1,6-diazaspiro[3.3]heptanyl; 2,6-diazaspiro[3.3]heptanyl; 3,4,6,7,9,9a-hexahydro-1H-pyrazino[2,1-c][1,4]oxazinyl; 3,8-diazabicyclo[3.2.1]octanyl; 3-oxa-7,9-diazabicyclo[3.3.1]nonanyl; 4,7-diazaspiro[2.5]octanyl; aminoazetidinyl; aminoC₁₋₆alkylamino; C₁₋₆alkoxycarbonylpiperazinyl; C₁₋₆alkyl; C₁₋₆alkylimidazolyl; C₁₋₆alkylpiperazinyl; C₃₋₇cycloalkylpiperazinyl; carboxypiperazinyl; haloC₁₋₆alkylpiperazinyl; halogen; morpholinylC₁₋₆alkyl; oxopiperazinyl; piperazinyl; piperidinyl; piperidinylC₁₋₆alkyl; pyrrolidinyl and pyrrolidinyloxy; or
   -NR^{6a}R^{6b}; wherein
      R^{6a} is H or C₁₋₆alkyl;
      R^{6b} is phenylC₁₋₆alkyl substituted by piperazinyl;
or a pharmaceutically acceptable salt, enantiomer or diastereomer thereof.

A another embodiment of present invention is (ii) a compound of formula (Ia), 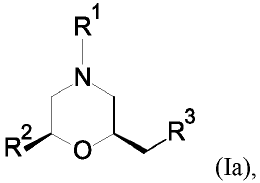 wherein R¹, R² and R³ are as described herein.
R¹ is
   A further embodiment of present invention is (iii) a compound of formula (I) or (Ia) according to (i) or (ii), wherein
R¹ is 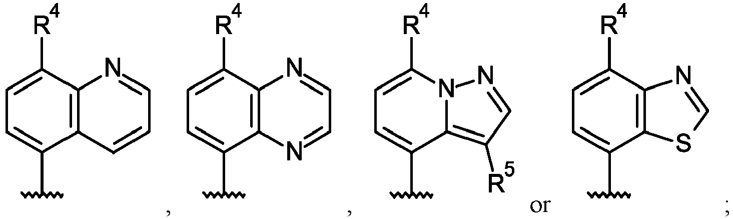 wherein R⁴ is cyano or chloro; R⁵ is H or fluoro;
R² is methyl;
R³ is piperazinyl-1,3-dihydropyrrolo[3,4-c]pyridinyl; piperazinyl-3,4-dihydro-1H-2,7-naphthyridinyl; piperazinyl-3,4-dihydro-1H-isoquinolinyl; methylpiperazinyl-3,4-dihydro-1H-isoquinolinyl; hydroxy(piperazinyl)-3,4-dihydro-1H-isoquinolinyl; piperazinyl-3,4-dihydro-2H-quinolinyl; piperazinyl-5,7-dihydropyrrolo[3,4-b]pyridinyl; piperazinyl-5,7-dihydropyrrolo[3,4-d]pyrimidinyl; methylpiperazinyl-5,7-dihydropyrrolo[3,4-d]pyrimidinyl; methylpiperazinyl-6,7-dihydro-4H-thiazolo[5,4-c]pyridinyl; piperazinyl-7 ,8-dihydro-5H -1, 6-naphthyridinyl; (difluoromethyl)piperazinylisoindolinyl; (dimethylamino)ethoxyisoindolinyl; (hydroxymethyl)piperazinylisoindolinyl; (methoxyethyl)piperazinylisoindolinyl; 1,3,4,6,7,8,9,9a-octahydropyrido[1,2-a]pyrazinylisoindolinyl; 1,6-diazaspiro[3.3]heptanylisoindolinyl; 2,6-diazaspiro[3.3]heptanylisoindolinyl; 3,4,6,7,9,9a-hexahydro-1H-pyrazino[2,1-c][1,4]oxazinylisoindolinyl; 3,8-diazabicyclo[3.2.1]octanylisoindolinyl; 3-oxa-7,9-diazabicyclo[3.3.1]nonanylisoindolinyl; 4,7-diazaspiro[2.5]octanylisoindolinyl; aminoazetidinylisoindolinyl; aminoethylaminoisoindolinyl; carboxypiperazinylisoindolinyl; cyclopropylpiperazinylisoindolinyl; dimethylpiperazinylisoindolinyl; dimethylpyridinylisoindolinyl; ethoxycarbonylpiperazinylisoindolinyl; methoxycarbonylpiperazinylisoindolinyl; methylimidazolylisoindolinyl; methylpiperazinyl(fluoro)isoindolinyl; methylpiperazinylisoindolinyl; morpholinylmethylisoindolinyl; oxopiperazinylisoindolinyl; piperazinyl(fluoro)isoindolinyl; piperazinyl(methyl)isoindolinyl; piperazinylisoindolinyl; piperidinylisoindolinyl; piperidinylmethylisoindolinyl; pyrrolidinylisoindolinyl; pyrrolidinyloxyisoindolinyl; (piperazinylphenyl)methylamino or (piperazinylphenyl)methyl(methyl)amino;
or a pharmaceutically acceptable salt, enantiomer or diastereomer thereof.

A further embodiment of present invention is (iv) a compound of formula (I) or (Ia) according to (iii), wherein R¹ is 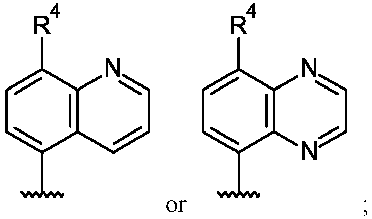 wherein R⁴ is cyano or halogen.

A further embodiment of present invention is (v) a compound of formula (I) or (Ia) according to (iv), wherein R⁴ is cyano or chloro.

A further embodiment of present invention is (vi) a compound of formula (I) or (Ia) according to (iv), wherein
R³ is
   1,3-dihydropyrrolo[3,4-c]pyridinyl substituted by piperazinyl;
   3,4-dihydro-1H-2,7-naphthyridinyl substituted by piperazinyl;
   3,4-dihydro-1H-isoquinolinyl substituted by one or two substituents independently selected from hydroxy, piperazinyl and C₁₋₆alkylpiperazinyl;
   3,4-dihydro-2H-quinolinyl substituted by piperazinyl;
   5,7-dihydropyrrolo[3,4-b]pyridinyl substituted by piperazinyl;
   5,7-dihydropyrrolo[3,4-d]pyrimidinyl substituted by piperazinyl or C₁₋₆alkylpiperazinyl;
   7,8-dihydro-SH-1,6-naphthyridinyl substituted by piperazinyl;
   isoindolinyl substituted by (C₁₋₆alkoxyC₁₋₆alkyl)piperazinyl, (C₁₋₆alkyl)₂piperazinyl, (hydroxyC₁₋₆alkyl)piperazinyl, C₁₋₆alkylpiperazinyl, C₃₋₇cycloalkylpiperazinyl, haloC₁₋₆alkylpiperazinyl, morpholinylC₁₋₆alkyl, piperazinyl, piperidinyl, piperidinylC₁₋₆alkyl or pyrrolidinyl; or
   -NR^{6a}R^{6b}; wherein
      R^{6a} is H;
      R^{6b} is phenylC₁₋₆alkyl substituted by piperazinyl.

A further embodiment of present invention is (vii) a compound of formula (I) or (Ia) according to (vi), wherein R³ is piperazinyl-1,3-dihydropyrrolo[3,4-c]pyridinyl; piperazinyl-3,4-dihydro-1H-2,7-naphthyridinyl; piperazinyl-3,4-dihydro-1H-isoquinolinyl; methylpiperazinyl-3,4-dihydro-1H-isoquinolinyl; hydroxy(piperazinyl)-3,4-dihydro-1H-isoquinolinyl; piperazinyl-3,4-dihydro-2H-quinolinyl; piperazinyl-5,7-dihydropyrrolo[3,4-b]pyridinyl; piperazinyl-5,7-dihydropyrrolo[3,4-d]pyrimidinyl; methylpiperazinyl-5,7-dihydropyrrolo[3,4-d]pyrimidinyl; piperazinyl-7,8-dihydro-5H-1,6-naphthyridinyl; (difluoromethyl)piperazinylisoindolinyl; (hydroxymethyl)piperazinylisoindolinyl; (methoxyethyl)piperazinylisoindolinyl; cyclopropylpiperazinylisoindolinyl; dimethylpiperazinylisoindolinyl; methylpiperazinylisoindolinyl; morpholinylmethylisoindolinyl; piperazinylisoindolinyl; piperidinylisoindolinyl; piperidinylmethylisoindolinyl; pyrrolidinylisoindolinyl; or (piperazinylphenyl)methylamino.

A further embodiment of present invention is (viii) a compound of formula (I) or (Ia) according to (i) or (ii), wherein
R¹ is 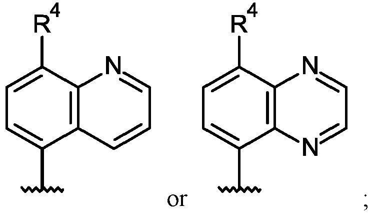 wherein R⁴ is cyano or halogen;
R² is C₁₋₆alkyl;
R³ is
   1,3-dihydropyrrolo[3,4-c]pyridinyl substituted by piperazinyl;
   3,4-dihydro-1H-2,7-naphthyridinyl substituted by piperazinyl;
   3,4-dihydro-1H-isoquinolinyl substituted by one or two substituents independently selected from hydroxy, piperazinyl and C₁₋₆alkylpiperazinyl;
   3,4-dihydro-2H-quinolinyl substituted by piperazinyl;
   5,7-dihydropyrrolo[3,4-b]pyridinyl substituted by piperazinyl;
   5,7-dihydropyrrolo[3,4-d]pyrimidinyl substituted by piperazinyl or C₁₋₆alkylpiperazinyl;
   7,8-dihydro-SH-1,6-naphthyridinyl substituted by piperazinyl;
   isoindolinyl substituted by (C₁₋₆alkoxyC₁₋₆alkyl)piperazinyl, (C₁₋₆alkyl)zpiperazinyl, (hydroxyC₁₋₆alkyl)piperazinyl, C₁₋₆alkylpiperazinyl, C₃₋₇cycloalkylpiperazinyl, haloC₁₋₆alkylpiperazinyl, morpholinylC₁₋₆alkyl, piperazinyl, piperidinyl, piperidinylC₁₋₆alkyl or pyrrolidinyl; or
   -NR^{6a}R^{6b}; wherein
      R^{6a} is H;
      R^{6b} is phenylC₁₋₆alkyl substituted by piperazinyl;
   or a pharmaceutically acceptable salt, enantiomer or diastereomer thereof.

A further embodiment of present invention is (ix) a compound of formula (I) or (Ia) according to (viii), wherein
R¹ is 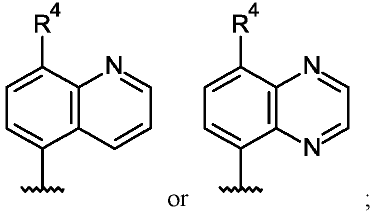 wherein R⁴ is cyano or chloro;
R² is methyl;
R³ is piperazinyl-1,3-dihydropyrrolo[3,4-c]pyridinyl; piperazinyl-3,4-dihydro-1H-2,7-naphthyridinyl; piperazinyl-3,4-dihydro-1H-isoquinolinyl; methylpiperazinyl-3,4-dihydro-1H-isoquinolinyl; hydroxy(piperazinyl)-3,4-dihydro-1H-isoquinolinyl; piperazinyl-3,4-dihydro-2H-quinolinyl; piperazinyl-5,7-dihydropyrrolo[3,4-b]pyridinyl; piperazinyl-5,7-dihydropyrrolo[3,4-d]pyrimidinyl; methylpiperazinyl-5,7-dihydropyrrolo[3,4-d]pyrimidinyl; piperazinyl-7,8-dihydro-5H-1,6-naphthyridinyl; (difluoromethyl)piperazinylisoindolinyl; (hydroxymethyl)piperazinylisoindolinyl; (methoxyethyl)piperazinylisoindolinyl; cyclopropylpiperazinylisoindolinyl; dimethylpiperazinylisoindolinyl; methylpiperazinylisoindolinyl; morpholinylmethylisoindolinyl; piperazinylisoindolinyl; piperidinylisoindolinyl; piperidinylmethylisoindolinyl; pyrrolidinylisoindolinyl; or (piperazinylphenyl)methylamino;
or a pharmaceutically acceptable salt, enantiomer or diastereomer thereof.

Another embodiment of present invention is that (x) particular compounds of formula (I) or (Ia) are the following:
4-[(2*R*,6*S*)-2-methyl-6-[(4-piperazin-1-ylisoindolin-2-yl)methyl]morpholin-4-yl]pyrazolo[1,5-a]pyridine-7-carbonitrile;
4-[(2*R*,6*S*)-2-methyl-6-[(5-piperazin-1-ylisoindolin-2-yl)methyl]morpholin-4-yl]pyrazolo[1,5-a]pyridine-7-carbonitrile;
4-[(2*R*,6*S*)-2-methyl-6-[(8-piperazin-1-yl-3,4-dihydro-1*H*-isoquinolin-2-yl)methyl]morpholin-4-yl]pyrazolo[1,5-a]pyridine-7-carbonitrile;
4-[(2*R*,6*S*)-2-methyl-6-[(7-piperazin-1-yl-3,4-dihydro-2*H*-quinolin-1-yl)methyl]morpholin-4-yl]pyrazolo[1,5-a]pyridine-7-carbonitrile;
3-fluoro-4-[(2*R*,6*S*)-2-methyl-6-[(5-piperazin-1-ylisoindolin-2-yl)methyl]morpholin-4-yl]pyrazolo[1,5-a]pyridine-7-carbonitrile;
4-[(2*R*,6*S*)-2-methyl-6-[[5-(4-piperidyl)isoindolin-2-yl]methyl]morpholin-4-yl]pyrazolo[1,5-a]pyridine-7-carbonitrile;
4-[(2*R*,6*S*)-2-methyl-6-[(5-pyrrolidin-3-ylisoindolin-2-yl)methyl]morpholin-4-yl]pyrazolo[1,5-a]pyridine-7-carbonitrile;
4-[(2*R*,6*S*)-2-methyl-6-[(6-piperazin-1-yl-3,4-dihydro-1H-isoquinolin-2-yl)methyl]morpholin-4-yl]pyrazolo[1,5-a]pyridine-7-carbonitrile;
4-[(2*R*,6*S*)-2-methyl-6-[(7-piperazin-1-yl-3,4-dihydro-1H-isoquinolin-2-yl)methyl]morpholin-4-yl]pyrazolo[1,5-a]pyridine-7-carbonitrile;
8-[(2*R*,6*S*)-2-methyl-6-[(5-piperazin-1-ylisoindolin-2-yl)methyl]morpholin-4-yl]quinoxaline-5-carbonitrile;
8-[(2*R*,6*S*)-2-methyl-6-[(6-piperazin-1-yl-3,4-dihydro-1H-isoquinolin-2-yl)methyl]morpholin-4-yl]quinoxaline-5-carbonitrile;
3-fluoro-4-[(2*R*,6*S*)-2-methyl-6-[(6-piperazin-1-yl-3,4-dihydro-1*H*-isoquinolin-2-yl)methyl]morpholin-4-yl]pyrazolo[1,5-a]pyridine-7-carbonitrile;
4-[(2*R*,6*S*)-2-methyl-6-[[6-(4-methylpiperazin-1-yl)-3,4-dihydro-1*H*-isoquinolin-2-yl]methyl]morpholin-4-yl]pyrazolo[1,5-a]pyridine-7-carbonitrile
4-[(2*R*,6*S*)-2-methyl-6-[[5-(4-methylpiperazin-1-yl)isoindolin-2-yl]methyl]morpholin-4-yl]pyrazolo[1,5-a]pyridine-7-carbonitrile;
4-[(2*R*,6*S*)-2-methyl-6-[(3-piperazin-1-yl-5,7-dihydropyrrolo[3,4-b]pyridin-6-yl)methyl]morpholin-4-yl]pyrazolo[1,5-a]pyridine-7-carbonitrile;
5-[(2*R*,6*S*)-2-methyl-6-[(6-piperazin-1-yl-3,4-dihydro-1*H*-isoquinolin-2-yl)methyl]morpholin-4-yl]quinoline-8-carbonitrile;
5-[(2*R*,6*S*)-2-methyl-6-[(3-piperazin-1-yl-5,7-dihydropyrrolo[3,4-b]pyridin-6-yl)methyl]morpholin-4-yl]quinoline-8-carbonitrile;
5-[(2*R*,6*S*)-2-methyl-6-[(5-piperazin-1-ylisoindolin-2-yl)methyl]morpholin-4-yl]quinoline-8-carbonitrile;
7-[(2*R*,6*S*)-2-methyl-6-[(5-piperazin-1-ylisoindolin-2-yl)methyl]morpholin-4-yl]-1,3-benzothiazole-4-carbonitrile;
7-[(2*R*,6*S*)-2-methyl-6-[(6-piperazin-1-yl-3,4-dihydro-1H-isoquinolin-2-yl)methyl]morpholin-4-yl]-1,3 -benzothiazole-4-carbonitrile;
7-[(2*R*,6*S*)-2-methyl-6-[(3-piperazin-1-yl-5,7-dihydropyrrolo[3,4-b]pyridin-6-yl)methyl]morpholin-4-yl]-1,3 -benzothiazole-4-carbonitrile;
4-[(2*S*,6*R*)-2-[[5-(2-aminoethylamino)isoindolin-2-yl]methyl]-6-methyl-morpholin-4-yl]pyrazolo[1,5-a]pyridine-7-carbonitrile;
4-[(2*R*,6*S*)-2-methyl-6-[[2-(4-methylpiperazin-1-yl)-6,7-dihydro-4*H*-thiazolo[5,4-c]pyridin-5-yl]methyl]morpholin-4-yl]pyrazolo[1,5-a]pyridine-7-carbonitrile;
4-[(2*S*,6*R*)-2-[[5-[2-(dimethylamino)ethoxy]isoindolin-2-yl]methyl]-6-methyl-morpholin-4-yl]pyrazolo[1,5-a]pyridine-7-carbonitrile;
3-fluoro-4-[(2*R*,6*S*)-2-methyl-6-[(3-piperazin-1-yl-5,7-dihydropyrrolo[3,4-b]pyridin-6-yl)methyl]morpholin-4-yl]pyrazolo[1,5-a]pyridine-7-carbonitrile;
8-[(2*R*,6*S*)-2-methyl-6-[(3-piperazin-1-yl-5,7-dihydropyrrolo[3,4-b]pyridin-6-yl)methyl]morpholin-4-yl]quinoxaline-5-carbonitrile;
(2*R*,6*S*)-4-(8-chloro-5-quinolyl)-2-methyl-6-[(5-piperazin-1-ylisoindolin-2-yl)methyl]morpholine;
(2*R*,6*S*)-4-(8-chloro-5-quinolyl)-2-methyl-6-[(6-piperazin-1-yl-3,4-dihydro-1*H-*isoquinolin-2-yl)methyl]morpholine;
(2*R*,6*S*)-4-(8-chloroquinoxalin-5-yl)-2-methyl-6-[(5-piperazin-1-ylisoindolin-2-yl)methyl]morpholine;
(2*R*,6*S*)-4-(8-chloroquinoxalin-5-yl)-2-methyl-6-[(6-piperazin-1-yl-3,4-dihydro-1*H-*isoquinolin-2-yl)methyl]morpholine;
8-[(2*S*,6*R*)-2-[[5-(4-cyclopropylpiperazin-1-yl)isoindolin-2-yl]methyl]-6-methyl-morpholin-4-yl]quinoxaline-5-carbonitrile;
8-[(2*R*,6*S*)-2-methyl-6-[[5-(3-methylpiperazin-1-yl)isoindolin-2-yl]methyl]morpholin-4-yl]quinoxaline-5-carbonitrile;
8-[(2*S*,6*R*)-2-[[5-[4-(2-methoxyethyl)piperazin-1-yl]isoindolin-2-yl]methyl]-6-methyl-morpholin-4-yl]quinoxaline-5-carbonitrile;
8-[(2*S*,6*R*)-2-[[5-(4,7-diazaspiro[2.5]octan-7-yl)isoindolin-2-yl]methyl]-6-methyl-morpholin-4-yl] quinoxaline-5-carbonitrile;
8-[(2*R*,6*S*)-2-methyl-6-[[5-(2-oxopiperazin-1-yl)isoindolin-2-yl]methyl]morpholin-4-yl]quinoxaline-5-carbonitrile;
8-[(2*S*,6*R*)-2-[[5-(1,3,4,6,7,8,9,9a-octahydropyrido[1,2-a]pyrazin-2-yl)isoindolin-2-yl]methyl]-6-methyl-morpholin-4-yl]quinoxaline-5-carbonitrile;
8-[(2*S*,6*R*)-2-[[5-(3,8-diazabicyclo[3.2.1]octan-8-yl)isoindolin-2-yl]methyl]-6-methyl-morpholin-4-yl] quinoxaline-5-carbonitrile;
5-[(2*S*,6*R*)-2-[[5-(3,8-diazabicyclo[3.2.1]octan-3-yl)isoindolin-2-yl]methyl]-6-methyl-morpholin-4-yl] quinoline-8-carbonitrile;
5-[(2*S*,6*R*)-2-[[5-(3,8-diazabicyclo[3.2.1]octan-8-yl)isoindolin-2-yl]methyl]-6-methyl-morpholin-4-yl] quinoline-8-carbonitrile;
8-[(2*S*,6*R*)-2-[[5-(3,8-diazabicyclo[3.2.1]octan-3-yl)isoindolin-2-yl]methyl]-6-methyl-morpholin-4-yl]quinoxaline-5-carbonitrile;
4-[(2*R*,6*S*)-2-methyl-6-[[5-(1-methylimidazol-4-yl)isoindolin-2-yl]methyl]morpholin-4-yl]pyrazolo[1,5-a]pyridine-7-carbonitrile;
5-[(2*R*,6*S*)-2-methyl-6-[[5-(3-oxa-7,9-diazabicyclo[3.3.1]nonan-9-yl)isoindolin-2-yl]methyl]morpholin-4-yl]quinoline-8-carbonitrile;
5-[(2*S*,6*R*)-2-[[5-(2,6-diazaspiro[3.3]heptan-2-yl)isoindolin-2-yl]methyl]-6-methyl-morpholin-4-yl] quinoline-8-carbonitrile;
5-[(2*R*,6*S*)-2-methyl-6-[(1-methyl-5-piperazin-1-yl-isoindolin-2-yl)methyl]morpholin-4-yl]quinoline-8-carbonitrile;
8-[(2*R*,6*S*)-2-methyl-6-[[5-(3-oxa-7,9-diazabicyclo[3.3.1]nonan-7-yl)isoindolin-2-yl]methyl]morpholin-4-yl]quinoxaline-5-carbonitrile;
Ethyl 1-[2-[[(2*S*,6*R*)-4-(8-cyano-5-quinolyl)-6-methyl-morpholin-2-yl]methyl]isoindolin-5-yl]piperazine-2-carboxylate;
4-[(2*R*,6*S*)-2-methyl-6-[[2-(4-methylpiperazin-1-yl)-5,7-dihydropyrrolo[3,4-d]pyrimidin-6-yl]methyl]morpholin-4-yl]pyrazolo[1,5-a]pyridine-7-carbonitrile;
4-[(2*R*,6*S*)-2-methyl-6-[(5-pyrrolidin-3-yloxyisoindolin-2-yl)methyl]morpholin-4-yl]pyrazolo[1,5-a]pyridine-7-carbonitrile;
4-[(2*S*,6*R*)-2-[[5-(2,6-dimethyl-4-pyridyl)isoindolin-2-yl]methyl]-6-methyl-morpholin-4-yl]pyrazolo[1,5-a]pyridine-7-carbonitrile;
4-[(2*S*,6*R*)-2-[[5-(2,6-dimethyl-4-pyridyl)isoindolin-2-yl]methyl]-6-methyl-morpholin-4-yl]-3-fluoro-pyrazolo[1,5-a]pyridine-7-carbonitrile;
5-[(2*S*,6*R*)-2-[[5-(1,6-diazaspiro[3.3]heptan-6-yl)isoindolin-2-yl]methyl]-6-methyl-morpholin-4-yl] quinoline-8-carbonitrile;
1-[2-[[(2*S*,6*R*)-4-(8-cyano-5-quinolyl)-6-methyl-morpholin-2-yl]methyl]isoindolin-5-yl]piperazine-2-carboxylic acid;
*Trans*-5-[(2*R*,6*S*)-2-methyl-6-[[5-[3,5-dimethylpiperazin-1-yl]isoindolin-2-yl]methyl]morpholin-4-yl]quinoline-8-carbonitrile;
5-[(2*S*,6*R*)-2-[[5-(3,4,6,7,9,9a-hexahydro-1*H*-pyrazino[2,1-c][1,4]oxazin-8-yl)isoindolin-2-yl]methyl]-6-methyl-morpholin-4-yl]quinoline-8-carbonitrile;
5-[(2*S*,6*R*)-2-[(4-fluoro-6-piperazin-1-yl-isoindolin-2-yl)methyl]-6-methyl-morpholin-4-yl]quinoline-8-carbonitrile;
8-[(2*R*,6*S*)-2-methyl-6-[(6-piperazin-1-yl-1,3-dihydropyrrolo[3,4-c]pyridin-2-yl)methyl]morpholin-4-yl]quinoxaline-5-carbonitrile;
5-[(2*R*,6*S*-2-methyl-6-[(6-piperazin-1-yl-1,3-dihydropyrrolo[3,4-c]pyridin-2-yl)methyl]morpholin-4-yl]quinoline-8-carbonitrile;
5-[(2*R*,6*S*)-2-methyl-6-[[5-[(3*R*)-3-methylpiperazin-1-yl]isoindolin-2-yl]methyl]morpholin-4-yl]quinoline-8-carbonitrile;
5-[(2*R*,6*S*)-2-methyl-6-[(2-piperazin-1-yl-5,7-dihydropyrrolo[3,4-d]pyrimidin-6-yl)methyl]morpholin-4-yl]quinoline-8-carbonitrile;
5-[(2*R*,6*S*)-2-methyl-6-[[5-[(3S)-3-methylpiperazin-1-yl]isoindolin-2-yl]methyl]morpholin-4-yl]quinoline-8-carbonitrile;
8-[(2*R*,6*S*)-2-methyl-6-[[5-[(3*R*)-3-methylpiperazin-1-yl]isoindolin-2-yl]methyl]morpholin-4-yl]quinoxaline-5-carbonitrile;
8-[(2*R*,6*S*)-2-methyl-6-[[5-[(3*S*)-3-methylpiperazin-1-yl]isoindolin-2-yl]methyl]morpholin-4-yl]quinoxaline-5-carbonitrile;
Methyl 4-[2-[[(2*S*,6*R*)-4-(8-cyano-5-quinolyl)-6-methyl-morpholin-2-yl]methyl]isoindolin-5-yl]piperazine-2-carboxylate;
4-[2-[[(2*S*,6*R*)-4-(8-cyano-5-quinolyl)-6-methyl-morpholin-2-yl]methyl]isoindolin-5-yl]piperazine-2-carboxylic acid;
5-[(2*S*,6*R*)-2-[[4-fluoro-6-(4-methylpiperazin-1-yl)isoindolin-2-yl]methyl]-6-methyl-morpholin-4-yl] quinoline-8-carbonitrile;
5-[(2*R*,6*S*)-2-methyl-6-[(6-piperazin-1-yl-3,4-dihydro-1*H*-2,7-naphthyridin-2-yl)methyl]morpholin-4-yl]quinoline-8-carbonitrile;
5-[(2*R*,6*S*)-2-methyl-6-[[5-[(3*R*,5*S*)-3,5-dimethylpiperazin-1-yl]isoindolin-2-yl]methyl]morpholin-4-yl]quinoline-8-carbonitrile;
5-[(2*R*,6*S*)-2-methyl-6-[[5-(3-oxa-7,9-diazabicyclo[3.3.1]nonan-7-yl)isoindolin-2-yl]methyl]morpholin-4-yl]quinoline-8-carbonitrile;
5-[(2*S*,6*R*)-2-[[5-[2-(hydroxymethyl)piperazin-1-yl]isoindolin-2-yl]methyl]-6-methyl-morpholin-4-yl] quinoline-8-carbonitrile;
5-[(2*R*,6*S*)-2-methyl-6-[[5-(1-piperidylmethyl)isoindolin-2-yl]methyl]morpholin-4-yl]quinoline-8-carbonitrile;
5-[(2*S*,6*R*)-2-[(4-hydroxy-6-piperazin-1-yl-3,4-dihydro-1*H*-isoquinolin-2-yl)methyl]-6-methyl-morpholin-4-yl]quinoline-8-carbonitrile;
5-[(2*S*,6*R*)-2-[[5-[2-(difluoromethyl)piperazin-1-yl]isoindolin-2-yl]methyl]-6-methyl-morpholin-4-yl] quinoline-8-carbonitrile;
5-[(2*S*,6*R*)-2-[[5-(3-aminoazetidin-1-yl)isoindolin-2-yl]methyl]-6-methyl-morpholin-4-yl]quinoline-8-carbonitrile;
5-[(2*R*,6*S*)-2-methyl-6-[[5-(2-methylpiperazin-1-yl)isoindolin-2-yl]methyl]morpholin-4-yl]quinoline-8-carbonitrile;
5-[(2*R*,6*S*)-2-methyl-6-[(2-piperazin-1-yl-7,8-dihydro-5*H*-1,6-naphthyridin-6-yl)methyl]morpholin-4-yl]quinoline-8-carbonitrile;
5-[(2*R*,6*S*)-2-methyl-6-[(2-piperazin-1-yl-5,7-dihydropyrrolo[3,4-b]pyridin-6-yl)methyl]morpholin-4-yl]quinoline-8-carbonitrile;
5-[(2S,6R)-2-[[5-[(2R)-2-(hydroxymethyl)piperazin-1-yl]isoindolin-2-yl]methyl]-6-methyl-morpholin-4-yl]quinoline-8-carbonitrile;
5-[(2S,6R)-2-[[5-[(2S)-2-(hydroxymethyl)piperazin-1-yl]isoindolin-2-yl]methyl]-6-methyl-morpholin-4-yl]quinoline-8-carbonitrile;
5-[(2*S*,6*R*)-2-[[5-[3-(hydroxymethyl)piperazin-1-yl]isoindolin-2-yl]methyl]-6-methyl-morpholin-4-yl] quinoline-8-carbonitrile;
   and
5-[(2*R*,6*S*)-2-methyl-6-[[5-(morpholinomethyl)isoindolin-2-yl]methyl]morpholin-4-yl]quinoline-8-carbonitrile;
or a pharmaceutically acceptable salt, enantiomer or diastereomer thereof.

A number of compounds were disclosed in patent US20150105370 showing TLR7 and TLR9 potency data summarized in table 1. From the chemical structure perspective, compounds in table 1 are characterized in having structures as shown in formula (A). More specifically, morpholine ring is substituted by a linear basic center (amino group) connected to aryl or heteroaryl in the tail part. According to the potency data disclosed in patent US20150105370, compounds of formula (A) only showed good TLR7 potency, but suffered from the loss of TLR9 potency.

Meanwhile, more analogues of the compounds disclosed in US20150105370, such as compound R1, compound R2 and compound R3 which are even closer to the compounds of this invention, were synthesized to confirm the SAR (structure-activity-relationship). However, according to the potency data shown in Table 2, the structural modification based on formula (A) could not improve the potency of TLR9. Therefore, the skill of the art shall not obtain any incitation from the information disclosed in US20150105370 to further optimize such chemical structures.

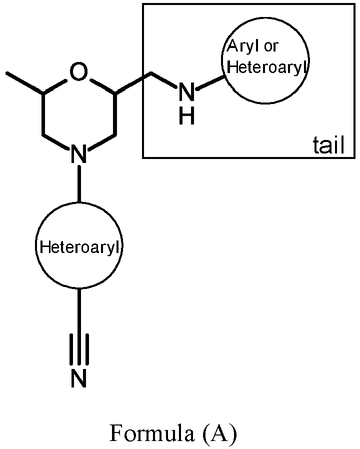

Surprisingly, the compounds of this invention significantly improved TLR9 potency (>8 folds compared to ER-886509) while keeping excellent TLR7 and TLR8 potency. In another embodiment, the human microsome stability of the compounds of this invention was greatly increased compared to the reference compound R3 and ER-886509 (see Table 3).

**Table 1. TLR7 and TLR9 potency of compounds disclosed in US20150105370**

| **Compound** | **Structure** | **HEK/hTLR7 IC50 (µM)** | **HEK/hTLR9 IC50 (µM)** |
|---|---|---|---|
| **ER-886509** | | 0.0440 | 3.94 |
| **ER-886601** | | 0.015 | >2.0 |
| **ER-886602** | | 0.1850 | >20 |
| **ER-886608** | | 0.0130 | 3.366 |
| **ER-886799** | | 0.0070 | >2.0 |

### SYNTHESIS

The compounds of the present invention can be prepared by any conventional means. Suitable processes for synthesizing these compounds as well as their starting materials are provided in the schemes below and in the examples. All substituents, in particular, R¹ to R⁵, R^{6a} and R^{6b} are as defined above unless otherwise indicated. Furthermore, and unless explicitly otherwise stated, all reactions, reaction conditions, abbreviations and symbols have the meanings well known to a person of ordinary skill in organic chemistry.

General synthetic routes for preparing the compound of formula (I), (Ia), (II), (III) and (IX) are shown below. 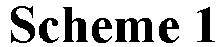 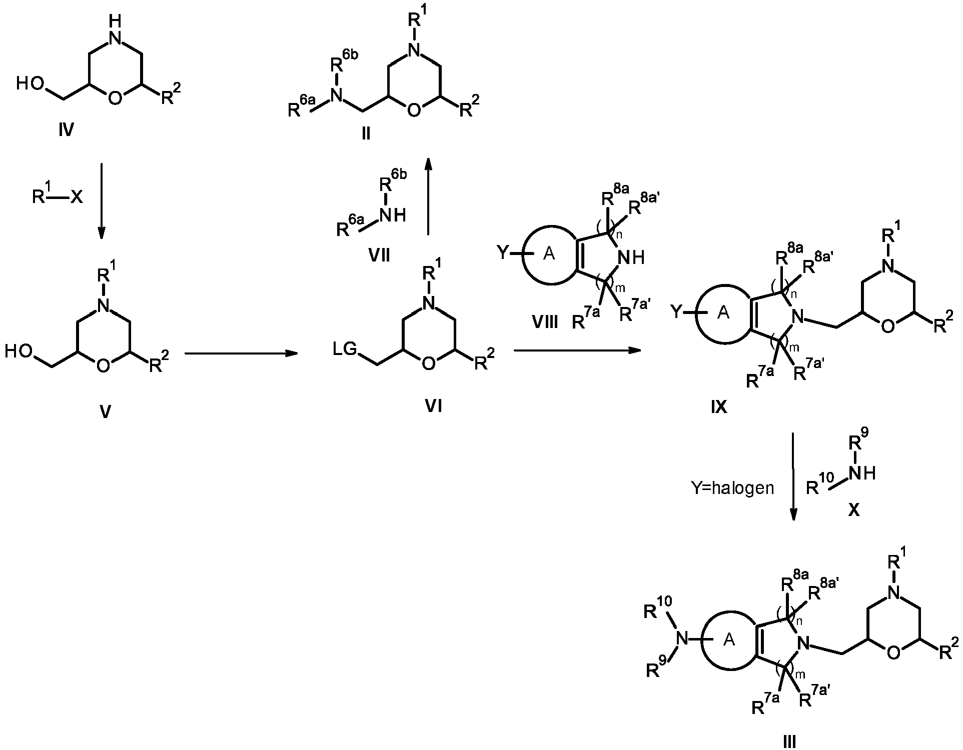 wherein X is halogen; Y is halogen, ((C₁₋₆alkyl)zamino)C₁₋₆alkoxy, hetercyclyloxy, - NR⁹R¹⁰, heterocyclyl or heterocyclylC₁₋₆alkyl; LG is leaving group, such as OTf, OTs and OMs; A is phenyl or 5-6 membered heteroaryl unsubstituted or substituted by halogen or C₁₋₆alkyl; m is 0, 1 or 2; n is 0, 1 or 2; m and n are not simultaneously 0; R^{7a}, R^{7b}, R^{8a}, R^{8b} are independently selected from H, OH and C₁₋₆alkyl; R⁹ and R¹⁰ are independently selected from H, aminoC₁₋₆alkylamino and aminoazetidinyl; or R⁹ and R¹⁰ together with the nitrogen they are attached to form a heterocyclyl.

The coupling of compound of formula (IV) with R¹-X can be achieved by direct coupling in the presence of a base, such as DIPEA or K₂CO₃, or under Buchwald-Hartwig amination conditions (ref: Acc. Chem. Res. 1998, 31, 805-818; Chem. Rev. 2016, 116, 12564-12649; Topics in Current Chemistry, 2002, 219, 131-209; and references cited therein) with a catalyst, such as RuPhos Pd G2, and a base, such as Cs₂CO₃, to provide compound of formula (V). Subsequently the hydroxy group of compound of formula (V) is converted to a leaving group, such as OTf, OTs, or OMs, under basic condition, such as DIPEA, TEA, K₂CO₃ or 2,6-dimethylpyridine, with Tf₂O, TsCl or MsCl. Compound of formula (VI) was further coupled with amine (VII) in the presence of base, such as K₂CO₃, DIPEA, or Cs₂CO₃, to afford compound of formula (II). On the other hand, in a basic condition, such as K₂CO₃, DIPEA, or Cs₂CO₃, compound of formula (VI) is reacted with amine (VIII) to give compound of formula (IX), which is further coupled with amine (X) under Buchwald-Hartwig amination conditions (ref: Acc. Chem. Res. 1998, 31, 805-818; Chem. Rev. 2016, 116, 12564-12649; Topics in Current Chemistry, 2002, 219, 131-209; and references cited therein) with a catalyst, such as Ruphos Pd G2, and a base, such as Cs₂CO₃, to provide compound of formula (III). In some embodiment, the coupling of compound of formula (VI) and amine (VII) or formula (IX) with amine (X) may give a product containing a protecting group, e.g. Boc, originated from amine (VII) or amine (X), which will be removed before affording the final compound of formula (II) or formula (III).

This invention also relates to a process for the preparation of a compound of formula (I) or (Ia) comprising any of the following steps:
a) the coupling of compound of formula (VI), 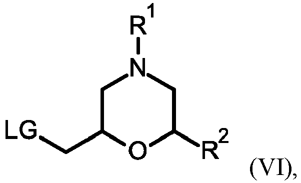 with amine (VII) in the presence of a base;
b) the coupling of compound of formula (VI), 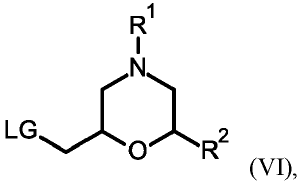 with amine (VIII) in the presence of a base;
c) the coupling of compound of formula (IX), 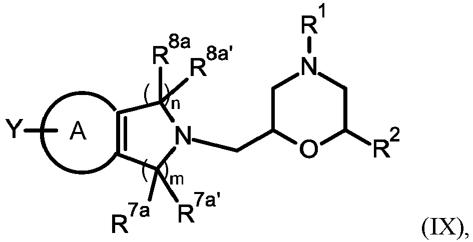 with amine (X) under Buchwald-Hartwig amination conditions;

In step a) and b) the base can be for example K₂CO₃, DIPEA, or Cs₂CO₃.

In step c) the Buchwald-Hartwig amination condition includes a catalyst which can be for example Ruphos Pd G2, and a base which can be for example Cs₂CO₃.

A compound of formula (I) or (Ia) when manufactured according to the above process is also an object of the invention.

Compounds of this invention can be obtained as mixtures of diastereomers or enantiomers, which can be separated by methods well known in the art, e.g. (chiral) HPLC or SFC.

### INDICATIONS AND METHODS OF TREATMENT

The present invention provides compounds that can be used as TLR7 and/or TLR8 and/or TLR9 antagonist, which inhibits pathway activation through TLR7 and/or TLR8 and/or TLR9 as well as respective downstream biological events including, innate and adaptive immune responses mediated through the production of all types of cytokines and all forms of auto-antibodies. Accordingly, the compounds of the invention are useful for blocking TLR7 and/or TLR8 and/or TLR9 in all types of cells that express such receptor(s) including, plasmacytoid dendritic cell, B cell, T cell, macrophage, monocyte, neutrophil, keratinocyte, epithelial cell. As such, the compounds can be used as a therapeutic or prophylactic agent for systemic lupus erythematosus and lupus nephritis.

The present application provides methods for treatment or prophylaxis of systemic lupus erythematosus and lupus nephritis in a patient in need thereof (not part of the invention).

Another embodiment of this application (not part of this invention) includes a method of treating or preventing systemic lupus erythematosus and lupus nephritis in a mammal in need of such treatment, wherein the method comprises administering to said mammal a therapeutically effective amount of a compound of formula (I), a stereoisomer, tautomer, prodrug or pharmaceutically acceptable salt thereof.

### EXAMPLES

The invention will be more fully understood by reference to the following examples.

### ABBREVIATIONS

The invention will be more fully understood by reference to the following examples. Abbreviations used herein are as follows:
- ACN:: acetonitrile
- BINAP:: (2,2'-bis(diphenylphosphino)-1,1'-binaphthyl)
- Boc₂O:: *di-tert* butyl dicarbonate
- *t*-Bu XPhosPd G3:: [(2-di-*tert*-butylphosphino-2',4',6'-triisopropyl-1,1'-biphenyl)-2-(2'-amino-1,1'-biphenyl)] palladium(II) methanesulfonate
- CbzCl:: benzylchloroformate
- cataCXium A Pd G2:: chloro[(di(1-adamantyl)-*N-*butylphosphine)-2-(2-aminobiphenyl)]palladium(II)
- DAST:: diethylaminosulfur trifluoride
- DCM:: dichloromethane
- DIAD:: diisopropyl azodicarboxylate
- DIPEA:: *N*,*N-*diisopropylethylamine
- DMA:: dimethylacetamide
- DMEDA:: 1,2-dimethylethylenediamine
- EtOAc or EA:: ethyl acetate
- FA:: formic acid
- HLM: human liver microsome
- IC₅₀:: half inhibition concentration
- IPA:: isopropanol
- LCMS: liquid chromatography-mass spectrometry
- MS:: mass spectrometry
- Pd₂(dba)₃:: tris(dibenzylideneacetone)dipalladium(0)
- PdCl₂(DPPF)-CH₂Cl₂:: [1,1'-bis(diphenylphosphino)ferrocene]dichloropalladium(II), complex with dichloromethane
- PE:: petroleum ether
- prep-HPLC:: preparative high performance liquid chromatography
- prep-TLC:: preparative thin layer chromatography
- PPh₃:: triphenylphosphine
- Rf:: retention factor
- rt:: room temperature
- RuPhos Pd G2:: chloro(2-dicyclohexylphosphino-2',6'-diisopropoxy-1,1'-biphenyl)[2-(2'-amino-1,1'-biphenyl)]palladium(II) 2nd generation
- SelectFluor:: 1-chloromethyl-4-fluoro-1,4-diazoniabicyclo[2.2.2]octane bis(tetrafluoroborate)
- SFC:: supercritical fluid chromatography
- TEA:: triethylamine
- TFA:: trifluoroacetic acid
- Tf₂O:: trifluoromethanesulfonic anhydride
- THF:: tetrahydrofuran
- v/v:: volume ratio
- XPhos:: 2-dicyclohexylphosphino-2',4',6'-triisopropylbiphenyl
- XPhos Pd G2:: chloro(2-dicyclohexylphosphino-2',4',6'-triisopropyl-1,1'-biphenyl)[2-(2'-amino-1,1 '-biphenyl)]palladiuM(II)

### GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using XBridge^{™} Prep-C18 (5 µm, OBDTM 30 × 100 mm) column, SunFire^{™} Prep-C18 (5 µm, OBD^{™} 30 × 100 mm) column, Phenomenex Synergi-C18 (10 µm, 25 × 150 mm) or Phenomenex Gemini-C18 (10 µm, 25 × 150 mm). Waters AutoP purification System (Sample Manager 2767, Pump 2525, Detector: Micromass ZQ and UV 2487, solvent system: acetonitrile and 0.1% ammonium hydroxide in water; acetonitrile and 0.1% FA in water or acetonitrile and 0.1% TFA in water). Or Gilson-281 purification System (Pump 322, Detector: UV 156, solvent system: acetonitrile and 0.05% ammonium hydroxide in water; acetonitrile and 0.225% FA in water; acetonitrile and 0.05% HCl in water; acetonitrile and 0.075% TFA in water; or acetonitrile and water).

For SFC chiral separation, intermediates were separated by chiral column (Daicel chiralpak IC, 5 µm, 30 × 250 mm), AS (10 µm, 30 × 250 mm) or AD (10 µm, 30 × 250 mm) using Mettler Toledo Multigram III system SFC, Waters 80Q preparative SFC or Thar 80 preparative SFC, solvent system: CO₂ and IPA (0.5% TEA in IPA) or CO₂ and MeOH (0.1% NH₃-H₂O in MeOH), back pressure 100bar, detection UV@ 254 or 220 nm.

LC/MS spectra of compounds were obtained using a LC/MS (Waters^{™} Alliance 2795-Micromass ZQ, Shimadzu Alliance 2020-Micromass ZQ or Agilent Alliance 6110-Micromass ZQ), LC/MS conditions were as follows (running time 3 or 1.5 mins):
Acidic condition I: A: 0.1% TFA in H₂O; B: 0.1% TFA in acetonitrile;
Acidic condition II: A: 0.0375% TFA in H₂O; B: 0.01875% TFA in acetonitrile;
Basic condition I: A: 0.1% NH₃·H₂O in H₂O; B: acetonitrile;
Basic condition II: A: 0.025% NH₃·H₂O in H₂O; B: acetonitrile;
Neutral condition: A: H₂O; B: acetonitrile.
Mass spectra (MS): generally only ions which indicate the parent mass are reported, and unless otherwise stated the mass ion quoted is the positive mass ion (MH)⁺.

NMR Spectra were obtained using Bruker Avance 400 MHz.

The microwave assisted reactions were carried out in a Biotage Initiator Sixty microwave synthesizer. All reactions involving air-sensitive reagents were performed under an argon or nitrogen atmosphere. Reagents were used as received from commercial suppliers without further purification unless otherwise noted.

### PREPARATIVE EXAMPLES

The following examples are intended to illustrate the meaning of the present invention:

### Intermediate A

### [(2R,6R)-4-(7-cyanopyrazolo [1,5-a] pyridin-4-yl)-6-methyl-morpholin-2-yl] methyl trifluoromethanesulfonate

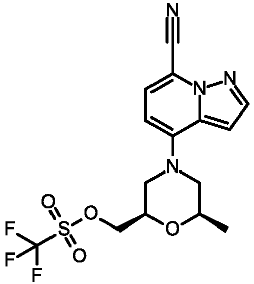

The title compound was prepared according to the following scheme:

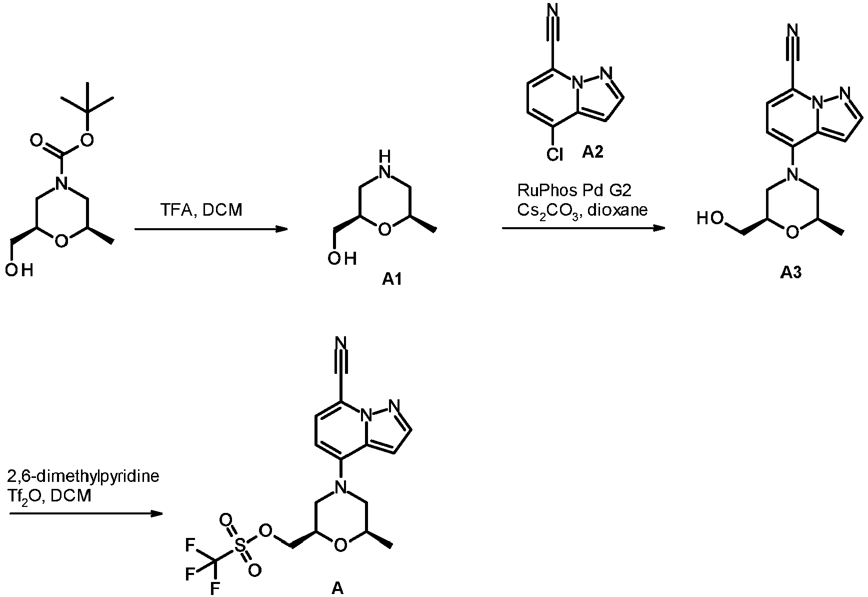

### Step 1: preparation of [(2R,6R)-6-methylmorpholin-2-yl]methanol (compound A1)

To a solution of *tert*-butyl (2*R*,6*R*)-2-(hydroxymethyl)-6-methyl-morpholine-4-carboxylate (CAS: 1700609-48-8, Vendor: WuXi Apptec, 231 mg, 1.0 mmol) in DCM (2 mL) was added TFA (1 mL) at 0 °C. The reaction mixture was stirred at rt for 2 hrs, then concentrated to give a crude compound **A1** (250 mg) which was directly used in next step. MS: calc'd 132 (MH⁺), measured 132 (MH⁺).

### Step 2: preparation of 4-[(2R,6R)-2-(hydroxymethyl)-6-methyl-morpholin-4-yl]pyrazolo[1,5-a]pyridine-7-carbonitrile (compound A3)

To a solution of crude [(2R,6R)-6-methylmorpholin-2-yl]methanol (compound **A1,** 250 mg, ~1.0 mmol), 4-chloropyrazolo[1,5-a]pyridine-7-carbonitrile (CAS: 1268520-74-6, Vendor: Pharmablock) (compound **A2,** 450 mg, 2.5 mmol) and Cs₂CO₃ (3.3 g, 10.1 mmol) in dioxane (5 mL) was added Ruphos Pd G2 (98 mg, 0.13 mmol). The reaction mixture was degassed and heated at 90 °C (oil bath) for 3 hrs, then cooled to rt, diluted with EtOAc (10 mL), and filtered through celite. The filtrate was concentrated to give a brown oil which was purified by column chromatography to give compound **A3** (521 mg) as a yellowish oil. MS: calc'd 273 (MH⁺), measured 273 (MH⁺).

### Step 3: preparation of [(2R,6R)-4-(7-cyanopyrazolo[1,5-a]pyridin-4-yl)-6-methyl-morpholin-2-yl]methyl trifluoromethanesulfonate (Intermediate A)

To a solution of 4-[(2*R*,6*R*)-2-(hydroxymethyl)-6-methyl-morpholin-4-yl]pyrazolo[1,5-a]pyridine-7-carbonitrile (compound **A3,** 190 mg, 0.70 mmol) and 2,6-dimethylpyridine (150 mg, 1.4 mmol) in DCM (4 mL) was added trifluoromethanesulfonic anhydride (295 mg, 1.05 mmol) dropwise at 0 °C. The mixture was stirred at 0 °C for 1h. The mixture was then diluted with DCM, washed with sat NH₄Cl and brine, dried over Na₂SO₄, and concentrated to give a crude product which was purified by column chromatography to give **Intermediate A** (166 mg) as a white solid. MS: calc'd 405 (MH⁺), measured 405 (MH⁺).

### Intermediate B

### [(2R,6R)-4-(7-cyano-3-fluoro-pyrazolo[1,5-a]pyridin-4-yl)-6-methyl-morpholin-2-yl]methyl trifluoromethanesulfonate

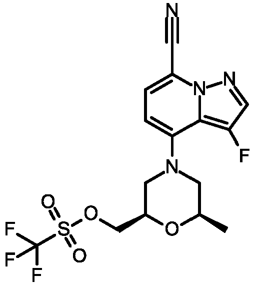

The title compound was prepared according to the following scheme:

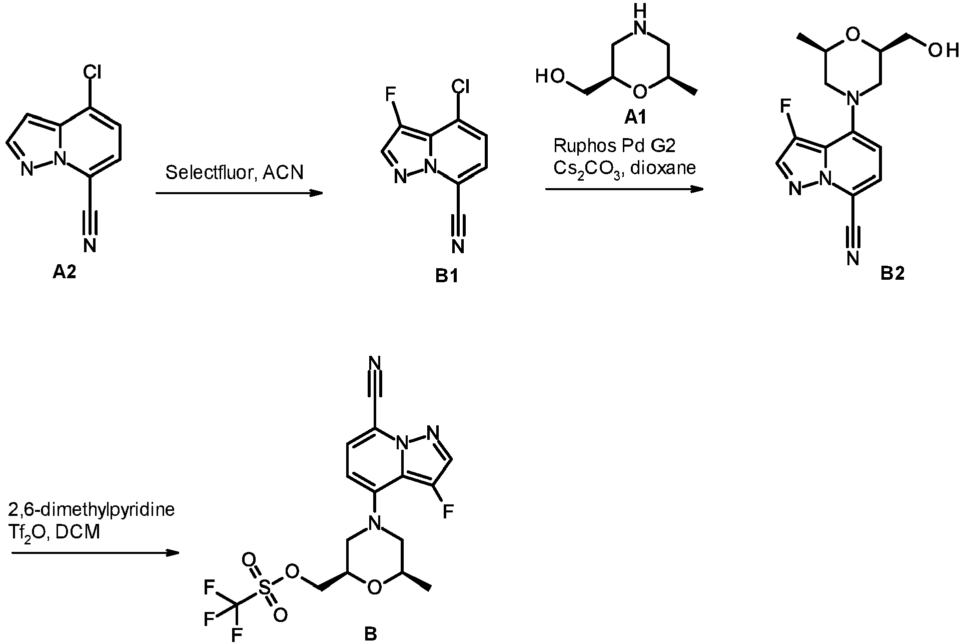

### Step 1: preparation of 4-chloro-3-fluoro-pyrazolo[1,5-a]pyridine-7-carbonitrile (compound 67a)

To a solution of 4-chloropyrazolo[1,5-a]pyridine-7-carbonitrile (compound **A2,** 600 mg, 3.38 mmol) in ACN (50 mL) was added SelectFluor (2.39 g, 6.76 mmol). The reaction mixture was stirred at rt for 24 hrs. The reaction mixture was concentrated to remove the solvent, then diluted with water (30 mL), extracted with DCM. The organic layer was washed with sat. NH₄Cl and brine, dried over Na₂SO₄, and concentrated to give a crude product which was purified by column chromatography to give compound **B1** (419 mg) as light yellow powder. MS: calc'd 196 (MH⁺), measured 196 (MH⁺). ¹H NMR (400MHz, METHANOL-d₄) *δ* 8.17 (d, *J=* 3.5 Hz, 1H), 7.55 (d, *J* = 7.6 Hz, 1H), 7.35 (d, *J* = 7.7 Hz, 1H).

### Step 2: preparation of 3-fluoro-4-[(2R,6R)-2-(hydroxymethyl)-6-methyl-morpholin-4-yl]pyrazolo[1,5-a]pyridine-7-carbonitrile (compound B2)

To a solution of 4-chloro-3-fluoropyrazolo[1,5-a]pyridine-7-carbonitrile (compound **67a,** 419 mg, 2.14 mmol), (2*R*,6*R*)-6-methylmorpholin-2-yl]methanol trifluoroacetate (compound **1a,** 526 mg, 2.14 mmol) and Cs₂CO₃ (2.79 g, 8.57 mmol) in dioxane (10 mL) was added RuPhos Pd G2 (116 mg, 0.15 mmol) under N₂. The reaction mixture was heated at 90 °C (oil bath) for 2 hrs, then cooled to rt, diluted with EtOAc and filtered through celite. The filtrate was concentrated to give a brown oil which was purified by column chromatography to give compound **B2** (325 mg) as a yellow oil. MS: calc'd 291 (MH⁺), measured 291 (MH⁺).

### Step 3: preparation of (2R,6R)-4-(7-cyanopyrazolo[1,5-a]pyridin-4-yl)-6-methyl-morpholin-2-yl]methyl trifluoromethanesulfonate (Intermediate B)

To a solution of 3-fluoro-4-[(2*R*,6*R*)-2-(hydroxymethyl)-6-methyl-morpholin-4-yl]pyrazolo[1,5-a]pyridine-7-carbonitrile (compound **B2,** 325 mg, 1.12 mmol) in DCM (3 mL) was added 2,6-dimethylpyridine (240 mg, 258 µL, 2.24 mmol) and Tf₂O (474 mg, 284 µL, 1.68 mmol) at rt. The reaction mixture was stirred at rt for 1.5 hrs. The mixture was then diluted with DCM, washed with sat NH₄Cl and brine. The organic layer was dried over Na₂SO₄ and concentrated to give the crude product which was purified by column chromatography to give **Intermediate B** (180 mg) as a yellow solid. MS: calc'd 423 (MH⁺), measured 423 (MH⁺).

### Intermediate C

### [(2R,6R)-4-(8-cyanoquinoxalin-5-yl)-6-methyl-morpholin-2-yl] methyl trifluoromethanesulfonate

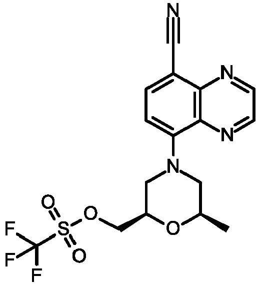

The title compound was prepared in analogy to the preparation of **Intermediate A** by using 8-bromoquinoxaline-5-carbonitrile (Synthesis refer to US 20170174653 A1) instead of 4-chloropyrazolo[1,5-a]pyridine-7-carbonitrile. **Intermediate** C (825 mg) was obtained as an off-white solid. MS: calc'd 417 (MH⁺), measured 417 (MH⁺).

### Intermediate D

### [(2R,6R)-4-(8-cyano-5-quinolyl)-6-methyl-morpholin-2-yl]methyl trifluoromethanesulfonate

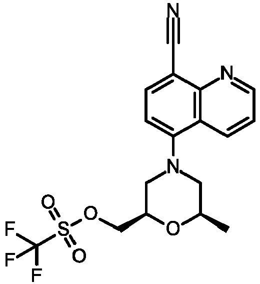

The title compound was prepared in analogy to the preparation of **Intermediate** A by using 8-bromoquinoline-5-carbonitrile instead of 4-chloropyrazolo[1,5-a]pyridine-7-carbonitrile (CAS: 1268520-74-6, Vendor: Pharmablock). **Intermediate D** (300 mg) was obtained as a yellow gum. MS: calc'd 416 (MH⁺), measured 416 (MH⁺).

### Intermediate E

### (2R,6R)-4-(4-cyano-1,3-benzothiazol-7-yl)-6-methyl-morpholin-2-yl]methyl trifluoromethanesulfonate

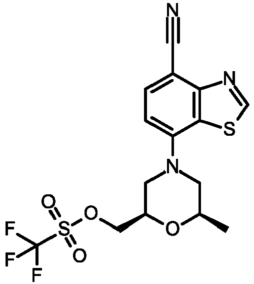

The title compound was prepared according to the following scheme:

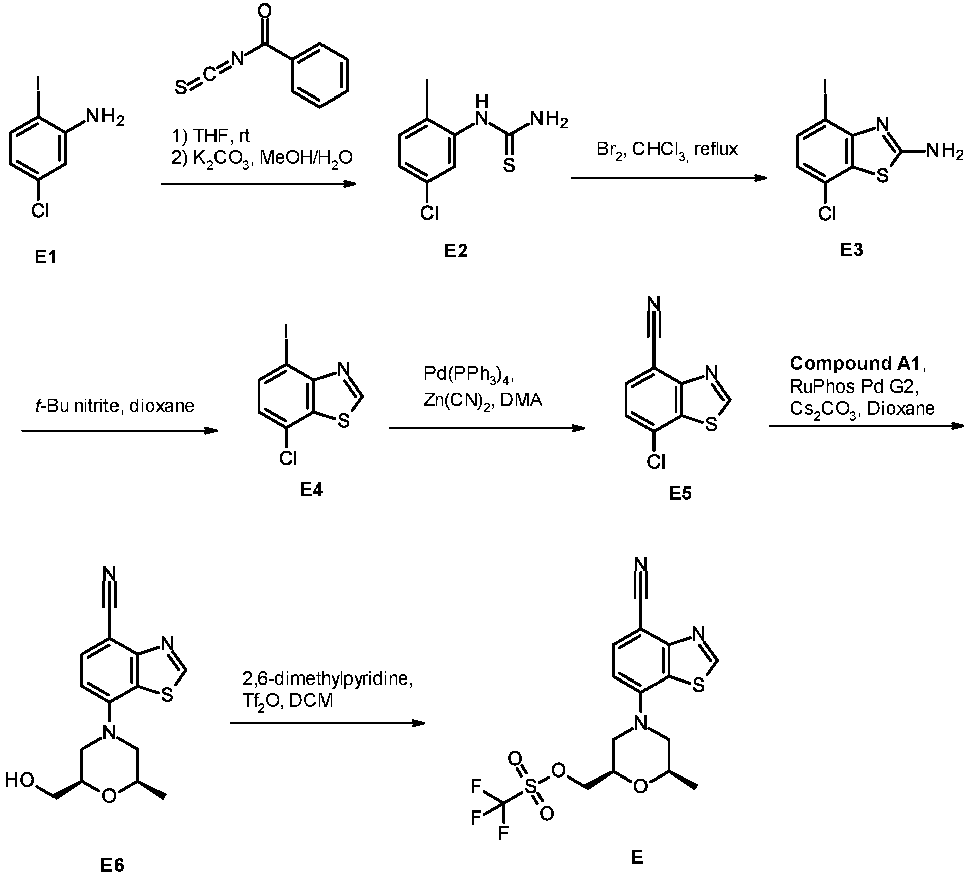

### Step 1: preparation of 1-(5-chloro-2-iodophenyl)thiourea (compound E2)

To a solution of 5-chloro-2-iodo-aniline (compound **E1,** 5.0 g, 19.7 mmol) in THF (87 mL) was added benzoyl isothiocyanate (6.4 g, 39.4 mmol) at 10-20 °C. After the reaction was stirred at 20 °C for 16 hrs, the solvent was removed and the solid was washed with EtOH/PE (50 mL, v/v = 4/1), followed by air-drying to afford an intermediate (8.7 g). The intermediate was dissolved in methanol (100 mL) and treated with a solution of K₂CO₃ (8.2 g, 59.2 mmol) in water (40 mL). The reaction mixture was heated at 70 °C for 4 hrs, then solvent was removed under vacuum followed by addition of water (20 mL). The solid was collected by filtration, further washed with water (20 mL), and dried to afford compound **E2** (5.0 g) as a yellow solid.

### Step2 : preparation of 7-chloro-4-iodo-1,3-benzothiazol-2-amine (compound E3)

To a solution of 1-(5-chloro-2-iodophenyl)thiourea (compound **E2,** 4.0 g, 12.8 mmol) in CHCl₃ (32 mL) was added bromine (0.66 mL, 12.8 mmol). The reaction mixture was heated at 78 °C for 18 hrs. After the reaction was cooled to rt, the solvent was removed under vacuum and the solid was dissolved in DCM/MeOH (100 mL, v/v = 1/1), to which aqueous solution of Na₂S₂C₃ (20 mL) was added. Then the solvent was removed under vacuum, and water (30 mL) was added. The solid was collected by filtration, and dried to give compound **E3** (3.5 g) as a yellow solid. MS: calc'd 311 (MH⁺), measured 311 (MH⁺).

### Step 3 : preparation of 7-chloro-4-iodo-1,3-benzothiazole (compound E4)

To a solution of 7-chloro-4-iodo-1,3-benzothiazol-2-amine (compound **E3,** 3.0 g, 9.7 mmol) in 1,4-dioxane (60 mL) was added *tert*-butyl nitrite (2.0 g, 19.3 mmol). The reaction mixture was heated at 90 °C for 18 hrs and then the solvent was removed under vacuum. The residue was purified by column chromatography to give a crude product, which was triturated in PE (15 mL) to give compound **E4** (1.5 g) as a white solid. MS: calc'd 296 (MH⁺), measured 296 (MH⁺).

### Step 4 : preparation of 7-chloro-1,3-benzothiazole-4-carbonitrile (compound E5)

To a solution of 7-chloro-4-iodo-1,3-benzothiazole (compound **E4,** 1.5 g, 5.1 mmol) in DMA (40 mL) was added Zn(CN)₂ (0.89 g, 7.6 mmol) and Pd(PPh₃)₄ (0.20 g, 0.17 mmol). The reaction mixture was heated at 100 °C for 18 hrs and then poured into water (100 mL). The solid was collected by filtration, and dissolved in EtOAc (400 mL). The organic layer was washed with brine, dried over anhydrous Na₂SO₄ and concentrated to give compound **E5** (0.9 g) as a white solid. MS: calc'd 195 (MH⁺), measured 195 (MH⁺).

### Step 5 : preparation of 7-[(2R,6R)-2-(hydroxymethyl)-6-methyl-morpholin-4-yl]-1,3-benzothiazole-4-carbonitrile (compound E6)

To a solution of tert-butyl (2R,6R)-2-(hydroxymethyl)-6-methyl-morpholine-4-carboxylate (61 mg, 0.26 mmol) in DCM (2 mL) was added HCl (0.5 mL, 10% in MeOH) at rt. After the reaction mixture was stirred at rt for 2 hrs, the solvent was removed, and the residue was dissolved in DMA (0.5 mL). To the solution was added 7-chloro-1,3-benzothiazole-4-carbonitrile (compound **E5,** 52 mg, 0.31 mmol) and DIPEA (0.13 mL, 0.77 mmol). After the reaction mixture was stirred at 150 °C for 2 hrs, water (10 mL) was added. The mixture was then cooled and extracted with EtOAc (10 mL). The organic layer was washed with brine, dried over anhydrous Na₂SO₄, and concentrated to give a crude product which was purified by prep-TLC (PE/EA, v/v = 2/1) to give compound **E6** (15 mg) as a yellow gum. MS: calc'd 290 (MH⁺), measured 290 (MH⁺).

### Step 6 : preparation of ((2R,6R)-4-(4-cyano-1,3-benzothiazol-7-yl)-6-methylmorpholin-2-yl)methyl trifluoromethanesulfonate (Intermediate E)

To a solution of 7-[(2*R*,6*R*)-2-(hydroxymethyl)-6-methyl-morpholin-4-yl]-1,3-benzothiazole-4-carbonitrile (compound **E6,** 15 mg, 0.050 mmol) in DCM (1 mL) was added 2,6-dimethylpyridine (22 mg, 0.21 mmol) and trifluoromethanesulfonic anhydride (29 mg, 0.10 mmol). The reaction mixture was stirred at 0 °C for 1h and then poured into ice-water (10 mL). The mixture was extracted with DCM (10 mL), which was dried over Na₂SO₄, and concentrated to give **Intermediate E** (20 mg) as a yellow solid. MS: calc'd 422 (MH⁺), measured 422 (MH⁺).

### Intermediate F

### [(2R,6R)-4-(8-chloro-5-quinolyl)-6-methyl-morpholin-2-yl]methyl trifluoromethanesulfonate

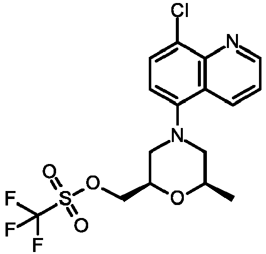

The title compound was prepared in analogy to the preparation of **Intermediate A** by using 5-bromo-8-chloro-quinoline (CAS: 927800-41-7, Vendor: Bepharm) instead of 4-chloropyrazolo[1,5-a]pyridine-7-carbonitrile. **Intermediate F** (86 mg) was obtained as an oil. MS: calc'd 425 (MH⁺), measured 425 (MH⁺).

### Intermediate G

### [(2R,6R)-4-(8-chloroquinoxalin-5-yl)-6-methyl-morpholin-2-yl] methyl trifluoromethanesulfonate

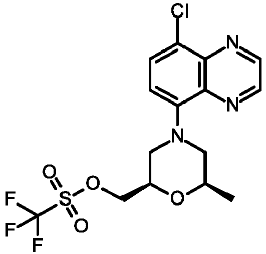

The title compound was prepared in analogy to the preparation of **Intermediate A** by using 5-bromo-8-chloro-quinoxaline (Catalog No: PB94364, Vendor: PharmaBlock) instead of 4-chloropyrazolo[1,5-a]pyridine-7-carbonitrile. **Intermediate G** (85 mg) was obtained as an oil. MS: calc'd 426 (MH⁺), measured 426 (MH⁺).

### Reference Compound R1

### 4-[(2S,6R)-2-(3,4-dihydro-1H-2,6-naphthyridin-2-ylmethyl)-6-methyl-morpholin-4-yl]pyrazolo[1,5-a]pyridine-7-carbonitrile

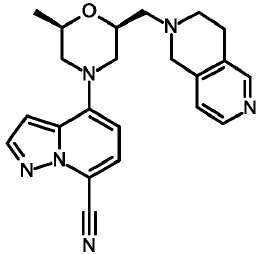

The title compound was prepared according to the following scheme:

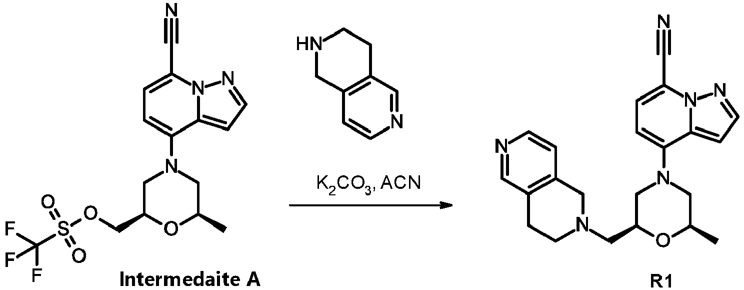

To a solution of [(2R,6R)-4-(7-cyanopyrazolo[1,5-a]pyridin-4-yl)-6-methyl-morpholin-2-yl]methyl trifluoromethanesulfonate (Intermediate **A,** 30 mg, 0.074 mmol) and 1,2,3,4-tetrahydro-2,6-naphthyridine (15 mg, 0.11 mmol) in ACN (3 mL) was added K₂CO₃ (20 mg, 0.14 mmol) at rt. The reaction mixture was refluxed for 6 hrs, then diluted with ACN (10 mL) and filtered through celite. The filtrate was concentrated to give a yellow solid which was purified by prep-HPLC to give **Compound R1** (8 mg) as a light brown solid. MS: calc'd 389 (MH⁺), measured 389 (MH⁺). ¹H NMR (400MHz, METHANOL-d4) *δ* 8.60 (br d, *J* = 18.0 Hz, 2H), 8.04 (d, *J* = 2.2 Hz, 1H), 7.64 (br d, *J* = 5.5 Hz, 1H), 7.49 (d, *J* = 7.9 Hz, 1H), 6.91 (d, *J =* 2.3 Hz, 1H), 6.64 (d, *J* = 7.9 Hz, 1H), 4.69 (s, 2H), 4.43 (br t, *J* = 9.8 Hz, 1H), 4.14 - 4.04 (m, 1H), 3.89 - 3.74 (m, 4H), 3.58 - 3.47 (m, 2H), 3.47 - 3.36 (m, 2H), 2.85 - 2.68 (m, 2H), 1.34 (d, *J* = 6.2 Hz, 3H).

### Reference Compound R2

### 4-[(2S,6R)-2-(1,3-dihydropyrrolo [3,4-c] pyridin-2-ylmethyl)-6-methyl-morpholin-4-yl]pyrazolo [1,5-a]pyridine-7-carbonitrile

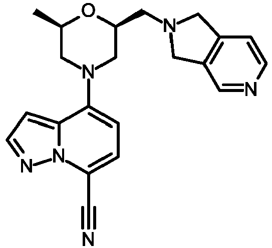

The title compound was prepared in analogy to the preparation of **Compound R1** by using 2,3-dihydro-1*H*-pyrrolo[3,4-c]pyridine instead of 1,2,3,4-tetrahydro-2,6-naphthyridine. **Compound R2** (15 mg) was obtained as a light yellow solid. MS: calc'd 375 (MH⁺), measured 375 (MH⁺). ¹H NMR (400 MHz, METHANOL-d4) *δ* 8.76 - 8.63 (m, 2H), 8.05 (d, *J* = 2.4 Hz, 1H), 7.67 (br d, *J* = 5.0 Hz, 1H), 7.49 (d, *J* = 7.9 Hz, 1H), 6.92 (d, *J* = 2.4 Hz, 1H), 6.65 (d, *J =* 7.9 Hz, 1H), 4.94 (br s, 4H), 4.37 - 4.28 (m, 1H), 4.11 - 4.02 (m, 1H), 3.89 - 3.77 (m, 2H), 3.74 - 3.60 (m, 2H), 2.84 - 2.71 (m, 2H), 1.35 (d, *J* = 6.4 Hz, 3H).

### Reference Compound R3

### 5-[(2S,6R)-2-(isoindolin-2-ylmethyl)-6-methyl-morpholin-4-yl]quinoline-8-carbonitrile

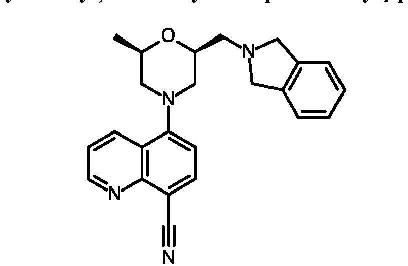

The title compound was prepared in analogy to the preparation of **Compound R1** by using isoindoline instead of 1,2,3,4-tetrahydro-2,6-naphthyridine. **Compound R3** (17 mg) was obtained as a light yellow solid. MS: calc'd 385 (MH⁺), measured 385 (MH⁺). ¹H NMR (400 MHz, METHANOL-d4) δ ¹H NMR (400MHz, METHANOL-d₄) *δ* = 8.90 (dd, *J* = 1.7, 4.2 Hz, 1H), 8.58 (dd, *J* = 1.7, 8.6 Hz, 1H), 8.07 (d, *J =* 7.9 Hz, 1H), 7.56 (dd, *J* = 4.2, 8.6 Hz, 1H), 7.41 - 7.29 (m, 4H), 7.20 (d, *J* = 7.9 Hz, 1H), 4.97 - 4.80 (m, 1H), 4.71 - 4.50 (m, 1H), 4.45 - 4.31 (m, 1H), 4.19 - 4.06 (m, 1H), 3.64 - 3.47 (m, 2H), 3.41 - 3.30 (m, 2H), 2.78 - 2.60 (m, 2H), 1.24 (d, *J* = 6.4 Hz, 3H).

### Example 3

### 4-[(2R,6S)-2-methyl-6-[(4-piperazin-1-ylisoindolin-2-yl)methyl] morpholin-4-yl]pyrazolo[1,5-a]pyridine-7-carbonitrile

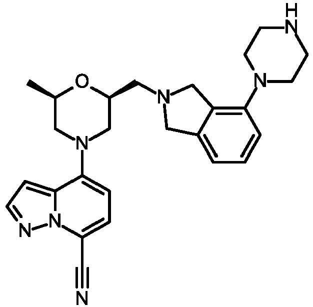

The title compound was prepared according to the following scheme:

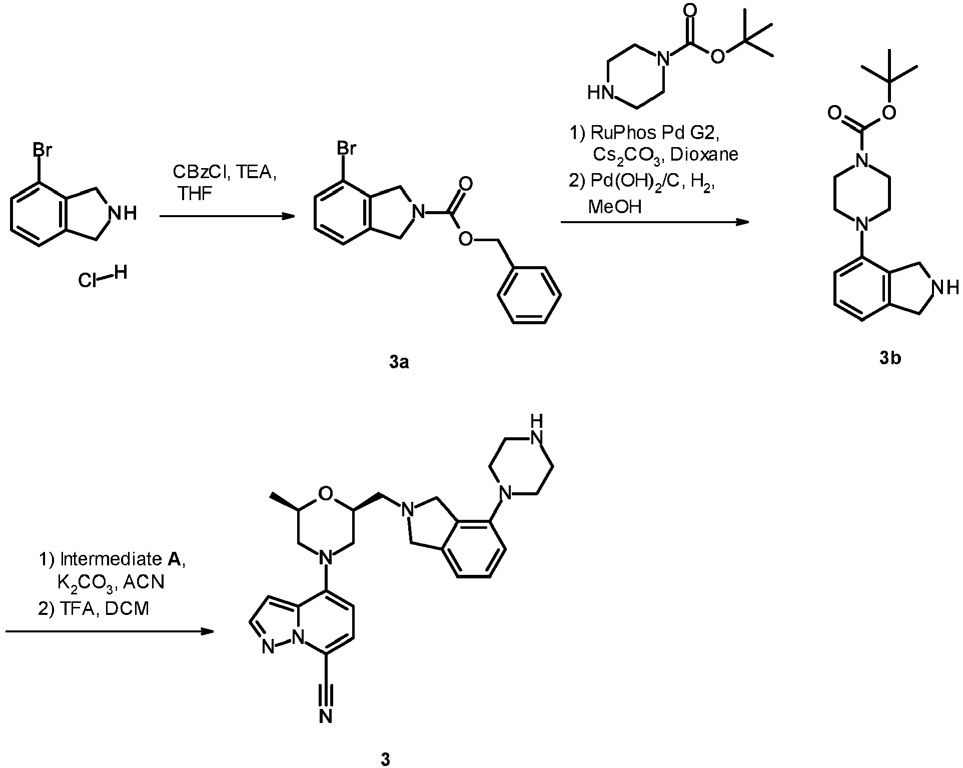

### Step 1: preparation of benzyl 4-bromoisoindoline-2-carboxylate (compound 3a)

To a solution of 4-bromoisoindoline hydrochloride (235 mg, 1.0 mmol) in THF (4 mL) was added TEA (355 mg, 0.49 mL, 3.5 mmol). The mixture was then cooled with ice-water bath. CbzCl (256 mg, 0.22 mL, 1.5 mmol) was added dropwise. The reaction mixture was stirred at rt overnight, then diluted with water (20 mL), and extracted with EtOAc (30 mL) twice. The combined organic layer was washed with brine, dried over Na₂SO₄ and concentrated to give a crude product which was purified by column chromatography to afford compound **3a** (255 mg) as a pink solid. MS: calc'd 332&334 (MH⁺), measured 332&334 (MH⁺).

### Step 2: preparation of tert-butyl 4-isoindolin-4-ylpiperazine-1-carboxylate (compound 3b)

To a solution of benzyl 4-bromoisoindoline-2-carboxylate (compound **3a,** 50 mg, 0.15 mmol) in dioxane (4 mL) was added tert-butyl piperazine-1-carboxylate (56 mg, 0.30 mmol) and Cs₂CO₃ (147 mg, 0.45 mmol). The suspension was bubbled with N₂ for 5 mins, then Ruphos Pd G2 (12 mg, 15 µmol) was added. The mixture was heated at 100 °C overnight, then cooled to rt, and diluted with EtOAc (10 mL). The mixture was filtered through celite, and the filtrate was concentrated. The residue was purified by column chromatography to give a pale yellow oil (37 mg) which was dissolved in MeOH (10 mL). To the solution was added Pd(OH)₂ on carbon (85 mg, 10% wet). The suspension was charged with hydrogen balloon and stirred at rt for 4 hrs. The mixture was then filtered through celite, the filtrated was concentrated to give a crude compound **3b** (25 mg) which was directly used in next step. MS: calc'd 304 (MH⁺), measured 304 (MH⁺).

### Step 3: preparation of 4-[(2R,6S)-2-methyl-6-[(4-piperazin-1-ylisoindolin-2-yl)methyl]morpholin-4-yl]pyrazolo[1,5-a]pyridine-7-carbonitrile (Example 3)

To a solution of [(2R,6R)-4-(7-cyanopyrazolo[1,5-a]pyridin-4-yl)-6-methyl-morpholin-2-yl]methyl trifluoromethanesulfonate (Intermediate **A,** 30 mg, 74 µmol) in ACN (4 mL) was added *tert*-butyl 4-(isoindolin-4-yl)piperazine-1-carboxylate (compound **3b,** 25 mg, 82 µmol) and K₂CO₃ (31 mg, 0.22 mmol) at rt. The suspension was heated to reflux for 3 hrs, then cooled to rt. The mixture was diluted with ACN (5 mL), filtered through celite. The filtrate was concentrated to give a yellow solid which was dissolved in DCM (2 mL). To the solution was added TFA (1 mL). The mixture was stirred at rt for 2 hrs, then concentrated to give a crude mixture which was purified by prep-HPLC to give **Example 3** (10 mg) as a grey powder. MS: calc'd 458 (MH⁺), measured 458 (MH⁺). ¹H NMR (400 MHz, METHANOL-d4) *δ* 8.06 (d, *J* = 2.4 Hz, 1H), 7.51 (d, *J* = 8.1 Hz, 1H), 7.38 (t, *J* = 7.8 Hz, 1H), 7.13 (d, *J* = 7.6 Hz, 1H), 7.05 (d, *J* = 8.2 Hz, 1H), 6.93 (d, *J* = 2.3 Hz, 1H), 6.66 (d, *J* = 8.1 Hz, 1H), 4.59 - 4.40 (m, 4H), 4.31 - 4.20 (m, 1H), 4.10 - 4.00 (m, 1H), 3.88 (br d, *J* = 12.1 Hz, 1H), 3.82 (br d, *J* = 12.0 Hz, 1H), 3.47 - 3.36 (m, 6H), 3.28 - 3.20 (m, 4H), 2.84 - 2.69 (m, 2H), 1.34 (d, *J =* 6.2 Hz, 3H).

### Example 4

### 4-[(2R,6S)-2-methyl-6-[(5-piperazin-1-ylisoindolin-2-yl)methyl]morpholin-4-yl]pyrazolo[1,5-a]pyridine-7-carbonitrile

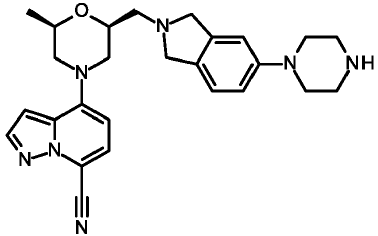

The title compound was prepared in analogy to the preparation of **Example 3** by using 5-bromoisoindoline hydrochloride instead of 4-bromoisoindoline hydrochloride. **Example 4** (17 mg) was obtained as a grey powder. MS: calc'd 458 (MH⁺), measured 458 (MH⁺). ¹H NMR (400 MHz, METHANOL-d4) *δ* 8.06 (d, *J* = 2.3 Hz, 1H), 7.51 (d, *J* = 8.1 Hz, 1H), 7.29 (d, *J* = 8.2 Hz, 1H), 7.08 - 7.01 (m, 2H), 6.93 (d, *J* = 2.4 Hz, 1H), 6.66 (d, *J* = 7.9 Hz, 1H), 4.54 - 4.35 (m, 4H), 4.25 - 4.16 (m, 1H), 4.09 - 3.98 (m, 1H), 3.84 (br dd, *J* = 12.0, 19.9 Hz, 2H), 3.47 - 3.35 (m, 10H), 2.82 - 2.69 (m, 2H), 1.34 (d, *J* = 6.2 Hz, 3H).

### Example 5

### 4-[(2R,6S)-2-methyl-6-[(8-piperazin-1-yl-3,4-dihydro-1H-isoquinolin-2-yl)methyl]morpholin-4-yl]pyrazolo[1,5-a]pyridine-7-carbonitrile

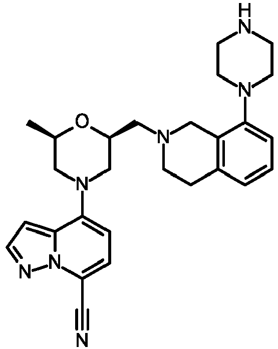

The title compound was prepared in analogy to the preparation of **Example 3** by using 8-bromo-1,2,3,4-tetrahydroisoquinoline instead of 4-bromoisoindoline hydrochloride. **Example 5** (22 mg) was obtained as a white powder. MS: calc'd 472 (MH⁺), measured 472 (MH⁺). ¹H NMR (400 MHz, METHANOL-d4) *δ* 8.05 (d, *J* = 2.4 Hz, 1H), 7.50 (d, *J* = 7.9 Hz, 1H), 7.43 - 7.37 (m, 1H), 7.22 (d, *J* = 7.8 Hz, 1H), 7.16 (d, *J* = 7.8 Hz, 1H), 6.92 (d, *J* = 2.4 Hz, 1H), 6.65 (d, *J* = 7.9 Hz, 1H), 4.44 (br t, *J* = 9.8 Hz, 2H), 4.14 - 3.98 (m, 1H), 3.93 - 3.77 (m, 3H), 3.68 - 3.33 (m, 8H), 3.29 - 3.07 (m, 6H), 2.83 - 2.70 (m, 2H), 1.35 (d, *J* = 6.2 Hz, 3H).

### Example 6

### 4-[(2R,6S)-2-methyl-6-[(7-piperazin-1-yl-3.4-dihydro-2H-quinolin-1-yl)methyl]morpholin-4-yl]pyrazolo[1,5-a]pyridine-7-carbonitrile

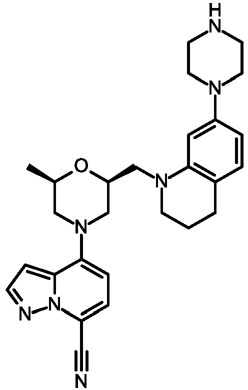

The title compound was prepared in analogy to the preparation of **Example 3** by using 7-bromo-1,2,3,4-tetrahydroquinoline instead of 4-bromoisoindoline hydrochloride. **Example 6** (5 mg) was obtained as a white powder. MS: calc'd 472 (MH⁺), measured 472 (MH⁺). ¹H NMR (400 MHz, METHANOL-d4) *δ* 8.00 (d, *J* = 2.3 Hz, 1H), 7.47 (d, *J* = 7.9 Hz, 1H), 6.88 - 6.83 (m, 1H), 6.71 (d, *J* = 2.4 Hz, 1H), 6.58 (d, *J* = 8.1 Hz, 1H), 6.35 (s, 1H), 6.26 (dd, *J* = 2.1, 8.2 Hz, 1H), 4.15 - 4.07 (m, 1H), 3.97 - 3.84 (m, 2H), 3.74 (br d, *J* = 12.2 Hz, 1H), 3.54 - 3.34 (m, 6H), 3.31 - 3.17 (m, 6H), 2.80 (dd, *J* = 10.6, 12.2 Hz, 1H), 2.73 - 2.65 (m, 3H), 1.92 (quin, *J* = 5.7 Hz, 2H), 1.28 (d, *J* = 6.2 Hz, 3H).

### Example 7

### 3-fluoro-4-[(2R,6S)-2-methyl-6-[(5-piperazin-1-ylisoindolin-2-yl)methyl]morpholin-4-yl]pyrazolo[1,5-a]pyridine-7-carbonitrile

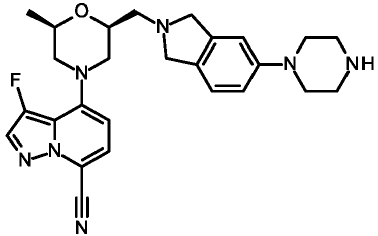

The title compound was prepared in analogy to the preparation of **Example 3** by using 5-bromoisoindoline hydrochloride and Intermediate **B** instead of 4-bromoisoindoline hydrochloride and intermediate **A. Example** 7 (19 mg) was obtained as a light yellow powder. MS: calc'd 472 (MH⁺), measured 472 (MH⁺). ¹H NMR (400 MHz, METHANOL-d4) *δ* 8.04 (d, *J* = 3.7 Hz, 1H), 7.47 (d, *J* = 7.9 Hz, 1H), 7.36 (d, *J* = 9.2 Hz, 1H), 7.14 - 7.08 (m, 2H), 6.59 (d, *J* = 7.9 Hz, 1H), 5.04 - 4.90 (m, 2H), 4.76 - 4.52 (m, 2H), 4.30 (br t, *J* = 9.8 Hz, 1H), 4.07 (ddd, *J* = 2.1, 6.4, 10.2 Hz, 1H), 3.72 - 3.59 (m, 4H), 3.49 - 3.44 (m, 4H), 3.43 - 3.37 (m, 4H), 2.84 - 2.68 (m, 2H), 1.34 (d, *J* = 6.4 Hz, 3H).

### Example 8

### 4-[(2R,6S)-2-methyl-6-[[5-(4-piperidyl)isoindolin-2-yl]methyl]morpholin-4-yl]pyrazolo[1,5-a]pyridine-7-carbonitrile

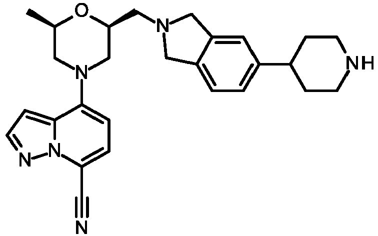

The title compound was prepared according to the following scheme:

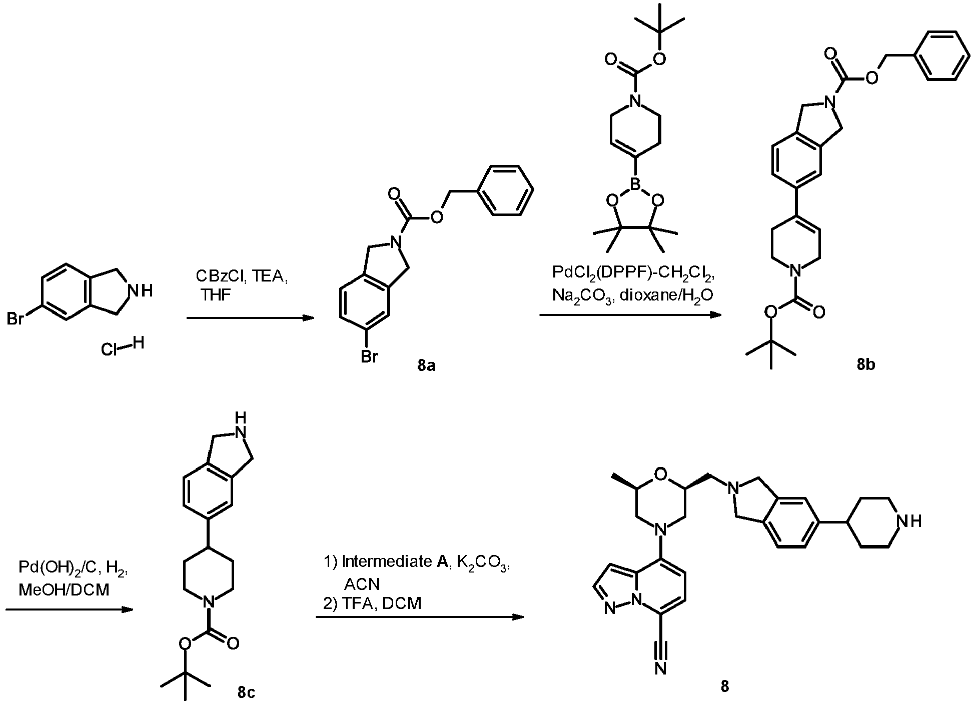

### Step 1: preparation of benzyl 5-bromoisoindoline-2-carboxylate (compound 8a)

To a solution of 5-bromoisoindoline hydrochloride (235 mg, 1.0 mmol) in THF (4 mL) was added TEA (355 mg, 0.49 mL, 3.5 mmol). The solution was cooled with ice-water bath and CbzCl (256 mg, 0.22 mL, 1.5 mmol) was added drop-wise. The reaction mixture was stirred at rt for 3 hrs, then diluted with water, extracted with EtOAc. The organic layer was washed with brine, dried over Na₂SO₄, and concentrated to give a crude product which was purified by column chromatography to give compound **8a** (265 mg) as a white solid. MS: calc'd 332&334 (MH⁺), measured 332&334 (MH⁺).

### Step 2: preparation of benzyl 5-(1-tert-butoxycarbonyl-3,6-dihydro-2H-pyridin-4-yl)isoindoline-2-carboxylate (compound 8b)

To a solution of benzyl 5-bromoisoindoline-2-carboxylate (166 mg, 500 µmol) in dioxane/water (4.5 mL, v/v = 8/1) was added *tert*-butyl 4-(4,4,5,5-tetramethyl-1,3,2-dioxaborolan-2-yl)-3,6-dihydro-2*H*-pyridine-1-carboxylate (232 mg, 0.75 mmol) and Na₂CO₃ (106 mg, 1.0 mmol). The suspension was bubbled with N₂ for 5 mins, PdCl₂(DPPF)-CH₂Cl₂ adduct (37 mg, 50 µmol) was added. The reaction mixture was heated at 100 °C (oil bath) overnight, then cooled to rt, diluted with EtOAc (10 mL) and filtered through celite, the filtrate was concentrated to give a crude product which was purified by column chromatography to give compound **8b** (141 mg) as a white solid. MS: calc'd 435 (MH⁺), measured 435 (MH⁺).

### Step 3: preparation of tert-butyl 4-isoindolin-5-ylpiperidine-1-carboxylate

To a solution of benzyl 5-(1-(*tert*-butoxycarbonyl)-1,2,3,6-tetrahydropyridin-4-yl)isoindoline-2-carboxylate (compound **8b,** 70 mg, 0.16 mmol) in MeOH/DCM (5 mL, v/v = 4/1) was added Pd(OH)₂ on carbon (10 mg, 10% wet). The reaction mixture was charged with hydrogen balloon and stirred at rt for 4 hrs, then filtered through celite, the filtrate was concentrated to give a crude compound **8c** (52 mg) as a brown solid which was used directly in next step. MS: calc'd 303 (MH⁺), measured 303 (MH⁺).

### Step 4: preparation of 4-[(2R,6S)-2-methyl-6-[[5-(4-piperidyl)isoindolin-2-yl]methyl]morpholin-4-yl]pyrazolo[1,5-a]pyridine-7-carbonitrile (Example 8)

To a solution of ((2*R*,6*R*)-4-(7-cyanopyrazolo[1,5-a]pyridin-4-yl)-6-methylmorpholin-2-yl)methyl trifluoromethanesulfonate (Intermediate **A,** 30 mg, 74 µmol) in ACN (4 mL) was added tert-butyl 4-(isoindolin-5-yl)piperidine-1-carboxylate (27 mg, 89 µmol) and K₂CO₃ (10 mg, 74 µmol). The suspension was heated to reflux for 2 hrs, then cooled to rt, diluted with ACN (10 mL) and filtered through celite, the filtrate was concentrated to give a yellow solid which was dissolved in DCM (2 mL), followed by addition of TFA (1 mL). The reaction mixture was stirred at rt for 2 hrs, then concentrated to give a crude mixture which was purified by prep-HPLC to afford **Example 8** (16 mg) as a light yellow powder. MS: calc'd 457 (MH⁺), measured 457 (MH⁺). ¹H NMR (400 MHz, METHANOL-d4) *δ* 8.06 (d, *J* = 2.3 Hz, 1H), 7.50 (d, *J* = 7.9 Hz, 1H), 7.45 - 7.40 (m, 1H), 7.38 - 7.34 (m, 2H), 6.92 (d, *J* = 2.4 Hz, 1H), 6.66 (d, *J* = 8.1 Hz, 1H), 4.94 - 4.91 (m, 2H), 4.32 (br t, *J* = 10.3 Hz, 1H), 4.12 - 4.02 (m, 1H), 3.87 - 3.77 (m, 2H), 3.71 - 3.58 (m, 2H), 3.56 - 3.52 (m, 1H), 3.52 - 3.46 (m, 2H), 3.23 - 3.09 (m, 3H), 2.99 (br t, *J* = 12.2 Hz, 1H), 2.83 - 2.71 (m, 2H), 2.09 (br d, *J* = 14.1 Hz, 2H), 1.97 - 1.85 (m, 2H), 1.35 (d, *J* = 6.2 Hz, 3H).

### Example 9

### 4-[(2R,6S)-2-methyl-6-[(5-pyrrolidin-3-ylisoindolin-2-yl)methyl]morpholin-4-yl]pyrazolo[1,5-a]pyridine-7-carbonitrile

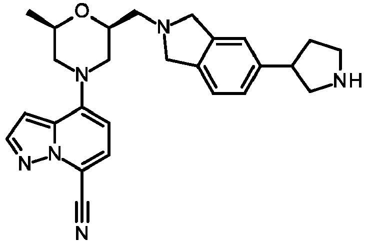

The title compound was prepared in analogy to the preparation of **Example 8** by using tert-butyl 3-(4,4,5,5-tetramethyl-1,3,2-dioxaborolan-2-yl)-2,5-dihydro-1*H*-pyrrole-1-carboxylate instead of tert-butyl 4-(4,4,5,5-tetramethyl-1,3,2-dioxaborolan-2-yl)-3,6-dihydro-2H-pyridine-1-carboxylate. **Example 9** (15 mg) was obtained as a white powder. MS: calc'd 443 (MH⁺), measured 443 (MH⁺). ¹H NMR (400 MHz, METHANOL-d4) *δ* 8.04 (d, *J* = 2.4 Hz, 1H), 7.49 (d, *J* = 7.9 Hz, 1H), 7.47 - 7.37 (m, 3H), 6.92 (d, *J* = 2.4 Hz, 1H), 6.65 (d, *J* = 7.9 Hz, 1H), 5.04 - 4.91 (m, 1H), 4.85 - 4.57 (m, 3H), 4.38 - 4.28 (m, 1H), 4.12 - 4.02 (m, 1H), 3.82 (br dd, *J =* 12.3, 19.4 Hz, 2H), 3.77 - 3.68 (m, 2H), 3.67 - 3.53 (m, 3H), 3.40 (dt, *J* = 7.2, 10.9 Hz, 1H), 3.22 (t, *J* = 10.9 Hz, 1H), 2.83 - 2.69 (m, 2H), 2.54 - 2.43 (m, 1H), 2.18 - 2.05 (m, 1H), 1.34 (d, *J* = 6.4 Hz, 3H).

### Example 10

### 4-[(2R,6S)-2-methyl-6-[(6-piperazin-1-yl-3,4-dihydro-1H-isoquinolin-2-yl)methyl]morpholin-4-yl]pyrazolo[1,5-a]pyridine-7-carbonitrile

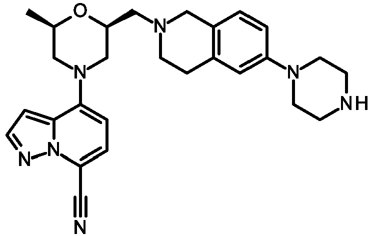

The title compound was prepared in analogy to the preparation of **Example 3** by using 6-bromo-1,2,3,4-tetrahydroisoquinoline instead of 4-bromoisoindoline hydrochloride. **Example 10** (5 mg) was obtained as a white powder. MS: calc'd 472 (MH⁺), measured 472 (MH⁺). ¹H NMR (400 MHz, METHANOL-d4) *δ* 8.04 (d, *J* = 2.3 Hz, 1H), 7.49 (d, *J* = 7.9 Hz, 1H), 7.17 (br d, *J* = 8.4 Hz, 1H), 7.00 (dd, *J* = 2.5, 8.5 Hz, 1H), 6.94 - 6.89 (m, 2H), 6.64 (d, *J* = 7.9 Hz, 1H), 4.64 - 4.32 (m, 3H), 4.13 - 4.01 (m, 1H), 3.81 (br t, *J* = 11.6 Hz, 3H), 3.57 - 3.35 (m, 11H), 3.28 - 3.15 (m, 2H), 2.82 - 2.68 (m, 2H), 1.33 (d, *J* = 6.2 Hz, 3H).

### Example 11

### 4-[(2R,6S)-2-methyl-6-[(7-piperazin-1-yl-3,4-dihydro-1H-isoquinolin-2-yl)methyl]morpholin-4-yl]pyrazolo[1,5-a]pyridine-7-carbonitrile

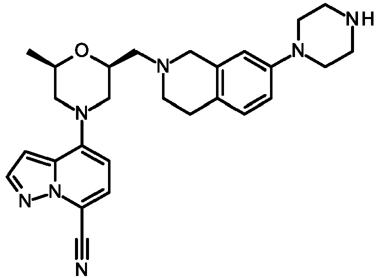

The title compound was prepared in analogy to the preparation of **Example 3** by using 7-bromo-1,2,3,4-tetrahydroisoquinoline instead of 4-bromoisoindoline hydrochloride. **Example 11** (5 mg) was obtained as a white powder. MS: calc'd 472 (MH⁺), measured 472 (MH⁺). ¹H NMR (400 MHz, METHANOL-d4) *δ* 8.05 (d, *J* = 2.3 Hz, 1H), 7.50 (d, *J* = 7.9 Hz, 1H), 7.23 (d, *J* = 8.6 Hz, 1H), 7.05 (dd, *J* = 2.5, 8.5 Hz, 1H), 6.92 (d, *J* = 2.4 Hz, 1H), 6.88 (s, 1H), 6.65 (d, *J* = 7.9 Hz, 1H), 4.71 - 4.36 (m, 3H), 4.15 - 4.03 (m, 1H), 3.82 (br t, *J =* 12.3 Hz, 3H), 3.60 - 3.35 (m, 11H), 3.27 - 3.09 (m, 2H), 2.83 - 2.71 (m, 2H), 1.34 (d, *J* = 6.2 Hz, 3H).

### Example 12

### 8-[(2R,6S)-2-methyl-6-[(5-piperazin-1-ylisoindolin-2-yl)methyl]morpholin-4-yl]quinoxaline-5-carbonitrile

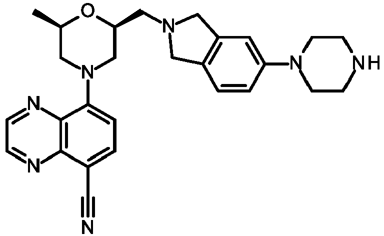

The title compound was prepared in analogy to the preparation of **Example 3** by using 5-bromoisoindoline hydrochloride and intermediate **C** instead of 4-bromoisoindoline hydrochloride and intermediate **A. Example 12** (17 mg) was obtained as a yellow powder. MS: calc'd 470 (MH⁺), measured 470 (MH⁺). ¹H NMR (400 MHz, METHANOL-d4) *δ* 8.98 (d, *J* = 1.8 Hz, 1H), 8.90 (d, *J* = 1.7 Hz, 1H), 8.16 (d, *J* = 8.3 Hz, 1H), 7.35 (d, *J* = 9.2 Hz, 1H), 7.27 (d, *J* = 8.4 Hz, 1H), 7.13 - 7.07 (m, 2H), 4.98 - 4.89 (m, 2H), 4.76 - 4.52 (m, 2H), 4.41 (br t, *J* = 10.1 Hz, 1H), 4.31 (br d, *J* = 11.9 Hz, 1H), 4.21 - 4.09 (m, 2H), 3.72 - 3.56 (m, 2H), 3.48 - 3.41 (m, 4H), 3.41 - 3.33 (m, 4H), 2.92 - 2.80 (m, 2H), 1.35 (d, *J =* 6.2 Hz, 3H).

### Example 13

### 8-[(2R,6S)-2-methyl-6-[(6-piperazin-1-yl-3,4-dihydro-1H-isoquinolin-2-yl)methyl]morpholin-4-yl]quinoxaline-5-carbonitrile

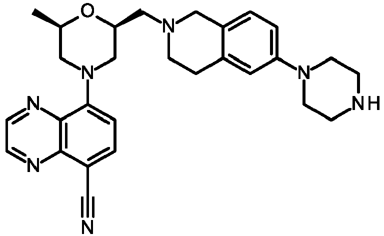

The title compound was prepared in analogy to the preparation of **Example 3** by using 6-bromo-1,2,3,4-tetrahydroisoquinoline and intermediate **C** instead of 4-bromoisoindoline hydrochloride and intermediate **A. Example 13** (14 mg) was obtained as a yellow powder. MS: calc'd 484 (MH⁺), measured 484 (MH⁺). ¹H NMR (400 MHz, METHANOL-d4) *δ* 8.98 (d, *J* = 1.8 Hz, 1H), 8.89 (d, *J* = 1.8 Hz, 1H), 8.16 (d, *J* = 8.3 Hz, 1H), 7.27 (d, *J* = 8.3 Hz, 1H), 7.17 (br d, *J =* 8.2 Hz, 1H), 7.01 (dd, *J* = 2.6, 8.6 Hz, 1H), 6.93 (s, 1H), 4.70 - 4.55 (m, 1H), 4.49 (br t, *J* = 10.0 Hz, 1H), 4.45 - 4.34 (m, 1H), 4.29 (br d, *J* = 12.1 Hz, 1H), 4.21 - 4.10 (m, 2H), 3.99 - 3.81 (m, 1H), 3.64 - 3.32 (m, 11H), 3.29 - 3.15 (m, 2H), 2.90 - 2.79 (m, 2H), 1.35 - 1.30 (m, 3H).

### Example 14

### 3-fluoro-4-[(2R,6S)-2-methyl-6-[(6-piperazin-1-yl-3,4-dihydro-1H-isoquinolin-2-yl)methyl]morpholin-4-yl]pyrazolo[1,5-a]pyridine-7-carbonitrile

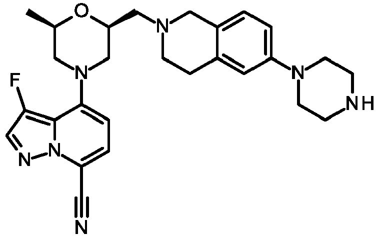

The title compound was prepared in analogy to the preparation of **Example 3** by using 6-bromo-1,2,3,4-tetrahydroisoquinoline and intermediate **B** instead of 4-bromoisoindoline hydrochloride and intermediate **A. Example 14** (18 mg) was obtained as a light yellow powder. MS: calc'd 490 (MH⁺), measured 490 (MH⁺). ¹H NMR (400 MHz, METHANOL-d4) *δ* 8.01 (d, *J* = 3.7 Hz, 1H), 7.44 (d, *J* = 7.8 Hz, 1H), 7.16 (br d, *J* = 8.6 Hz, 1H), 7.00 (dd, *J* = 2.4, 8.6 Hz, 1H), 6.92 (d, *J* = 2.2 Hz, 1H), 6.55 (d, *J* = 8.1 Hz, 1H), 4.66 - 4.47 (m, 1H), 4.47 - 4.32 (m, 2H), 4.12 - 4.01 (m, 1H), 3.97 - 3.78 (m, 1H), 3.60 (br t, *J* = 12.8 Hz, 2H), 3.51 - 3.34 (m, 11H), 3.29 - 3.14 (m, 2H), 2.77 (t, *J* = 11.2 Hz, 1H), 2.74 - 2.66 (m, 1H), 1.31 (d, *J* = 6.2 Hz, 3H).

### Example 15

### 4-[(2R,6S)-2-methyl-6-[[6-(4-methylpiperazin-1-yl)-3,4-dihydro-1H-isoquinolin-2-yl]methyl]morpholin-4-yl]pyrazolo[1,5-a]pyridine-7-carbonitrile

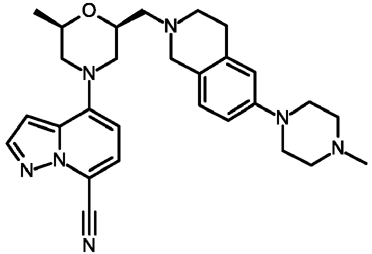

The title compound was prepared according to the following scheme:

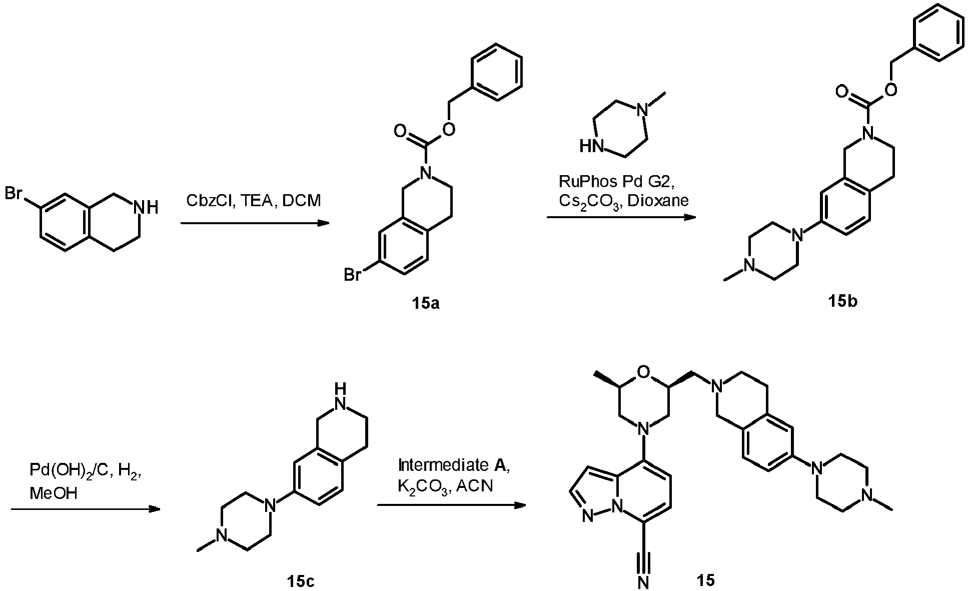

### Step 1: preparation of benzyl 7-bromo-3,4-dihydro-1H-isoquinoline-2-carboxylate (compound 15a)

To a solution of 6-bromo-1,2,3,4-tetrahydroisoquinoline (500 mg, 2.4 mmol) in DCM (10 mL) was added TEA (477 mg, 0.66 mL, 4.7 mmol). To the solution was added CbzCl (603 mg, 0.50 mL, 3.5 mmol) at 0 °C. The reaction mixture was stirred at rt for 3 hrs, then diluted with water (20 mL). The mixture was extracted with EtOAc (20 mL) twice. The combined organic layer was washed with brine, dried over Na₂SO₄ and concentrated to give a crude product which was purified by column chromatography to give compound **15a** (715 mg) as an oil. MS: calc'd 346&348 (MH⁺), measured 346&348 (MH⁺).

### Step 2: preparation of benzyl 7-(4-methylpiperazin-1-yl)-3,4-dihydro-1H-isoquinoline-2-carboxylate (compound 15b)

To a solution of benzyl 6-bromo-3,4-dihydroisoquinoline-2(1*H*)-carboxylate (compound **15a,** 173 mg, 0.50 mmol) in dioxane (5mL) was added 1-methylpiperazine (100 mg, 0.11 mL, 1.0 mmol) and Cs₂CO₃ (326 mg, 1.0 mmol). The suspension was bubbled with N₂ for 5 mins, then Ruphos Pd G2 (39 mg, 50 µmol) was added. The reaction mixture was heated at 100 °C (oil bath) for 12 hrs, then cooled to rt, diluted with EtOAc (10 mL), and filtered through celite. The filtrate was concentrated to give a crude product which was purified by column chromatography to give compound **15b** (142 mg) as a yellow oil. MS: calc'd 366 (MH⁺), measured 366 (MH⁺).

### Step 3: preparation of 7-(4-methylpiperazin-1-yl)-1,2,3,4-tetrahydroisoquinoline (compound 15c)

To a solution of benzyl 6-(4-methylpiperazin-1-yl)-3,4-dihydroisoquinoline-2(1*H*)-carboxylate (compound **15b,** 142 mg, 0.39 mmol) in MeOH (8 mL) was added Pd(OH)₂ on carbon (40 mg, 10% wet). The suspension was charged with hydrogen balloon and stirred at rt for 2 hrs. The mixture was then filtered through celite, the filtrate was concentrated to give compound **15c** (94 mg) as a dark solid which was directly used in next step. MS: calc'd 232 (MH⁺), measured 232 (MH⁺).

### Step 4: preparation of 4-[(2R,6S)-2-methyl-6-[[6-(4-methylpiperazin-1-yl)-3,4-dihydro-1H-isoquinolin-2-yl]methyl]morpholin-4-yl]pyrazolo[1,5-a]pyridine-7-carbonitrile

### (Example 15)

To a solution of ((2R,6R)-4-(7-cyanopyrazolo[1,5-a]pyridin-4-yl)-6-methylmorpholin-2-yl)methyl trifluoromethanesulfonate (Intermediate **A,** 25 mg, 62 µmol) in ACN (4 mL) was added K₂CO₃ (34 mg, 0.25 mmol) and 6-(4-methylpiperazin-1-yl)-1,2,3,4-tetrahydroisoquinoline (compound 15c, 21 mg, 93 µmol). The reaction mixture was refluxed for 3 hrs, then cooled to rt, diluted with ACN (10 mL), and filtered through celite, the filtrate was concentrated to give a crude product which was purified by prep-HPLC to give **Example 15** (15 mg) as a light yellow solid. MS: calc'd 486 (MH⁺), measured 486 (MH⁺). ¹H NMR (400 MHz, METHANOL-d4) *δ* 8.01 (d, *J* = 2.4 Hz, 1H), 7.46 (d, *J* = 7.9 Hz, 1H), 6.95 (d, *J* = 8.4 Hz, 1H), 6.87 (d, *J* = 2.4 Hz, 1H), 6.80 (dd, *J* = 2.5, 8.4 Hz, 1H), 6.73 (d, *J* = 2.2 Hz, 1H), 6.59 (d, *J* = 7.9 Hz, 1H), 4.14 - 4.05 (m, 1H), 3.99 - 3.90 (m, 1H), 3.84 (br d, *J* = 12.3 Hz, 1H), 3.76 (br d, *J* = 12.3 Hz, 1H), 3.73 - 3.62 (m, 2H), 3.18 - 3.11 (m, 4H), 2.97 - 2.87 (m, 3H), 2.83 - 2.64 (m, 5H), 2.63 - 2.58 (m, 4H), 2.34 (s, 3H), 1.27 (d, *J* = 6.2 Hz, 3H).

### Example 16 (reference)

### 4-[(2R,6S)-2-methyl-6-[[(4-piperazin-1-ylphenyl)methylamino]methyl] morpholin-4-yl]pyrazolo[1,5-a]pyridine-7-carbonitrile

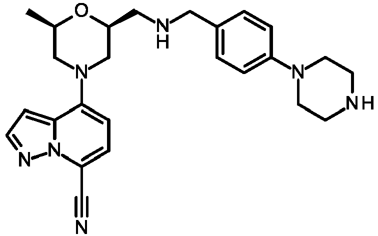

The title compound was prepared according to the following scheme:

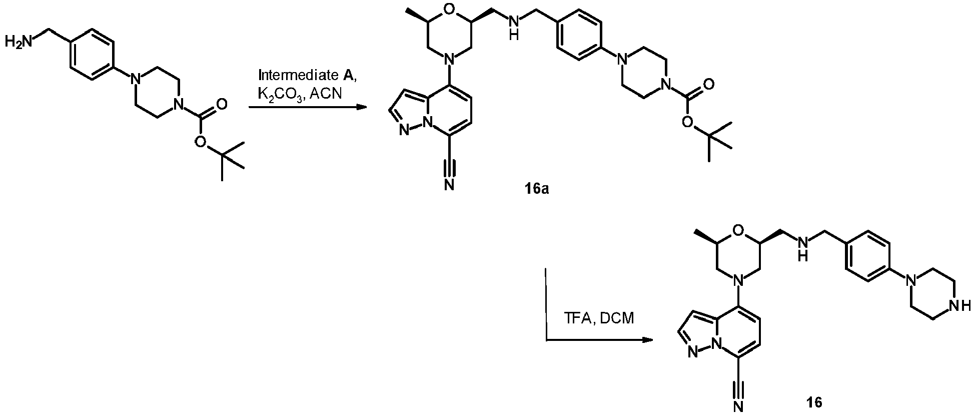

### Step 1: preparation of tert-butyl 4-[4-[[[(2S,6R)-4-(7-cyanopyrazolo[1,5-a]pyridin-4-yl)-6-methyl-morpholin-2-yl] methylamino] methyl] phenyl] piperazine-1-carboxylate (compound 16a)

To a solution of ((2*R*,6*R*)-4-(7-cyanopyrazolo[1,5-a]pyridin-4-yl)-6-methylmorpholin-2-yl)methyl trifluoromethanesulfonate (Intermediate **A,** 40 mg, 99 µmol) in ACN (4 mL) was added *tert*-butyl 4-(4-(aminomethyl)phenyl)piperazine-1-carboxylate (43 mg, 0.15 mmol) and K₂CO₃ (41 mg, 0.30 mmol). The reaction mixture was refluxed for 3h, then cooled to rt, diluted with ACN (10 mL), and filtered through celite, the filtrate was concentrated to give a yellow solid which was purified by column chromatography to give compound **16a** (52 mg) as a yellow solid. MS: calc'd 546 (MH⁺), measured 546 (MH⁺).

### Step 2: preparation of 4-[(2R,6S)-2-methyl-6-[[(4-piperazin-1-ylphenyl)methylamino]methyl]morpholin-4-yl]pyrazolo[1,5-a]pyridine-7-carbonitrile

### (Example 16)

To a solution of *tert*-butyl 4-[4-[[[(2*S*,6*R*)-4-(7-cyanopyrazolo[1,5-a]pyridin-4-yl)-6-methyl-morpholin-2-yl]methylamino]methyl]phenyl]piperazine-1-carboxylate (compound **16a,** 20 mg, 37 µmol) in DCM (2 mL) was added TFA (1 mL). The reaction mixture was stirred at rt for 2 hrs, then concentrated to give a crude product which was purified by prep-HPLC to give **Example 16** (5mg) as a light yellow powder. MS: calc'd 446 (MH⁺), measured 446 (MH⁺). ¹H NMR (400 MHz, METHANOL-d4) *δ* 8.09 - 8.01 (m, 1H), 7.47 (d, *J* = 8.1 Hz, 1H), 7.29 - 7.23 (m, 2H), 6.99 - 6.93 (m, 2H), 6.87 (d, *J* = 2.4 Hz, 1H), 6.58 (d, *J* = 7.9 Hz, 1H), 4.65 - 4.54 (m, 2H), 3.97 - 3.89 (m, 1H), 3.79 - 3.71 (m, 3H), 3.22 - 3.18 (m, 1H), 3.18 - 3.09 (m, 4H), 3.02 - 2.96 (m, 3H), 2.74 - 2.59 (m, 4H), 1.15 (d, *J* = 6.2 Hz, 1H).

### Example 17

### 4-[(2R,6S)-2-methyl-6-[[5-(4-methylpiperazin-1-yl)isoindolin-2-yl]methyl]morpholin-4-yl]pyrazolo[1,5-a]pyridine-7-carbonitrile

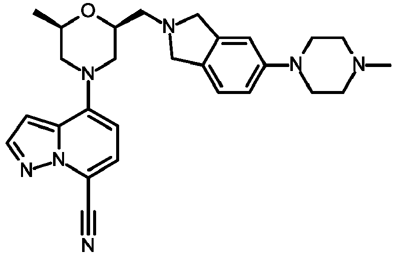

The title compound was prepared in analogy to the preparation of **Example 15** by using 6-bromo-1,2,3,4-tetrahydroisoquinoline instead of 5-bromoisoindoline hydrochloride. **Example 17** (7 mg) was obtained as a light yellow powder. MS: calc'd 472 (MH⁺), measured 472 (MH⁺). ¹H NMR (400 MHz, METHANOL-d4) *δ* 8.03 (d, *J* = 2.3 Hz, 1H), 7.48 (d, *J* = 7.9 Hz, 1H), 7.24 (d, *J* = 8.2 Hz, 1H), 7.03 - 6.95 (m, 2H), 6.90 (d, *J* = 2.4 Hz, 1H), 6.62 (d, *J* = 8.1 Hz, 1H), 4.50 - 4.34 (m, 4H), 4.25 - 4.14 (m, 1H), 4.06 - 3.95 (m, 1H), 3.88 - 3.75 (m, 2H), 3.42 - 3.32 (m, 6H), 3.12 - 3.03 (m, 4H), 2.81 - 2.66 (m, 2H), 2.70 (s, 3H), 1.32 (d, *J* = 6.2 Hz, 3H).

### Example 18

### 4-[(2R,6S)-2-methyl-6-[(3-piperazin-1-yl-5,7-dihydropyrrolo[3,4-b]pyridin-6-yl)methyl]morpholin-4-yl]pyrazolo[1,5-a]pyridine-7-carbonitrile

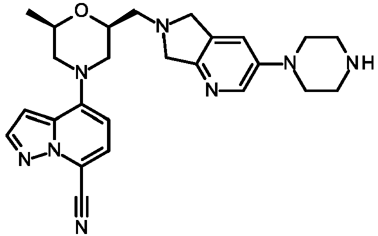

The title compound was prepared in analogy to the preparation of **Example 3** by using 3-bromo-6,7-dihydro-5*H*-pyrrolo[3,4-b]pyridine (CAS: 1394117-24-8, Vendor: Pharmablock) instead of 4-bromoisoindoline hydrochloride. **Example 18** (8 mg) was obtained as a light yellow powder. MS: calc'd 459 (MH⁺), measured 459 (MH⁺). ¹H NMR (400 MHz, METHANOL-d4) *δ* 8.12 (d, *J* = 2.6 Hz, 1H), 8.02 (d, *J* = 2.3 Hz, 1H), 7.46 (d, *J* = 7.9 Hz, 1H), 7.44 (d, *J* = 2.3 Hz, 1H), 6.88 (d, *J* = 2.4 Hz, 1H), 6.61 (d, *J* = 7.9 Hz, 1H), 4.20 - 4.02 (m, 5H), 4.01 - 3.91 (m, 1H), 3.84 (br d, *J* = 12.2 Hz, 1H), 3.78 (br d, *J* = 12.3 Hz, 1H), 3.49 - 3.41 (m, 4H), 3.41 - 3.35 (m, 4H), 3.05 - 3.00 (m, 2H), 2.77 (dd, *J* = 10.6, 12.2 Hz, 1H), 2.68 (dd, *J* = 10.5, 12.3 Hz, 1H), 1.29 (d, *J* = 6.2 Hz, 3H).

### Example 19

### 5-[(2R,6S)-2-methyl-6-[(6-piperazin-1-yl-3,4-dihydro-1H-isoquinolin-2-yl)methyl]morpholin-4-yl]quinoline-8-carbonitrile

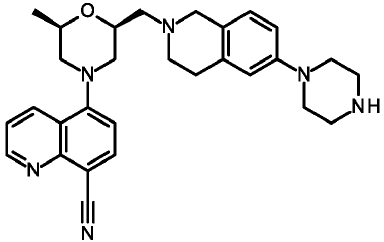

The title compound was prepared in analogy to the preparation of **Example 3** by using Intermediate **D** and 6-bromo-1,2,3,4-tetrahydroisoquinoline instead of Intermediate **A** and 4-bromoisoindoline hydrochloride. **Example 19** (20 mg) was obtained as a light yellow powder. MS: calc'd 483 (MH⁺), measured 483 (MH⁺). ¹H NMR (400 MHz, METHANOL-d4) *δ* 8.99 (dd, *J =* 1.5, 4.2 Hz, 1H), 8.67 (dd, *J* = 1.6, 8.6 Hz, 1H), 8.17 (d, *J =* 8.1 Hz, 1H), 7.65 (dd, *J =* 4.2, 8.6 Hz, 1H), 7.28 (d, *J* = 8.1 Hz, 1H), 7.18 (br d, *J* = 7.8 Hz, 1H), 7.01 (dd, *J* = 2.4, 8.6 Hz, 1H), 6.93 (d, *J* = 2.2 Hz, 1H), 4.67 - 4.50 (m, 2H), 4.49 - 4.33 (m, 1H), 4.28 - 4.16 (m, 1H), 4.00 - 3.80 (m, 1H), 3.52 - 3.35 (m, 13H), 3.28 - 3.15 (m, 2H), 2.84 - 2.69 (m, 2H), 1.32 (d, *J =* 6.4 Hz, 3H).

### Example 20

### 5-[(2R,6S)-2-methyl-6-[(3-piperazin-1-yl-5,7-dihydropyrrolo[3,4-b]pyridin-6-yl)methyl]morpholin-4-yl]quinoline-8-carbonitrile

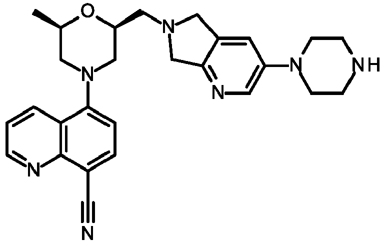

The title compound was prepared in analogy to the preparation of **Example 3** by using Intermediate **D** and 3-bromo-6,7-dihydro-5*H*-pyrrolo[3,4-b]pyridine (CAS: 1394117-24-8, Vendor: Pharmablock) instead of Intermediate **A** and 4-bromoisoindoline hydrochloride. **Example 20** (7.8 mg) was obtained as a light yellow powder. MS: calc'd 470 (MH⁺), measured 470 (MH⁺). ¹H NMR (400 MHz, METHANOL-d4) *δ* 8.99 (dd, *J* = 1.6, 4.2 Hz, 1H), 8.68 (dd, *J* = 1.5, 8.6 Hz, 1H), 8.34 (d, *J* = 2.3 Hz, 1H), 8.16 (d, *J =* 8.1 Hz, 1H), 7.65 (dd, *J* = 4.3, 8.6 Hz, 1H), 7.55 (d, *J* = 2.3 Hz, 1H), 7.29 (d, *J* = 8.1 Hz, 1H), 5.01 - 4.89 (m, 2H), 4.82 - 4.70 (m, 2H), 4.54 - 4.44 (m, 1H), 4.26 - 4.16 (m, 1H), 3.75 - 3.62 (m, 2H), 3.58 - 3.51 (m, 4H), 3.49 - 3.38 (m, 6H), 2.86 - 2.70 (m, 2H), 1.33 (d, *J* = 6.2 Hz, 3H).

### Example 21

### 5-[(2R,6S)-2-methyl-6-[(5-piperazin-1-ylisoindolin-2-yl)methyl]morpholin-4-yl]quinoline-8-carbonitrile

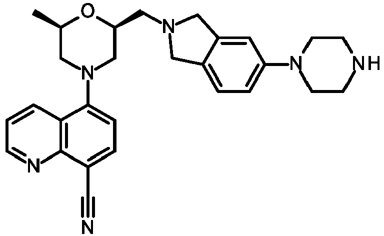

The title compound was prepared in analogy to the preparation of **Example 3** by using Intermediate **D** and 5-bromoisoindoline hydrochloride instead of Intermediate **A** and 4-bromoisoindoline hydrochloride. **Example 21** (7.8 mg) was obtained as a light yellow powder. MS: calc'd 469 (MH⁺), measured 469 (MH⁺). ¹H NMR (400 MHz, METHANOL-d4) *δ* 9.00 (dd, *J =* 1.6, 4.3 Hz, 1H), 8.68 (dd, *J =* 1.7, 8.6 Hz, 1H), 8.17 (d, *J* = 8.1 Hz, 1H), 7.66 (dd, *J* = 4.2, 8.6 Hz, 1H), 7.39 - 7.33 (m, 1H), 7.29 (d, *J* = 8.1 Hz, 1H), 7.10 (dd, *J* = 2.0, 4.3 Hz, 2H), 5.02 - 4.88 (m, 2H), 4.77 - 4.55 (m, 2H), 4.47 (br t, *J=* 9.9 Hz, 1H), 4.26 - 4.16 (m, 1H), 3.69 - 3.56 (m, 2H), 3.48 - 3.33 (m, 10H), 2.84 - 2.70 (m, 2H), 1.33 (d, *J =* 6.2 Hz, 3H).

### Example 22 (reference)

### 4-[(2R,6S)-2-methyl-6-[[methyl-[(4-piperazin-1-ylphenyl)methyl] amino] methyl] morpholin-4-yl]pyrazolo[1,5-a]pyridine-7-carbonitrile

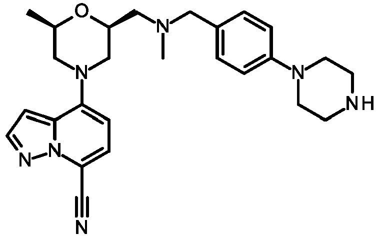

The title compound was prepared according to the following scheme:

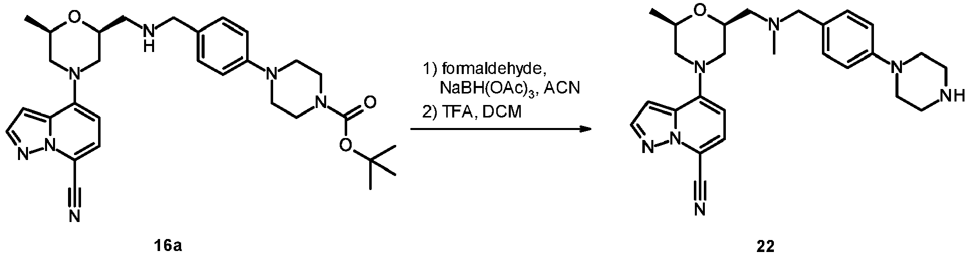

To a solution of tert-butyl 4-[4-[[[(2S,6R)-4-(7-cyanopyrazolo[1,5-a]pyridin-4-yl)-6-methyl-morpholin-2-yl]methylamino]methyl]phenyl]piperazine-1-carboxylate (compound **16a,** 592 mg, 1.1 mmol) in ACN (2 mL) was added formaldehyde (33 mg, 1.1 mmol) and NaBH(OAc)₃ (460 mg, 2.2 mmol). The suspension was stirred at rt for 2 hrs, then concentrated. The residue was dissolved in DCM (2 mL), then TFA (1 mL) was added. The reaction mixture was stirred at rt for 2 hrs, then concentrated to give a crude product which was purified by prep-HPLC to give **Example 22** (2 mg) as a light yellow solid. MS: calc'd 460 (MH⁺), measured 460 (MH⁺). ¹H NMR (400 MHz, METHANOL-d4) *δ* 8.06 (s, 1H), 7.53 - 7.43 (m, 3H), 7.15 (d, *J =* 8.7 Hz, 2H), 6.90 (d, *J* = 2.1 Hz, 1H), 6.63 (d, *J* = 7.9 Hz, 1H), 4.59 - 4.15 (m, 3H), 4.09 - 4.00 (m, 1H), 3.79 (br d, *J* = 11.7 Hz, 2H), 3.55 - 3.48 (m, 4H), 3.44 - 3.38 (m, 4H), 3.32 - 3.20 (m, 2H), 2.97-2.86 (m, 3H), 2.80 - 2.64 (m, 2H), 1.40 - 1.29 (m, 3H).

### Example 23

### 7-[(2R,6S)-2-methyl-6-[(5-piperazin-1-ylisoindolin-2-yl)methyl]morpholin-4-yl]-1,3-benzothiazole-4-carbonitrile

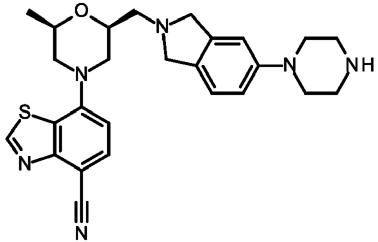

The title compound was prepared in analogy to the preparation of **Example 3** by using Intermediate **E** and 5-bromoisoindoline hydrochloride instead of Intermediate **A** and 4-bromoisoindoline hydrochloride. **Example 23** (16 mg) was obtained as a light yellow powder. MS: calc'd 475 (MH⁺), measured 475 (MH⁺). ¹H NMR (400 MHz, METHANOL-d4) *δ* 9.41 (s, 1H), 7.92 (d, *J* = 8.2 Hz, 1H), 7.37 - 7.32 (m, 1H), 7.17 (d, *J =* 8.3 Hz, 1H), 7.12 - 7.07 (m, 2H), 4.76 - 4.53 (m, 2H), 4.30 (br t, *J* = 10.0 Hz, 1H), 4.11 - 4.01 (m, 1H), 3.77 (br d, *J=* 12.3 Hz, 1H), 3.74 - 3.67 (m, 2H), 3.67 - 3.59 (m, 1H), 3.48 - 3.43 (m, 4H), 3.41 - 3.36 (m, 4H), 3.35 - 3.32 (m, 1H), 3.29 - 3.16 (m, 1H), 2.89 - 2.82 (m, 1H), 2.77 (dd, *J* = 10.5, 12.3 Hz, 1H), 1.35 (d, *J* = 6.2 Hz, 3H).

### Example 24

### 7-[(2R,6S)-2-methyl-6-[(6-piperazin-1-yl-3,4-dihydro-1H-isoquinolin-2-yl)methyl] morpholin-4-yl]-1,3-benzothiazole-4-carbonitrile

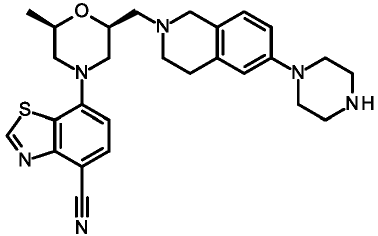

The title compound was prepared in analogy to the preparation of **Example 3** by using Intermediate **E** and 6-bromo-1,2,3,4-tetrahydroisoquinoline instead of Intermediate **A** and 4-bromoisoindoline hydrochloride. **Example 24** (16 mg) was obtained as a light yellow powder. MS: calc'd 489 (MH⁺), measured 489 (MH⁺). ¹H NMR (400 MHz, METHANOL-d4) *δ* 9.41 (s, 1H), 7.91 (d, *J* = 8.2 Hz, 1H), 7.21 - 7.12 (m, *J* = 8.2 Hz, 2H), 7.01 (dd, *J* = 2.4, 8.6 Hz, 1H), 6.93 (s, 1H), 4.69 - 4.53 (m, 1H), 4.47 - 4.33 (m, 2H), 4.13 - 4.03 (m, 1H), 3.98 - 3.82 (m, 1H), 3.77 (br d, *J* = 12.1 Hz, 1H), 3.70 (br d, *J* = 11.9 Hz, 1H), 3.60 - 3.46 (m, 3H), 3.46 - 3.33 (m, 9H), 3.23 - 3.16 (m, 1H), 2.89 - 2.81 (m, 1H), 2.79 - 2.71 (m, 1H), 1.34 (d, *J* = 6.2 Hz, 3H).

### Example 25

### 7-[(2R,6S)-2-methyl-6-[(3-piperazin-1-yl-5,7-dihydropyrrolo[3,4-b]pyridin-6-yl)methyl] morpholin-4-yl]-1,3-benzothiazole-4-carbonitrile

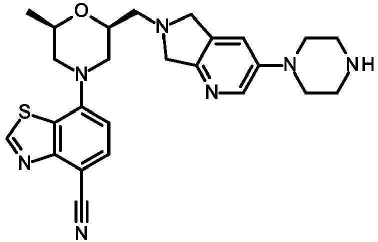

The title compound was prepared in analogy to the preparation of **Example 3** by using Intermediate **E** and 3-bromo-6,7-dihydro-5*H*-pyrrolo[3,4-b]pyridine instead of Intermediate **A** and 4-bromoisoindoline hydrochloride. **Example 25** (10 mg) was obtained as a light yellow powder. MS: calc'd 476 (MH⁺), measured 476 (MH⁺). ¹H NMR (400 MHz, METHANOL-d4) *δ* 9.41 (s, 1H), 8.34 (d, *J* = 2.6 Hz, 1H), 7.91 (d, *J =* 8.3 Hz, 1H), 7.55 (d, *J* = 2.4 Hz, 1H), 7.17 (d, *J* = 8.3 Hz, 1H), 4.75 (br s, 2H), 4.33 (br t, *J* = 10.0 Hz, 1H), 4.12 - 4.02 (m, 1H), 3.80 - 3.65 (m, 4H), 3.58 - 3.50 (m, 4H), 3.46 - 3.38 (m, 4H), 3.37 - 3.32 (m, 2H), 2.87 (t, *J* = 11.0 Hz, 1H), 2.77 (dd, *J* = 10.5, 12.2 Hz, 1H), 1.35 (d, *J* = 6.2 Hz, 3H).

### Example 26

### 4-[(2S,6R)-2-[[5-(2-aminoethylamino)isoindolin-2-yl]methyl]-6-methyl-morpholin-4-yl]pyrazolo[1,5-a]pyridine-7-carbonitrile

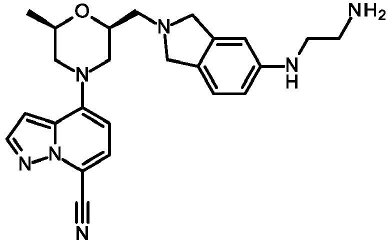

The title compound was prepared in analogy to the preparation of **Example 3** by using 5-bromoisoindoline hydrochloride and *tert*-butyl *N*-(2-aminoethyl)carbamate instead of 4-bromoisoindoline hydrochloride and tert-butyl piperazine-1-carboxylate. **Example 26** (1.7 mg) was obtained as a white powder. MS: calc'd 432 (MH⁺), measured 432 (MH⁺). ¹H NMR (400 MHz, METHANOL-d4) *δ* 8.05 (d, *J* = 2.3 Hz, 1H), 7.50 (d, *J* = 7.9 Hz, 1H), 7.20 (d, *J* = 8.1 Hz, 1H), 6.92 (d, *J* = 2.4 Hz, 1H), 6.76 - 6.70 (m, 2H), 6.65 (d, *J* = 8.1 Hz, 1H), 4.84 - 4.75 (m, 2H), 4.67 - 4.44 (m, 2H), 4.30 (br t, *J=* 10.0 Hz, 1H), 4.11 - 4.01 (m, 1H), 3.82 (br t, *J* = 13.1 Hz, 2H), 3.70 - 3.63 (m, 1H), 3.62 - 3.54 (m, 1H), 3.44 (t, *J* = 6.1 Hz, 2H), 3.15 (t, *J* = 6.0 Hz, 2H), 2.81 - 2.69 (m, 2H), 1.34 (d, *J* = 6.2 Hz, 3H).

### Example 27

### 4-[(2R,6S)-2-methyl-6-[[2-(4-methylpiperazin-1-yl)-6,7-dihydro-4H-thiazolo[5,4-c]pyridin-5-yl]methyl]morpholin-4-yl]pyrazolo[1,5-a]pyridine-7-carbonitrile

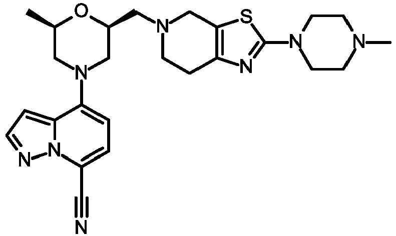

The title compound was prepared according to the following scheme:

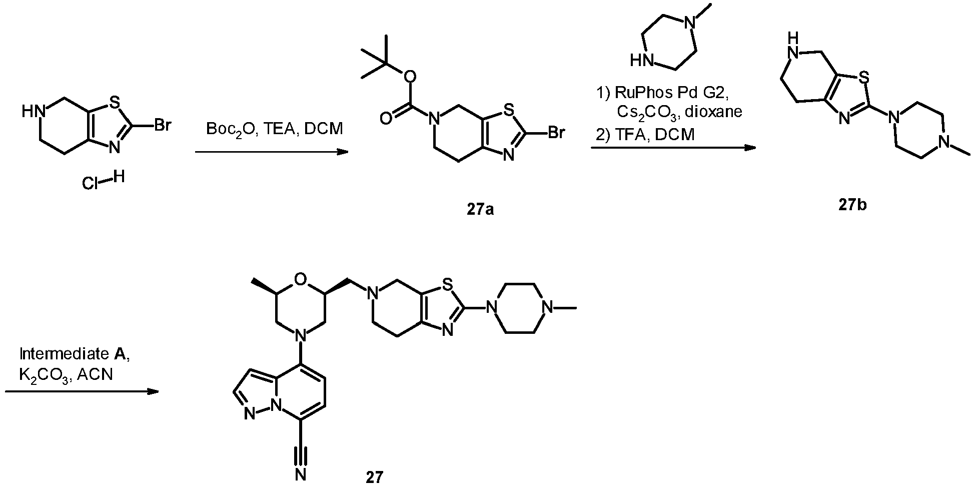

### Step 1: Preparation of tert-butyl 2-bromo-6,7-dihydro-4H-thiazolo[5,4-c]pyridine-5-carboxylate (compound 27a)

To a solution of 2-bromo-4,5,6,7-tetrahydrothiazolo[5,4-c]pyridine hydrochloride (CAS: 949922-52-5, vendor: Pharmablock, 130 mg, 0.51 mmol) in DCM (4 mL) was added TEA (206 mg, 0.28 mL, 2.0 mmol) and Boc₂O (167 mg, 0.18 mL, 0.76 mmol). The reaction mixture was stirred at rt for 2 hrs, then diluted with water (20 mL), and extracted with DCM (15 mL2) twice. The combined organic layer was washed with brine, dried over Na₂SO₄ and concentrated to give a brown oil which was purified by column chromatography to give compound 27a (131 mg) as an oil. MS: calc'd 319 and 321 (MH⁺), measured 319 and 321 (MH⁺).

### Step 2: preparation of 2-(4-methylpiperazin-1-yl)-4,5,6,7-tetrahydrothiazolo[5,4-c]pyridine (compound 27b)

To a solution of *tert*-butyl 2-bromo-6,7-dihydrothiazolo[5,4-c]pyridine-5(4*H*)-carboxylate (compound 21a, 131 mg, 0.41 mmol) in dioxane (5 mL) was added 1-methylpiperazine (82 mg, 91 µL, 0.82 mmol) and Cs₂CO₃ (267 mg, 0.82 mmol). The suspension was bubbled with N₂ for 5 min, then RuPhos Pd G2 (32 mg, 41 µmol) was added. The reaction mixture was heated at 100 °C for 18 hrs. The mixture was then diluted with EtOAc (10 mL), filtered through celite. The filtrate was concentrated, and the residue was purified by column chromatography to give a yellow solid (100 mg) which was dissolved in DCM (2 mL). To the solution was added TFA (1 mL). The reaction mixture was then stirred at rt for 2 hrs, then concentrated to give a crude compound **27b** (100 mg) which was directly used in next step. MS: calc'd 239 (MH⁺), measured 239 (MH⁺).

### Step 3: preparation of 4-[(2R,6S)-2-methyl-6-[[2-(4-methylpiperazin-1-yl)-6,7-dihydro-4H-thiazolo[5,4-c]pyridin-5-yl]methyl]morpholin-4-yl]pyrazolo[1,5-a]pyridine-7-carbonitrile (Example 27)

To a solution of ((2*R*,6*R*)-4-(7-cyanopyrazolo[1,5-a]pyridin-4-yl)-6-methylmorpholin-2-yl)methyl trifluoromethanesulfonate (Intermediate **A,** 25 mg, 62 µmol) in ACN (4 mL) was added K₂CO₃ (34 mg, 0.25 mmol) and 2-(4-methylpiperazin-1-yl)-4,5,6,7-tetrahydrothiazolo[5,4-c]pyridine (compound **27b,** 22 mg, 93 µmol). The suspension was heated under reflux for 2 hrs. The mixture was then cooled to rt, diluted with ACN (10 mL), and filtered through celite. The filtrated was concentrated to give a crude product which was purified by prep-HPLC to give **Example 27** (3.9 mg) as a light yellow solid. MS: calc'd 493 (MH⁺), measured 493 (MH⁺). ¹H NMR (400 MHz, METHANOL-d4) *δ* 8.06 (d, *J* = 2.3 Hz, 1H), 7.51 (d, *J* = 7.9 Hz, 1H), 6.92 (d, *J* = 2.3 Hz, 1H), 6.66 (d, *J* = 7.9 Hz, 1H), 4.61 - 4.46 (m, 2H), 4.39 (br t, *J* = 10.1 Hz, 1H), 4.13 - 4.05 (m, 1H), 4.05 - 3.65 (m, 6H), 3.64 - 3.35 (m, 6H), 3.13 - 3.02 (m, 2H), 2.99 (s, 3H), 2.82 - 2.70 (m, 2H), 1.34 (d, *J* = 6.2 Hz, 3H).

### Example 28

### 4-[(2S,6R)-2-[[5-[2-(dimethylamino)ethoxy]isoindolin-2-yl]methyl]-6-methyl-morpholin-4-yl]pyrazolo[1,5-a]pyridine-7-carbonitrile

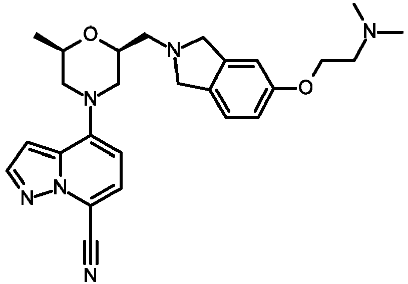

The title compound was prepared according to the following scheme:

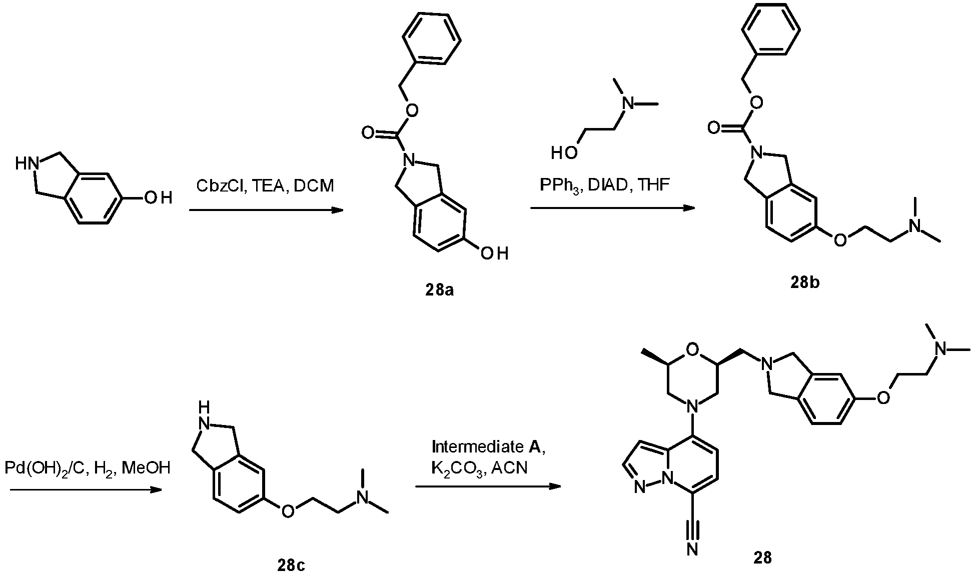

### Step 1: preparation of benzyl 5-hydroxyisoindoline-2-carboxylate (compound 28a)

To a solution of isoindolin-5-ol hydrobromide (216 mg, 1.0 mmol) in DCM (5 mL) was added TEA (405 mg, 0.56 mL, 4.0 mmol) and CbzCl (256 mg, 0.22 mL, 1.5 mmol) at 0 °C. The reaction mixture was stirred at rt for 2 hrs, then diluted with water (20 mL), and extracted with DCM (15 mL) twice. The combined organic layer was washed with brine, dried over Na₂SO₄ and concentrated to give a crude product which was purified by column chromatography to give compound **28a** (71 mg) as a brown solid. MS: calc'd 270 (MH⁺), measured 270 (MH⁺).

### Step 2: preparation of benzyl 5-[2-(dimethylamino)ethoxy]isoindoline-2-carboxylate (compound 28b)

To a solution of benzyl 5-hydroxyisoindoline-2-carboxylate (compound **28a,** 71 mg, 0.26 mmol) in THF (5 mL) was added 2-(dimethylamino)ethan-1-ol (23 mg, 26 µL, 0.26 mmol), triphenylphosphine (138 mg, 0.53 mmol) and DIAD (80 mg, 77 µL, 0.40 mmol). The reaction mixture was heated at 65 °C overnight, then cooled to rt, quenched with water, and extracted with DCM. The organic layer was washed with brine, dried over Na₂SO₄ and concentrated to give a crude product which was purified by column chromatography to afford compound **28b** (109 mg) as an oil. MS: calc'd 341 (MH⁺), measured 341 (MH⁺).

### Step 3: preparation of 2-isoindolin-5-yloxy-N,N-dimethyl-ethanamine (compound 28c)

To a solution of benzyl 5-(2-(dimethylamino)ethoxy)isoindoline-2-carboxylate (compound **28b,** 109 mg, 0.32 mmol) in MeOH (10 mL) was added Pd(OH)₂ on carbon (20 mg, 10% wet). The suspension was charged with hydrogen balloon at rt for 2 hrs, then filtered through celite, the filtrate was concentrated to give a crude compound **28c** (63 mg) which was directly used in next step. MS: calc'd 207 (MH⁺), measured 207 (MH⁺).

### Step 4: preparation of 4-[(2S,6R)-2-[[5-[2-(dimethylamino)ethoxy]isoindolin-2-yl]methyl]-6-methyl-morpholin-4-yl]pyrazolo[1,5-a]pyridine-7-carbonitrile (Example 28)

To a solution of ((2*R*,6*R*)-4-(7-cyanopyrazolo[1,5-a]pyridin-4-yl)-6-methylmorpholin-2-yl)methyl trifluoromethanesulfonate (intermediate **A,** 25 mg, 62 µmol) in ACN (4 mL) was added K₂CO₃ (34 mg, 0.25 mmol) and 2-isoindolin-5-yloxy-*N,N-*dimethyl-ethanamine (compound **28c,** 19 mg, 93 µmol). The suspension was heated under reflux for 2 hrs. The mixture was then cooled to rt, diluted with ACN (10 mL), and filtered through celite. The filtrated was concentrated to give a crude product which was purified by prep-HPLC to give **Example 28** (14.4 mg) as a light yellow solid. MS: calc'd 461 (MH⁺), measured 461 (MH⁺). ¹H NMR (400 MHz, METHANOL-d4) *δ* 8.06 (d, *J* = 2.4 Hz, 1H), 7.51 (d, *J* = 8.1 Hz, 1H), 7.40 (d, *J =* 8.3 Hz, 1H), 7.14 - 7.08 (m, 2H), 6.94 (d, *J* = 2.4 Hz, 1H), 6.67 (d, *J* = 8.1 Hz, 1H), 4.83 - 4.67 (m, 2H), 4.45 - 4.38 (m, 2H), 4.38 - 4.30 (m, 1H), 4.14 - 4.03 (m, 1H), 3.90 - 3.80 (m, 2H), 3.80 - 3.55 (m, 5H), 3.42 - 3.34 (m, 1H), 3.01 (s, 6H), 2.84 - 2.78 (m, 1H), 2.78 - 2.72 (m, 1H), 1.36 (d, *J* = 6.2 Hz, 3H).

### Example 29

### 3-fluoro-4-[(2R,6S)-2-methyl-6-[(3-piperazin-1-yl-5,7-dihydropyrrolo[3,4-b]pyridin-6-yl)methyl]morpholin-4-yl]pyrazolo[1,5-a]pyridine-7-carbonitrile

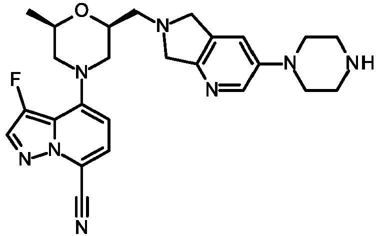

The title compound was prepared in analogy to the preparation of **Example 3** by using Intermediate **B** and 3-bromo-6,7-dihydro-5*H*-pyrrolo[3,4-b]pyridine instead of Intermediate **A** and 4-bromoisoindoline hydrochloride. **Example 29** (7.4 mg) was obtained as dark brown powder. MS: calc'd 477 (MH⁺), measured 477 (MH⁺). ¹H NMR (400 MHz, METHANOL-d4) *δ* 8.37 (d, *J* = 2.6 Hz, 1H), 8.04 (d, *J* = 3.7 Hz, 1H), 7.56 (d, *J* = 2.2 Hz, 1H), 7.47 (d, *J* = 7.8 Hz, 1H), 6.59 (d, *J* = 7.9 Hz, 1H), 4.78 - 4.69 (m, 2H), 4.36 - 4.29 (m, 1H), 4.12 - 4.03 (m, 1H), 3.73 - 3.61 (m, 4H), 3.58 - 3.51 (m, 5H), 3.47 - 3.39 (m, 5H), 2.85 - 2.71 (m, 2H), 1.35 (d, *J* = 6.2 Hz, 3H).

### Example 30

### 8-[(2R,6S)-2-methyl-6-[(3-piperazin-1-yl-5,7-dihydropyrrolo[3,4-b]pyridin-6-yl)methyl]morpholin-4-yl]quinoxaline-5-carbonitrile

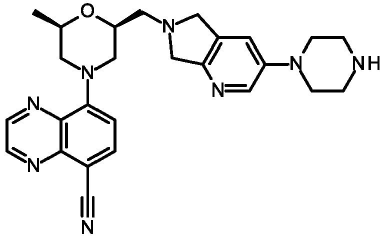

The title compound was prepared in analogy to the preparation of **Example 3** by using Intermediate **C** and 3-bromo-6,7-dihydro-5*H*-pyrrolo[3,4-b]pyridine instead of Intermediate **A** and 4-bromoisoindoline hydrochloride. **Example 30** (1.6 mg) was obtained as brown powder. MS: calc'd 471 (MH⁺), measured 471 (MH⁺). ¹H NMR (400 MHz, METHANOL-d4) *δ* 8.98 (d, *J =* 1.7 Hz, 1H), 8.90 (d, *J* = 1.8 Hz, 1H), 8.35 (d, *J* = 2.6 Hz, 1H), 8.17 (d, *J* = 8.3 Hz, 1H), 7.54 (d, *J* = 2.2 Hz, 1H), 7.28 (d, *J* = 8.4 Hz, 1H), 4.78 - 4.72 (m, 2H), 4.43 (br t, *J* = 10.0 Hz, 1H), 4.33 (br d, *J* = 11.9 Hz, 1H), 4.19 - 4.09 (m, 2H), 3.76 - 3.66 (m, 2H), 3.58 - 3.49 (m, 5H), 3.44 - 3.36 (m, 5H), 2.93 - 2.81 (m, 2H), 1.35 (d, *J* = 6.1 Hz, 3H).

### Example 31

### (2R,6S)-4-(8-chloro-5-quinolyl)-2-methyl-6-[(5-piperazin-1-ylisoindolin-2-yl)methyl]morpholine

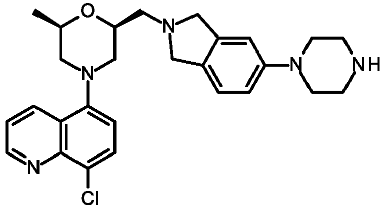

The title compound was prepared in analogy to the preparation of **Example 3** by using Intermediate **F** and 5-bromoisoindoline hydrochloride instead of Intermediate **A** and 4-bromoisoindoline hydrochloride. **Example 31** (2.2 mg) was obtained as a light brown powder. MS: calc'd 478 (MH⁺), measured 478 (MH⁺). ¹H NMR (400 MHz, METHANOL-d4) *δ* 8.96 (dd, *J* = 1.6, 4.3 Hz, 1H), 8.78 (dd, *J* = 1.6, 8.6 Hz, 1H), 7.87 (d, *J* = 8.2 Hz, 1H), 7.67 (dd, *J* = 4.3, 8.6 Hz, 1H), 7.38 - 7.33 (m, 1H), 7.27 (d, *J* = 8.2 Hz, 1H), 7.13 - 7.07 (m, 2H), 4.84 - 4.52 (m, 2H), 4.50 - 4.40 (m, 1H), 4.25 - 4.14 (m, 1H), 3.68 - 3.57 (m, 2H), 3.49 - 3.33 (m, 9H), 3.30 - 3.19 (m, 3H), 2.77 - 2.64 (m, 2H), 1.32 (d, *J* = 6.2 Hz, 3H).

### Example 32

### (2R,6S)-4-(8-chloro-5-quinolyl)-2-methyl-6-[(6-piperazin-1-yl-3,4-dihydro-1H-isoquinolin-2-yl)methyl]morpholine

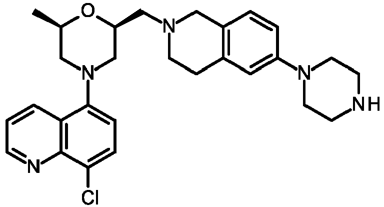

The title compound was prepared in analogy to the preparation of **Example 3** by using Intermediate **F** and 6-bromo-1,2,3,4-tetrahydroisoquinoline instead of Intermediate **A** and 4-bromoisoindoline hydrochloride. **Example 32** (21mg) was obtained as a yellow solid. MS: calc'd 492 (MH⁺), measured 492 (MH⁺). ¹H NMR (400 MHz, METHANOL-d4) *δ* 8.97 (dd, *J =* 1.6, 4.4 Hz, 1H), 8.81 (dd, *J=* 1.7, 8.5 Hz, 1H), 7.87 (d, *J* = 8.2 Hz, 1H), 7.69 (dd, *J* = 4.3, 8.6 Hz, 1H), 7.28 (d, *J* = 8.2 Hz, 1H), 7.17 (br d, *J* = 7.0 Hz, 1H), 7.00 (dd, *J* = 2.4, 8.5 Hz, 1H), 6.93 (d, *J =* 2.1 Hz, 1H), 4.75 - 4.49 (m, 2H), 4.49 - 4.31 (m, 1H), 4.24 - 4.15 (m, 1H), 4.01 - 3.81 (m, 1H), 3.55 - 3.33 (m, 11H), 3.30 - 3.14 (m, 4H), 2.77 - 2.63 (m, 2H), 1.31 (d, *J* = 6.4 Hz, 3H).

### Example 33

### (2R,6S)-4-(8-chloroquinoxalin-5-yl)-2-methyl-6-[(5-piperazin-1-ylisoindolin-2-yl)methyl]morpholine

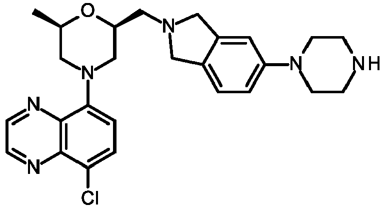

The title compound was prepared in analogy to the preparation of **Example 3** by using Intermediate **G** and 5-bromoisoindoline hydrochloride instead of Intermediate **A** and 4-bromoisoindoline hydrochloride. **Example 33** (2.6 mg) was obtained as a brown powder. MS: calc'd 479 (MH⁺), measured 479 (MH⁺). ¹H NMR (400 MHz, METHANOL-d4) *δ* 8.96 (d, *J =* 1.8 Hz, 1H), 8.91 (d, *J* = 1.8 Hz, 1H), 7.88 (d, *J* = 8.4 Hz, 1H), 7.40 - 7.32 (m, 1H), 7.27 (d, *J* = 8.6 Hz, 1H), 7.15 - 7.06 (m, 2H), 4.76 - 4.52 (m, 2H), 4.49 - 4.38 (m, 1H), 4.23 - 4.11 (m, 1H), 3.93 (br d, *J =* 11.5 Hz, 1H), 3.82 (br d, *J* = 11.7 Hz, 1H), 3.69 - 3.55 (m, 2H), 3.51 - 3.33 (m, 10H), 2.75 - 2.63 (m, 2H), 1.33 (d, *J* = 6.2 Hz, 3H).

### Example 34

### (2R,6S)-4-(8-chloroquinoxalin-5-yl)-2-methyl-6-[(6-piperazin-1-yl-3,4-dihydro-1H-isoquinolin-2-yl)methyl]morpholine

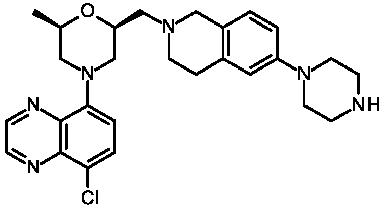

The title compound was prepared in analogy to the preparation of **Example 3** by using Intermediate **G** and 6-bromo-1,2,3,4-tetrahydroisoquinoline instead of Intermediate **A** and 4-bromoisoindoline hydrochloride. **Example 34** (9.9 mg) was obtained as a yellow solid. MS: calc'd 493 (MH⁺), measured 493 (MH⁺). ¹H NMR (400 MHz, METHANOL-d4) *δ* 8.97 (d, *J =* 1.8 Hz, 1H), 8.93 (d, *J* = 1.8 Hz, 1H), 7.89 (d, *J =* 8.4 Hz, 1H), 7.28 (d, *J* = 8.6 Hz, 1H), 7.19 (br s, 1H), 7.02 (dd, *J* = 2.5, 8.5 Hz, 1H), 6.95 (d, *J* = 2.1 Hz, 1H), 4.69 - 4.49 (m, 2H), 4.48 - 4.33 (m, 1H), 4.25 - 4.15 (m, 1H), 4.01 - 3.78 (m, 3H), 3.57 - 3.37 (m, 11H), 3.31 - 3.17 (m, 2H), 2.75 - 2.66 (m, 2H), 1.34 (d, *J* = 6.2 Hz, 3H).

### Example 35

### 8-[(2S,6R)-2-[[5-(4-cyclopropylpiperazin-1-yl)isoindolin-2-yl]methyl]-6-methyl-morpholin-4-yl]quinoxaline-5-carbonitrile

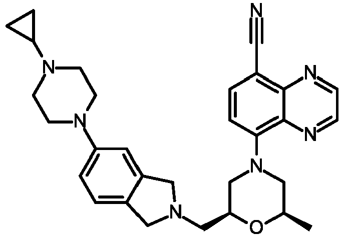

The title compound was prepared in analogy to the preparation of **Example 15** by using Intermediate **C,** 5-bromoisoindoline hydrochloride and 1-cyclopropylpiperazine instead of Intermediate **A,** 7-bromo-1,2,3,4-tetrahydroisoquinoline and 1-methylpiperazine. **Example 35** (16.9 mg) was obtained as a yellow solid. MS: calc'd 510 (MH⁺), measured 510 (MH⁺). ¹H NMR (400 MHz, METHANOL-d4) *δ* 8.96 (d, *J* = 1.8 Hz, 1H), 8.89 (d, *J* = 1.8 Hz, 1H), 8.13 (d, *J* = 8.3 Hz, 1H), 7.24 (d, *J* = 8.3 Hz, 2H), 7.03 - 6.95 (m, 2H), 4.66 - 4.49 (m, 4H), 4.39 - 4.25 (m, 2H), 4.21 - 4.05 (m, 2H), 3.53 - 3.39 (m, 2H), 3.25 - 3.16 (m, 4H), 2.91 - 2.77 (m, 6H), 1.87 - 1.76 (m, 1H), 1.33 (d, *J =* 6.0 Hz, 3H), 0.60 - 0.47 (m, 4H).

### Example 36

### 8-[(2R,6S)-2-methyl-6-[[5-(3-methylpiperazin-1-yl)isoindolin-2-yl]methyl]morpholin-4-yl]quinoxaline-5-carbonitrile

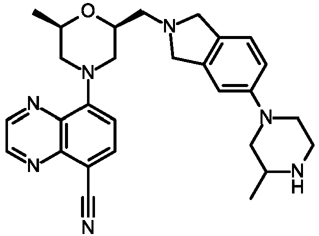

The title compound was prepared in analogy to the preparation of **Example 3** by using Intermediate **C,** 5-bromoisoindoline hydrochloride and *tert*-butyl 2-methylpiperazine-1-carboxylate instead of Intermediate **A,** 4-bromoisoindoline hydrochloride and *tert*-butyl piperazine-1-carboxylate. **Example 36** (21 mg) was obtained as a yellow solid. MS: calc'd 484 (MH⁺), measured 484 (MH⁺). ¹H NMR (400 MHz, METHANOL-d4) *δ* 8.95 (d, *J=* 1.5 Hz, 1H), 8.88 (d, *J* = 1.5 Hz, 1H), 8.12 (d, *J* = 8.3 Hz, 1H), 7.24 (t, *J* = 8.0 Hz, 2H), 7.04 - 6.97 (m, 2H), 4.46 - 4.25 (m, 6H), 4.17 (br d, *J* = 12.0 Hz, 1H), 4.12 - 4.01 (m, 1H), 3.76 (br t, *J* = 14.1 Hz, 2H), 3.51 - 3.42 (m, 2H), 3.30 - 3.21 (m, 3H), 3.03 (br t, *J* = 11.3 Hz, 1H), 2.88 - 2.74 (m, 3H), 1.39 (d, *J* = 6.5 Hz, 3H), 1.31 (d, *J* = 6.0 Hz, 3H).

### Example 37

### 8-[(2S,6R)-2-[[5-[4-(2-methoxyethyl)piperazin-1-yl]isoindolin-2-yl]methyl]-6-methyl-morpholin-4-yl]quinoxaline-5-carbonitrile

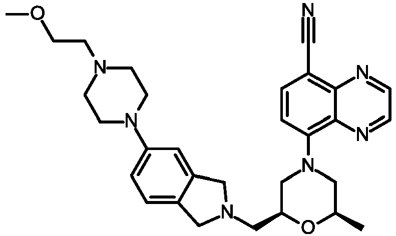

The title compound was prepared in analogy to the preparation of **Example 15** by using Intermediate **C,** 5-bromoisoindoline hydrochloride and 1-(2-methoxyethyl)piperazine instead of Intermediate **A,** 7-bromo-1,2,3,4-tetrahydroisoquinoline and 1-methylpiperazine. **Example 37** (3.8 mg) was obtained as a yellow gum. MS: calc'd 528 (MH⁺), measured 528 (MH⁺). ¹H NMR (400 MHz, METHANOL-d4) *δ* 8.97 (d, *J =* 1.8 Hz, 1H), 8.90 (d, *J =* 1.8 Hz, 1H), 8.15 (d, *J =* 8.3 Hz, 1H), 7.25 (dd, *J* = 2.8, 8.3 Hz, 2H), 7.03 - 6.95 (m, 2H), 4.54 - 4.37 (m, 4H), 4.31 (br d, *J =* 11.0 Hz, 2H), 4.21 - 4.08 (m, 2H), 3.67 (t, *J =* 5.3 Hz, 2H), 3.43 - 3.32 (m, 9H), 3.08 - 3.01 (m, 4H), 2.99 (t, *J* = 5.3 Hz, 2H), 2.88 - 2.77 (m, 2H), 1.32 (d, *J* = 6.3 Hz, 3H).

### Example 38

### 8-[(2S,6R)-2-[[5-(4,7-diazaspiro[2.5]octan-7-yl)isoindolin-2-yl]methyl]-6-methyl-morpholin-4-yl]quinoxaline-5-carbonitrile

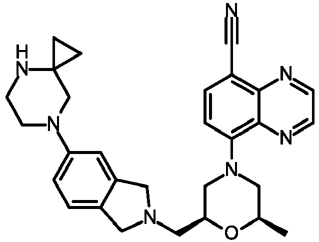

The title compound was prepared according to the following scheme:

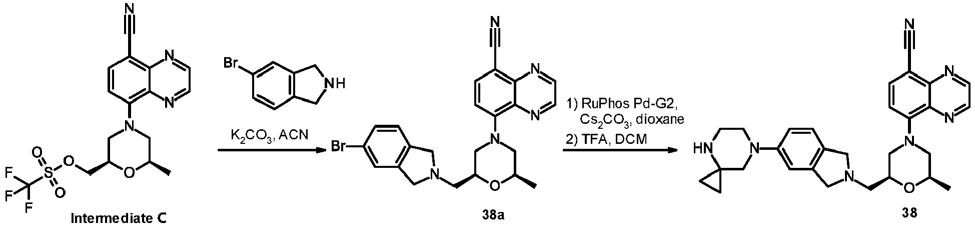

### Step 1: preparation of 8-[(2S,6R)-2-[(5-bromoisoindolin-2-yl)methyl]-6-methyl-morpholin-4-yl]quinoxaline-5-carbonitrile (compound 38a)

To the solution of [(2*R*,6*R*)-4-(8-cyanoquinoxalin-5-yl)-6-methyl-morpholin-2-yl]methyl trifluoromethanesulfonate (Intermediate **C,** 825 mg, 2.0 mmol) in ACN (8 mL) was added 5-bromoisoindoline hydrochloride (392 mg, 2.0 mmol) and K₂CO₃ (685 mg, 5.0 mmol). The mixture was stirred at 50 °C for 3 hrs, then diluted with EtOAc (100 mL) and filtered. The filtrate was concentrated to give a crude product which was purified by column chromatography to afford compound **38a** (450 mg) as a yellow solid. MS: calc'd 464 &466 (MH⁺), measured 464&466 (MH⁺).

### Step 2: preparation of 8-[(2S,6R)-2-[[5-(4,7-diazaspiro[2.5]octan-7-yl)isoindolin-2-yl]methyl]-6-methyl-morpholin-4-yl]quinoxaline-5-carbonitrile (Example 38)

To a solution of 8-[(2*S*,6*R*)-2-[(5-bromoisoindolin-2-yl)methyl]-6-methyl-morpholin-4-yl]quinoxaline-5-carbonitrile (compound **38a,** 60 mg, 0.13 mmol) and *tert*-butyl 4,7-diazaspiro[2.5]octane-4-carboxylate (27 mg, 0.13 mmol) in 1,4-dioxane (5 mL) was added RuPhos Pd G2 (10 mg, 0.010 mmol) and Cs₂CO₃ (105 mg, 0.32 mmol). The mixture was stirred at 90 °C for 12 hrs under N₂. The reaction mixture was then concentrated, the residue was dissolved in EtOAc (100 mL). The mixture was washed with water and brine, dried over Na₂SO₄ and concentrated to give a crude product which was purified by prep-TLC (EA/PE, v/v = 1/3, Rf = 0.25) to give the intermediate (15 mg) as a yellow gum which was dissolved in DCM (2 mL). To the solution was added TFA (1 mL). The reaction mixture was stirred at rt for 1h, then concentrated. The residue was purified by prep-HPLC to give **Example 5** (8.9 mg) as a yellow solid. MS: calc'd 496 (MH⁺), measured 496 (MH⁺). ¹H NMR (400 MHz, METHANOL-d4) *δ* 8.96 (d, *J* = 1.5 Hz, 1H), 8.89 (d, *J =* 1.5 Hz, 1H), 8.14 (d, *J* = 8.3 Hz, 1H), 7.24 (dd, *J* = 4.9, 8.2 Hz, 2H), 7.01 - 6.91 (m, 2H), 4.49 - 4.35 (m, 4H), 4.30 (br d, *J* = 10.3 Hz, 2H), 4.20 - 4.05 (m, 2H), 3.36 (br d, *J* = 6.0 Hz, 2H), 3.31 - 3.24 (m, 4H), 3.19 (s, 2H), 2.88 - 2.74 (m, 2H), 1.31 (d, *J* = 6.3 Hz, 3H), 0.98 - 0.85 (m, 4H).

### Example 39

### 8-[(2R,6S)-2-methyl-6-[[5-(2-oxopiperazin-1-yl)isoindolin-2-yl]methyl]morpholin-4-yl]quinoxaline-5-carbonitrile

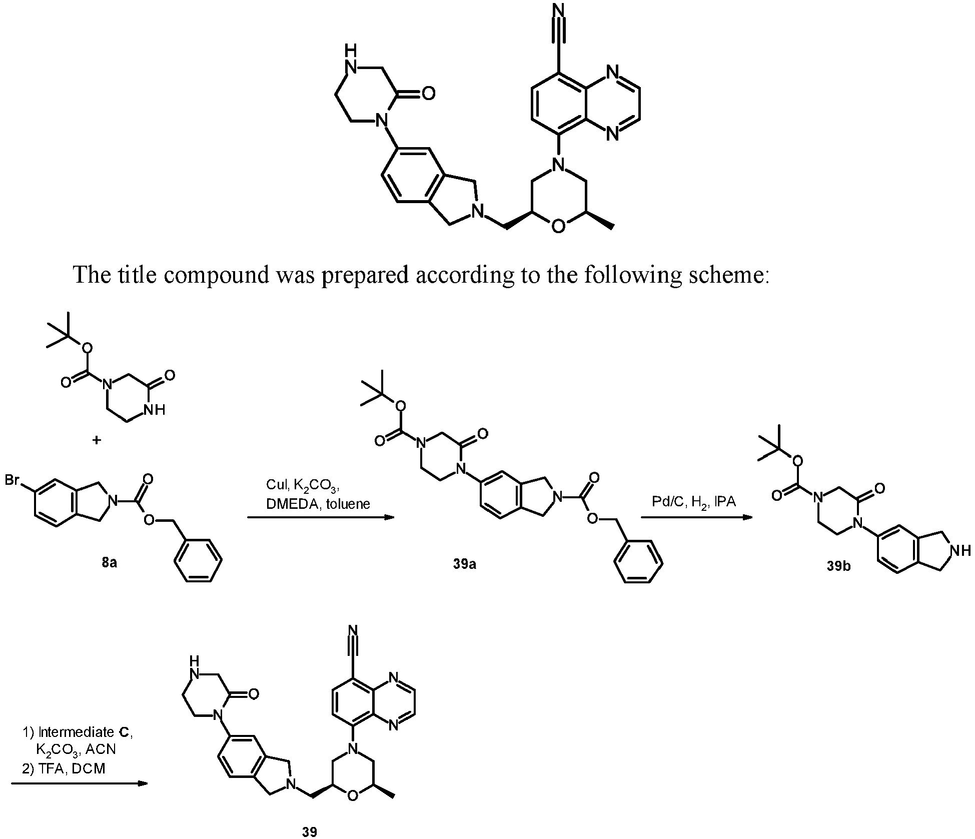

### Step 1: preparation of benzyl 5-(4-tert-butoxycarbonyl-2-oxo-piperazin-1-yl)isoindoline-2-carboxylate (compound 39a)

To a solution of benzyl 5-bromoisoindoline-2-carboxylate (compound **8a,** 100 mg, 0.30 mmol) and tert-butyl 3-oxopiperazine-1-carboxylate (181 mg, 0.90 mmol) in toluene (3 mL) was added CuI (29 mg, 0.15 mmol), K₂CO₃ (159 mg, 1.5 mmol) and DMEDA (2.6 mg, 0.030 mmol). The mixture was degassed with N₂, and stirred at 150 °C for 4 hrs under N₂. The solvent was then removed to give a crude product which was purified by prep-TLC (PE/EA, v/v = 1/1) to give compound **39a** (150 mg) as a white solid. MS: calc'd 452 (MH⁺), measured 452 (MH⁺).

### Step 2: preparation of tert-butyl 4-isoindolin-5-yl-3-oxo-piperazine-1-carboxylate (compound 39b)

To a solution of benzyl 5-(4-tert-butoxycarbonyl-2-oxo-piperazin-1-yl)isoindoline-2-carboxylate (compound **39a,** 150 mg, 0.33 mmol) in IPA (5 mL) was added Pd on carbon (20 mg, 10%). The reaction was stirred at rt for 18 hrs under H₂. The mixture was then filtered, the filtrate was concentrated to give compound **39b** (50 mg) as a yellow gum. MS: calc'd 318 (MH⁺), measured 318 (MH⁺).

### Step 3: preparation of 8-[(2R,6S)-2-methyl-6-[[5-(2-oxopiperazin-1-yl)isoindolin-2-yl]methyl]morpholin-4-yl]quinoxaline-5-carbonitrile (Example 39)

To a solution of *tert*-butyl 4-isoindolin-5-yl-3-oxo-piperazine-1-carboxylate (compound **39b,** 37 mg, 0.12 mmol) in ACN (6 mL) was added K₂CO₃ (33 mg, 0.24 mmol) and [(2*R*,6*R*)-4-(8-cyanoquinoxalin-5-yl)-6-methyl-morpholin-2-yl]methyl trifluoromethanesulfonate (Intermediate **C,** 40 mg, 0.10 mmol). The mixture was stirred at 55 °C for 3 hrs. The mixture was then diluted with EtOAc (10 mL) and filtered. The filtrate was concentrated. The residue was purified by prep-TLC (EA/PE, v/v = 2/1) to afford the intermediate (40 mg) as yellow gum which was dissolved in DCM (2 mL). To the solution was added TFA (1 mL). The reaction mixture was stirred at rt for 1h, then solvent was removed. The residue was purified by prep-HPLC to give **Example 39** (4 mg) as a yellow gum. MS: calc'd 484 (MH⁺), measured 484 (MH⁺). ¹H NMR (400 MHz, METHANOL-d4) *δ* 9.00 (d, *J* = 1.6 Hz, 1H), 8.92 (d, *J* = 1.6 Hz, 1H), 8.18 (d, *J=* 8.4 Hz, 1H), 7.57 - 7.55 (m, 1H), 7.47 (s, 1H), 7.45 - 7.43 (m, 1H), 7.30 (d, *J* = 8.4 Hz, 1H), 4.95 - 4.88 (m, 4H), 4.47 - 4.44 (m, 1H), 4.36 - 4.33 (m, 1H), 4.19 - 4.15 (m, 2H), 4.04 (s, 2H), 4.01 - 3.98 (m, 2H), 3.72 - 3.66 (m, 4H), 2.93 - 2.84 (m, 2H), 1.37 (d, *J* = 6 Hz, 3H).

### Example 40

### 8-[(2S,6R)-2-[[5-(1,3,4,6,7,8,9,9a-octahydropyrido[1,2-a]pyrazin-2-yl)isoindolin-2-yl]methyl]-6-methyl-morpholin-4-yl]quinoxaline-5-carbonitrile

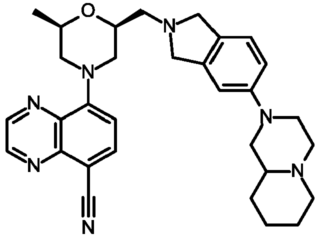

The title compound was prepared according to the following scheme:

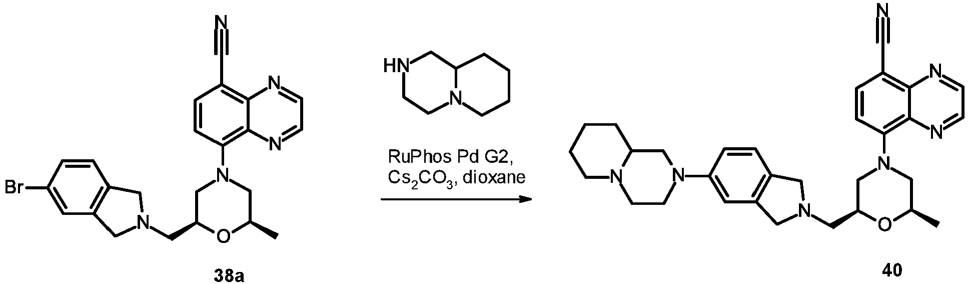

To a solution of 8-[(2*S*,6*R*)-2-[(5-bromoisoindolin-2-yl)methyl]-6-methyl-morpholin-4-yl]quinoxaline-5-carbonitrile (compound **38a,** 50 mg, 0.11 mmol) in dioxane (4 mL) was added Cs₂CO₃ (107 mg, 0.33 mmol), RuPhos Pd G2 (7.7 mg, 0.01 mmol) and 2,3,4,6,7,8,9,9a-octahydro-1*H*-pyrido[1,2-a]pyrazine (22 mg, 0.16 mmol). The mixture was degassed and stirred at 80 °C for 18 hrs under N₂. The mixture was then concentrated, the residue was dissolved in EtOAc (20 mL), washed with brine, dried over Na₂SO₄ and concentrated to give a crude product which was purified by prep-HPLC to give **Example 40** (7 mg) as a yellow solid. MS: calc'd 524 (MH⁺), measured 524 (MH⁺). ¹H NMR (400 MHz, METHANOL-d4) *δ* 8.97 (d, *J=* 1.5 Hz, 1H), 8.91 (d, *J* = 1.5 Hz, 1H), 8.15 (d, *J =* 8.3 Hz, 1H), 7.24 (dd, *J* = 8.3, 12.6 Hz, 2H), 7.02 - 6.93 (m, 2H), 4.38 - 4.05 (m, 8H), 3.74 - 3.59 (m, 2H), 3.27 - 2.48 (m, 11H), 1.93 - 1.67 (m, 4H), 1.58 - 1.38 (m, 2H), 1.33 (d, *J* = 6.3 Hz, 3H).

### Example 41

### 8-[(2S,6R)-2-[[5-(3,8-diazabicyclo[3.2.1]octan-8-yl)isoindolin-2-yl]methyl]-6-methyl-morpholin-4-yl]quinoxaline-5-carbonitrile

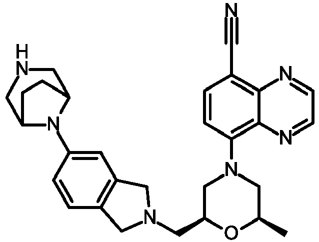

The title compound was prepared in analogy to the preparation of **Example 38** by using *tert*-butyl 3,8-diazabicyclo[3.2.1]octane-3-carboxylate instead of *tert*-butyl 4,7-diazaspiro[2.5]octane-4-carboxylate. **Example 41** (3.9 mg) was obtained as a yellow solid. MS: calc'd 496 (MH⁺), measured 496 (MH⁺). ¹H NMR (400 MHz, METHANOL-d4) *δ* 9.00 (d, *J =* 1.6 Hz, 1H), 8.92 (d, *J* = 1.6 Hz, 1H), 8.19 (d, *J* = 8.3 Hz, 1H), 7.36 (d, *J* = 9.0 Hz, 1H), 7.30 (d, *J* = 8.3 Hz, 1H), 6.98 - 7.04 (m, 2H), 4.56 - 4.75 (m, 2H), 4.55 - 4.45 (m, 2H), 4.43 (br t, *J* = 9.8 Hz, 1H), 4.33 (br d, *J=* 12.3 Hz, 1H), 4.11 - 4.23 (m, 2H), 3.58 - 3.74 (m, 2H), 3.40 - 3.30 (m, 4H), 3.16 (br d, *J* = 12.8 Hz, 2H), 2.81 - 2.95 (m, 2H), 2.24 - 2.35 (m, 2H), 2.05 - 2.14 (m, 2H), 1.37 (d, *J* = 6.1 Hz, 3H).

### Example 42

### 5-[(2S,6R)-2-[[5-(3,8-diazabicyclo[3.2.1]octan-3-yl)isoindolin-2-yl]methyl]-6-methyl-morpholin-4-yl]quinoline-8-carbonitrile

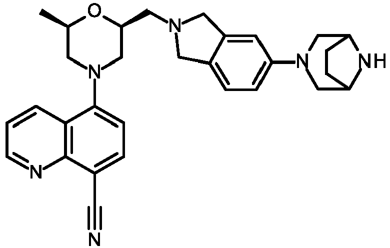

The title compound was prepared in analogy to the preparation of **Example 3** by using Intermediate **D,** 5-bromoisoindoline hydrochloride and *tert*-butyl 3,8-diazabicyclo[3.2.1]octane-8-carboxylate instead of Intermediate **A,** 4-bromoisoindoline hydrochloride and *tert*-butyl piperazine-1-carboxylate. **Example 42** (25mg) was obtained as a light yellow powder. MS: calc'd 495 (MH⁺), measured 495 (MH⁺). ¹H NMR (400 MHz, METHANOL-d4) *δ* 9.02 (dd, *J* = 1.6, 4.2 Hz, 1H), 8.70 (dd, *J* = 1.7, 8.6 Hz, 1H), 8.20 (d, *J* = 8.1 Hz, 1H), 7.68 (dd, *J* = 4.3, 8.6 Hz, 1H), 7.37 - 7.30 (m, 2H), 7.05 (br d, *J* = 5.1 Hz, 2H), 5.06 - 4.93 (m, 2H), 4.80 - 4.52 (m, 2H), 4.52 - 4.42 (m, 1H), 4.27 - 4.18 (m, 3H), 3.77 (br d, *J* = 10.9 Hz, 2H), 3.69 - 3.58 (m, 2H), 3.50 - 3.44 (m, 2H), 3.16 (br d, *J* = 11.6 Hz, 2H), 2.86 - 2.73 (m, 2H), 2.20 - 2.11 (m, 4H), 1.35 (d, *J* = 6.2 Hz, 3H).

### Example 43

### 5-[(2S,6R)-2-[[5-(3,8-diazabicyclo[3.2.1]octan-8-yl)isoindolin-2-yl]methyl]-6-methyl-morpholin-4-yl]quinoline-8-carbonitrile

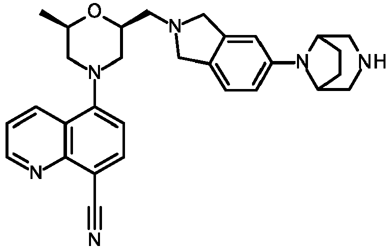

The title compound was prepared in analogy to the preparation of **Example 3** by using Intermediate **D,** 5-bromoisoindoline hydrochloride and *tert*-butyl 3,8-diazabicyclo[3.2.1]octane-3-carboxylate instead of Intermediate **A,** 4-bromoisoindoline hydrochloride and *tert*-butyl piperazine-1-carboxylate. **Example 43** (24mg) was obtained as a light brown powder. MS: calc'd 495 (MH⁺), measured 495 (MH⁺). ¹H NMR (400 MHz, METHANOL-d4) *δ* 9.00 (dd, *J* = 1.6, 4.3 Hz, 1H), 8.68 (dd, *J* = 1.6, 8.7 Hz, 1H), 8.18 (d, *J=* 7.9 Hz, 1H), 7.66 (dd, *J* = 4.3, 8.6 Hz, 1H), 7.34 (br d, *J* = 8.9 Hz, 1H), 7.30 (d, *J* = 8.1 Hz, 1H), 7.04 - 6.97 (m, 2H), 5.04 - 4.90 (m, 2H), 4.80 - 4.55 (m, 2H), 4.53 - 4.43 (m, 3H), 4.27 - 4.16 (m, 1H), 3.69 - 3.57 (m, 2H), 3.46 (br d, *J=* 11.7 Hz, 2H), 3.37 - 3.32 (m, 2H), 3.14 (br d, *J=* 12.2 Hz, 2H), 2.86 - 2.71 (m, 2H), 2.34 - 2.23 (m, 2H), 2.13 - 2.03 (m, 2H), 1.34 (d, *J* = 6.4 Hz, 3H).

### Example 44

### 8-[(2S,6R)-2-[[5-(3,8-diazabicyclo[3.2.1]octan-3-yl)isoindolin-2-yl]methyl]-6-methyl-morpholin-4-yl]quinoxaline-5-carbonitrile

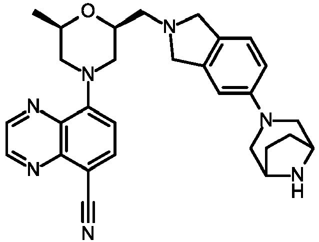

The title compound was prepared in analogy to the preparation of **Example 38** by using *tert*-butyl 3,8-diazabicyclo[3.2.1]octane-8-carboxylate instead of *tert*-butyl 4,7-diazaspiro[2.5]octane-4-carboxylate. **Example 44** (3.9 mg) was obtained as a yellow solid. MS: calc'd 496 (MH⁺), measured 496 (MH⁺). ¹H NMR (400 MHz, METHANOL-d4) *δ* 9.00 (d, *J =* 1.6 Hz, 1H), 8.92 (d, *J* = 1.8 Hz, 1H), 8.19 (d, *J=* 8.3 Hz, 1H), 7.36 - 7.28 (m, 2H), 7.04 (br s, 2H), 4.42 (br t, *J* = 9.8 Hz, 1H), 4.33 (br d, *J =* 11.7 Hz, 1H), 4.24 - 4.13 (m, 4H), 3.80 - 3.57 (m, 4H), 3.39 - 3.30 (m, 4H), 3.16 (br d, *J* = 12.0 Hz, 2H), 2.92 - 2.81 (m, 2H), 2.20 - 2.19 (m, 4H), 1.36 (d, *J* = 6.1 Hz, 3H).

### Example 45

### 4-[(2R,6S)-2-methyl-6-[[5-(1-methylimidazol-4-yl)isoindolin-2-yl]methyl]morpholin-4-yl]pyrazolo[1,5-a]pyridine-7-carbonitrile

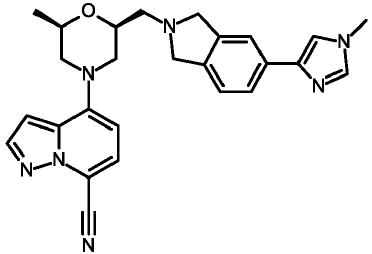

The title compound was prepared according to the following scheme:

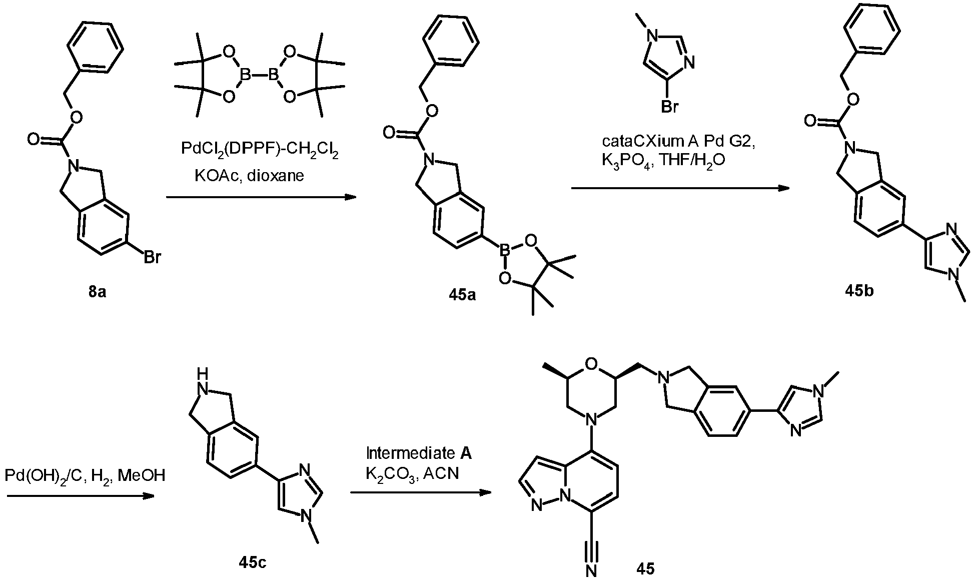

### Step 1: preparation of benzyl 5-(4,4,5,5-tetramethyl-1,3,2-dioxaborolan-2-yl)isoindoline-2-carboxylate (compound 45a)

To a solution of 4,4,4',4',5,5,5',5'-octamethyl-2,2'-bi(1,3,2-dioxaborolane) (459 mg, 1.8 mmol) in dioxane (4 mL) was added benzyl 5-bromoisoindoline-2-carboxylate (compound **8a,** 400 mg, 1.2 mmol), and KOAc (118 mg, 1.2 mmol). The solution was bubbled with N₂ for 5 mins, then PdCl₂(DPPF)-CH₂Cl₂ adduct (881 mg, 1.2 mmol) was added. The mixture was heated at 100 °C for 3 hrs, then cooled to rt and concentrated to give a dark oil which was purified by column chromatography to give compound **45a** (469 mg) as an oil. MS: calc'd 380 (MH⁺), measured 380 (MH⁺).

### Step 2: preparation of benzyl 5-(1-methylimidazol-4-yl)isoindoline-2-carboxylate (compound 45b)

To a solution of benzyl 5-(4,4,5,5-tetramethyl-1,3,2-dioxaborolan-2-yl)isoindoline-2-carboxylate (compound **45a,** 76 mg, 0.20 mmol) in THF/H₂O (4.5 mL, v/v = 8/1) was added 4-bromo-1-methyl-1*H*-imidazole (32 mg, 0.20 mmol), and K₃PO₄ (85 mg, 0.40 mmol). The solution was bubbled with N₂ for 5 mins, then cataCXium A Pd G2 (134 mg, 0.20 mmol) was added. The mixture was heated at 75 °C overnight, then cooled to rt and concentrated to give a crude product which was purified by column chromatography to give compound **45b** (43 mg) as an oil. MS: calc'd 334 (MH⁺), measured 334 (MH⁺).

### Step 2: preparation of 5-(1-methylimidazol-4-yl)isoindoline (compound 45c)

To a solution of benzyl 5-(1-methyl-1*H*-imidazol-4-yl)isoindoline-2-carboxylate (compound **45b,** 43 mg, 0.13 mmol) in MeOH (10 mL) was added Pd(OH)₂ on carbon (10 mg, 20% wet). The suspension was charged with hydrogen balloon and stirred at rt for 2 hrs, then filtered through celite. The filtrate was concentrated to give crude compound **45c** (27 mg) which was directly used in next step.

### Step 3: preparation of 4-[(2R,6S)-2-methyl-6-[[5-(1-methylimidazol-4-yl)isoindolin-2-yl]methyl]morpholin-4-yl]pyrazolo[1,5-a]pyridine-7-carbonitrile (Example 45)

To a solution of ((2*R*,6*R*)-4-(7-cyanopyrazolo[1,5-a]pyridin-4-yl)-6-methylmorpholin-2-yl)methyl trifluoromethanesulfonate (Intermediate **A,** 29 mg, 72 µmol) in ACN (4 mL) was added K₂CO₃ (34 mg, 0.25 mmol) and 5-(1-methyl-1*H*-imidazol-4-yl)isoindoline (compound **45c,** 21 mg, 0.11 mmol). The suspension was heated under reflux for 2 hrs. The mixture was then cooled to rt, diluted with ACN (10 mL), and filtered through celite. The filtrated was concentrated to give a crude product which was purified by prep-HPLC to give **Example 45** (21 mg) as a light yellow solid. MS: calc'd 454 (MH⁺), measured 454 (MH⁺). ¹H NMR (400 MHz, METHANOL-d4) *δ* 8.83 (s, 1H), 8.05 (d, *J* = 2.4 Hz, 1H), 7.93 (d, *J* = 1.2 Hz, 1H), 7.82 - 7.75 (m, 2H), 7.59 (d, *J* = 8.6 Hz, 1H), 7.50 (d, *J* = 7.9 Hz, 1H), 6.93 (d, *J* = 2.4 Hz, 1H), 6.66 (d, *J =* 8.1 Hz, 1H), 5.10 - 4.90 (m, 4H), 4.35 (br t, *J=* 10.0 Hz, 1H), 4.13 - 4.03 (m, 1H), 3.97 (s, 3H), 3.86 (br d, *J* = 12.2 Hz, 1H), 3.81 (br d, *J* = 12.3 Hz, 1H), 3.76 - 3.61 (m, 2H), 2.84 - 2.71 (m, 2H), 1.35 (d, *J* = 6.2 Hz, 3H).

### Example 46

### 5-[(2R,6S)-2-methyl-6-[[5-(3-oxa-7,9-diazabicyclo[3.3.1]nonan-9-yl)isoindolin-2-yl]methyl]morpholin-4-yl]quinoline-8-carbonitrile

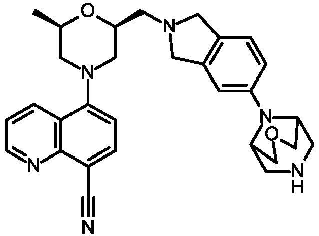

The title compound was prepared according to the following scheme:

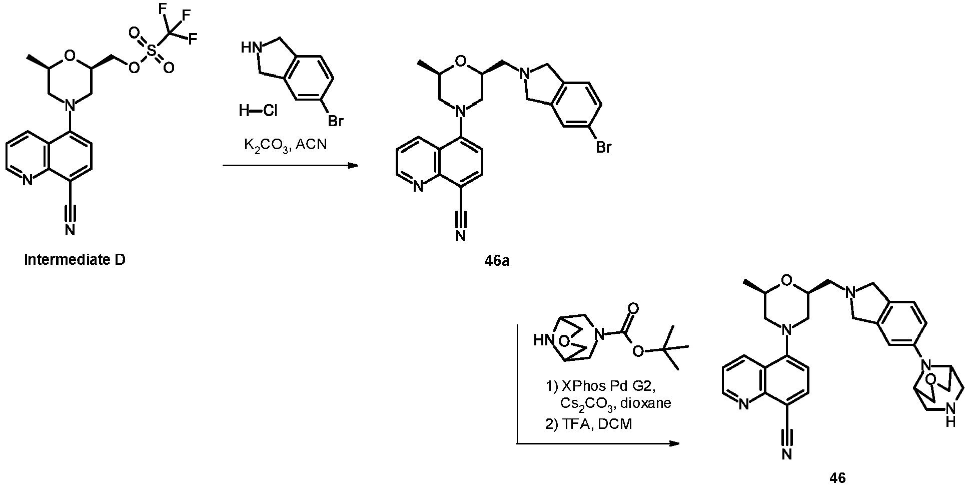

### Step 1: preparation of 5-[(2S,6R)-2-[(5-bromoisoindolin-2-yl)methyl]-6-methyl-morpholin-4-yl]quinoline-8-carbonitrile (compound 46a)

To a solution of 5-bromoisoindoline hydrochloride (352 mg, 1.50 mmol) in ACN/DCM (50 mL, v/v = 3/2) was added K₂CO₃ (300 mg, 2.17 mmol). The suspension was stirred at rt for 48 hrs, then filtered through celite. The filtrate was concentrated to give 5-bromoisoindoline (90 mg) which was dissolved in ACN (5 mL). To the solution was added ((2R,6R)-4-(8-cyanoquinolin-5-yl)-6-methylmorpholin-2-yl)methyl trifluoromethanesulfonate (Intermediate **D,** 519 mg, 1.25 mmol) and K₂CO₃ (518 mg, 3.75 mmol). The suspension was heated under reflux for 2 hrs, then diluted with ACN (10 mL), and filtered through celite. The filtrate was concentrated to give a crude product which was purified by column chromatography to give compound **46a** (500 mg) as a brown foamy solid. MS: calc'd 463 and 465 (MH⁺), measured 463 and 465 (MH⁺).

### Step 2: preparation of 5-[(2R,6S)-2-methyl-6-[[5-(3-oxa-7,9-diazabicyclo [3.3.1] nonan-9-yl)isoindolin-2-yl] methyl] morpholin-4-yl] quinoline-8-carbonitrile (Example 46)

To a solution of 5-((2*R*,6*S*)-2-((5-bromoisoindolin-2-yl)methyl)-6-methylmorpholino)quinoline-8-carbonitrile (compound **46a,** 30 mg, 65 µmol) in dioxane (3 mL) was added *tert*-butyl 3-oxa-7,9-diazabicyclo[3.3.1]nonane-7-carboxylate (22 mg, 97 µmol) and Cs₂CO₃ (63 mg, 0.19 mmol). The mixture was bubbled with N₂ for 5 mins, then XPhos Pd G2 (51 mg, 65 µmol) was added. The mixture was heated at 85 °C (oil bath) for 3 hrs, then cooled to rt, and filtered through celite. The filtrate was concentrated to give a brown oil which was dissolved in DCM (2 mL). To the solution was added TFA (1 mL). The reaction mixture was stirred at rt for 2 hrs, then concentrated to give a crude product which was purified by prep-HPLC to give **Example 46** (2.8 mg) as a yellow powder. MS: calc'd 511 (MH⁺), measured 511 (MH⁺). ¹H NMR (400 MHz, METHANOL-d4) *δ* 9.01 (dd, *J* = 1.6, 4.3 Hz, 1H), 8.68 (dd, *J =* 1.7, 8.6 Hz, 1H), 8.18 (d, *J* = 8.1 Hz, 1H), 7.66 (dd, *J* = 4.2, 8.6 Hz, 1H), 7.37 (d, *J* = 9.2 Hz, 1H), 7.30 (d, *J* = 8.1 Hz, 1H), 7.07 - 7.02 (m, 2H), 4.97 - 4.93 (m, 2H), 4.75 - 4.54 (m, 1H), 4.50 - 4.42 (m, 1H), 4.24 - 4.15 (m, 5H), 4.06 - 4.00 (m, 2H), 3.68 - 3.56 (m, 4H), 3.50 - 3.34 (m, 5H), 2.85 - 2.71 (m, 2H), 1.34 (d, *J* = 6.4 Hz, 3H).

### Example 47

### 5-[(2S,6R)-2-[[5-(2,6-diazaspiro[3.3]heptan-2-yl)isoindolin-2-yl]methyl]-6-methyl-morpholin-4-yl]quinoline-8-carbonitrile

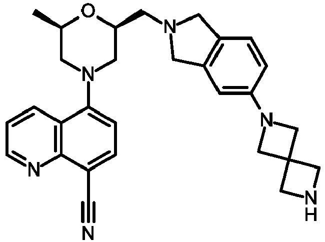

The title compound was prepared in analogy to the preparation of **Example 46** by using *tert*-butyl 2,6-diazaspiro[3.3]heptane-2-carboxylate oxalate instead of *tert*-butyl 3-oxa-7,9-diazabicyclo[3.3.1]nonane-7-carboxylate. **Example 47** (15mg) was obtained as a light brown powder. MS: calc'd 481 (MH⁺), measured 481 (MH⁺). ¹H NMR (400 MHz, METHANOL-d4) *δ* 9.02 (dd, *J* = 1.7, 4.2 Hz, 1H), 8.70 (dd, *J* = 1.7, 8.6 Hz, 1H), 8.19 (d, *J=* 8.1 Hz, 1H), 7.68 (dd, *J* = 4.2, 8.6 Hz, 1H), 7.31 (d, *J* = 8.1 Hz, 1H), 7.26 (br d, *J* = 8.7 Hz, 1H), 6.60 - 6.51 (m, 2H), 4.87 - 4.78 (m, 2H), 4.73 - 4.51 (m, 2H), 4.50 - 4.41 (m, 1H), 4.33 (s, 4H), 4.27 - 4.17 (m, 1H), 4.09 (s, 4H), 3.68 - 3.56 (m, 2H), 3.49 - 3.42 (m, 2H), 2.86 - 2.71 (m, 2H), 1.35 (d, *J* = 6.2 Hz, 3H).

### Example 48

### 5-[(2R,6S)-2-methyl-6-[(1-methyl-5-piperazin-1-yl-isoindolin-2-yl)methyl]morpholin-4-yl]quinoline-8-carbonitrile

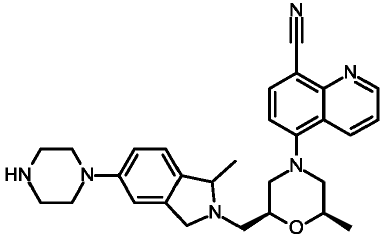

The title compound was prepared in analogy to the preparation of **Example 3** by using Intermediate **D** and 5-bromo-1-methyl-isoindoline (CAS: 335428-62-1, Vendor: Bepharm) instead of Intermediate **A** and 4-bromoisoindoline hydrochloride. **Example 48** (5.5 mg) was obtained as a yellow solid. MS: calc'd 483 (MH⁺), measured 483 (MH⁺). ¹H NMR (400 MHz, METHANOL-d4) *δ* 9.00 (dd, *J* = 1.6, 4.1 Hz, 1H), 8.70 (d, *J* = 8.5 Hz, 1H), 8.21 - 8.16 (m, 1H), 7.67 (dd, *J* = 4.3, 8.8 Hz, 1H), 7.30 (d, *J* = 8.0 Hz, 2H), 7.15 - 7.07 (m, 2H), 4.99 - 4.89 (m, 2H), 4.68 (br d, *J =* 15.1 Hz, 1H), 4.48 (br t, *J* = 9.9 Hz, 1H), 4.26 - 4.16 (m, 1H), 3.77 - 3.65 (m, 1H), 3.57 (br d, *J=* 10.8 Hz, 1H), 3.50 - 3.43 (m, 6H), 3.41 - 3.35 (m, 4H), 2.85 (t, *J* = 10.9 Hz, 1H), 2.79 - 2.70 (m, 1H), 1.79 (d, *J* = 5.5 Hz, 3H), 1.32 (d, *J* = 6.3 Hz, 3H).

### Example 49

### 8-[(2R,6S)-2-methyl-6-[[5-(3-oxa-7,9-diazabicyclo[3.3.1]nonan-7-yl)isoindolin-2-yl]methyl]morpholin-4-yl]quinoxaline-5-carbonitrile

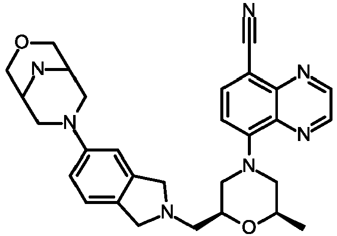

The title compound was prepared in analogy to the preparation of **Example 38** by using *tert*-butyl 3-oxa-7,9-diazabicyclo[3.3.1]nonane-9-carboxylate instead of *tert*-butyl 4,7-diazaspiro[2.5]octane-4-carboxylate. **Example 49** (6 mg) was obtained as a yellow solid. MS: calc'd 512 (MH⁺), measured 512 (MH⁺). ¹H NMR (400 MHz, METHANOL-d4) *δ* 8.98 (d, *J* = 1.7 Hz, 1H), 8.90 (d, *J* = 1.7 Hz, 1H), 8.16 (d, *J =* 8.3 Hz, 1H), 7.38 - 7.30 (m, 1H), 7.27 (d, *J* = 8.3 Hz, 1H), 7.07 - 7.00 (m, 2H), 4.82 - 4.48 (m, 4H), 4.41 (br t, *J* = 9.9 Hz, 1H), 4.31 (br d, *J =* 11.7 Hz, 1H), 4.21 - 4.03 (m, 8H), 3.72 - 3.52 (m, 4H), 3.42 (br d, *J =* 11.2 Hz, 2H), 2.92 - 2.78 (m, 2H), 1.35 (d, *J* = 6.1 Hz, 3H).

### Example 50

### Ethyl 1-[2-[[(2S,6R)-4-(8-cyano-5-quinolyl)-6-methyl-morpholin-2-yl]methyl]isoindolin-5-yl]piperazine-2-carboxylate

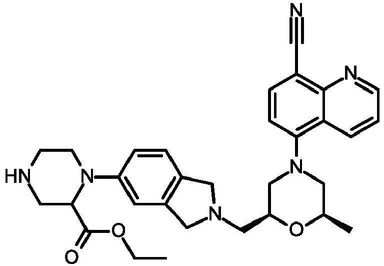

The title compound was prepared according to the following scheme:

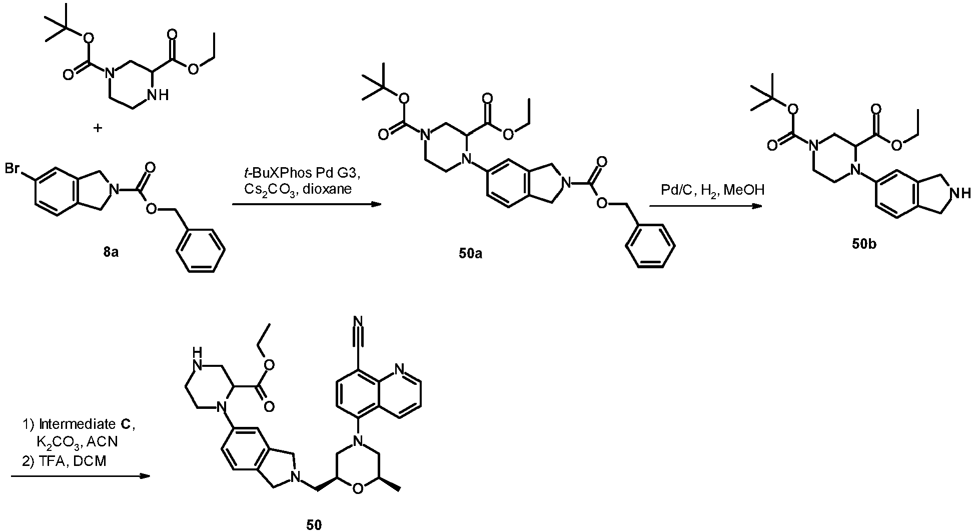

### Step 1: preparation of O1-tert-butyl O3-ethyl 4-(2-benzyloxycarbonylisoindolin-5-yl)piperazine-1,3-dicarboxylate (compound 50a)

To a solution of benzyl 5-bromoisoindoline-2-carboxylate (compound **8a,** 400 mg, 1.2 mmol) in 1,4-dioxane (5 mL) was added *O*1-*tert*-butyl *O*3-ethyl piperazine-1,3-dicarboxylate (353 mg, 1.44 mmol), Cs₂CO₃ (1.18 g, 3.61 mmol) and *t*-BuXPhos Pd G3 (40 mg, 0.12 mmol). The reaction mixture was stirred at 90 °C for 12 hrs under N₂, then concentrated. The residue was dissolved in EtOAc (100 mL) and filtered, the filtrate was concentrated. The crude product was purified by prep-TLC (EA/PE, v/v = 1/1, Rf = 0.25) to give compound **50a** (40 mg) as a yellow solid. MS: calc'd 510 (MH⁺), measured 510 (MH⁺).

### Step 2: preparation of O1-tert-butyl O3-ethyl 4-isoindolin-5-ylpiperazine-1,3-dicarboxylate (compound 50b)

To a solution of O1-*tert*-butyl *O*3-ethyl 4-(2-benzyloxycarbonylisoindolin-5-yl)piperazine-1,3-dicarboxylate (compound **50a,** 60 mg, 0.12 mmol) in MeOH (5 mL) was added Pd on carbon (6 mg, 10%). The mixture was stirred for at rt for 2 hrs under hydrogen balloon. The reaction mixture was then filtered, the filtrate was concentrated to afford compound **50b** (40 mg) as a brown oil. MS: calc'd 376 (MH⁺), measured 376 (MH⁺).

### Step 3: preparation of ethyl 1-[2-[[(2S,6R)-4-(8-cyano-5-quinolyl)-6-methyl-morpholin-2-yl]methyl]isoindolin-5-yl]piperazine-2-carboxylate (Example 50)

To a solution of *O*1-*tert*-butyl *O*3-ethyl 4-isoindolin-5-ylpiperazine-1,3-dicarboxylate (compound **50b,** 40 mg, 0.11 mmol) in ACN (5 mL) was added K₂CO₃ (37 mg, 0.27 mmol) and [(2*R*,6*A*)-4-(8-cyano-5-quinolyl)-6-methyl-morpholin-2-yl]methyl trifluoromethanesulfonate (Intermediate **D,** 53 mg, 0.13 mmol). The mixture was stirred at 55 °C for 3 hrs, then filtered. The filtrate was concentrated. The residue was purified by prep-TLC (EA/PE, v/v = 1/1, Rf = 0.5) to afford the intermediate (40 mg) as a yellow oil which was dissolved in DCM (2 mL). To the solution was added TFA (1 mL). The reaction mixture was then concentrated to give a crude product (30 mg). 10 mg of the crude product was purified by prep-HPLC to give **Example 50** (7 mg) as a white solid. MS: calc'd 541 (MH⁺), measured 541 (MH⁺). ¹H NMR (400 MHz, METHANOL-*d*₄) δ ppm 9.01 (dd, *J* = 4.3, 1.6 Hz, 1H), 8.69 (dd, *J* = 8.6, 1.6 Hz, 1H), 8.18 (d, *J* = 7.9 Hz, 1H), 7.67 (dd, *J =* 8.5, 4.3 Hz, 1H), 7.35 (d, *J =* 8.3 Hz, 1H), 7.30 (d, *J* = 8.2 Hz, 1H), 7.05 - 7.14 (m, 2H), 5.01 (br d, *J* = 3.8 Hz, 1H), 4.59 - 4.85 (m, 4H), 4.49 (br t, *J* = 9.9 Hz, 1H), 4.09 - 4.28 (m, 3H), 3.92 (d, *J=* 13.1 Hz, 1H), 3.75 - 3.84 (m, 1H), 3.42 - 3.71 (m, 7H), 3.24 - 3.32 (m, 1H), 2.69 - 2.88 (m, 2H), 1.35 (d, *J* = 6.3 Hz, 3H), 1.20 (t, *J* = 7.2 Hz, 3H).

### Example 51

### 4-[(2R,6S)-2-methyl-6-[[2-(4-methylpiperazin-1-yl)-5,7-dihydropyrrolo[3,4-d]pyrimidin-6-yl]methyl]morpholin-4-yl]pyrazolo[1,5-a]pyridine-7-carbonitrile

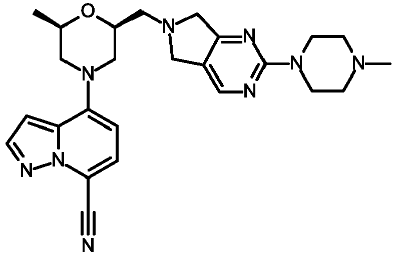

The title compound was prepared according to the following scheme:

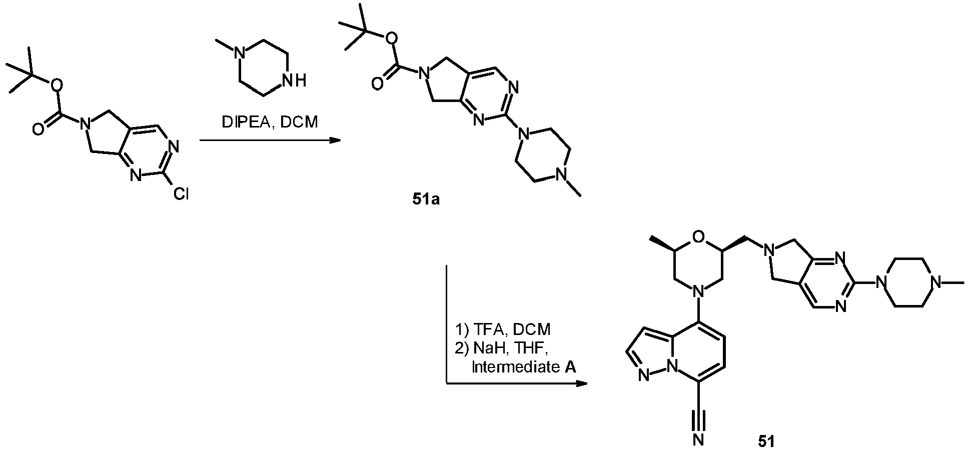

### Step 1: preparation of tert-butyl 2-(4-methylpiperazin-1-yl)-5,7-dihydropyrrolo[3,4-d]pyrimidine-6-carboxylate (compound 51a)

To a solution of *tert*-butyl 2-chloro-5,7-dihydro-6*H*-pyrrolo[3,4-d]pyrimidine-6-carboxylate (CAS: 1211581-47-3, Vendor: Pharmablock, 256 mg, 1.0 mmol) in DCM (3 mL) was added 1-methylpiperazine (150 mg, 0.16 mL, 1.5 mmol) and DIPEA (1.85 g, 2.5 mL, 14.3 mmol) . The suspension was heated at 150 °C under microwave for 2 hrs. The mixture was then concentrated to give a crude product which was purified by column chromatography to give compound **51a** (301 mg) as a brown solid. MS: calc'd 320 (MH⁺), measured 320 (MH⁺).

### Step 2: preparation of 4-[(2R,6S)-2-methyl-6-[[2-(4-methylpiperazin-1-yl)-5,7-dihydropyrrolo[3,4-d]pyrimidin-6-yl]methyl]morpholin-4-yl]pyrazolo[1,5-a]pyridine-7-carbonitrile (Example 51)

To a solution of *tert*-butyl 2-(4-methylpiperazin-1-yl)-5,7-dihydro-6*H*-pyrrolo[3,4-d]pyrimidine-6-carboxylate (compound **51a,** 35 mg, 0.11 mmol) in DCM (2 mL) was added TFA (1 mL). The mixture was stirred at rt for 2 hrs, then concentrated to give an oil which was dissolved in THF (4 mL). To the solution was added NaH (60% in mineral oil, 59 mg, 1.49 mmol). The reaction mixture was stirred at rt for 20 min, then was added ((2*R*,6*R*)-4-(7-cyanopyrazolo[1,5-a]pyridin-4-yl)-6-methylmorpholin-2-yl)methyl trifluoromethanesulfonate (Intermediate **A,** 30 mg, 74 µmol). The reaction mixture was stirred at rt for 2 hrs, then quenched with MeOH, the mixture was concentrated to give a crude mixture which was purified by prep-HPLC to give **Example 51** (8.2 mg) as a white powder. MS: calc'd 474 (MH⁺), measured 474 (MH⁺). ¹H NMR (400 MHz, METHANOL-d4) *δ* 8.47 (s, 1H), 8.07 (d, *J* = 2.0 Hz, 1H), 7.52 (d, *J* = 8.1 Hz, 1H), 6.93 (d, *J* = 2.3 Hz, 1H), 6.67 (d, *J* = 7.9 Hz, 1H), 5.11 - 4.90 (m, 2H), 4.86 - 4.71 (m, 2H), 4.71 - 4.60 (m, 2H), 4.38 - 4.28 (m, 1H), 4.12 - 4.02 (m, 1H), 3.84 (br dd, *J* = 12.5, 18.2 Hz, 2H), 3.74 - 3.51 (m, 4H), 3.46 - 3.34 (m, 2H), 3.30 - 3.14 (m, 2H), 2.98 (s, 3H), 2.85 - 2.71 (m, 2H), 1.36 (d, *J* = 6.2 Hz, 3H).

### Example 52

### 4-[(2R,6S)-2-methyl-6-[(5-pyrrolidin-3-yloxyisoindolin-2-yl)methyl]morpholin-4-yl]pyrazolo[1,5-a]pyridine-7-carbonitrile

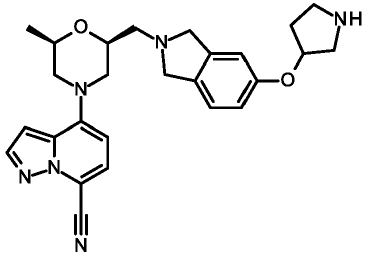

The title compound was prepared according to the following scheme:

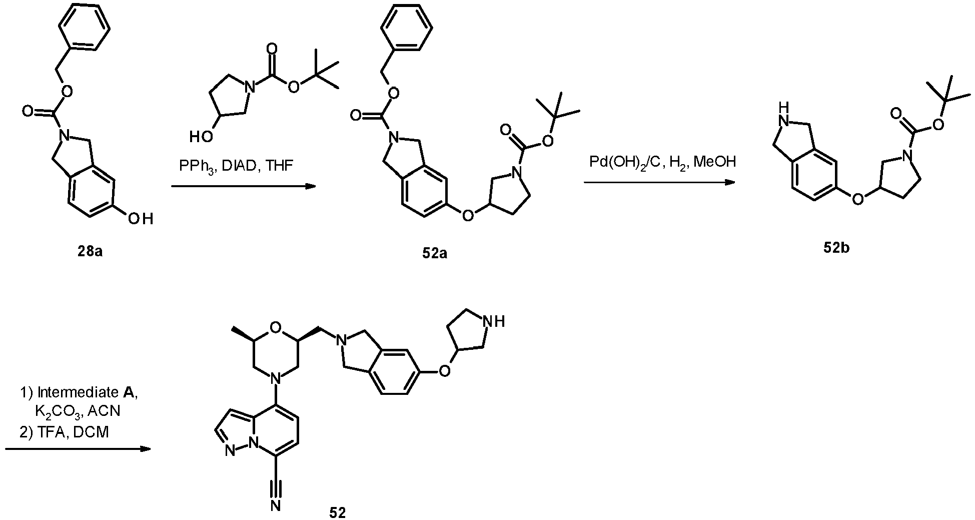

### Step 1: preparation of benzyl 5-(1-tert-butoxycarbonylpyrrolidin-3-yl)oxyisoindoline-2-carboxylate (compound 52a)

To a solution of benzyl 5-hydroxyisoindoline-2-carboxylate (compound **28a,** 62 mg, 0.23 mmol) in THF (5 mL) was added *tert*-butyl 3-hydroxypyrrolidine-1-carboxylate (43 mg, 0.23 mmol), and triphenylphosphine (121 mg, 0.46 mmol). To the solution was added DIAD (70 mg, 0.35 mmol) in one portion. The mixture was heated at 65 °C and stirred overnight, then concentrated to give an oil which was purified by column chromatography to give compound **52a** (83 mg) as an oil. MS: calc'd 439 (MH⁺), measured 439 (MH⁺).

### Step 2: preparation of tert-butyl 3-isoindolin-5-yloxypyrrolidine-1-carboxylate (compound 52b)

To a solution of benzyl 5-((1-(*tert*-butoxycarbonyl)pyrrolidin-3-yl)oxy)isoindoline-2-carboxylate (compound **52a,** 83 mg, 0.19 mmol) in MeOH (10 mL) was added Pd(OH)₂ on carbon (20 mg, 10% wet). The suspension was charged with hydrogen balloon and stirred at rt for 2 hrs, then filtered through celite, the filtrated was concentrated to give compound **52b** (56 mg) as a yellow oil. MS: calc'd 305 (MH⁺), measured 305 (MH⁺).

### Step 3: preparation of 4-[(2R,6S)-2-methyl-6-[(5-pyrrolidin-3-yloxyisoindolin-2-yl)methyl]morpholin-4-yl]pyrazolo[1,5-a]pyridine-7-carbonitrile (Example 52)

To a solution of ((2*R*,6*R*)-4-(7-cyanopyrazolo[1,5-a]pyridin-4-yl)-6-methylmorpholin-2-yl)methyl trifluoromethanesulfonate (Intermediate **A,** 50 mg, 0.12 mmol) in ACN (4 mL) was added *tert*-butyl 3-(isoindolin-5-yloxy)pyrrolidine-1-carboxylate (compound **52b,** 56 mg, 0.18 mmol) and K₂CO₃ (51 mg, 0.37 mmol). The suspension was heated under reflux for 4 hrs, then cooled to rt, diluted with ACN (10 mL), and filtered through celite. The filtrate was concentrated to give a yellow oil which was dissolved in DCM (4 mL). To the solution was added TFA (1 mL). The reaction mixture was stirred at rt for 2 hrs, then concentrated to give a crude product which was purified by prep-HPLC to give **Example 52** (28 mg) as a white powder. MS: calc'd 459 (MH⁺), measured 459 (MH⁺). ¹H NMR (400 MHz, METHANOL-d4) *δ* 8.07 (s, 1H), 7.52 (d, *J* = 7.9 Hz, 1H), 7.40 (d, *J* = 8.7 Hz, 1H), 7.12 - 7.04 (m, 2H), 6.94 (d, *J* = 2.4 Hz, 1H), 6.67 (d, *J* = 7.9 Hz, 1H), 5.25 (br s, 1H), 5.03 - 4.91 (m, 2H), 4.83 - 4.54 (m, 2H), 4.34 (br t, *J* = 10.1 Hz, 1H), 4.13 - 4.04 (m, 1H), 3.84 (br t, *J* = 13.8 Hz, 2H), 3.75 - 3.62 (m, 2H), 3.62 - 3.56 (m, 2H), 3.55 - 3.45 (m, 2H), 2.85 - 2.72 (m, 2H), 2.39 - 2.31 (m, 2H), 1.36 (d, *J* = 6.2 Hz, 3H).

### Example 53

### 4-[(2S,6R)-2-[[5-(2,6-dimethyl-4-pyridyl)isoindolin-2-yl]methyl]-6-methyl-morpholin-4-yl]pyrazolo[1,5-a]pyridine-7-carbonitrile

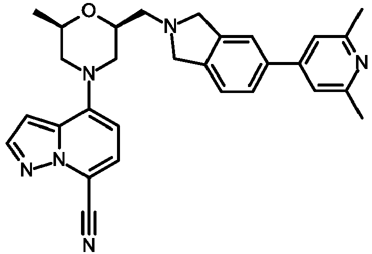

The title compound was prepared in analogy to the preparation of **Example 45** by using 4-bromo-2,6-dimethylpyridine instead of 4-bromo-1-methyl-imidazole. **Example 53** (6 mg) was obtained as a light yellow powder. MS: calc'd 479 (MH⁺), measured 479 (MH⁺). ¹H NMR (400 MHz, METHANOL-d4) *δ* 8.05 (d, *J* = 2.3 Hz, 1H), 7.68 - 7.61 (m, 2H), 7.50 (d, *J* = 7.9 Hz, 1H), 7.45 - 7.37 (m, 3H), 6.92 (d, *J* = 2.3 Hz, 1H), 6.64 (d, *J* = 7.9 Hz, 1H), 4.28 - 4.07 (m, 5H), 4.05 - 3.95 (m, 1H), 3.88 (br d, *J=* 12.6 Hz, 1H), 3.82 (br d, *J* = 12.5 Hz, 1H), 3.12 - 3.02 (m, 2H), 2.80 (t, *J* = 11.2 Hz, 1H), 2.75 - 2.67 (m, 1H), 2.58 (s, 6H), 1.33 (d, *J* = 6.2 Hz, 3H).

### Example 54

### 4-[(2S,6R)-2-[[5-(2,6-dimethyl-4-pyridyl)isoindolin-2-yl]methyl]-6-methyl-morpholin-4-yl]-3-fluoro-pyrazolo[1,5-a]pyridine-7-carbonitrile

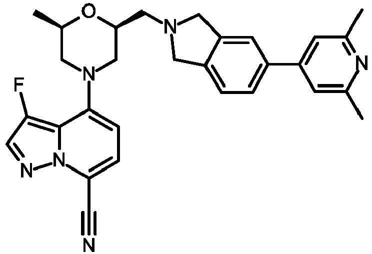

The title compound was prepared in analogy to the preparation of **Example 45** by using Intermediate **B** and 4-bromo-2,6-dimethylpyridine instead of Intermediate **A** and 4-bromo-1-methyl-imidazole. **Example 54** (6.5 mg) was obtained as a light yellow powder. MS: calc'd 497 (MH⁺), measured 497 (MH⁺). ¹H NMR (400 MHz, METHANOL-d4) *δ* 8.03 (d, *J* = 3.5 Hz, 1H), 7.72 - 7.65 (m, 2H), 7.48 - 7.41 (m, 4H), 6.57 (d, *J* = 7.9 Hz, 1H), 4.45 - 4.26 (m, 4H), 4.21 - 4.10 (m, 1H), 4.04 - 3.94 (m, 1H), 3.69 (br d, *J* = 11.7 Hz, 1H), 3.60 (br d, *J* = 11.6 Hz, 1H), 3.25 - 3.16 (m, 2H), 2.82 - 2.74 (m, 1H), 2.73 - 2.66 (m, 1H), 2.59 (s, 6H), 1.32 (d, *J* = 6.2 Hz, 3H).

### Example 55

### 5-[(2S,6R)-2-[[5-(1,6-diazaspiro[3.3]heptan-6-yl)isoindolin-2-yl]methyl]-6-methyl-morpholin-4-yl]quinoline-8-carbonitrile

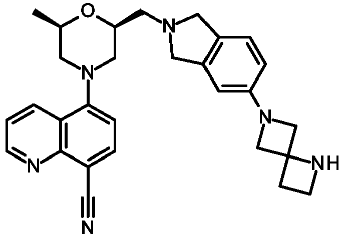

The title compound was prepared in analogy to the preparation of **Example 46** by using *tert*-butyl 1,6-diazaspiro[3.3]heptane-1-carboxylate oxalate instead of *tert*-butyl 3-oxa-7,9-diazabicyclo[3.3.1]nonane-7-carboxylate. **Example 55** (3 mg) was obtained as a brown solid. MS: calc'd 481 (MH⁺), measured 481 (MH⁺). ¹H NMR (400 MHz, METHANOL-d4) *δ* 9.01 (dd, *J =* 1.7, 4.2 Hz, 1H), 8.69 (dd, *J =* 1.7, 8.6 Hz, 1H), 8.18 (d, *J* = 8.1 Hz, 1H), 7.67 (dd, *J* = 4.2, 8.6 Hz, 1H), 7.29 (d, *J* = 8.1 Hz, 1H), 7.19 (d, *J* = 8.2 Hz, 1H), 6.53 (s, 1H), 6.49 (br d, *J* = 8.1 Hz, 1H), 4.39 - 4.27 (m, 4H), 4.24 (d, *J* = 9.0 Hz, 2H), 4.21 - 4.13 (m, 1H), 4.10 (d, *J =* 9.0 Hz, 2H), 3.92 (t, *J* = 8.1 Hz, 2H), 3.51 - 3.39 (m, 2H), 3.32 - 3.18 (m, 3H), 2.85 - 2.66 (m, 4H), 1.32 (d, *J* = 6.2 Hz, 3H).

### Example 56

### 1-[2-[[(2S,6R)-4-(8-cyano-5-quinolyl)-6-methyl-morpholin-2-yl]methyl]isoindolin-5-yl]piperazine-2-carboxylic acid

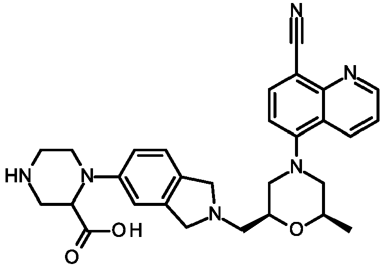

To a solution of crude ethyl 1-[2-[[(2*S*,6*R*)-4-(8-cyano-5-quinolyl)-6-methyl-morpholin-2-yl]methyl]isoindolin-5-yl]piperazine-2-carboxylate **(Example 50,** 15 mg, 0.03 mmol) in THF (1.0 mL) was added water (0.5 mL) and lithium hydroxide (0.7 mg, 0.03 mmol) at 0 °C. The mixture was stirred at rt for 12 hrs, then concentrated under reduced pressure. The residue was purified by prep-HPLC to give **Example 56** (8.1 mg) as a yellow solid. MS: calc'd 513 (MH⁺), measured 513 (MH⁺). ¹H NMR (400 MHz, METHANOL-*d*₄) δ ppm 9.02 (dd, *J =* 4.3, 1.5 Hz, 1H), 8.70 (dd, *J* = 8.6, 1.6 Hz, 1H), 8.19 (d, *J* = 8.0 Hz, 1H), 7.68 (dd, *J* = 8.7, 4.3 Hz, 1H), 7.38 - 7.29 (m, 2H), 7.14 - 7.07 (m, 2H), 5.03 - 4.94 (m, 2H), 4.93 - 4.91 (m, 1H), 4.81 - 4.54 (m, 2H), 4.48 (br t, *J* = 9.5 Hz, 1H), 4.30 - 4.18 (m, 1H), 3.93 (br d, *J* = 12.8 Hz, 1H), 3.81 (br d, *J* = 13.2 Hz, 1H), 3.71 - 3.58 (m, 2H), 3.57 - 3.43 (m, 5H), 3.29 (br d, *J* = 3.8 Hz, 1H), 2.87 - 2.72 (m, 2H), 1.35 (d, *J* = 6.3 Hz, 3H).

### Example 57

### Trans-5-[(2R,6S)-2-methyl-6-[[5-[3,5-dimethylpiperazin-1-yl]isoindolin-2-yl]methyl]morpholin-4-yl]quinoline-8-carbonitrile

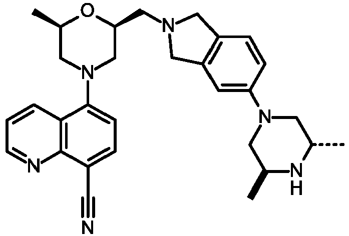

The title compound was prepared in analogy to the preparation of **Example 46** by using *trans-tert*-butyl 2,6-dimethylpiperazine-1-carboxylate instead of *tert*-butyl 3-oxa-7,9-diazabicyclo[3.3.1]nonane-7-carboxylate. **Example 57** (5.5 mg) was obtained as a light yellow solid. MS: calc'd 497 (MH⁺), measured 497 (MH⁺). ¹H NMR (400 MHz, METHANOL-d4) *δ* 9.03 (dd, *J* = 1.6, 4.3 Hz, 1H), 8.70 (dd, *J* = 1.6, 8.6 Hz, 1H), 8.20 (d, *J* = 7.9 Hz, 1H), 7.68 (dd, *J* = 4.3, 8.6 Hz, 1H), 7.37 (br d, *J* = 8.9 Hz, 1H), 7.32 (d, *J* = 8.1 Hz, 1H), 7.11 (br s, 2H), 4.77 - 4.55 (m, 2H), 4.52 - 4.44 (m, 1H), 4.28 - 4.18 (m, 1H), 3.87 - 3.76 (m, 2H), 3.70 - 3.60 (m, 2H), 3.55 - 3.45 (m, 4H), 3.31 - 3.18 (m, 4H), 2.87 - 2.73 (m, 2H), 1.49 (s, 3H), 1.47 (s, 3H), 1.36 (d, *J* = 6.2 Hz, 3H).

### Example 58

### 5-[(2S,6R)-2-[[5-(3,4,6,7,9,9a-hexahydro-1H-pyrazino[2,1-c][1,4]oxazin-8-yl)isoindolin-2-yl]methyl]-6-methyl-morpholin-4-yl]quinoline-8-carbonitrile

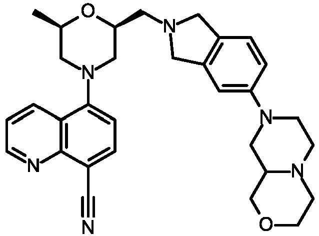

The title compound was prepared according to the following scheme:

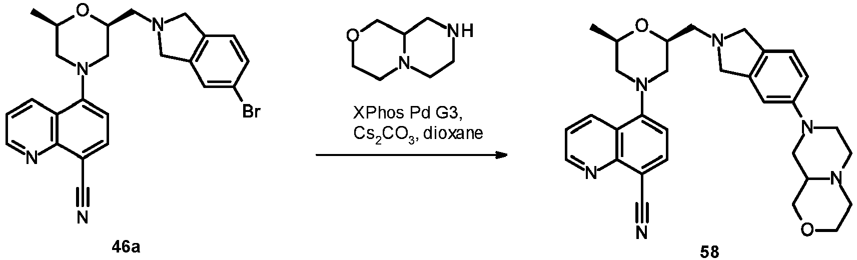

To a solution of 5-((2*R*,6*S*)-2-((5-bromoisoindolin-2-yl)methyl)-6-methylmorpholino)quinoline-8-carbonitrile (compound **46a,** 30 mg) in dioxane (4 mL) was added octahydropyrazino[2,1-c][1,4]oxazine dihydrochloride (21 mg, 97 µmol) and Cs₂CO₃ (105 mg, 0.32 mmol). The mixture was bubbled with N₂ for 5 mins and XPhos Pd G3 (11 mg, 13 µmol) was added. The mixture was heated at 90 °C (oil bath) overnight. The mixture was filtered through celite, the filtrate was concentrated to give a brown oil which was purified by prep-HPLC to give **Example 58** (1 mg) as a light yellow powder. MS: calc'd 525 (MH⁺), measured 525 (MH⁺). ¹H NMR (400 MHz, METHANOL-d4) *δ* 9.02 (dd, *J* = 1.6, 4.3 Hz, 1H), 8.70 (dd, *J* = 1.7, 8.6 Hz, 1H), 8.20 (d, *J* = 8.1 Hz, 1H), 7.68 (dd, *J* = 4.4, 8.6 Hz, 1H), 7.37 (br d, *J* = 9.3 Hz, 1H), 7.32 (d, *J* = 8.1 Hz, 1H), 7.15 - 7.10 (m, 2H), 4.80 - 4.53 (m, 4H), 4.48 (br t, *J* = 9.5 Hz, 1H), 4.28 - 4.19 (m, 1H), 4.15 - 4.03 (m, 2H), 3.97 - 3.82 (m, 4H), 3.71 - 3.55 (m, 4H), 3.54 - 3.39 (m, 4H), 3.30 - 3.17 (m, 2H), 2.87 - 2.73 (m, 3H), 1.35 (d, *J* = 6.1 Hz, 3H).

### Example 59

### 5-[(2S,6R)-2-[(4-fluoro-6-piperazin-1-yl-isoindolin-2-yl)methyl]-6-methyl-morpholin-4-yl]quinoline-8-carbonitrile

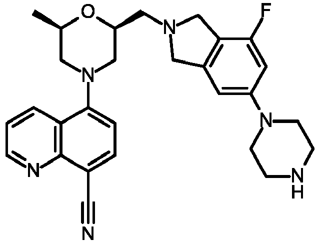

The title compound was prepared in analogy to the preparation of **Example 3** by using Intermediate **D** and 6-bromo-4-fluoroisoindoline hydrochloride instead of Intermediate **A** and 4-bromoisoindoline hydrochloride. **Example 59** (24 mg) was obtained as a light yellow powder. MS: calc'd 487 (MH⁺), measured 487 (MH⁺). ¹H NMR (400 MHz, METHANOL-d4) *δ* 9.02 (dd, *J =* 1.7, 4.2 Hz, 1H), 8.70 (dd, *J =* 1.7, 8.6 Hz, 1H), 8.19 (d, *J* = 7.9 Hz, 1H), 7.68 (dd, *J* = 4.3, 8.6 Hz, 1H), 7.31 (d, *J* = 8.1 Hz, 1H), 6.95 (s, 1H), 6.89 (d, *J=* 12.1 Hz, 1H), 4.88 - 4.63 (m, 4H), 4.53 - 4.44 (m, 1H), 4.28 - 4.17 (m, 1H), 3.71 - 3.59 (m, 2H), 3.55 - 3.47 (m, 5H), 3.47 - 3.36 (m, 5H), 2.86 - 2.72 (m, 2H), 1.35 (d, *J* = 6.2 Hz, 3H).

### Example 60

### 8-[(2R,6S)-2-methyl-6-[(6-piperazin-1-yl-1,3-dihydropyrrolo[3,4-c]pyridin-2-yl)methyl]morpholin-4-yl]quinoxaline-5-carbonitrile

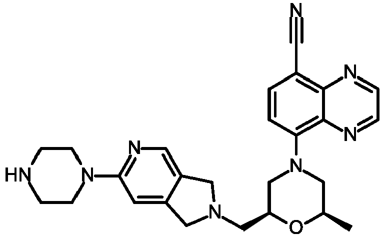

The title compound was prepared in analogy to the preparation of **Example 3** by using Intermediate **C** and 6-chloro-2,3-dihydro-1*H*-pyrrolo[3,4-c]pyridine (CAS: 905273-90-7, Vendor: Pharmablock) instead of Intermediate **A** and 4-bromoisoindoline hydrochloride. **Example 60** (24 mg) was obtained as a yellow solid. MS: calc'd 471 (MH⁺), measured 471 (MH⁺). ¹H NMR (400 MHz, METHANOL-d4) *δ* 8.96 (d, *J* = 1.7 Hz, 1H), 8.89 (d, *J* = 2.0 Hz, 1H), 8.22 (s, 1H), 8.14 (d, *J=* 8.3 Hz, 1H), 7.25 (d, *J* = 8.3 Hz, 1H), 7.01 (s, 1H), 4.81 - 4.72 (m, 4H), 4.48 - 4.37 (m, 1H), 4.31 (br d, *J* = 11.7 Hz, 1H), 4.20 - 4.09 (m, 2H), 3.90 - 3.80 (m, 4H), 3.74 - 3.58 (m, 2H), 3.34 (br d, *J* = 5.4 Hz, 4H), 2.91 - 2.79 (m, 2H), 1.34 (d, *J* = 6.1 Hz, 3H).

### Example 61

### 5-[(2R,6S)-2-methyl-6-[(6-piperazin-1-yl-1,3-dihydropyrrolo[3,4-c]pyridin-2-yl)methyl]morpholin-4-yl]quinoline-8-carbonitrile

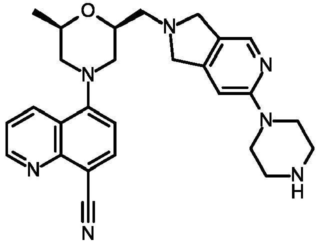

The title compound was prepared in analogy to the preparation of **Example 3** by using Intermediate **D** and 6-bromo-2,3-dihydro-1*H*-pyrrolo[3,4-c]pyridine hydrochloride instead of Intermediate **A** and 4-bromoisoindoline hydrochloride. **Example 61** (15 mg) was obtained as a light yellow solid. MS: calc'd 470 (MH⁺), measured 470 (MH⁺). ¹H NMR (400 MHz, METHANOL-d4) *δ* 8.88 (dd, *J* = 1.6, 4.3 Hz, 1H), 8.57 (dd, *J* = 1.7, 8.6 Hz, 1H), 8.05 (d, *J =* 7.9 Hz, 1H), 7.99 (s, 1H), 7.55 (dd, *J* = 4.3, 8.7 Hz, 1H), 7.17 (d, *J* = 8.1 Hz, 1H), 6.81 (s, 1H), 4.72 - 4.69 (m, 2H), 4.20 - 3.98 (m, 6H), 3.72 - 3.62 (m, 4H), 3.40 - 3.28 (m, 2H), 3.25 - 3.22 (m, 2H), 3.02 - 2.92 (m, 2H), 2.72 - 2.64 (m, 1H), 2.59 (dd, *J* = 10.3, 11.8 Hz, 1H), 1.19 (d, *J* = 6.2 Hz, 3H).

### Example 62

### 5-[(2R,6S)-2-methyl-6-[[5-[(3R)-3-methylpiperazin-1-yl]isoindolin-2-yl]methyl]morpholin-4-yl]quinoline-8-carbonitrile

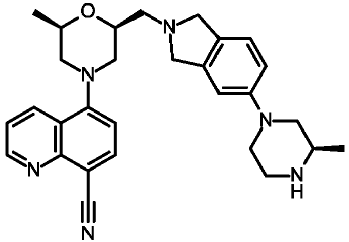

The title compound was prepared in analogy to the preparation of **Example 46** by using *tert*-butyl (R)-2-methylpiperazine-1-carboxylate instead of *tert*-butyl 3-oxa-7,9-diazabicyclo[3.3.1]nonane-7-carboxylate. **Example 62** (2.9 mg) was obtained as a light yellow powder. MS: calc'd 483 (MH⁺), measured 483 (MH⁺). ¹H NMR (400 MHz, METHANOL-d4) *δ* 9.03 (dd, *J* = 1.7, 4.2 Hz, 1H), 8.70 (dd, *J* = 1.6, 8.7 Hz, 1H), 8.20 (d, *J =* 8.1 Hz, 1H), 7.68 (dd, *J* = 4.2, 8.6 Hz, 1H), 7.38 (br d, *J =* 8.9 Hz, 1H), 7.32 (d, *J* = 8.2 Hz, 1H), 7.16 - 7.11 (m, 2H), 4.87 - 4.54 (m, 2H), 4.51 - 4.42 (m, 1H), 4.29 - 4.19 (m, 1H), 3.86 (br t, *J* = 14.7 Hz, 2H), 3.70 - 3.59 (m, 2H), 3.58 - 3.43 (m, 5H), 3.38 - 3.35 (m, 2H), 3.11 - 3.01 (m, 1H), 2.89 - 2.73 (m, 3H), 1.43 (d, *J* = 6.6 Hz, 3H), 1.36 (d, *J* = 6.2 Hz, 3H).

### Example 63

### 5-[(2R,6S)-2-methyl-6-[(2-piperazin-1-yl-5,7-dihydropyrrolo[3,4-d]pyrimidin-6-yl)methyl]morpholin-4-yl]quinoline-8-carbonitrile

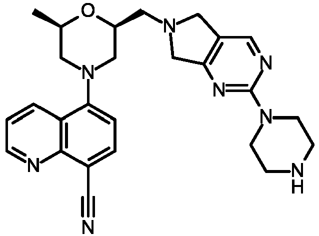

The title compound was prepared according to the following scheme:

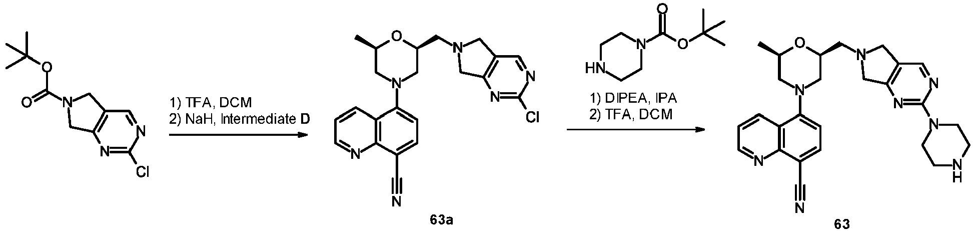

### Step 1: preparation of 5-[(2S,6R)-2-[(2-chloro-5,7-dihydropyrrolo[3,4-d]pyrimidin-6-yl)methyl]-6-methyl-morpholin-4-yl]quinoline-8-carbonitrile (compound 63a)

The a solution of *tert*-butyl 2-chloro-5,7-dihydro-6*H*-pyrrolo[3,4-d]pyrimidine-6-carboxylate (CAS: 1211581-47-3, Vendor: Pharmablock, 75 mg, 0.29 mmol) in DCM (2 mL) was added TFA (1 mL). The reaction mixture was stirred at rt for 2 hrs, then concentrated to give an oil which was dissolved in THF (4 mL). To the solution was added NaH (60% in mineral oil, 85 mg, 2.14 mmol) at 0 °C under N₂. The mixture was stirred at rt for 30 mins, then the solution of ((2*R*,6*R*)-4-(8-cyanoquinolin-5-yl)-6-methylmorpholin-2-yl)methyl trifluoromethanesulfonate (Intermediate **D,** 111 mg, 0.27 mmol) in THF (1 mL) was added. The reaction mixture was stirred at rt for 24 hrs, then quenched with water, extracted with EtOAc. The organic layer was then washed with sat NH₄Cl, dried over Na₂SO₄, and concentrated. The residue was purified by column chromatography to give compound **63a** (50 mg) as a yellow solid. MS: calc'd 421 (MH⁺), measured 421 (MH⁺).

### Step 2: preparation of 5-[(2R,6S)-2-methyl-6-[(2-piperazin-1-yl-5,7-dihydropyrrolo[3,4-d]pyrimidin-6-yl)methyl]morpholin-4-yl]quinoline-8-carbonitrile

### (Example 63)

To a solution of 5-[(2*S*,6*R*)-2-[(2-chloro-5,7-dihydropyrrolo[3,4-d]pyrimidin-6-yl)methyl]-6-methyl-morpholin-4-yl]quinoline-8-carbonitrile (compound **63a,** 50 mg, 0.12 mmol) in IPA (3 mL) was added *tert*-butyl piperazine-1-carboxylate (27 mg, 0.14 mmol) and DIPEA (2.22 g, 3.0 mL, 17 mmol). The reaction mixture was heated under microwave at 150 °C for 2 hrs, then solvent was removed. The residue was dissolved in DCM (2 mL). To the solution was added TFA (1 mL). The reaction mixture was stirred at rt for 2 hrs, then concentrated to give a crude product which was purified by prep-HPLC to give **Example 63** (14 mg) as a light yellow powder. MS: calc'd 471 (MH⁺), measured 471 (MH⁺). ¹H NMR (400 MHz, METHANOL-d4) *δ* 8.90 (dd, *J* = 1.7, 4.2 Hz, 1H), 8.58 (dd, *J* = 1.7, 8.6 Hz, 1H), 8.36 (s, 1H), 8.07 (d, *J* = 7.9 Hz, 1H), 7.56 (dd, *J* = 4.2, 8.6 Hz, 1H), 7.19 (d, *J* = 8.1 Hz, 1H), 4.74 - 4.66 (m, 2H), 4.65 - 4.52 (m, 2H), 4.42 - 4.32 (m, 1H), 4.16 - 4.06 (m, 1H), 4.06 - 3.99 (m, 4H), 3.62 - 3.48 (m, 2H), 3.40 - 3.31 (m, 2H), 3.26 - 3.22 (m, 2H), 3.20 - 3.11 (m, 2H), 2.71 (dd, *J =* 10.3, 11.8 Hz, 1H), 2.65 (dd, *J* = 10.3, 12.2 Hz, 1H), 1.23 (d, *J* = 6.2 Hz, 3H).

### Example 64

### 5-[(2R,6S)-2-methyl-6-[[5-[(3S)-3-methylpiperazin-1-yl]isoindolin-2-yl]methyl]morpholin-4-yl]quinoline-8-carbonitrile

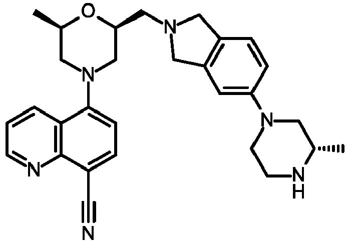

The title compound was prepared in analogy to the preparation of **Example 46** by using *tert*-butyl (*S*)-2-methylpiperazine-1-carboxylate instead of *tert*-butyl 3-oxa-7,9-diazabicyclo[3.3.1]nonane-7-carboxylate. **Example 64** (2.1 mg) was obtained as a light yellow solid. MS: calc'd 483 (MH⁺), measured 483 (MH⁺). ¹H NMR (400 MHz, METHANOL-d4) *δ* 8.90 (dd, *J* = 1.5, 4.2 Hz, 1H), 8.58 (dd, *J* = 1.5, 8.6 Hz, 1H), 8.08 (d, *J* = 7.9 Hz, 1H), 7.56 (dd, *J=* 4.2, 8.6 Hz, 1H), 7.28 - 7.17 (m, 2H), 7.01 (br s, 2H), 4.64 - 4.43 (m, 2H), 4.37 (br t, *J* = 9.8 Hz, 1H), 4.16 - 4.06 (m, 1H), 3.79 - 3.67 (m, 2H), 3.59 - 3.46 (m, 2H), 3.46 - 3.32 (m, 4H), 3.20 - 3.08 (m, 3H), 2.96 (br t, *J* = 11.1 Hz, 1H), 2.77 - 2.61 (m, 3H), 1.31 (d, *J* = 6.5 Hz, 3H), 1.23 (d, *J* = 6.2 Hz, 3H).

### Example 65

### 8-[(2R,6S)-2-methyl-6-[[5-[(3R)-3-methylpiperazin-1-yl]isoindolin-2-yl]methyl]morpholin-4-yl]quinoxaline-5-carbonitrile

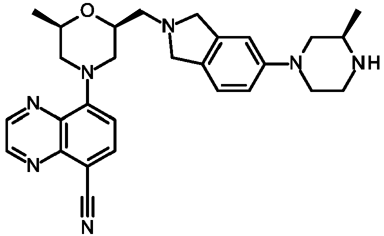

The title compound was prepared in analogy to the preparation of **Example 3** by using Intermediate **C,** 5-bromoisoindoline hydrochloride and *tert*-butyl (*R*)-2-methylpiperazine-1-carboxylate instead of Intermediate **A,** 4-bromoisoindoline hydrochloride and *tert*-butyl piperazine-1-carboxylate. **Example 65** (23 mg) was obtained as a yellow powder. MS: calc'd 484 (MH⁺), measured 484 (MH⁺). ¹H NMR (400 MHz, METHANOL-d4) *δ* 9.00 (d, *J* = 1.8 Hz, 1H), 8.92 (d, *J* = 1.8 Hz, 1H), 8.18 (d, *J* = 8.3 Hz, 1H), 7.39 - 7.33 (m, 1H), 7.29 (d, *J* = 8.4 Hz, 1H), 7.15 - 7.07 (m, 2H), 4.78 - 4.53 (m, 2H), 4.43 (br t, *J* = 10.1 Hz, 1H), 4.33 (br d, *J* = 12.0 Hz, 1H), 4.21 - 4.10 (m, 2H), 3.91 - 3.78 (m, 2H), 3.74 - 3.59 (m, 2H), 3.57 - 3.48 (m, 2H), 3.38 - 3.34 (m, 2H), 3.32 - 3.27 (m, 1H), 3.12 - 3.02 (m, 1H), 2.94 - 2.80 (m, 3H), 1.42 (d, *J* = 6.6 Hz, 3H), 1.37 (d, *J* = 6.1 Hz, 3H).

### Example 66

### 8-[(2R,6S)-2-methyl-6-[[5-[(3S)-3-methylpiperazin-1-yl]isoindolin-2-yl]methyl]morpholin-4-yl]quinoxaline-5-carbonitrile

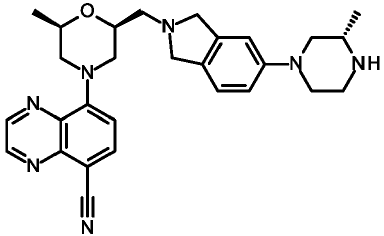

The title compound was prepared in analogy to the preparation of **Example 3** by using Intermediate **C,** 5-bromoisoindoline hydrochloride and *tert*-butyl (*S*)-2-methylpiperazine-1-carboxylate instead of Intermediate **A,** 4-bromoisoindoline hydrochloride and *tert*-butyl piperazine-1-carboxylate. **Example 66** (28 mg) was obtained as a yellow powder. MS: calc'd 484 (MH⁺), measured 484 (MH⁺). ¹H NMR (400 MHz, METHANOL-d4) *δ* 9.00 (d, *J* = 1.7 Hz, 1H), 8.92 (d, *J* = 1.7 Hz, 1H), 8.19 (d, *J* = 8.3 Hz, 1H), 7.39 - 7.34 (m, 1H), 7.30 (d, *J* = 8.4 Hz, 1H), 7.15 - 7.09 (m, 2H), 4.80 - 4.53 (m, 2H), 4.43 (br t, *J* = 10.1 Hz, 1H), 4.33 (br d, *J* = 12.0 Hz, 1H), 4.21 - 4.12 (m, 2H), 3.85 (br t, *J* = 13.9 Hz, 2H), 3.73 - 3.59 (m, 2H), 3.56 - 3.47 (m, 2H), 3.38 - 3.34 (m, 2H), 3.31 - 3.27 (m, 1H), 3.11 - 3.01 (m, 1H), 2.93 - 2.79 (m, 3H), 1.42 (d, *J* = 6.6 Hz, 3H), 1.37 (d, *J* = 6.1 Hz, 3H).

### Example 67

### Methyl 4-[2-[[(2S,6R)-4-(8-cyano-5-quinolyl)-6-methyl-morpholin-2-yl]methyl]isoindolin-5-yl]piperazine-2-carboxylate

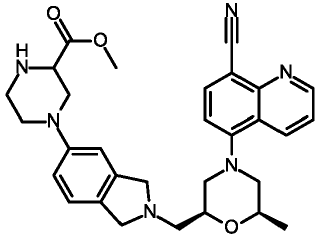

The title compound was prepared in analogy to the preparation of **Example 50** by using *O*1*-tert*-butyl *O*2-methyl piperazine-1,2-dicarboxylate instead of O1-tert-butyl *O*3-ethyl piperazine-1,3-dicarboxylate. **Example** 67 (23 mg) was obtained as a yellow powder. MS: calc'd 527 (MH⁺), measured 527 (MH⁺). ¹H NMR (400 MHz, METHANOL-d4) *δ* 9.06 - 8.89 (m, 1H), 8.71 (d, *J* = 8.5 Hz, 1H), 8.20 (d, *J* = 8.0 Hz, 1H), 7.68 (dd, *J* = 8.7, 4.3 Hz, 1H), 7.39 (br d, *J* = 8.8 Hz, 1H), 7.32 (d, *J* = 7.9 Hz, 1H), 7.18 - 7.11 (m, 2H), 4.82 - 4.60 (m, 2H), 4.50 - 4.40 (m, 2H), 4.30 - 4.19 (m, 1H), 3.93 (s, 3H), 3.90 - 3.88 (m, 1H), 3.70 - 3.56 (m, 4H), 3.52 - 3.35 (m, 6H), 3.31 - 3.28 (m, 1H), 2.88 - 2.72 (m, 2H), 1.35 (d, *J=* 6.1 Hz, 3H).

### Example 68

### 4-[2-[[(2S,6R)-4-(8-cyano-5-quinolyl)-6-methyl-morpholin-2-yl]methyl]isoindolin-5-yl]piperazine-2-carboxylic acid

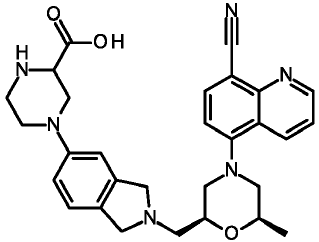

The title compound was prepared in analogy to the preparation of **Example 56** by using **Example 67** instead of **Example 50. Example 68** (13 mg) was obtained as a yellow powder. MS: calc'd 513 (MH⁺), measured 513 (MH⁺). ¹H NMR (400 MHz, METHANOL-d4) *δ* 9.00 (dd, *J =* 4.3, 1.5 Hz, 1H), 8.69 (dd, *J* = 8.5, 1.6 Hz, 1H), 8.17 (d, *J* = 8.0 Hz, 1H), 7.67 (dd, *J* = 8.5, 4.3 Hz, 1H), 7.38 (d, *J =* 8.4 Hz, 1H), 7.30 (d, *J* = 8.0 Hz, 1H), 7.17 - 7.11 (m, 2H), 4.89 - 4.60 (m, 4H), 4.50 (br t, *J* = 9.9 Hz, 1H), 4.37 (dd, *J* = 8.7, 3.6 Hz, 1H), 4.28 - 4.18 (m, 1H), 3.93 (dd, *J* = 13.4, 3.3 Hz, 1H), 3.71 - 3.56 (m, 4H), 3.48 (br d, *J* = 9.2 Hz, 4H), 3.31 - 3.23 (m, 1H) 2.88 - 2.71 (m, 2H), 1.35 (d, *J* = 6.3 Hz, 3H).

### Example 69

### 5-[(2S,6R)-2-[[4-fluoro-6-(4-methylpiperazin-1-yl)isoindolin-2-yl] methyl]-6-methyl-morpholin-4-yl]quinoline-8-carbonitrile

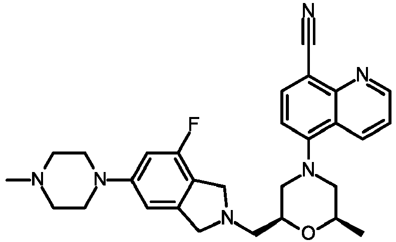

The title compound was prepared according to the following scheme:

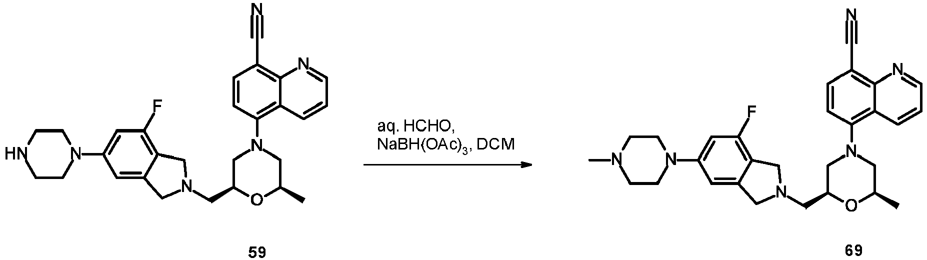

To a solution of 5-[(2*S*,6*R*)-2-[(4-fluoro-6-piperazin-1-yl-isoindolin-2-yl)methyl]-6-methyl-morpholin-4-yl]quinoline-8-carbonitrile **(Example 59,** 20 mg, 0.040 mmol) in DCM (2 mL) was added formaldehyde (0.5 mL, 0.12 mmol) and NaBH(OAc)₃ (26 mg, 0.12 mmol). The reaction mixture was stirred at rt for 2 hrs. After the solvent was removed, the residue was purified by prep-HPLC to give **Example 69** (21 mg) as a white solid. MS: calc'd 501 (MH⁺), measured 501 (MH⁺). ¹H NMR (400 MHz, METHANOL-d4) *δ* 8.98 (dd, *J* = 1.5, 4.3 Hz, 1H), 8.66 (dd, *J* = 1.8, 8.5 Hz, 1H), 8.15 (d, *J* = 8.0 Hz, 1H), 7.64 (dd, *J* = 4.3, 8.5 Hz, 1H), 7.27 (d, *J* = 8.0 Hz, 1H), 6.93 (s, 1H), 6.87 (dd, *J=* 1.6, 12.2 Hz, 1H), 4.87 - 4.75 (m, 4H), 4.54 - 4.41 (m, 1H), 4.27 - 4.16 (m, 1H), 3.93 (br d, *J =* 11.5 Hz, 2H), 3.74 - 3.54 (m, 4H), 3.44 (br t, *J* = 10.8 Hz, 2H), 3.30 - 3.04 (m, 3H), 3.30 - 3.04 (m, 1H), 2.98 (s, 3H), 2.85 - 2.66 (m, 2H), 1.33 (d, *J* = 6.3 Hz, 3H).

### Example 70

### 5-[(2R,6S)-2-methyl-6-[(6-piperazin-1-yl-3,4-dihydro-1H-2,7-naphthyridin-2-yl)methyl]morpholin-4-yl]quinoline-8-carbonitrile

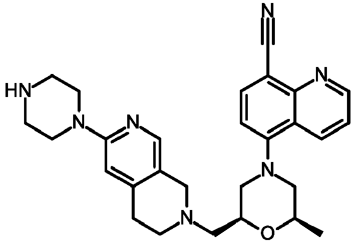

The title compound was prepared in analogy to the preparation of **Example 3** by using Intermediate **D** and 6-chloro-1,2,3,4-tetrahydro-2,7-naphthyridine hydrochloride (CAS: 1196151-85-5, Vendor: Bepharm) instead of Intermediate **A** and 4-bromoisoindoline hydrochloride. **Example 70** (19 mg) was obtained as a yellow gum. MS: calc'd 484 (MH⁺), measured 484 (MH⁺). ¹H NMR (400 MHz, METHANOL-d4) *δ* 8.99 (dd, *J* = 1.5, 4.3 Hz, 1H), 8.68 (dd, *J* = 1.5, 8.5 Hz, 1H), 8.17 (d, *J* = 8.0 Hz, 1H), 8.11 (s, 1H), 7.66 (dd, *J* = 4.3, 8.8 Hz, 1H), 7.29 (d, *J* = 8.0 Hz, 1H), 6.88 (s, 1H), 4.71 - 4.40 (m, 3H), 4.30 - 4.17 (m, 1H), 3.90 - 3.79 (m, 4H), 3.75 (br s, 2H), 3.50 - 3.39 (m, 4H), 3.34 (br d, *J* = 6.0 Hz, 4H), 3.29 - 3.15 (m, 2H), 2.84 - 2.69 (m, 2H), 1.33 (d, *J* = 6.3 Hz, 3H).

### Example 71

### 5-[(2R,6S)-2-methyl-6-[[5-[(3R,5S)-3,5-dimethylpiperazin-1-yl]isoindolin-2-yl]methyl]morpholin-4-yl]quinoline-8-carbonitrile

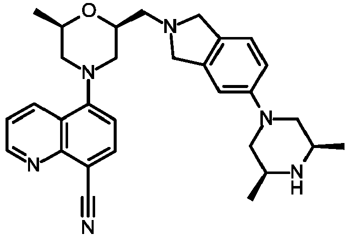

The title compound was prepared in analogy to the preparation of **Example 46** by using *tert*-butyl (2*R*,6*S*)-2,6-dimethylpiperazine-1-carboxylate instead of *tert*-butyl 3-oxa-7,9-diazabicyclo[3.3.1]nonane-7-carboxylate. **Example 71** (3.8 mg) was obtained as a light yellow powder. MS: calc'd 497 (MH⁺), measured 497 (MH⁺). ¹H NMR (400 MHz, METHANOL-d4) *δ* 8.91 (dd, *J* = 1.6, 4.3 Hz, 1H), 8.58 (dd, *J* = 1.7, 8.6 Hz, 1H), 8.08 (d, *J=* 8.1 Hz, 1H), 7.56 (dd, *J* = 4.2, 8.5 Hz, 1H), 7.25 (d, *J* = 8.7 Hz, 1H), 7.20 (d, *J* = 8.1 Hz, 1H), 7.05 - 6.99 (m, 2H), 4.75 - 4.42 (m, 2H), 4.40 - 4.31 (m, 1H), 4.15 - 4.07 (m, 1H), 3.82 (br d, *J* = 14.9 Hz, 2H), 3.58 - 3.49 (m, 2H), 3.45 - 3.33 (m, 4H), 3.27 - 3.22 (m, 2H), 2.75 - 2.67 (m, 1H), 2.66 - 2.56 (m, 3H), 1.30 (d, *J* = 6.6 Hz, 6H), 1.24 (d, *J* = 6.2 Hz, 3H).

### Example 72

### 5-[(2R,6S)-2-methyl-6-[[5-(3-oxa-7,9-diazabicyclo[3.3.1]nonan-7-yl)isoindolin-2-yl]methyl]morpholin-4-yl]quinoline-8-carbonitrile

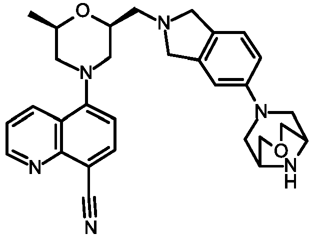

The title compound was prepared in analogy to the preparation of **Example 3** by using Intermediate **D,** 5-bromoisoindoline hydrochloride and *tert*-butyl 3-oxa-7,9-diazabicyclo[3.3.1]nonane-9-carboxylate instead of Intermediate **A,** 4-bromoisoindoline hydrochloride and *tert*-butyl piperazine-1-carboxylate. **Example 72** (3.9 mg) was obtained as a light yellow powder. MS: calc'd 511 (MH⁺), measured 511 (MH⁺). ¹H NMR (400 MHz, METHANOL-d4) *δ* 9.03 (dd, *J* = 1.6, 4.3 Hz, 1H), 8.70 (dd, *J* = 1.6, 8.6 Hz, 1H), 8.20 (d, *J =* 8.1 Hz, 1H), 7.68 (dd, *J* = 4.3, 8.7 Hz, 1H), 7.39 - 7.30 (m, 2H), 7.10 - 7.04 (m, 2H), 4.87 - 4.54 (m, 2H), 4.48 (br t, *J* = 9.5 Hz, 1H), 4.27 - 4.20 (m, 1H), 4.20 - 4.08 (m, 6H), 3.78 - 3.72 (m, 2H), 3.70 - 3.59 (m, 2H), 3.51 - 3.40 (m, 4H), 3.32 - 3.23 (m, 2H), 2.87 - 2.73 (m, 2H), 1.36 (d, *J* = 6.4 Hz, 3H).

### Example 73

### 5-[(2S,6R)-2-[[5-[2-(hydroxymethyl)piperazin-1-yl]isoindolin-2-yl]methyl]-6-methyl-morpholin-4-yl]quinoline-8-carbonitrile

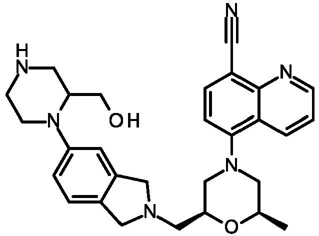

The title compound was prepared in analogy to the preparation of **Example 50** by using *tert*-butyl 3-(hydroxymethyl)piperazine-1-carboxylate instead of *O*1-*tert*-butyl *O*3-ethyl piperazine-1,3-dicarboxylate . **Example 73** (10 mg) was obtained as a yellow solid. MS: calc'd 499 (MH⁺), measured 499 (MH⁺). ¹H NMR (400 MHz, METHANOL-d4) *δ* 8.98 (dd, *J* = 1.3, 4.3 Hz, 1H), 8.67 (dd, *J* = 1.4, 8.7 Hz, 1H), 8.15 (d, *J* = 8.0 Hz, 1H), 7.64 (dd, *J* = 4.3, 8.5 Hz, 1H), 7.34 (br d, *J* = 8.8 Hz, 1H), 7.27 (d, *J* = 8.0 Hz, 1H), 7.13 - 7.05 (m, 2H), 4.86 - 4.54 (m, 4H), 4.47 (br t, *J =* 10.0 Hz, 1H), 4.25 - 4.15 (m, 1H), 4.13 - 4.05 (m, 1H), 3.84 - 3.77 (m, 1H), 3.75 - 3.55 (m, 6H), 3.51 - 3.40 (m, 4H), 3.29 - 3.22 (m, 1H), 2.84 - 2.69 (m, 2H), 1.32 (d, *J* = 6.3 Hz, 3H).

### Example 74

### 5-[(2R,6S)-2-methyl-6-[[5-(1-piperidylmethyl)isoindolin-2-yl]methyl]morpholin-4-yl]quinoline-8-carbonitrile

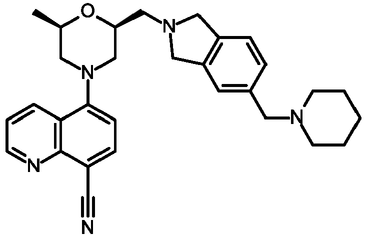

The title compound was prepared according to the following scheme:

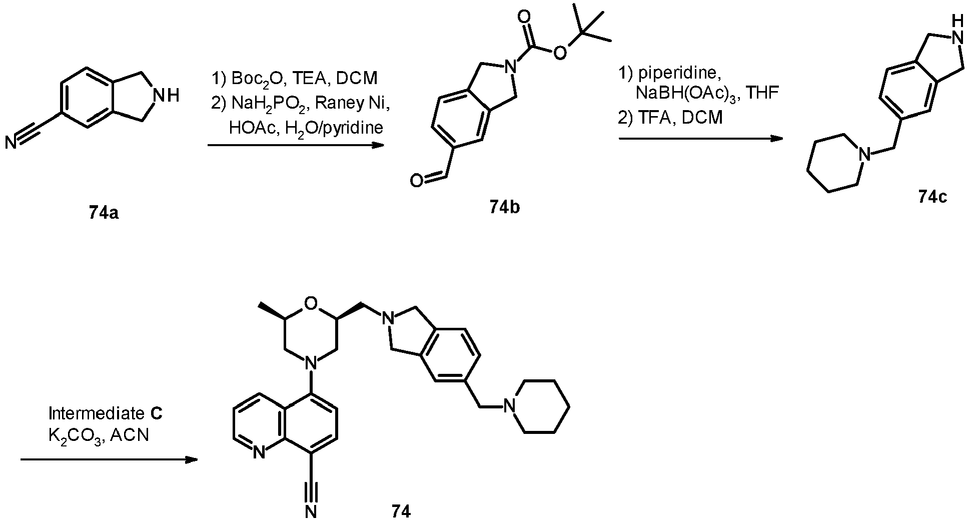

### Step 1: preparation of tert-butyl 5-formylisoindoline-2-carboxylate (compound 74b)

To a solution of isoindoline-5-carbonitrile hydrochloride (compound **74a,** 181 mg, 1.0 mmol) in DCM (4 mL) was added TEA (406 mg, 0.56 mL, 4.0 mmol). Boc-Anhydride (328 mg, 0.35 mL, 1.5 mmol) was added drop-wise at 0 °C. The reaction mixture was stirred at rt for 6 hrs, then diluted with water, and extracted with DCM. The organic layer dried over Na₂SO₄ and concentrated to give a brown oil which was purified by column chromatography to give the intermediate (222 mg) as a white solid which was dissolved in HOAc/H₂O/Pyridine (8 mL, v/v/v = 1/1/2). To the solution was added NaH₂PO₂ (560 mg, 6.4 mmol) and Raney Ni (30 mg). The reaction mixture was stirred at 60 °C overnight, then filtered through celite. The filtrate was concentrated, the residue was dissolved in water (20 mL), and the mixture was extracted with DCM. The organic layer was washed with sat NH₄Cl, dried over Na₂SO₄ and concentrated to give a yellow oil which was purified by column chromatography to give compound **74b** (124 mg) as a white solid. MS: calc'd 192 (MH⁺-56), measured 192 (MH⁺-56).

### Step 2: preparation of 5-(1-piperidylmethyl)isoindoline (compound 74c)

To a solution of *tert*-butyl 5-formylisoindoline-2-carboxylate (compound **74b,** 72 mg, 0.29 mmol) in THF (2 mL) was added piperidine (99 mg, 0.12 mL, 1.2 mmol) and NaBH(OAc)₃ (185 mg, 0.87 mmol). The reaction mixture was stirred at 40 °C for 3 hrs, then quenched with water, and the pH was adjusted to 8. The mixture was then extracted with DCM, the organic layer was washed with brine, dried over Na₂SO₄, and concentrated to give a crude product which was purified by column chromatography to give an oil (95 mg) which was dissolved in DCM (2 mL). To the solution was added TFA (1 mL). The reaction mixture was stirred at rt for 2h, then concentrated to give compound **74c** (94 mg) as an oil. MS: calc'd 217 (MH⁺), measured 217 (MH⁺).

### Step 3: preparation of 5-[(2R,6S)-2-methyl-6-[[5-(1-piperidylmethyl)isoindolin-2-yl]methyl]morpholin-4-yl]quinoline-8-carbonitrile (Example 74)

To a solution of ((2*R*,6*R*)-4-(8-cyanoquinolin-5-yl)-6-methylmorpholin-2-yl)methyl trifluoromethanesulfonate (Intermediate **D,** 30 mg, 72 µmol) and 5-(piperidin-1-ylmethyl)isoindoline trifluoroacetate (compound **74c,** 36 mg, 0.11 mmol) in ACN ( 4 mL) was added K₂CO₃ (40 mg, 0.29 mmol). The reaction mixture was stirred at 85 °C (oil bath) overnight, then filtered through celite, the filtrate was concentrated to give a brown oil which was purified by prep-HPLC to give **Example 74** (14.5 mg) as a light yellow solid. MS: calc'd 482 (MH⁺), measured 482 (MH⁺). ¹H NMR (400 MHz, METHANOL-d4) *δ* 9.02 (dd, *J* = 1.6, 4.3 Hz, 1H), 8.70 (dd, *J* = 1.7, 8.6 Hz, 1H), 8.19 (d, *J* = 7.9 Hz, 1H), 7.68 (dd, *J* = 4.3, 8.6 Hz, 1H), 7.62 - 7.56 (m, 3H), 7.32 (d, *J* = 8.1 Hz, 1H), 5.10 - 4.90 (m, 4H), 4.56 - 4.46 (m, 1H), 4.40 - 4.30 (m, 2H), 4.28 - 4.18 (m, 1H), 3.76 - 3.62 (m, 2H), 3.54 - 3.42 (m, 4H), 3.06 - 2.94 (m, 2H), 2.88 - 2.72 (m, 2H), 1.97 (br d, *J* = 13.9 Hz, 2H), 1.91 - 1.69 (m, 3H), 1.61 - 1.47 (m, 1H), 1.36 (d, *J* = 6.2 Hz, 3H).

### Example 75

### 5-[(2S,6R)-2-[(4-hydroxy-6-piperazin-1-yl-3,4-dihydro-1H-isoquinolin-2-yl)methyl]-6-methyl-morpholin-4-yl]quinoline-8-carbonitrile

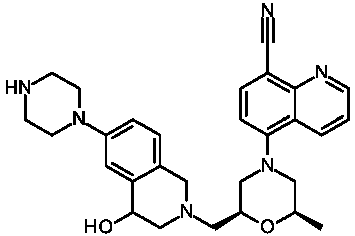

The title compound was prepared according to the following scheme:

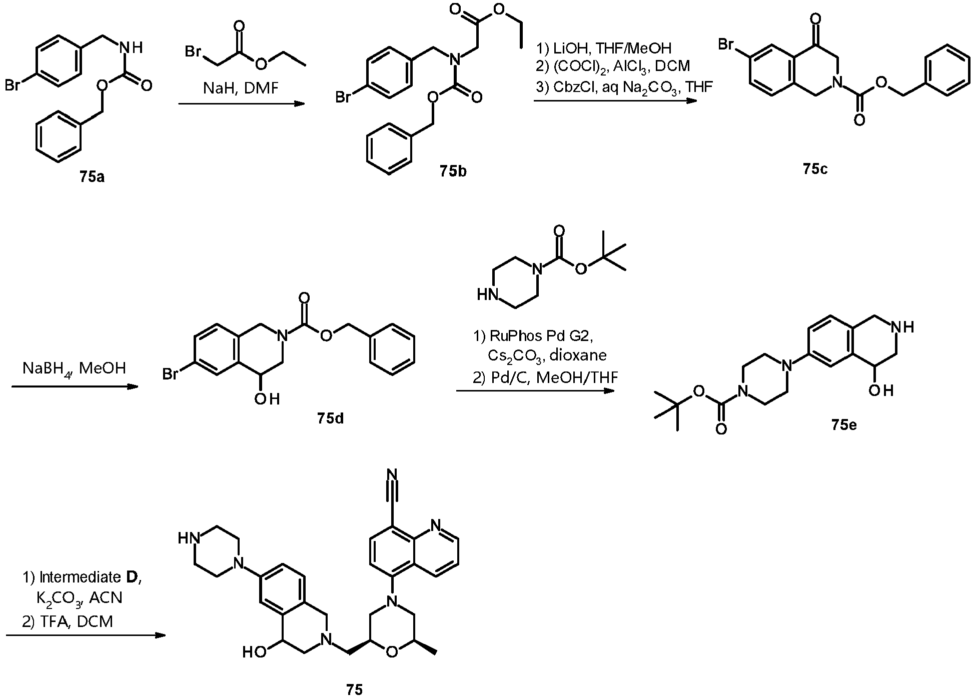

### Step 1: preparation of ethyl 2-[benzyloxycarbonyl-[(4-bromophenyl)methyl]amino]acetate (compound 75b)

To a solution of benzyl A-[(4-bromophenyl)methyl]carbamate (compound 75a, 7.0 g, 21.9 mmol) in DMF (84 mL) was added NaH (1.14 g, 28.4 mmol) at 0°C. After being stirred at 0 °C for 30 mins, ethyl bromoacetate (2.9 mL, 26.2 mmol) was added. The reaction mixture was stirred at rt for 18 hrs, then quenched with water (100 mL), and extracted with EtOAc (50 mL) twice. The combined organic layer was washed with brine, dried over anhydrous Na₂SO₄ and concentrated to give a crude product which was purified by column chromatography to give compound 75b (2.4 g) as a colorless oil. MS: calc'd 406&408 (MH⁺), measured 406&408 (MH⁺).

### Step 2: preparation of benzyl 6-bromo-4-oxo-1,3-dihydroisoquinoline-2-carboxylate (compound 75c)

To a solution of ethyl 2-[benzyloxycarbonyl-[(4-bromophenyl)methyl]amino]acetate (compound **75b,** 2.20 g, 5.4 mmol) in THF (30 mL) was added a solution of LiOH·H₂O (0.45 g, 10.8 mmol) in water (8 mL). The mixture was stirred at rt for 5 hrs, then quenched with aq. HCl, the pH was adjusted to 5. The mixture was extracted with EtOAc (20 mL) twice, the combined organic layer was washed with brine, dried over Na₂SO₄, and concentrated to give the crude intermediate (1.8 g) as a yellow gum which was dissolved in DCM (40 mL). To the solution was added thionyl chloride (0.94 g, 7.9 mmol). The reaction mixture was refluxed for 3 hrs, then AlCl₃ (2.82 g, 21.1 mmol) was added. The mixture was stirred at rt for 5 hrs, then poured into ice-water, the pH was adjusted to 8 with aq. NaHCO₃, and extracted with DCM (50 mL) twice. The combined organic layer was washed with brine, dried over Na₂SO₄ and concentrated to give a crude 6-bromo-2,3-dihydro-1*H*-isoquinolin-4-one (1.20 g) which was dissolved in THF/water (18 mL, v/v = 2/1). To the solution was added Na₂CO₃ (1.69 g, 15.9 mmol) and CbzCl (1.09 g, 6.4 mmol). The reaction mixture was stirred at rt for 18 hrs, then extracted with DCM (50 mL) three times. The combined organic layer was dried over Na₂SO₄ and concentrated. The crude product was purified by column chromatography to give compound **75c** (300 mg) as a yellow gum. MS: calc'd 360&362 (MH⁺), measured 360&362 (MH⁺).

### Step 3: preparation of benzyl 6-bromo-4-hydroxy-3,4-dihydro-1H-isoquinoline-2-carboxylate (compound 75d)

To a solution of benzyl 6-bromo-4-oxo-1,3-dihydroisoquinoline-2-carboxylate (compound **75c,** 270 mg, 0.75 mmol) in MeOH (13 mL) was added NaBH₄ (34 mg, 0.90 mmol) at 0 °C. The reaction mixture was stirred at 0 °C for 2 hrs, then concentrated. The crude product was purified by column chromatography to give compound **75d** (180 mg) as a yellow gum. MS: calc'd 362&364 (MH⁺), measured 362&364 (MH⁺).

### Step 4: preparation of tert-butyl 4-(4-hydroxy-1,2,3,4-tetrahydroisoquinolin-6-yl)piperazine-1-carboxylate (compound 75e)

To a solution of benzyl 6-bromo-4-hydroxy-3,4-dihydro-1*H*-isoquinoline-2-carboxylate (compound **75d,** 180 mg, 0.50 mmol) in dioxane (5 mL) was added *tert*-butyl piperazine-1-carboxylate (111 mg, 0.60 mmol), Cs₂CO₃ (486 mg, 1.49 mmol) and RuPhos Pd G2 (22 mg, 0.030 mmol). The mixture was stirred at 90 °C for 12 hrs under N₂. The mixture was concentrated, the crude product was purified by column chromatography to give the intermediate (50 mg) which was dissolved in MeOH/THF ( 5 mL, v/v = 1/1). To the solution was added Pd/C (10 mg, 10%). The reaction mixture was charged with H₂ and stirred at rt for 4 hrs, then filtered. The filtrate was concentrated to give compound **75e** (25 mg) as a yellow gum. MS: calc'd 334 (MH⁺), measured 334 (MH⁺).

### Step 5: preparation of 5-[(2S,6R)-2-[(4-hydroxy-6-piperazin-1-yl-3,4-dihydro-1H-isoquinolin-2-yl)methyl]-6-methyl-morpholin-4-yl]quinoline-8-carbonitrile (Example 75)

To a solution of *tert*-butyl 4-(4-hydroxy-1,2,3,4-tetrahydroisoquinolin-6-yl)piperazine-1-carboxylate (compound **75e,** 25 mg, 0.070 mmol) in ACN (3 mL) was added K₂CO₃ (17 mg, 0.12 mmol) and [(2*R*,6*R*)-4-(8-cyano-5-quinolyl)-6-methyl-morpholin-2-yl]methyl trifluoromethanesulfonate (Intermediate **D,** 20 mg, 0.050 mmol). The mixture was stirred at 50 °C for 1h, then diluted with EtOAc (10 mL). The mixture was filtered, and the filtrate was concentrated to give the intermediate (40 mg) which was dissolved in DCM (2 mL). To the solution was added TFA (1 mL). The reaction mixture was stirred at rt for 1h, then concentrated to give a crude product which was purified by prep-HPLC to afford **Example 75** (12 mg) as a yellow solid. MS: calc'd 499 (MH⁺), measured 499 (MH⁺). ¹H NMR (400 MHz, METHANOL-d4) *δ* 9.02 (d, *J=* 4.0 Hz, 1H), 8.69 - 8.67 (m, 1H), 8.20 (d, *J=* 8.4 Hz, 1H), 7.68 (d, *J=* 7.2 Hz, 1H), 7.33 - 7.32 (m, 1H), 7.31 - 7.12 (m, 3H), 5.01 - 4.98 (m, 1H), 4.58 - 4.57 (m, 3H), 4.30 - 4.25 (m, 1H), 3.50 - 3.33 (m, 14H), 2.83 - 2.80 (m, 2H), 1.34 (d, *J* = 6.0 Hz, 3H).

### Example 76

### 5-[(2S,6R)-2-[[5-[2-(difluoromethyl)piperazin-1-yl]isoindolin-2-yl]methyl]-6-methyl-morpholin-4-yl]quinoline-8-carbonitrile

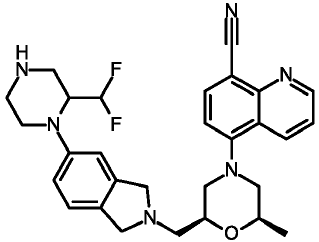

The title compound was prepared according to the following scheme:

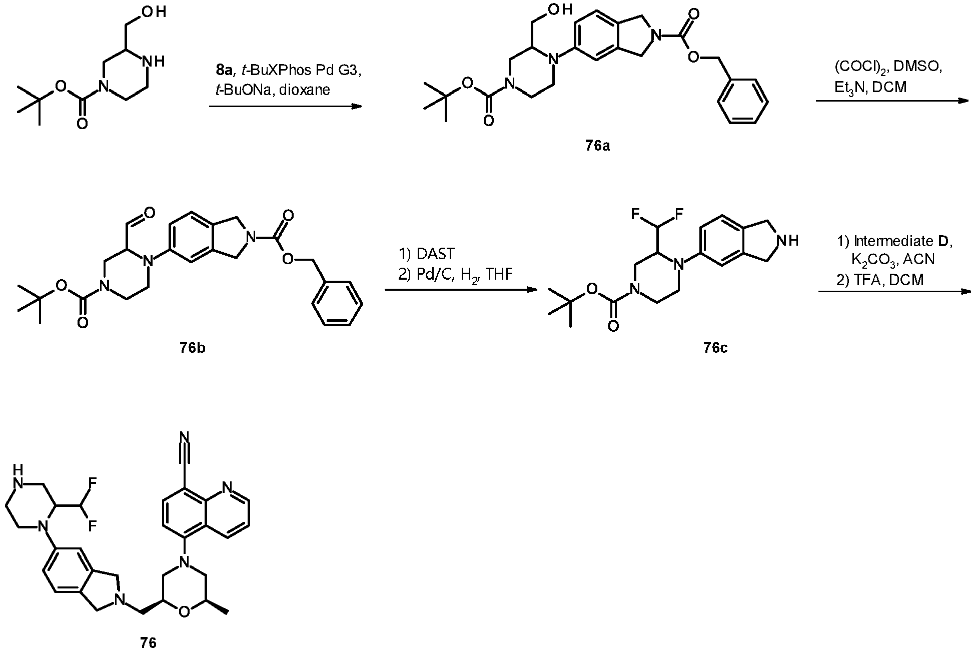

### Step 1: preparation of benzyl 5-[4-tert-butoxycarbonyl-2-(hydroxymethyl)piperazin-1-yl]isoindoline-2-carboxylate (compound 76a)

To a solution of benzyl 5-bromoisoindoline-2-carboxylate (compound **8a,** 1.0 g, 3.0 mmol) in 1,4-dioxane (20 mL) was added *tert*-butyl 3-(hydroxymethyl)piperazine-1-carboxylate (0.98 g, 4.5 mmol), t-BuONa (0.87 g, 9.0 mmol) and *t*-BuXPhos Pd G3 (239 mg, 0.30 mmol). The mixture was stirred at 100 °C for 3 hrs under N₂, then concentrated. The residue was dissolved in EtOAc (150 mL), washed with brine, dried over MgSO₄, and concentrated. The crude product was purified by column chromatography to give compound **76a** (370 mg) as a colorless gum. MS: calc'd 468 (MH⁺), measured 468 (MH⁺).

### Step 2 : preparation of benzyl 5-(4-tert-butoxycarbonyl-2-formyl-piperazin-1-yl)isoindoline-2-carboxylate (compound 76b)

To a solution of oxalyl chloride (122 mg, 0.96 mmol) in DCM (30 mL) was added DMSO (100 mg, 1.28 mmol) at -78 °C. After release of gas, the reaction mixture was stirred at - 78 °C under N₂ for 30 mins. To the solution was added benzyl 5-[4-*tert*-butoxycarbonyl-2-(hydroxymethyl)-piperazin-1-yl]isoindoline-2-carboxylate (compound **76a,** 300 mg, 0.64 mmol) in DCM (5 mL) dropwise at -78 °C. The reaction mixture was stirred for another 1 h at this temperature. To the solution was added TEA (0.45 mL, 3.21 mmol) at -78 °C, the reaction mixture was warmed to rt over 30 mins, and stirred for another 30 min, then quenched with sat NH₄Cl (40 mL). The organic layer was separated, and the aqueous phase was extracted with DCM. The combined organic layer was washed with sat NH₄Cl and brine, dried over Na₂SO₄ and concentrated. The residue was purified by column chromatography to afford compound **76b** (400 mg) as a yellow oil. MS: calc'd 466 (MH⁺), measured 466 (MH⁺).

### Step 3 : preparation of tert-butyl 3-(difluoromethyl)-4-isoindolin-5-yl-piperazine-1-carboxylate (compound 76c)

To a solution of benzyl 5-(4-*tert*-butoxycarbonyl-2-formyl-piperazin-1-yl)isoindoline-2-carboxylate (compound **76b,** 350 mg, 0.75 mmol) in DCM (30 mL) was added DAST (1.21 g, 7.5 mmol) dropwise at 0 °C. The reaction mixture was kept at this temperature and stirred for 2.5 hrs, then quenched with sat NaHCO₃ (30 mL). The mixture was extracted with DCM. The organic layer was washed with brine, dried over Na₂SO₄, and concentrated. The residue was purified by prep-TLC (EA/PE, v/v = 1/10, Rf = 0.2) to afford the intermediate (150 mg) as a yellow oil.

The above intermediate (130 mg, 0.27 mmol) was dissolved in THF (5 mL). To the solution was added Pd on carbon (50 mg, 10%). The reaction mixture was charged with hydrogen and stirred at rt for 2 hrs. The mixture was then filtered, the filtrate was concentrated to give compound **76c** (90 mg) as a colorless gum which was directly used in next step. MS: calc'd 354 (MH⁺), measured 354 (MH⁺).

### Step 4 : preparation of 5-[(2S,6R)-2-[[5-[2-(difluoromethyl)piperazin-1-yl]isoindolin-2-yl]methyl]-6-methyl-morpholin-4-yl]quinoline-8-carbonitrile (Example 76)

To a solution of *tert*-butyl 3-(difluoromethyl)-4-isoindolin-5-yl-piperazine-1-carboxylate (compound **76c,** 51 mg, 0.14 mmol) in ACN (3 mL) was added K₂CO₃ (25 mg, 0.18 mmol) and [(2*R*,6*R*)-4-(8-cyano-5-quinolyl)-6-methyl-morpholin-2-yl]methyl trifluoromethanesulfonate (Intermediate **D,** 30 mg, 0.070 mmol). The reaction mixture was stirred at 60 °C for 3 hrs. The mixture was then diluted with EtOAc (10 mL). The mixture was filtered, the filtrate was concentrated. The residue was purified by prep-TLC (EA/PE, v/v = 1/1, Rf = 0.1) to give the intermediate (20 mg) as colorless gum which was dissolved in DCM (2 mL). To the solution was added TFA (1 mL). The mixture was stirred at rt for 2 hrs, then concentrated. The residue was purified by prep-HPLC to give **Example 76** (12.6 mg) as a yellow solid. MS: calc'd 519 (MH⁺), measured 519 (MH⁺). ¹H NMR (400MHz, METHANOL-d4) *δ* 8.99 (dd, *J* = 1.8, 4.3 Hz, 1H), 8.68 (dd, *J* = 1.8, 8.5 Hz, 1H), 8.17 (d, *J* = 8.0 Hz, 1H), 7.66 (dd, *J* = 4.3, 8.5 Hz, 1H), 7.37 (d, *J* = 8.8 Hz, 1H), 7.29 (d, *J* = 8.0 Hz, 1H), 7.21 - 7.10 (m, 2H), 6.27 - 5.98 (m, 1H), 4.71 (br s, 4H), 4.58 - 4.44 (m, 2H), 4.25 - 4.16 (m, 1H), 3.77 - 3.41 (m, 9H), 3.34 - 3.32 (m, 1H), 2.84 - 2.70 (m, 2H), 1.33 (d, *J* = 6.3 Hz, 3H).

### Example 77

### 5-[(2S,6R)-2-[[5-(3-aminoazetidin-1-yl)isoindolin-2-yl] methyl]-6-methyl-morpholin-4-yl]quinoline-8-carbonitrile

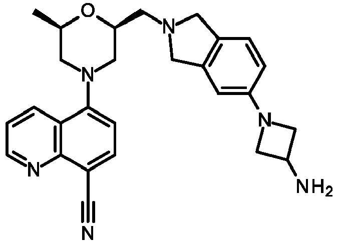

The title compound was prepared in analogy to the preparation of **Example 46** by using *tert*-butyl azetidin-3-ylcarbamate instead of *tert*-butyl 3-oxa-7,9-diazabicyclo[3.3.1]nonane-7-carboxylate. **Example 77** (5.4 mg) was obtained as a light yellow solid. MS: calc'd 455 (MH⁺), measured 455 (MH⁺). ¹H NMR (400 MHz, METHANOL-d4) *δ* 9.02 (dd, *J* = 1.7, 4.3 Hz, 1H), 8.70 (dd, *J* = 1.7, 8.6 Hz, 1H), 8.20 (d, *J* = 7.9 Hz, 1H), 7.68 (dd, *J* = 4.2, 8.5 Hz, 1H), 7.34 - 7.27 (m, 2H), 6.63 - 6.56 (m, 2H), 4.77 - 4.52 (m, 2H), 4.51 - 4.42 (m, 1H), 4.29 - 4.17 (m, 4H), 3.98 - 3.91 (m, 2H), 3.69 - 3.58 (m, 2H), 3.50 - 3.43 (m, 2H), 3.32 - 3.20 (m, 2H), 2.86 - 2.71 (m, 2H), 1.35 (d, *J* = 6.2 Hz, 3H).

### Example 78

### 5-[(2R,6S)-2-methyl-6-[[5-(2-methylpiperazin-1-yl)isoindolin-2-yl]methyl]morpholin-4-yl]quinoline-8-carbonitrile

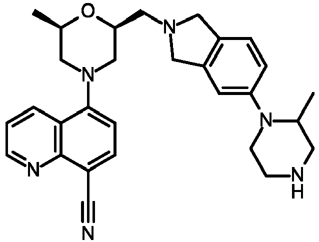

The title compound was prepared in analogy to the preparation of **Example 3** by using Intermediate **D,** 5-bromoisoindoline hydrochloride and *tert*-butyl 3-methylpiperazine-1-carboxylate instead of Intermediate **A,** 4-bromoisoindoline hydrochloride and *tert*-butyl piperazine-1-carboxylate. **Example 78** (6.2 mg) was obtained as a grey powder. MS: calc'd 483 (MH⁺), measured 483 (MH⁺). ¹H NMR (400 MHz, METHANOL-d4) *δ* 8.90 (dd, *J =* 1.7, 4.2 Hz, 1H), 8.58 (dd, *J=* 1.7, 8.6 Hz, 1H), 8.08 (d, *J* = 7.9 Hz, 1H), 7.56 (dd, *J* = 4.2, 8.6 Hz, 1H), 7.31 - 7.24 (m, 1H), 7.22 - 7.16 (m, 1H), 7.08 - 7.00 (m, 2H), 4.70 - 4.43 (m, 2H), 4.37 (br t, *J* = 9.8 Hz, 1H), 4.16 - 4.06 (m, 1H), 3.99 - 3.86 (m, 1H), 3.60 - 3.46 (m, 2H), 3.41 - 3.25 (m, 6H), 3.20 - 3.11 (m, 4H), 2.75 - 2.58 (m, 2H), 1.23 (d, *J* = 6.2 Hz, 3H), 0.99 (d, *J* = 6.7 Hz, 3H).

### Example 79

### 5-[(2R,6S)-2-methyl-6-[(2-piperazin-1-yl-7,8-dihydro-5H-1,6-naphthyridin-6-yl)methyl]morpholin-4-yl]quinoline-8-carbonitrile

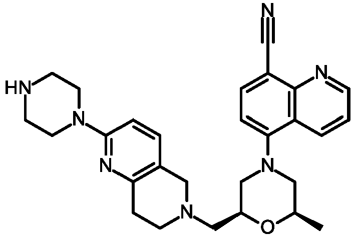

The title compound was prepared in analogy to the preparation of **Example 3** by using Intermediate **D** and 2-chloro-5,6,7,8-tetrahydro-1,6-naphthyridine (CAS: 210539-05-2, Vendor: Bepharm) instead of Intermediate **A** and 4-bromoisoindoline hydrochloride. **Example 79** (42 mg) was obtained as a yellow solid. MS: calc'd 484 (MH⁺), measured 484 (MH⁺). ¹H NMR (400 MHz, METHANOL-d4) *δ* 9.00 (dd, *J* = 1.5, 4.3 Hz, 1H), 8.69 (dd, *J* = 1.6, 8.6 Hz, 1H), 8.18 (d, *J* = 7.9 Hz, 1H), 7.67 (dd, *J* = 4.3, 8.7 Hz, 1H), 7.52 (d, *J* = 8.8 Hz, 1H), 7.30 (d, *J* = 8.0 Hz, 1H), 6.91 (d, *J* = 8.8 Hz, 1H), 4.64 - 4.40 (m, 3H), 4.29 - 4.18 (m, 1H), 4.06 - 3.64 (m, 6H), 3.56 - 3.41 (m, 4H), 3.35 (br s, 2H), 3.32 - 3.12 (m, 4H), 2.86 - 2.72 (m, 2H), 1.34 (d, *J=* 6.1 Hz, 3H).

### Example 80

### 5-[(2R,6S)-2-methyl-6-[(2-piperazin-1-yl-5,7-dihydropyrrolo[3,4-b]pyridin-6-yl)methyl]morpholin-4-yl]quinoline-8-carbonitrile

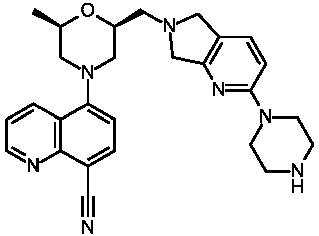

The title compound was prepared according to the following scheme:

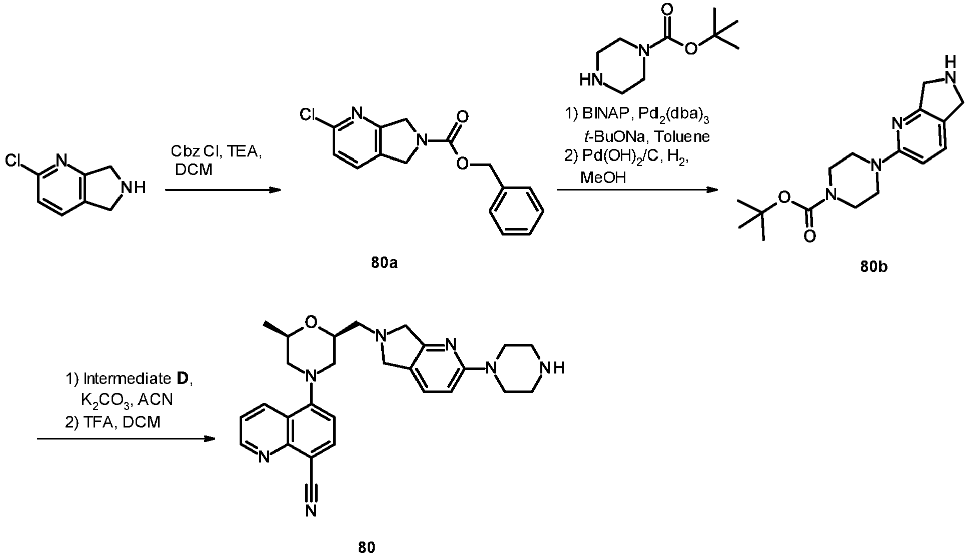

### Step 1: preparation of benzyl 2-chloro-5,7-dihydropyrrolo[3,4-b]pyridine-6-carboxylate (compound 80a)

To a solution of 2-chloro-6,7-dihydro-5//-pyrrolo[3,4-b]pyridine hydrochloride (CAS: 1841081-37-5, Vendor: Pharmablock, 181 mg, 0.95 mmol) in DCM (5 mL) was added TEA (383 mg, 0.53 mL, 3.8 mmol). To the solution was added CbzCl (242 mg, 0.20 mL, 1.4 mmol) drop-wise at 0 °C. The mixture was stirred at rt for 2h, then diluted with water and extracted with DCM, the organic layer was dried over Na₂SO₄ and concentrated. The residue was purified by column chromatography to afford compound **80a** (218 mg) as a white solid. MS: calc'd 289 (MH⁺), measured 289 (MH⁺).

### Step 2: preparation of tert-butyl 4-(6,7-dihydro-5H-pyrrolo[3,4-b]pyridin-2-yl)piperazine-1-carboxylate (compound 80b)

To a solution of benzyl 2-chloro-5,7-dihydro-6*H*-pyrrolo[3,4-b]pyridine-6-carboxylate (compound **80a,** 109 mg, 0.38 mmol) in toluene (5 mL) was added *tert*-butyl piperazine-1-carboxylate (105 mg, 0.57 mmol), and *t*-BuONa (73 mg, 0.76 mmol). The suspension was bubbled with N₂ for 5 mins, BINAP (47 mg, 75 µmol) and Pd₂(dba)₃ (35 mg, 38 µmol) was added. The mixture was heated at 90 °C overnight, then diluted with EtOAc (10 mL), and filtered through celite, the filtrate was concentrated. The residue was purified by column chromatography to give the intermediate (116 mg) as a white solid which was dissolved in DCM/MeOH (6 mL, v/v = 1/2). To the solution was added Pd(OH)₂ on carbon (20 mg, 20% wet). The suspension was charged with hydrogen balloon, and stirred at rt for 2h. The mixture was then filtered, the filtrate was concentrated to give compound **80b** (83 mg) as an oil. MS: calc'd 305 (MH⁺), measured 305 (MH⁺).

### Step 3: preparation of 5-[(2R,6S)-2-methyl-6-[(2-piperazin-1-yl-5,7-dihydropyrrolo[3,4-b]pyridin-6-yl)methyl]morpholin-4-yl]quinoline-8-carbonitrile

### (Example 80)

To a solution of ((2*R*,6*R*)-4-(8-cyanoquinolin-5-yl)-6-methylmorpholin-2-yl)methyl trifluoromethanesulfonate (Intermediate **D,** 42 mg, 0.10 mmol) in ACN (4 mL) was added *tert-*butyl 4-(6,7-dihydro-5*H*-pyrrolo[3,4-b]pyridin-2-yl)piperazine-1-carboxylate (compound **80b,** 40 mg, 0.13 mmol) and K₂CO₃ (42 mg, 0.30 mmol). The mixture was then heated at 85 °C for 3 hrs, then filtered through celite, the filtrate was concentrated to give a brown oil which was dissolved in DCM (2 mL). To the solution was added TFA (1 mL). The mixture was stirred at rt for 2 hrs, then solvent was removed. The residue was purified by prep-HPLC to afford **Example 80** (33 mg) as an orange powder. MS: calc'd 470 (MH⁺), measured 470 (MH⁺). ¹H NMR (400 MHz, METHANOL-d4) *δ* 9.02 (d, *J* = 4.2 Hz, 1H), 8.70 (d, *J* = 8.4 Hz, 1H), 8.19 (d, *J* = 8.1 Hz, 1H), 7.72 - 7.64 (m, 2H), 7.31 (d, *J* = 8.1 Hz, 1H), 6.97 (d, *J* = 8.6 Hz, 1H), 4.87 - 4.61 (m, 4H), 4.54 - 4.45 (m, 1H), 4.26 - 4.19 (m, 1H), 3.92 - 3.84 (m, 4H), 3.74 - 3.61 (m, 2H), 3.50 - 3.43 (m, 2H), 3.37 - 3.34 (m, 4H), 2.87 - 2.72 (m, 2H), 1.35 (d, *J* = 6.2 Hz, 3H).

### Example 82

### 5-[(2S,6R)-2-[[5-[(2R)-2-(hydroxymethyl)piperazin-1-yl]isoindolin-2-yl]methyl]-6-methyl-morpholin-4-yl]quinoline-8-carbonitrile

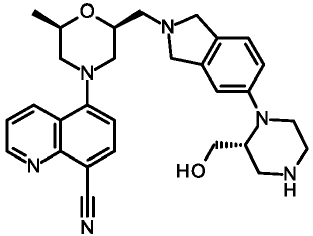

The title compound was prepared in analogy to the preparation of **Example 50** by using *tert*-butyl (3*R*)-3-(hydroxymethyl)piperazine-1-carboxylate instead of *O*1-*tert*-butyl *O*3-ethyl piperazine-1,3-dicarboxylate. **Example 82** (19.2 mg) was obtained as a light yellow powder. MS: calc'd 499 (MH⁺), measured 499 (MH⁺). ¹H NMR (400MHz, METHANOL-d₄) *δ* 8.90 (dd, *J* = 1.6, 4.3 Hz, 1H), 8.58 (dd, *J* = 1.6, 8.6 Hz, 1H), 8.08 (d, *J* = 7.9 Hz, 1H), 7.56 (dd, *J* = 4.3, 8.7 Hz, 1H), 7.25 (d, *J* = 9.2 Hz, 1H), 7.20 (d, *J* = 8.1 Hz, 1H), 7.04 - 6.95 (m, 2H), 4.69 - 4.44 (m, 2H), 4.36 (br t, *J* = 9.4 Hz, 1H), 4.18 - 4.06 (m, 1H), 4.00 (br s, 1H), 3.76 - 3.68 (m, 1H), 3.66 - 3.60 (m, 1H), 3.59 - 3.46 (m, 5H), 3.43 - 3.30 (m, 4H), 2.76 - 2.59 (m, 2H), 1.23 (d, *J*=6.2 Hz, 3H).

### Example 83

### 5-[(2S,6R)-2-[[5-[(2S)-2-(hydroxymethyl)piperazin-1-yl]isoindolin-2-yl]methyl]-6-methyl-morpholin-4-yl]quinoline-8-carbonitrile

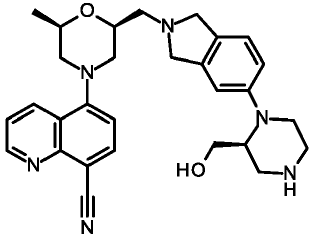

The title compound was prepared in analogy to the preparation of **Example 50** by using *tert*-butyl (3*S*)-3-(hydroxymethyl)piperazine-1-carboxylate instead of *O*1-*tert*-butyl *O*3-ethyl piperazine-1,3-dicarboxylate. **Example 83** (16.6 mg) was obtained as a light brown powder. MS: calc'd 499 (MH⁺), measured 499 (MH⁺). ¹H NMR (400MHz, METHANOL-d₄) *δ* 8.90 (dd, *J* = 1.6, 4.3 Hz, 1H), 8.58 (dd, *J* = 1.6, 8.6 Hz, 1H), 8.08 (d, *J* = 7.9 Hz, 1H), 7.56 (dd, *J* = 4.3, 8.6 Hz, 1H), 7.26 (d, *J* = 9.2 Hz, 1H), 7.20 (d, *J* = 8.1 Hz, 1H), 7.03 - 6.97 (m, 2H), 4.65 - 4.42 (m, 2H), 4.36 (br t, *J* = 8.1 Hz, 1H), 4.18 - 4.07 (m, 1H), 4.05 - 3.95 (m, 1H), 3.76 - 3.69 (m, 1H), 3.67 - 3.61 (m, 1H), 3.60 - 3.46 (m, 5H), 3.42 - 3.32 (m, 4H), 2.77 - 2.60 (m, 2H), 1.24 (d, *J* = 6.2 Hz, 3H).

### Example 84 (reference)

### 8-[(2R,6S)-2-methyl-6-[[(4-piperazin-1-ylphenyl)methylamino]methyl]morpholin-4-yl]quinoxaline-5-carbonitrile

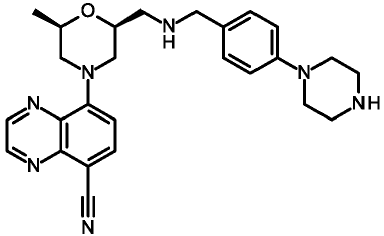

The title compound was prepared in analogy to the preparation of **Example 16** by using Intermediate **C** instead of Intermediate **A. Example 84** (13 mg) was obtained as a yellow solid. MS: calc'd 458 (MH⁺), measured 458 (MH⁺). ¹H NMR (400MHz, METHANOL-d₄) *δ* 8.96 (d, *J* = 1.8 Hz, 1H), 8.87 (d, *J=* 1.7 Hz, 1H), 8.13 (d, *J=* 8.3 Hz, 1H), 7.44 (d, *J=* 8.8 Hz, 2H), 7.23 (d, *J* = 8.3 Hz, 1H), 7.11 (d, *J=* 8.8 Hz, 2H), 4.29 - 4.19 (m, 4H), 4.18 - 4.01 (m, 2H), 3.50 - 3.45 (m, 4H), 3.40 - 3.35 (m, 4H), 3.24 (dd, *J=* 2.8, 12.9 Hz, 1H), 3.15 - 3.05 (m, 1H), 2.80 (ddd, *J=* 3.2, 10.5, 12.3 Hz, 2H), 1.31 (d, *J* = 6.1 Hz, 3H).

### Example 85

### 5-[(2S,6R)-2-[[5-[3-(hydroxymethyl)piperazin-1-yl]isoindolin-2-yl]methyl]-6-methyl-morpholin-4-yl]quinoline-8-carbonitrile

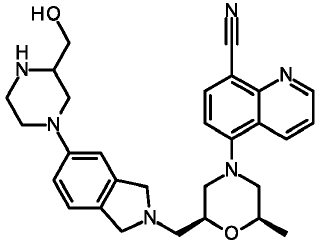

The title compound was prepared in analogy to the preparation of **Example 50** by using tert-butyl 2-(hydroxymethyl)piperazine-1-carboxylate instead of *O*1-*tert*-butyl *O*3-ethyl piperazine-1,3-dicarboxylate. **Example 85** (22.6 mg) was obtained as a yellow solid. MS: calc'd 484 (MH⁺), measured 484 (MH⁺). ¹H NMR (400 MHz, METHANOL-d4) *δ* 9.02 (dd, *J* = 4.3, 1.6 Hz, 1H), 8.71 (dd, *J* = 8.6, 1.7 Hz, 1H), 8.20 (d, *J* = 7.9 Hz, 1H), 7.69 (dd, *J* = 8.7, 4.3 Hz, 1H), 7.36 (d, *J* = 8.9 Hz, 1H), 7.32 (d, *J* = 8.0 Hz, 1H), 7.15 - 7.09 (m, 2H), 4.71 - 4.55 (m, 4H), 4.49 - 4.43 (m, 1H), 4.27 - 4.18 (m, 1H), 3.93 - 3.72 (m, 4H), 3.62 - 3.55 (m, 1H), 3.55 - 3.45 (m, 5H), 3.14 - 2.94 (m, 3H), 2.87 - 2.72 (m, 2H), 1.35 (d, *J* = 6.3 Hz, 3H).

### Example 86

### 5-[(2R,6S)-2-methyl-6-[[(4-piperazin-1-ylphenyl)methylamino]methyl]morpholin-4-yl]quinoline-8-carbonitrile

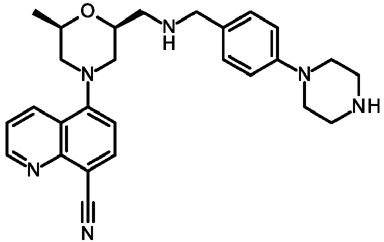

The title compound was prepared in analogy to the preparation of **Example 16** by using Intermediate **D** instead of Intermediate **A. Example 86** (13 mg) was obtained as a yellow solid. MS: calc'd 457 (MH⁺), measured 457 (MH⁺). ¹H NMR (400 MHz, METHANOL-d4) *δ* 9.18 - 8.98 (m, 2H), 8.35 (d, *J* = 8.2 Hz, 1H), 7.94 (dd, *J* = 4.8, 8.6 Hz, 1H), 7.59 - 7.39 (m, 3H), 7.15 (d, *J* = 8.7 Hz, 2H), 4.47 - 4.12 (m, 4H), 3.63 - 3.46 (m, 6H), 3.23 (m, 5H), 3.12 (dd, *J* = 9.8, 12.9 Hz, 1H), 2.96 - 2.67 (m, 2H), 1.33 (d, *J* = 6.2 Hz, 3H).

### Example 87

### 5-[(2R,6S)-2-methyl-6-[[5-(morpholinomethyl)isoindolin-2-yl]methyl]morpholin-4-yl]quinoline-8-carbonitrile

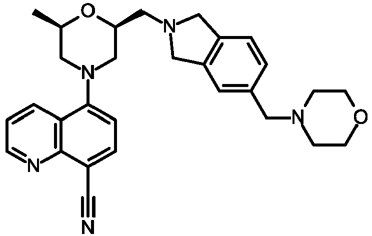

The title compound was prepared in analogy to the preparation of **Example 74** by using morpholine instead of piperidine. **Example 87** (23 mg) was obtained as a light yellow powder. MS: calc'd 484 (MH⁺), measured 484 (MH⁺). ¹H NMR (400 MHz, METHANOL-d4) *δ* 9.02 (dd, *J* = 1.6, 4.2 Hz, 1H), 8.70 (dd, *J* = 1.7, 8.6 Hz, 1H), 8.19 (d, *J* = 7.9 Hz, 1H), 7.68 (dd, *J* = 4.3, 8.7 Hz, 1H), 7.64 - 7.57 (m, 3H), 7.32 (d, *J* = 8.1 Hz, 1H), 5.16 - 4.90 (m, 4H), 4.55 - 4.46 (m, 1H), 4.41 (s, 2H), 4.29 - 4.19 (m, 1H), 3.92 (br s, 4H), 3.74 - 3.62 (m, 2H), 3.52 - 3.43 (m, 2H), 3.32 - 3.19 (m, 4H), 2.87 - 2.73 (m, 2H), 1.36 (d, *J* = 6.2 Hz, 3H).

### Example 88

The following tests were carried out in order to determine the activity of the compounds of formula (I) or (Ia) in HEK293-Blue-hTLR-7/8/9 cells assay.

### HEK293-Blue-hTLR-7 cells assay:

A stable HEK293-Blue-hTLR-7 cell line was purchased from InvivoGen (Cat.#: hkb-htlr7, San Diego, California, USA). These cells were originally designed for studying the stimulation of human TLR7 by monitoring the activation of NF-κB. A SEAP (secreted embryonic alkaline phosphatase) reporter gene was placed under the control of the IFN-β minimal promoter fused to five NF-xB and AP-1-binding sites. The SEAP was induced by activating NF-κB and AP-1 *via* stimulating HEK-Blue hTLR7 cells with TLR7 ligands. Therefore the reporter expression was declined by TLR7 antagonist under the stimulation of a ligand, such as R848 (Resiquimod), for incubation of 20 hrs. The cell culture supernatant SEAP reporter activity was determined using QUANTI-Blue^{™} kit (Cat.#: rep-qb1, Invivogen, San Diego, Ca, USA) at a wavelength of 640 nm, a detection medium that turns purple or blue in the presence of alkaline phosphatase.

HEK293-Blue-hTLR7 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 20uM R848 in above DMEM, perform incubation under 37 °C in a CO₂ incubator for 20 hrs. Then 20 µL of the supernatant from each well was incubated with 180 µL Quanti-blue substrate solution at 37 °C for 2 hrs and the absorbance was read at 620-655 nm using a spectrophotometer. The signalling pathway that TLR7 activation leads to downstream NF-κB activation has been widely accepted, and therefore similar reporter assay was modified for evaluating TLR7 antagonist.

### HEK293-Blue-hTLR-8 cells assay:

A stable HEK293-Blue-hTLR-8 cell line was purchased from InvivoGen (Cat.#: hkb-htlr8, San Diego, California, USA). These cells were originally designed for studying the stimulation of human TLR8 by monitoring the activation of NF-xB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR8 cells with TLR8 ligands. Therefore the reporter expression was declined by TLR8 antagonist under the stimulation of a ligand, such as R848, for incubation of 20 hrs. The cell culture supernatant SEAP reporter activity was determined using QUANTI-Blue^{™} kit (Cat.#: rep-qb1, Invivogen, San Diego, Ca, USA) at a wavelength of 640 nm, a detection medium that turns purple or blue in the presence of alkaline phosphatase.

HEK293-Blue-hTLR8 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 60uM R848 in above DMEM, perform incubation under 37 °C in a CO₂ incubator for 20 hrs. Then 20 µL of the supernatant from each well was incubated with 180 µL Quanti-blue substrate solution at 37°C for 2 hrs and the absorbance was read at 620-655 nm using a spectrophotometer. The signalling pathway that TLR8 activation leads to downstream NF-κB activation has been widely accepted, and therefore similar reporter assay was modified for evaluating TLR8 antagonist.

### HEK293-Blue-hTLR-9 cells assay:

A stable HEK293-Blue-hTLR-9 cell line was purchased from InvivoGen (Cat.#: hkb-htlr9, San Diego, California, USA). These cells were originally designed for studying the stimulation of human TLR9 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-xB and AP-1 *via* stimulating HEK-Blue hTLR9 cells with TLR9 ligands. Therefore the reporter expression was declined by TLR9 antagonist under the stimulation of a ligand, such as ODN2006 (Cat.#: tlrl-2006-1, Invivogen, San Diego, California, USA), for incubation of 20 hrs. The cell culture supernatant SEAP reporter activity was determined using QUANTI-Blue^{™} kit (Cat.#: rep-qb1, Invivogen, San Diego, California, USA) at a wavelength of 640 nm, a detection medium that turns purple or blue in the presence of alkaline phosphatase.

HEK293-Blue-hTLR9 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 20uM ODN2006 in above DMEM, perform incubation under 37 °C in a CO₂ incubator for 20 hrs. Then 20 µL of the supernatant from each well was incubated with 180 µL Quanti-blue substrate solution at 37 °C for 2 hrs and the absorbance was read at 620-655 nm using a spectrophotometer. The signalling pathway that TLR9 activation leads to downstream NF-κB activation has been widely accepted, and therefore similar reporter assay was modified for evaluating TLR9 antagonist.

The compounds of formula (I) or (Ia) have human TLR7 and/or TLR8 inhibitory activities (IC₅₀ value) <0.5 µM. Moreover, some compounds also have human TLR9 inhibitory activity <1 µM. Activity data of the compounds of the present invention were shown in Table 2.

**Table 2: The activity of the compounds of present invention in HEK293-Blue-hTLR-7/8/9 cells assays**

| Example No | HEK/hTLR7 IC₅₀ (µM) | HEK/hTLR8 IC₅₀ (µM) | HEK/hTLR9 IC₅₀ (µM) |
|---|---|---|---|
| **ER-886509 (reference compound)** | 0.088 | 0.139 | 2.08 |
| **R1 (reference compound)** | 0.55 | 0.118 | 50.6 |
| **R2 (reference compound)** | 0.722 | 0.784 | 47.0 |
| **R3 (reference compound)** | 0.186 | 0.146 | 5.54 |
| **4** | 0.056 | 0.057 | 0.055 |
| **7** | 0.042 | 0.013 | 0.091 |
| **8** | 0.206 | 0.079 | 0.062 |
| **9** | 0.195 | 0.09 | 0.064 |
| **10** | 0.081 | 0.052 | 0.067 |
| **11** | 0.192 | 0.229 | 0.06 |
| **12** | <0.003^{∗} | <0.003 | 0.052 |
| **13** | 0.003 | <0.003 | 0.054 |
| **14** | 0.021 | 0.029 | 0.117 |
| **15** | 0.037 | 0.021 | 0.046 |
| **16** | 0.104 | 0.016 | 0.082 |
| **17** | 0.065 | 0.052 | 0.048 |
| **18** | 0.068 | 0.031 | 0.048 |
| **19** | 0.011 | 0.006 | 0.064 |
| **20** | 0.014 | 0.004 | 0.048 |
| **21** | 0.009 | 0.005 | 0.055 |
| **23** | 0.006 | <0.003 | 0.075 |
| **24** | 0.009 | 0.004 | 0.081 |
| **25** | 0.011 | <0.003 | 0.063 |
| **28** | 0.227 | 0.058 | 0.093 |
| **29** | 0.049 | 0.014 | 0.134 |
| **30** | 0.016 | 0.003 | 0.136 |
| **31** | 0.029 | 0.013 | 0.168 |
| **32** | 0.039 | 0.017 | 0.123 |
| **33** | 0.009 | 0.005 | 0.174 |
| **34** | 0.023 | 0.005 | 0.125 |
| **35** | 0.041 | 0.009 | 0.216 |
| **36** | 0.01 | <0.003 | 0.057 |
| **37** | 0.05 | 0.009 | 0.26 |
| **38** | 0.023 | 0.005 | 0.227 |
| **40** | 0.033 | 0.004 | 0.122 |
| **41** | 0.059 | 0.009 | 0.227 |
| **42** | 0.062 | 0.013 | 0.097 |
| **43** | 0.06 | 0.012 | 0.088 |
| **46** | 0.074 | 0.019 | 0.075 |
| **47** | 0.084 | 0.023 | 0.083 |
| **48** | 0.238 | 0.018 | 0.105 |
| **49** | 0.05 | 0.014 | 0.164 |
| **50** | 0.085 | 0.033 | 0.259 |
| **55** | 0.08 | 0.007 | 0.084 |
| **57** | 0.125 | 0.012 | 0.098 |
| **59** | 0.018 | <0.003 | 0.066 |
| **60** | 0.011 | <0.003 | 0.046 |
| **61** | 0.023 | <0.003 | 0.059 |
| **62** | 0.026 | 0.005 | 0.062 |
| **63** | 0.013 | <0.003 | 0.12 |
| **64** | 0.04 | 0.006 | 0.075 |
| **65** | 0.008 | <0.003 | 0.059 |
| **66** | 0.006 | <0.003 | 0.083 |
| **67** | 0.056 | 0.036 | 0.221 |
| **69** | 0.043 | <0.003 | 0.081 |
| **70** | 0.014 | <0.003 | <0.032^{∗∗} |
| **71** | 0.108 | 0.006 | 0.073 |
| **72** | 0.045 | 0.009 | 0.066 |
| **73** | 0.048 | 0.01 | 0.06 |
| **74** | 0.059 | <0.003 | 0.073 |
| **75** | 0.042 | 0.004 | 0.055 |
| **76** | 0.009 | 0.004 | 0.19 |
| **77** | 0.032 | 0.008 | 0.239 |
| **78** | 0.011 | <0.003 | 0.163 |
| **79** | 0.015 | 0.004 | <0.032 |
| **80** | 0.007 | <0.003 | 0.056 |
| **82** | 0.047 | 0.014 | 0.054 |
| **83** | 0.034 | 0.020 | 0.063 |
| **84** | 0.012 | 0.022 | 0.050 |
| **85** | 0.02 | 0.017 | 0.051 |
| **86** | 0.015 | 0.026 | <0.032 |

| | | | |
|---|---|---|---|
| * 0.003 is the lowest tested concentration for TLR7 and TLR8 ** 0.032 is the lowest tested concentration for TLR9 | | | |

### Example 89

### Human microsome stability assay

The human microsomal stability assay is used for early assessment of metabolic stability of a test compound in human liver microsomes.

Human liver microsomes (Cat.NO.: 452117, Corning, USA;Cat.NO.:H2610, Xenotech, USA) were preincubated with test compound for 10 minutes at 37°C in 100 mM potassium phosphate buffer, pH 7.4. The reactions were initiated by adding NADPH regenerating system. The final incubation mixtures contained 1 µM test compound, 0.5 mg/mL liver microsomal protein, 1 mM MgClz, 1 mM NADP, 1 unit/mL isocitric dehydrogenase and 6 mM isocitric acid in 100 mM potassium phosphate buffer, pH 7.4. After incubation times of 0, 3, 6, 9, 15 and 30 minutes at 37°C, 300 µL of cold acetonitrile (including internal standard) was added to 100 µL incubation mixture to terminate the reaction. Following precipitation and centrifugation, the amount of compound remaining in the samples were determined by LC-MS/MS. Controls of no NADPH regenerating system at zero and 30 minutes were also prepared and analyzed. The compounds of present invention showed good human liver microsome stability determined in the above assay, results are shown in Table 3 below.

**Table 3. Human liver microsome stability of the compounds of present invention**

| **Example No** | **Clearance of Human microsome (mL/min/kg)** |
|---|---|
| **R3 (reference compound)** | 15.4 |
| **ER-886509 (reference compound)** | 15.2 |
| **16** | 7.31 |
| **19** | 6.17 |
| **21** | 6.15 |
| **44** | 6.94 |
| **55** | 7.09 |
| **56** | 6.51 |
| **62** | 6.39 |

## Claims

1. A compound of formula (I), 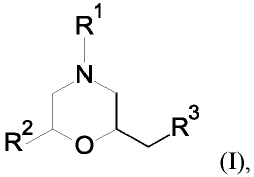 wherein
R¹ is 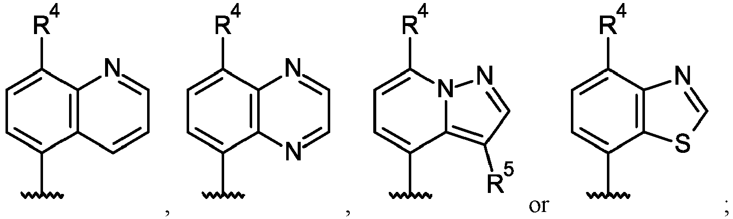 wherein R⁴ is cyano or halogen; R⁵ is H or halogen;
R² is C₁₋₆alkyl;
R³ is
1,3-dihydropyrrolo[3,4-c]pyridinyl substituted by piperazinyl;
3,4-dihydro-1H-2,7-naphthyridinyl substituted by piperazinyl;
3,4-dihydro-1H-isoquinolinyl substituted by one or two substituents independently selected from hydroxy, piperazinyl and C₁₋₆alkylpiperazinyl;
3,4-dihydro-2H-quinolinyl substituted by piperazinyl;
5,7-dihydropyrrolo[3,4-b]pyridinyl substituted by piperazinyl;
5,7-dihydropyrrolo[3,4-d]pyrimidinyl substituted by piperazinyl or C₁₋₆alkylpiperazinyl;
6,7-dihydro-4H-thiazolo[5,4-c]pyridinyl substituted by C₁₋₆alkylpiperazinyl;
7,8-dihydro-SH-1,6-naphthyridinyl substituted by piperazinyl;
isoindolinyl substituted by one or two substituents independently selected from ((C₁₋₆alkyl)₂amino)C₁₋₆alkoxy; (C₁₋₆alkoxyC₁₋₆alkyl)piperazinyl; (C₁-₆alkyl)₂piperazinyl; (C₁₋₆alkyl)₂pyridinyl; (hydroxyC₁₋₆alkyl)piperazinyl; 1,3,4,6,7,8,9,9a-octahydropyrido[1,2-a]pyrazinyl; 1,6-diazaspiro[3.3]heptanyl; 2,6-diazaspiro[3.3]heptanyl; 3,4,6,7,9,9a-hexahydro-1H-pyrazino[2,1-c][1,4]oxazinyl; 3,8-diazabicyclo[3.2.1]octanyl; 3-oxa-7,9-diazabicyclo[3.3.1]nonanyl; 4,7-diazaspiro[2.5]octanyl; aminoazetidinyl; aminoC₁₋₆alkylamino; C₁₋₆alkoxycarbonylpiperazinyl; C₁₋₆alkyl; C₁₋₆alkylimidazolyl; C₁₋₆alkylpiperazinyl; C₃₋₇cycloalkylpiperazinyl; carboxypiperazinyl; haloC₁₋₆alkylpiperazinyl; halogen; morpholinylC₁₋₆alkyl; oxopiperazinyl; piperazinyl; piperidinyl; piperidinylC₁₋₆alkyl; pyrrolidinyl and pyrrolidinyloxy; or
-NR^{6a}R^{6b}; wherein
R^{6a} is H or C₁₋₆alkyl;
R^{6b} is phenylC₁₋₆alkyl substituted by piperazinyl;
or a pharmaceutically acceptable salt, enantiomer or diastereomer thereof.

2. A compound according to claim 1 of formula (Ia), 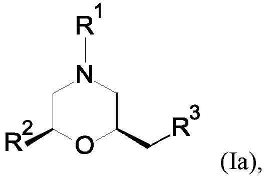 wherein R¹, R² and R³ are as described in claim 1.

3. A compound according to claim 1 or 2, wherein
R¹ is 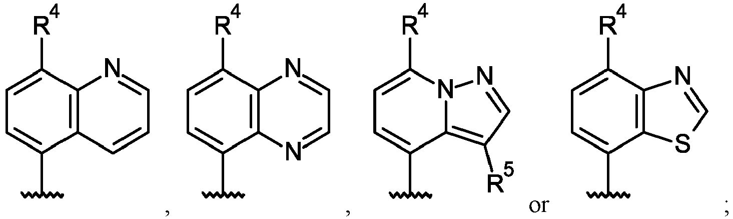 wherein R⁴ is cyano or chloro; R⁵ is H or fluoro;
R² is methyl;
R³ is piperazinyl-1,3-dihydropyrrolo[3,4-c]pyridinyl; piperazinyl-3,4-dihydro-1H-2,7-naphthyridinyl; piperazinyl-3,4-dihydro-1H-isoquinolinyl; methylpiperazinyl-3,4-dihydro-1H-isoquinolinyl; hydroxy(piperazinyl)-3,4-dihydro-1H-isoquinolinyl; piperazinyl-3,4-dihydro-2H-quinolinyl; piperazinyl-5,7-dihydropyrrolo[3,4-b]pyridinyl; piperazinyl-5,7-dihydropyrrolo[3,4-d]pyrimidinyl; methylpiperazinyl-5,7-dihydropyrrolo[3,4-d]pyrimidinyl; methylpiperazinyl-6,7-dihydro-4H-thiazolo[5,4-c]pyridinyl; piperazinyl-7 ,8-dihydro-5H -1, 6-naphthyridinyl; (difluoromethyl)piperazinylisoindolinyl; (dimethylamino)ethoxyisoindolinyl; (hydroxymethyl)piperazinylisoindolinyl; (methoxyethyl)piperazinylisoindolinyl; 1,3,4,6,7,8,9,9a-octahydropyrido[1,2-a]pyrazinylisoindolinyl; 1,6-diazaspiro[3.3]heptanylisoindolinyl; 2,6-diazaspiro[3.3]heptanylisoindolinyl; 3,4,6,7,9,9a-hexahydro-1H-pyrazino[2,1-c][1,4]oxazinylisoindolinyl; 3,8-diazabicyclo[3.2.1]octanylisoindolinyl; 3-oxa-7,9-diazabicyclo[3.3.1]nonanylisoindolinyl; 4,7-diazaspiro[2.5]octanylisoindolinyl; aminoazetidinylisoindolinyl; aminoethylaminoisoindolinyl; carboxypiperazinylisoindolinyl; cyclopropylpiperazinylisoindolinyl; dimethylpiperazinylisoindolinyl; dimethylpyridinylisoindolinyl; ethoxycarbonylpiperazinylisoindolinyl; methoxycarbonylpiperazinylisoindolinyl; methylimidazolylisoindolinyl; methylpiperazinyl(fluoro)isoindolinyl; methylpiperazinylisoindolinyl; morpholinylmethylisoindolinyl; oxopiperazinylisoindolinyl; piperazinyl(fluoro)isoindolinyl; piperazinyl(methyl)isoindolinyl; piperazinylisoindolinyl; piperidinylisoindolinyl; piperidinylmethylisoindolinyl; pyrrolidinylisoindolinyl; pyrrolidinyloxyisoindolinyl; (piperazinylphenyl)methylamino or (piperazinylphenyl)methyl(methyl)amino;
or a pharmaceutically acceptable salt, enantiomer or diastereomer thereof.

4. A compound according to claim 1 or 2, wherein R¹ is

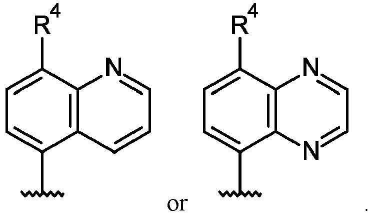

5. A compound according to claim 4, wherein R⁴ is cyano or chloro.

6. A compound according to claim 4, wherein
R³
is 1,3-dihydropyrrolo[3,4-c]pyridinyl substituted by piperazinyl;
3,4-dihydro-1H-2,7-naphthyridinyl substituted by piperazinyl;
3,4-dihydro-1H-isoquinolinyl substituted by one or two substituents independently selected from hydroxy, piperazinyl and C₁₋₆alkylpiperazinyl;
3,4-dihydro-2H-quinolinyl substituted by piperazinyl;
5,7-dihydropyrrolo[3,4-b]pyridinyl substituted by piperazinyl;
5,7-dihydropyrrolo[3,4-d]pyrimidinyl substituted by piperazinyl or C₁₋₆alkylpiperazinyl;
7,8-dihydro-5H-1,6-naphthyridinyl substituted by piperazinyl;
isoindolinyl substituted by (C₁₋₆alkoxyC₁₋₆alkyl)piperazinyl, (C₁₋₆alkyl)₂piperazinyl, (hydroxyC₁₋₆alkyl)piperazinyl, C₁₋₆alkylpiperazinyl, C₃₋₇cycloalkylpiperazinyl, haloC₁₋₆alkylpiperazinyl, morpholinylC₁₋₆alkyl, piperazinyl, piperidinyl, piperidinylC₁₋₆alkyl or pyrrolidinyl; or
-NR^{6a}R^{6b}; wherein
R^{6a} is H;
R^{6b} is phenylC₁₋₆alkyl substituted by piperazinyl.

7. A compound according to claim 6, wherein R³ is piperazinyl-1,3-dihydropyrrolo[3,4-c]pyridinyl; piperazinyl-3,4-dihydro-1H-2,7-naphthyridinyl; piperazinyl-3,4-dihydro-1H-isoquinolinyl; methylpiperazinyl-3,4-dihydro-1H-isoquinolinyl; hydroxy(piperazinyl)-3,4-dihydro-1H-isoquinolinyl; piperazinyl-3,4-dihydro-2H-quinolinyl; piperazinyl-5,7-dihydropyrrolo[3,4-b]pyridinyl; piperazinyl-5,7-dihydropyrrolo[3,4-d]pyrimidinyl; methylpiperazinyl-5,7-dihydropyrrolo[3,4-d]pyrimidinyl; piperazinyl-7,8-dihydro-5H-1,6-naphthyridinyl; (difluoromethyl)piperazinylisoindolinyl; (hydroxymethyl)piperazinylisoindolinyl; (methoxyethyl)piperazinylisoindolinyl; cyclopropylpiperazinylisoindolinyl; dimethylpiperazinylisoindolinyl; methylpiperazinylisoindolinyl; morpholinylmethylisoindolinyl; piperazinylisoindolinyl; piperidinylisoindolinyl; piperidinylmethylisoindolinyl; pyrrolidinylisoindolinyl; or (piperazinylphenyl)methylamino.

8. A compound according to claim 1 or 2, wherein
R¹ is 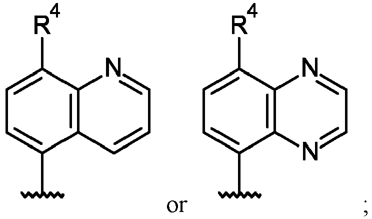 wherein R⁴ is cyano or halogen;
R² is C₁₋₆alkyl;
R³ is
1,3-dihydropyrrolo[3,4-c]pyridinyl substituted by piperazinyl;
3,4-dihydro-1H-2,7-naphthyridinyl substituted by piperazinyl;
3,4-dihydro-1H-isoquinolinyl substituted by one or two substituents independently selected from hydroxy, piperazinyl and C₁₋₆alkylpiperazinyl;
3,4-dihydro-2H-quinolinyl substituted by piperazinyl;
5,7-dihydropyrrolo[3,4-b]pyridinyl substituted by piperazinyl;
5,7-dihydropyrrolo[3,4-d]pyrimidinyl substituted by piperazinyl or C₁₋₆alkylpiperazinyl;
7,8-dihydro-5H-1,6-naphthyridinyl substituted by piperazinyl;
isoindolinyl substituted by (C₁₋₆alkoxyC₁₋₆alkyl)piperazinyl, (C₁₋₆alkyl)₂piperazinyl, (hydroxyC₁₋₆alkyl)piperazinyl, C₁₋₆alkylpiperazinyl, C₃₋₇cycloalkylpiperazinyl, haloC₁₋₆alkylpiperazinyl, morpholinylC₁₋₆alkyl, piperazinyl, piperidinyl, piperidinylC₁₋₆alkyl or pyrrolidinyl; or
-NR^{6a}R^{6b}; wherein
R^{6a} is H;
R^{6b} is phenylC₁₋₆alkyl substituted by piperazinyl;
or a pharmaceutically acceptable salt, enantiomer or diastereomer thereof.

9. A compound according to claim 8, wherein
R¹ is 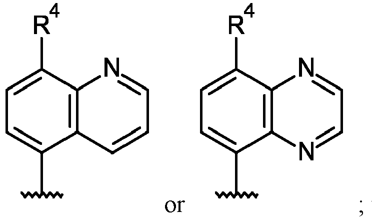 wherein R⁴ is cyano or chloro;
R² is methyl;
R³ is piperazinyl-1,3-dihydropyrrolo[3,4-c]pyridinyl; piperazinyl-3,4-dihydro-1H-2,7-naphthyridinyl; piperazinyl-3,4-dihydro-1H-isoquinolinyl; methylpiperazinyl-3,4-dihydro-1H-isoquinolinyl; hydroxy(piperazinyl)-3,4-dihydro-1H-isoquinolinyl; piperazinyl-3,4-dihydro-2H-quinolinyl; piperazinyl-5,7-dihydropyrrolo[3,4-b]pyridinyl; piperazinyl-5,7-dihydropyrrolo[3,4-d]pyrimidinyl; methylpiperazinyl-5,7-dihydropyrrolo[3,4-d]pyrimidinyl; piperazinyl-7,8-dihydro-5H-1,6-naphthyridinyl; (difluoromethyl)piperazinylisoindolinyl; (hydroxymethyl)piperazinylisoindolinyl; (methoxyethyl)piperazinylisoindolinyl; cyclopropylpiperazinylisoindolinyl; dimethylpiperazinylisoindolinyl; methylpiperazinylisoindolinyl; morpholinylmethylisoindolinyl; piperazinylisoindolinyl; piperidinylisoindolinyl; piperidinylmethylisoindolinyl; pyrrolidinylisoindolinyl; or (piperazinylphenyl)methylamino;
or a pharmaceutically acceptable salt, enantiomer or diastereomer thereof.

10. A compound according to claim 1 or 2, selected from:
4-[(2*R*,6*S*)-2-methyl-6-[(4-piperazin-1-ylisoindolin-2-yl)methyl]morpholin-4-yl]pyrazolo[1,5-a]pyridine-7-carbonitrile;
4-[(2*R*,6*S*)-2-methyl-6-[(5-piperazin-1-ylisoindolin-2-yl)methyl]morpholin-4-yl]pyrazolo[1,5-a]pyridine-7-carbonitrile;
4-[(2*R*,6*S*)-2-methyl-6-[(8-piperazin-1-yl-3,4-dihydro-1*H*-isoquinolin-2-yl)methyl]morpholin-4-yl]pyrazolo[1,5-a]pyridine-7-carbonitrile;
4-[(2*R*,6*S*)-2-methyl-6-[(7-piperazin-1-yl-3,4-dihydro-2*H*-quinolin-1-yl)methyl]morpholin-4-yl]pyrazolo[1,5-a]pyridine-7-carbonitrile;
3-fluoro-4-[(2*R*,6*S*)-2-methyl-6-[(5-piperazin-1-ylisoindolin-2-yl)methyl]morpholin-4-yl]pyrazolo[1,5-a]pyridine-7-carbonitrile;
4-[(2*R*,6*S*)-2-methyl-6-[[5-(4-piperidyl)isoindolin-2-yl]methyl]morpholin-4-yl]pyrazolo[1,5-a]pyridine-7-carbonitrile;
4-[(2*R*,6*S*)-2-methyl-6-[(5-pyrrolidin-3-ylisoindolin-2-yl)methyl]morpholin-4-yl]pyrazolo[1,5-a]pyridine-7-carbonitrile;
4-[(2*R*,6*S*)-2-methyl-6-[(6-piperazin-1-yl-3,4-dihydro-1*H*-isoquinolin-2-yl)methyl]morpholin-4-yl]pyrazolo[1,5-a]pyridine-7-carbonitrile;
4-[(2*R*,6*S*)-2-methyl-6-[(7-piperazin-1-yl-3,4-dihydro-1*H*-isoquinolin-2-yl)methyl]morpholin-4-yl]pyrazolo[1,5-a]pyridine-7-carbonitrile;
8-[(2*R*,6*S*)-2-methyl-6-[(5-piperazin-1-ylisoindolin-2-yl)methyl]morpholin-4-yl]quinoxaline-5-carbonitrile;
8-[(2*R*,6*S*)-2-methyl-6-[(6-piperazin-1-yl-3,4-dihydro-1*H*-isoquinolin-2-yl)methyl]morpholin-4-yl]quinoxaline-5-carbonitrile;
3-fluoro-4-[(2*R*,6*S*)-2-methyl-6-[(6-piperazin-1-yl-3,4-dihydro-1*H*-isoquinolin-2-yl)methyl]morpholin-4-yl]pyrazolo[1,5-a]pyridine-7-carbonitrile;
4-[(2*R*,6*S*)-2-methyl-6-[[6-(4-methylpiperazin-1-yl)-3,4-dihydro-1*H*-isoquinolin-2-yl]methyl]morpholin-4-yl]pyrazolo[1,5-a]pyridine-7-carbonitrile
4-[(2*R*,6*S*)-2-methyl-6-[[5-(4-methylpiperazin-1-yl)isoindolin-2-yl]methyl]morpholin-4-yl]pyrazolo[1,5-a]pyridine-7-carbonitrile;
4-[(2*R*,6*S*)-2-methyl-6-[(3-piperazin-1-yl-5,7-dihydropyrrolo[3,4-b]pyridin-6-yl)methyl]morpholin-4-yl]pyrazolo[1,5-a]pyridine-7-carbonitrile;
5-[(2*R*,6*S*)-2-methyl-6-[(6-piperazin-1-yl-3,4-dihydro-1*H*-isoquinolin-2-yl)methyl]morpholin-4-yl]quinoline-8-carbonitrile;
5-[(2*R*,6*S*)-2-methyl-6-[(3-piperazin-1-yl-5,7-dihydropyrrolo[3,4-b]pyridin-6-yl)methyl]morpholin-4-yl]quinoline-8-carbonitrile;
5-[(2*R*,6*S*)-2-methyl-6-[(5-piperazin-1-ylisoindolin-2-yl)methyl]morpholin-4-yl]quinoline-8-carbonitrile;
7-[(2*R*,6*S*)-2-methyl-6-[(5-piperazin-1-ylisoindolin-2-yl)methyl]morpholin-4-yl]-1,3-benzothiazole-4-carbonitrile;
7-[(2*R*,6*S*)-2-methyl-6-[(6-piperazin-1-yl-3,4-dihydro-1*H*-isoquinolin-2-yl)methyl]morpholin-4-yl]-1,3-benzothiazole-4-carbonitrile;
7-[(2*R*,6*S*)-2-methyl-6-[(3-piperazin-1-yl-5,7-dihydropyrrolo[3,4-b]pyridin-6-yl)methyl]morpholin-4-yl]-1,3-benzothiazole-4-carbonitrile;
4-[(2*S*,6*R*)-2-[[5-(2-aminoethylamino)isoindolin-2-yl]methyl]-6-methyl-morpholin-4-yl]pyrazolo[1,5-a]pyridine-7-carbonitrile;
4-[(2*R*,6*S*)-2-methyl-6-[[2-(4-methylpiperazin-1-yl)-6,7-dihydro-4*H*-thiazolo[5,4-c]pyridin-5-yl]methyl]morpholin-4-yl]pyrazolo[1,5-a]pyridine-7-carbonitrile;
4-[(2*S*,6*R*)-2-[[5-[2-(dimethylamino)ethoxy]isoindolin-2-yl]methyl]-6-methyl-morpholin-4-yl]pyrazolo[1,5-a]pyridine-7-carbonitrile;
3-fluoro-4-[(2*R*,6*S*)-2-methyl-6-[(3-piperazin-1-yl-5,7-dihydropyrrolo[3,4-b]pyridin-6-yl)methyl]morpholin-4-yl]pyrazolo[1,5-a]pyridine-7-carbonitrile;
8-[(2*R*,6*S*)-2-methyl-6-[(3-piperazin-1-yl-5,7-dihydropyrrolo[3,4-b]pyridin-6-yl)methyl]morpholin-4-yl]quinoxaline-5-carbonitrile;
(2*R*,6*S*)-4-(8-chloro-5-quinolyl)-2-methyl-6-[(5-piperazin-1-ylisoindolin-2-yl)methyl]morpholine;
(2*R*,6*S*))-4-(8-chloro-5-quinolyl)-2-methyl-6-[(6-piperazin-1-yl-3,4-dihydro-1*H-*isoquinolin-2-yl)methyl]morpholine;
(2*R*,6*S*)-4-(8-chloroquinoxalin-5-yl)-2-methyl-6-[(5-piperazin-1-ylisoindolin-2-yl)methyl]morpholine;
(2*R*,6*S*)-4-(8-chloroquinoxalin-5-yl)-2-methyl-6-[(6-piperazin-1-yl-3,4-dihydro-1*H-*isoquinolin-2-yl)methyl]morpholine;
8-[(2*S*,6*R*)-2-[[5-(4-cyclopropylpiperazin-1-yl)isoindolin-2-yl]methyl]-6-methyl-morpholin-4-yl]quinoxaline-5-carbonitrile;
8-[(2*R*,6*S*)-2-methyl-6-[[5-(3-methylpiperazin-1-yl)isoindolin-2-yl]methyl]morpholin-4-yl]quinoxaline-5-carbonitrile;
8-[(2*S*,6*R*)-2-[[5-[4-(2-methoxyethyl)piperazin-1-yl]isoindolin-2-yl]methyl]-6-methyl-morpholin-4-yl]quinoxaline-5-carbonitrile;
8-[(2*S*,6*R*)-2-[[5-(4,7-diazaspiro[2.5]octan-7-yl)isoindolin-2-yl]methyl]-6-methyl-morpholin-4-yl]quinoxaline-5-carbonitrile;
8-[(2*R*,6*S*)-2-methyl-6-[[5-(2-oxopiperazin-1-yl)isoindolin-2-yl]methyl]morpholin-4-yl]quinoxaline-5-carbonitrile;
8-[(2*R*,6*S*)-2-[[5-(1,3,4,6,7,8,9,9a-octahydropyrido[1,2-a]pyrazin-2-yl)isoindolin-2-yl]methyl]-6-methyl-morpholin-4-yl]quinoxaline-5-carbonitrile;
8-[(2*S*,6*R*)-2-[[5-(3,8-diazabicyclo[3.2.1]octan-8-yl)isoindolin-2-yl]methyl]-6-methyl-morpholin-4-yl]quinoxaline-5-carbonitrile;
5-[(2*S*,6*R*)-2-[[5-(3,8-diazabicyclo[3.2.1]octan-3-yl)isoindolin-2-yl]methyl]-6-methyl-morpholin-4-yl]quinoline-8-carbonitrile;
5-[(2*S*,6*R*)-2-[[5-(3,8-diazabicyclo[3.2.1]octan-8-yl)isoindolin-2-yl]methyl]-6-methyl-morpholin-4-yl]quinoline-8-carbonitrile;
8-[(2*S*,6*R*)-2-[[5-(3,8-diazabicyclo[3.2.1]octan-3-yl)isoindolin-2-yl]methyl]-6-methyl-morpholin-4-yl]quinoxaline-5-carbonitrile;
4-[(2*R*,6*S*)-2-methyl-6-[[5-(1-methylimidazol-4-yl)isoindolin-2-yl]methyl]morpholin-4-yl]pyrazolo[1,5-a]pyridine-7-carbonitrile;
5-[(2*R*,6*S*)-2-methyl-6-[[5-(3-oxa-7,9-diazabicyclo[3.3.1]nonan-9-yl)isoindolin-2-yl]methyl]morpholin-4-yl]quinoline-8-carbonitrile;
5-[(2*S*,6*R*)-2-[[5-(2,6-diazaspiro[3.3]heptan-2-yl)isoindolin-2-yl]methyl]-6-methyl-morpholin-4-yl]quinoline-8-carbonitrile;
5-[(2R,6S)-2-methyl-6-[(1-methyl-5-piperazin-1-yl-isoindolin-2-yl)methyl]morpholin-4-yl]quinoline-8-carbonitrile;
8-[(2*R*,6*S*)-2-methyl-6-[[5-(3-oxa-7,9-diazabicyclo[3.3.1]nonan-7-yl)isoindolin-2-yl]methyl]morpholin-4-yl]quinoxaline-5-carbonitrile;
Ethyl 1-[2-[[(2*S*,6*R*)-4-(8-cyano-5-quinolyl)-6-methyl-morpholin-2-yl]methyl]isoindolin-5-yl]piperazine-2-carboxylate;
4-[(2*R*,6*S*)-2-methyl-6-[[2-(4-methylpiperazin-1-yl)-5,7-dihydropyrrolo[3,4-d]pyrimidin-6-yl]methyl]morpholin-4-yl]pyrazolo[1,5-a]pyridine-7-carbonitrile;
4-[(2*R*,6*S*)-2-methyl-6-[(5-pyrrolidin-3-yloxyisoindolin-2-yl)methyl]morpholin-4-yl]pyrazolo[1,5-a]pyridine-7-carbonitrile;
4-[(2*S*,6*R*)-2-[[5-(2,6-dimethyl-4-pyridyl)isoindolin-2-yl]methyl]-6-methyl-morpholin-4-yl]pyrazolo[1,5-a]pyridine-7-carbonitrile;
4-[(2*S*,6*R*)-2-[[5-(2,6-dimethyl-4-pyridyl)isoindolin-2-yl]methyl]-6-methyl-morpholin-4-yl]-3-fluoro-pyrazolo[1,5-a]pyridine-7-carbonitrile;
5-[(2*S*,6*R*)-2-[[5-(1,6-diazaspiro[3.3]heptan-6-yl)isoindolin-2-yl]methyl]-6-methyl-morpholin-4-yl]quinoline-8-carbonitrile;
1-[2-[[(2*S*,6*R*)-4-(8-cyano-5-quinolyl)-6-methyl-morpholin-2-yl]methyl]isoindolin-5-yl]piperazine-2-carboxylic acid;
*Trans*-5-[(2*R*,6*S*)-2-methyl-6-[[5-[3,5-dimethylpiperazin-1-yl]isoindolin-2-yl]methyl]morpholin-4-yl]quinoline-8-carbonitrile;
5-[(2*S*,6*R*)-2-[[5-(3,4,6,7,9,9a-hexahydro-1*H*-pyrazino[2,1-c][1,4]oxazin-8-yl)isoindolin-2-yl]methyl]-6-methyl-morpholin-4-yl]quinoline-8-carbonitrile;
5-[(2*S*,6*R*)-2-[(4-fluoro-6-piperazin-1-yl-isoindolin-2-yl)methyl]-6-methyl-morpholin-4-yl]quinoline-8-carbonitrile;
8-[(2*R*,6*S*)-2-methyl-6-[(6-piperazin-1-yl-1,3-dihydropyrrolo[3,4-c]pyridin-2-yl)methyl]morpholin-4-yl]quinoxaline-5-carbonitrile;
5-[(2*R*,6*S*)-2-methyl-6-[(6-piperazin-1-yl-1,3-dihydropyrrolo[3,4-c]pylidin-2-yl)methyl]morpholin-4-yl]quinoline-8-carbonitrile;
5-[(2*R*,6*S*)-2-methyl-6-[[5-[(3*R*)-3-methylpiperazin-1-yl]isoindolin-2-yl]methyl]morpholin-4-yl]quinoline-8-carbonitrile;
5-[(2*R*,6*S*)-2-methyl-6-[(2-piperazin-l-yl-5,7-dihydropyrrolo[3,4-d]pyrimidin-6-yl)methyl]morpholin-4-yl]quinoline-8-carbonitrile;
5-[(2*R*,6*S*)-2-methyl-6-[[5-[(3*S*)-3-methylpiperazin-1-yl]isoindolin-2-yl]methyl]morpholin-4-yl]quinoline-8-carbonitrile;
8-[(2*R*,6*S*)-2-methyl-6-[[5-[(3*R*)-3-methylpiperazin-1-yl]isoindolin-2-yl]methyl]morpholin-4-yl]quinoxaline-5-carbonitrile;
8-[(2*R*,6*S*)-2-methyl-6-[[5-[(3*S*)-3-methylpiperazin-1-yl]isoindolin-2-yl]methyl]morpholin-4-yl]quinoxaline-5-carbonitrile;
Methyl 4-[2-[[(2*S*,6*R*)-4-(8-cyano-5-quinolyl)-6-methyl-morpholin-2-yl]methyl]isoindolin-5-yl]piperazine-2-carboxylate;
4-[2-[[(2*S*,6*R*)-4-(8-cyano-5-quinolyl)-6-methyl-morpholin-2-yl]methyl]isoindolin-5-yl]piperazine-2-carboxylic acid;
5-[(2*S*,6*R*)-2-[[4-fluoro-6-(4-methylpiperazin-1-yl)isoindolin-2-yl]methyl]-6-methyl-morpholin-4-yl]quinoline-8-carbonitrile;
5-[(2*R*,6*S*)-2-methyl-6-[(6-piperazin-1-yl-3,4-dihydro-1*H*-2,7-naphthyridin-2-yl)methyl]morpholin-4-yl]quinoline-8-carbonitrile;
5-[(2*R*,6*S*)-2-methyl-6-[[5-[(3*R*,5*S*)-3,5-dimethylpiperazin-1-yl]isoindolin-2-yl]methyl]morpholin-4-yl]quinoline-8-carbonitrile;
5-[(2*R*,6*S*)-2-methyl-6-[[5-(3-oxa-7,9-diazabicyclo[3.3.1]nonan-7-yl)isoindolin-2-yl]methyl]morpholin-4-yl]quinoline-8-carbonitrile;
5-[(2*S*,6*R*)-2-[[5-[2-(hydroxymethyl)piperazin-1-yl]isoindolin-2-yl]methyl]-6-methyl-morpholin-4-yl]quinoline-8-carbonitrile;
5-[(2*R*,6*S*)-2-methyl-6-[[5-(1-piperidylmethyl)isoindolin-2-yl]methyl]morpholin-4-yl]quinoline-8-carbonitrile;
5-[(2S,6R)-2-[(4-hydroxy-6-piperazin-1-yl-3,4-dihydro-1H-isoquinolin-2-yl)methyl]-6-methyl-morpholin-4-yl]quinoline-8-carbonitrile;
5-[(2*S*,6*R*)-2-[[5-[2-(difluoromethyl)piperazin-1-yl]isoindolin-2-yl]methyl]-6-methyl-morpholin-4-yl]quinoline-8-carbonitrile;
5-[(2*S*,6*R*)-2-[[5-(3-aminoazetidin-1-yl)isoindolin-2-yl]methyl]-6-methyl-morpholin-4-yl]quinoline-8-carbonitrile;
5-[(2*R*,6*S*)-2-methyl-6-[[5-(2-methylpiperazin-1-yl)isoindolin-2-yl]methyl]morpholin-4-yl]quinoline-8-carbonitrile;
5-[(2*R*,6*S*)-2-methyl-6-[(2-piperazin-1-yl-7,8-dihydro-5*H*-1,6-naphthyridin-6-yl)methyl]morpholin-4-yl]quinoline-8-carbonitrile;
5-[(2*R*,6*S*)-2-methyl-6-[(2-piperazin-1-yl-5,7-dihydropyrrolo[3,4-b]pyridin-6-yl)methyl]morpholin-4-yl]quinoline-8-carbonitrile;
5-[(2S,6R)-2-[[5-[(2R)-2-(hydroxymethyl)piperazin-1-yl]isoindolin-2-yl]methyl]-6-methyl-morpholin-4-yl]quinoline-8-carbonitrile;
5-[(2S,6R)-2-[[5-[(2S)-2-(hydroxymethyl)piperazin-1-yl]isoindolin-2-yl]methyl]-6-methyl-morpholin-4-yl]quinoline-8-carbonitrile;
5-[(2*S*,6*R*)-2-[[5-[3-(hydroxymethyl)piperazin-1-yl]isoindolin-2-yl]methyl]-6-methyl-morpholin-4-yl]quinoline-8-carbonitrile;
5-[(2*R*,6*S*)-2-methyl-6-[[5-(morpholinomethyl)isoindolin-2-yl]methyl]morpholin-4-yl]quinoline-8-carbonitrile;
or a pharmaceutically acceptable salt, enantiomer or diastereomer thereof.

11. A process for the preparation of a compound according to any one of claims 1 to 10 comprising any of the following steps:
a) the coupling of compound of formula (VI), 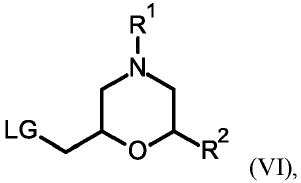 with amine (VII) in the presence of a base;
b) the coupling of compound of formula (VI), 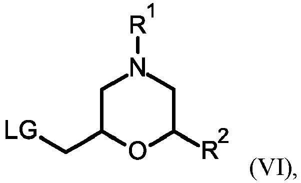 with amine (VIII) in the presence of a base;
c) the coupling of compound of formula (IX), 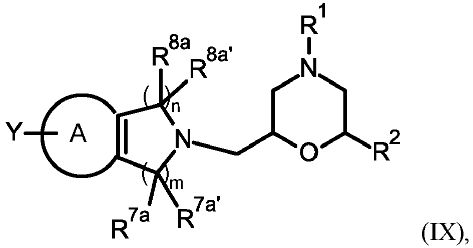 with amine (X) under Buchwald-Hartwig amination conditions;
wherein the base in step a) and b) is K₂CO₃, DIPEA, or Cs₂CO_{3;} the Buchwald-Hartwig amination condition in step c) includes a catalyst which is Ruphos Pd G2, and a base which is Cs₂CO₃; LG is OTf, OTs or OMs; Y is halogen, ((C₁₋₆alkyl)₂amino)C₁₋₆alkoxy, -NR⁹R¹⁰, heterocyclyl or heterocyclylC₁₋₆alkyl; R^{7a}, R^{7b}, R^{8a}, R^{8b} are independently selected from H and C₁₋₆alkyl; R⁹ and R¹⁰ are independently selected from H, aminoC₁₋₆alkylamino and aminoazetidinyl; or R⁹ and R¹⁰ together with the nitrogen they are attached to form a heterocyclyl; R¹ and R² are defined as in any one of claims 1 to 9.

12. A compound or pharmaceutically acceptable salt, enantiomer or diastereomer according to any one of claims 1 to 10 for use as therapeutically active substance.

13. A pharmaceutical composition comprising a compound in accordance with any one of claims 1 to 10 and a therapeutically inert carrier.

14. A compound or pharmaceutically acceptable salt, enantiomer or diastereomer according to any one of claims 1 to 10 for use in the treatment or prophylaxis of systemic lupus erythematosus or lupus nephritis.

## Patentansprüche

1. Verbindung der Formel (I) 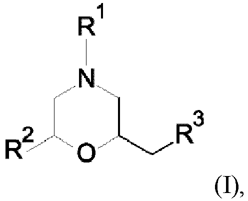 wobei
R¹ 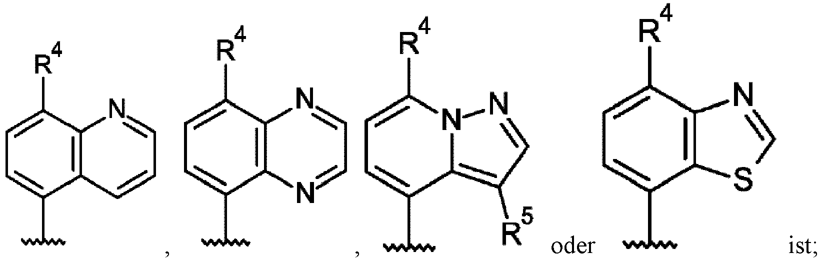 wobei R⁴ Cyano oder Halogen ist; R⁵ H oder Halogen ist;
R² C₁₋₆-Alkyl ist;
R³ mit Piperazinyl substituiertes 1,3-Dihydropyrrolo[3,4-c]pyridinyl;
mit Piperazinyl substituiertes 3,4-Dihydro-1H-2,7-naphthyridinyl;
3,4-Dihydro-1H-isochinolinyl, das mit einem oder zwei Substituenten substituiert ist, die unabhängig voneinander aus Hydroxy, Piperazinyl und C₁₋₆-Alkylpiperazinyl ausgewählt sind;
mit Piperazinyl substituiertes 3,4-Dihydro-2H-chinolinyl;
mit Piperazinyl substituiertes 5,7-Dihydropyrrolo[3,4-b]pyridinyl;
mit Piperazinyl oder C₁₋₆-Alkylpiperazinyl substituiertes 5,7-Dihydropyrrolo[3,4-d]pyrimidinyl;
mit C₁₋₆-Alkylpiperazinyl substituiertes 6,7-Dihydro-4H-thiazolo[5,4-c]pyridinyl;
mit Piperazinyl substituiertes 7,8-Dihydro-5H-1,6-naphthyridinyl,
Isoindolinyl, das mit einem oder zwei Substituenten substituiert ist, die unabhängig voneinander aus ((C₁₋₆-Alkyl)₂-amino)-C₁₋₆-alkoxy; (C₁₋₆-Alkoxy-C₁₋₆-alkyl)-piperazinyl; (C₁₋₆-Alkyl)₂-piperazinyl; (C₁₋₆-Alkyl)₂-pyridinyl; (Hydroxy-C₁₋₆-alkyl)-piperazinyl; 1,3,4,6,7,8,9,9a-Octahydropyrido[1,2-a]pyrazinyl, 1,6-Diazaspiro[3.3]heptanyl; 2,6-Diazaspiro[3.3]heptanyl; 3,4,6,7,9,9a-Hexahydro-1H-pyrazino[2,1-c][1,4]oxazinyl; 3,8-Diazabicyclo[3.2.1]octanyl; 3-Oxa-7,9-diazabicyclo[3.3.1]nonanyl; 4,7-Diazaspiro[2.5]octanyl; Aminoazetidinyl; Amino-C₁₋₆-alkylamino; C₁₋₆-Alkoxycarbonylpiperazinyl; C₁₋₆-Alkyl; C₁₋₆-Alkylimidazolyl; C₁₋₆-Alkylpiperazinyl; C₃₋₇-Cycloalkylpiperazinyl; Carboxypiperazinyl; Halogen-C₁₋₆-alkylpiperazinyl; Halogen; Morpholinyl-C₁₋₆-alkyl; Oxopiperazinyl; Piperazinyl; Piperidinyl; Piperidinyl-C₁₋₆-alkyl; Pyrrolidinyl und Pyrrolidinyloxy ausgewählt sind; oder
-NR^{6a}R^{6b} ist; wobei
R^{6a} H oder C₁₋₆-Alkyl ist;
R^{6b} mit Piperazinyl substituiertes Phenyl-C₁₋₆-alkyl ist;
oder ein pharmazeutisch unbedenkliches Salz, Enantiomer oder Diastereomer davon.

2. Verbindung nach Anspruch 1 der Formel (Ia) 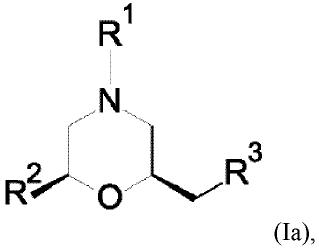 wobei R¹, R² und R³ wie in Anspruch 1 beschrieben sind.

3. Verbindung nach Anspruch 1 oder 2, wobei
R¹ 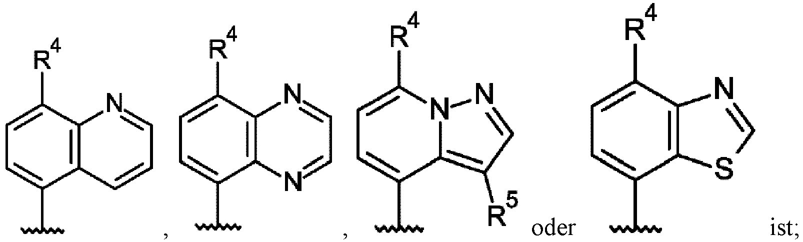 wobei R⁴ Cyano oder Chlor ist; R⁵ H oder Fluor ist;
R² Methyl ist;
R³ Piperazinyl-1,3-dihydropyrrolo[3,4-c]pyridinyl; Piperazinyl-3,4-dihydro-1H-2,7-naphthyridinyl; Piperazinyl-3,4-dihydro-1H-isochinolinyl; Methylpiperazinyl-3,4-dihydro-1H-isochinolinyl; Hydroxy-(piperazinyl)-3,4-dihydro-1H-isochinolinyl; Piperazinyl-3,4-dihydro-2H-chinolinyl; Piperazinyl-5,7-dihydropyrrolo[3,4-b]pyridinyl; Piperazinyl-5,7-dihydropyrrolo[3,4-d]pyrimidinyl; Methylpiperazinyl-5,7-dihydropyrrolo[3,4-d]pyrimidinyl; Methylpiperazinyl-6,7-dihydro-4H-thiazolo[5,4-c]pyridinyl; Piperazinyl-7,8-dihydro-5H-1,6-naphthyridinyl, (Difluormethyl)-piperazinylisoindolinyl; (Dimethylamino)-ethoxyisoindolinyl; (Hydroxymethyl)-piperazinylisoindolinyl; (Methoxyethyl)-piperazinylisoindolinyl; 1,3,4,6,7,8,9,9a-Octahydropyrido[1,2-a]pyrazinylisoindolinyl, 1,6-Diazaspiro[3.3]heptanylisoindolinyl; 2,6-Diazaspiro[3.3]heptanylisoindolinyl; 3,4,6,7,9,9a-Hexahydro-1H-pyrazino[2,1-c][1,4]oxazinylisoindolinyl; 3,8-Diazabicyclo[3.2.1]octanylisoindolinyl; 3-Oxa-7,9-diazabicyclo[3.3.1]nonanylisoindolinyl; 4,7-Diazaspiro[2.5]octanylisoindolinyl; Aminoazetidinylisoindolinyl; Aminoethylaminoisoindolinyl; Carboxypiperazinylisoindolinyl; Cyclopropylpiperazinylisoindolinyl; Dimethylpiperazinylisoindolinyl; Dimethylpyridinylisoindolinyl; Ethoxycarbonylpiperazinylisoindolinyl; Methoxycarbonylpiperazinylisoindolinyl; Methylimidazolylisoindolinyl; Methylpiperazinyl-(fluor)-isoindolinyl; Methylpiperazinylisoindolinyl; Morpholinylmethylisoindolinyl; Oxopiperazinylisoindolinyl; Piperazinyl-(fluor)-isoindolinyl; Piperazinyl-(methyl)-isoindolinyl; Piperazinylisoindolinyl; Piperidinylisoindolinyl; Piperidinylmethylisoindolinyl; Pyrrolidinylisoindolinyl; Pyrrolidinyloxyisoindolinyl; (Piperazinylphenyl)-methylamino oder (Piperazinylphenyl)-methyl-(methyl)-amino ist;
oder ein pharmazeutisch unbedenkliches Salz, Enantiomer oder Diastereomer davon.

4. Verbindung nach Anspruch 1 oder 2, wobei R¹

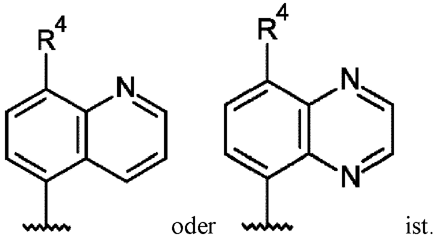

5. Verbindung nach Anspruch 4, wobei R⁴ Cyano oder Chlor ist.

6. Verbindung nach Anspruch 4, wobei
R³
mit Piperazinyl substituiertes 1,3-Dihydropyrrolo[3,4-c]pyridinyl,
mit Piperazinyl substituiertes 3,4-Dihydro-1H-2,7-naphthyridinyl;
3,4-Dihydro-1H-isochinolinyl, das mit einem oder zwei Substituenten substituiert ist, die unabhängig voneinander aus Hydroxy, Piperazinyl und C₁₋₆-Alkylpiperazinyl ausgewählt sind;
mit Piperazinyl substituiertes 3,4-Dihydro-2H-chinolinyl;
mit Piperazinyl substituiertes 5,7-Dihydropyrrolo[3,4-b]pyridinyl;
mit Piperazinyl oder C₁₋₆-Alkylpiperazinyl substituiertes 5,7-Dihydropyrrolo[3,4-d]pyrimidinyl;
mit Piperazinyl substituiertes 7,8-Dihydro-5H-1,6-naphthyridinyl,
Isoindolinyl, das mit (C₁₋₆-Alkoxy-C₁₋₆-alkyl)-piperazinyl, (C₁₋₆-Alkyl)₂-piperazinyl, (Hydroxy-C₁₋₆-alkyl)-piperazinyl, C₁₋₆-Alkylpiperazinyl, C₃₋₇-Cycloalkylpiperazinyl, Halogen-C₁₋₆-alkylpiperazinyl, Morpholinyl-C₁₋₆-alkyl, Piperazinyl, Piperidinyl, Piperidinyl-C₁₋₆-alkyl oder Pyrrolidinyl substituiert ist; oder
-NR^{6a}R^{6b} ist; wobei
R^{6a} H ist;
R^{6b} mit Piperazinyl substituiertes Phenyl-C₁₋₆-alkyl ist.

7. Verbindung nach Anspruch 6, wobei R³ Piperazinyl-1,3-dihydropyrrolo[3,4-c]pyridinyl, Piperazinyl-3,4-dihydro-1H-2,7-naphthyridinyl; Piperazinyl-3,4-dihydro-1H-isochinolinyl; Methylpiperazinyl-3,4-dihydro-1H-isochinolinyl; Hydroxy-(piperazinyl)-3,4-dihydro-1H-isochinolinyl; Piperazinyl-3,4-dihydro-2H-chinolinyl; Piperazinyl-5,7-dihydropyrrolo[3,4-b]pyridinyl; Piperazinyl-5,7-dihydropyrrolo[3,4-d]pyrimidinyl; Methylpiperazinyl-5,7-dihydropyrrolo[3,4-d]pyrimidinyl; Piperazinyl-7,8-dihydro-5H-1,6-naphthyridinyl; (Difluormethyl)-piperazinylisoindolinyl; (Hydroxymethyl)-piperazinylisoindolinyl; (Methoxyethyl)-piperazinylisoindolinyl; Cyclopropylpiperazinylisoindolinyl; Dimethylpiperazinylisoindolinyl; Methylpiperazinylisoindolinyl; Morpholinylmethylisoindolinyl; Piperazinylisoindolinyl; Piperidinylisoindolinyl; Piperidinylmethylisoindolinyl; Pyrrolidinylisoindolinyl oder (Piperazinylphenyl)-methylamino ist.

8. Verbindung nach Anspruch 1 oder 2, wobei
R¹ 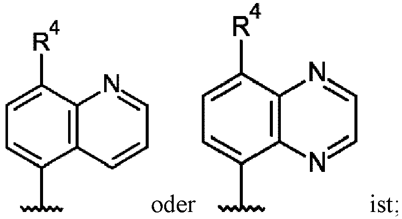 wobei R⁴ Cyano oder Halogen ist;
R² C₁₋₆-Alkyl ist;
R³ mit Piperazinyl substituiertes 1,3-Dihydropyrrolo[3,4-c]pyridinyl;
mit Piperazinyl substituiertes 3,4-Dihydro-1H-2,7-naphthyridinyl;
3,4-Dihydro-1H-isochinolinyl, das mit einem oder zwei Substituenten substituiert ist, die unabhängig voneinander aus Hydroxy, Piperazinyl und C₁₋₆-Alkylpiperazinyl ausgewählt sind;
mit Piperazinyl substituiertes 3,4-Dihydro-2H-chinolinyl;
mit Piperazinyl substituiertes 5,7-Dihydropyrrolo[3,4-b]pyridinyl;
mit Piperazinyl oder C₁₋₆-Alkylpiperazinyl substituiertes 5,7-Dihydropyrrolo[3,4-d]pyrimidinyl;
mit Piperazinyl substituiertes 7,8-Dihydro-5H-1,6-naphthyridinyl,
Isoindolinyl, das mit (C₁₋₆-Alkoxy-C₁₋₆-alkyl)-piperazinyl, (C₁₋₆-Alkyl)₂-piperazinyl, (Hydroxy-C₁₋₆-alkyl)-piperazinyl, C₁₋₆-Alkylpiperazinyl, C₃₋₇-Cycloalkylpiperazinyl, Halogen-C₁₋₆-alkylpiperazinyl, Morpholinyl-C₁₋₆-alkyl, Piperazinyl, Piperidinyl, Piperidinyl-C₁₋₆-alkyl oder Pyrrolidinyl substituiert ist; oder
-NR^{6a}R^{6b} ist; wobei
R^{6a} H ist;
R^{6b} mit Piperazinyl substituiertes Phenyl-C₁₋₆-alkyl ist;
oder ein pharmazeutisch unbedenkliches Salz, Enantiomer oder Diastereomer davon.

9. Verbindung nach Anspruch 8, wobei
R¹ 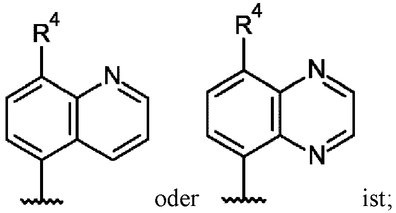 wobei R⁴ Cyano oder Chlor ist;
R² Methyl ist;
R³ Piperazinyl-1,3-dihydropyrrolo[3,4-c]pyridinyl; Piperazinyl-3,4-dihydro-1H-2,7-naphthyridinyl; Piperazinyl-3,4-dihydro-1H-isochinolinyl; Methylpiperazinyl-3,4-dihydro-1H-isochinolinyl; Hydroxy-(piperazinyl)-3,4-dihydro-1H-isochinolinyl; Piperazinyl-3,4-dihydro-2H-chinolinyl; Piperazinyl-5,7-dihydropyrrolo[3,4-b]pyridinyl; Piperazinyl-5,7-dihydropyrrolo[3,4-d]pyrimidinyl; Methylpiperazinyl-5,7-dihydropyrrolo[3,4-d]pyrimidinyl; Piperazinyl-7,8-dihydro-5H-1,6-naphthyridinyl, (Difluormethyl)-piperazinylisoindolinyl; (Hydroxymethyl)-piperazinylisoindolinyl; (Methoxyethyl)-piperazinylisoindolinyl; Cyclopropylpiperazinylisoindolinyl; Dimethylpiperazinylisoindolinyl; Methylpiperazinylisoindolinyl; Morpholinylmethylisoindolinyl; Piperazinylisoindolinyl; Piperidinylisoindolinyl; Piperidinylmethylisoindolinyl; Pyrrolidinylisoindolinyl; oder (Piperazinylphenyl)-methylamino ist;
oder ein pharmazeutisch unbedenkliches Salz, Enantiomer oder Diastereomer davon.

10. Verbindung nach Anspruch 1 oder 2, ausgewählt aus:
4-[(2*R*,6*S*)-2-Methyl-6-[(4-piperazin-1-ylisoindolin-2-yl)methyl]morpholin-4-yl]pyrazolo[1,5-a]pyridin-7-carbonitril;
4-[(2*R*,6*S*)-2-Methyl-6-[(5-piperazin-1-ylisoindolin-2-yl)methyl]morpholin-4-yl]pyrazolo[1,5-a]pyridin-7-carbonitril;
4-[(2*R*,6*S*)-2-Methyl-6-[(8-piperazin-1-yl-3,4-dihydro-1*H*-isochinolin-2-yl)methyl]morpholin-4-yl]pyrazolo[1,5-a]pyridin-7-carbonitril;
4-[(2*R*,6*S*)-2-Methyl-6-[(7-piperazin-1-yl-3,4-dihydro-2*H*-chinolin-1-yl)methyl]morpholin-4-yl]pyrazolo[1,5-a]pyridin-7-carbonitril;
3-Fluor-4-[(2*R*,6*S*)-2-methyl-6-[(5-piperazin-1-ylisoindolin-2-yl)methyl]morpholin-4-yl]pyrazolo[1,5-a]pyridin-7-carbonitril;
4-[(2*R*,6*S*)-2-Methyl-6-[[5-(4-piperidyl)isoindolin-2-yl]methyl]morpholin-4-yl]pyrazolo[1,5-a]pyridin-7-carbonitril;
4-[(2*R*,6*S*)-2-Methyl-6-[(5-pyrrolidin-3-ylisoindolin-2-yl)methyl]morpholin-4-yl]pyrazolo[1,5-a]pyridin-7-carbonitril;
4-[(2*R*,6*S*)-2-Methyl-6-[(6-piperazin-1-yl-3,4-dihydro-1*H*-isochinolin-2-yl)methyl]morpholin-4-yl]pyrazolo[1,5-a]pyridin-7-carbonitril;
4-[(2*R*,6*S*)-2-Methyl-6-[(7-piperazin-1-yl-3,4-dihydro-1*H*-isochinolin-2-yl)methyl]morpholin-4-yl]pyrazolo[1,5-a]pyridin-7-carbonitril;
8-[(2*R*,6*S*)-2-Methyl-6-[(5-piperazin-1-ylisoindolin-2-yl)methyl]morpholin-4-yl]chinoxalin-5-carbonitril;
8-[(2*R*,6*S*)-2-Methyl-6-[(6-piperazin-1-yl-3,4-dihydro-1*H*-isochinolin-2-yl)methyl]morpholin-4-yl]chinoxalin-5-carbonitril;
3-Fluor-4-[(2*R*,6*S*)-2-methyl-6-[(6-piperazin-1-yl-3,4-dihydro-1*H*-isochinolin-2-yl)methyl]morpholin-4-yl]pyrazolo[1,5-a]pyridin-7-carbonitril;
4-[(2*R*,6*S*)-2-Methyl-6-[[6-(4-methylpiperazin-1-yl)-3,4-dihydro-1*H*-isochinolin-2-yl]methyl]morpholin-4-yl]pyrazolo[1,5-a]pyridin-7-carbonitril
4-[(2*R*,6*S*)-2-Methyl-6-[[5-(4-methylpiperazin-1-yl)isoindolin-2-yl]methyl]morpholin-4-yl]pyrazolo[1,5-a]pyridin-7-carbonitril;
4-[(2*R*,6*S*)-2-Methyl-6-[(3-piperazin-1-yl-5,7-dihydropyrrolo[3,4-b]pyridin-6-yl)methyl]morpholin-4-yl]pyrazolo[1,5-a]pyridin-7-carbonitril;
5-[(2*R*,6*S*)-2-Methyl-6-[(6-piperazin-1-yl-3,4-dihydro-1*H*-isochinolin-2-yl)methyl]morpholin-4-yl]chinolin-8-carbonitril;
5-[(2*R*,6*S*)-2-Methyl-6-[(3-piperazin-1-yl-5,7-dihydropyrrolo[3,4-b]pyridin-6-yl)methyl]morpholin-4-yl]chinolin-8-carbonitril;
5-[(2*R*,6*S*)-2-Methyl-6-[(5-piperazin-1-ylisoindolin-2-yl)methyl]morpholin-4-yl]chinolin-8-carbonitril;
7-[(2*R*,6*S*)-2-Methyl-6-[(5-piperazin-1-ylisoindolin-2-yl)methyl]morpholin-4-yl]-1,3-benzothiazol-4-carbonitril;
7-[(2*R*,6*S*)-2-Methyl-6-[(6-piperazin-1-yl-3,4-dihydro-1*H*-isochinolin-2-yl)methyl]morpholin-4-yl]-1,3-benzothiazol-4-carbonitril;
7-[(2*R*,6*S*)-2-Methyl-6-[(3-piperazin-1-yl-5,7-dihydropyrrolo[3,4-b]pyridin-6-yl)methyl]morpholin-4-yl]-1,3-benzothiazol-4-carbonitril;
4-[(2*S*,6*R*)-2-[[5-(2-Aminoethylamino)isoindolin-2-yl]methyl]-6-methylmorpholin-4-yl]pyrazolo[1,5-a]pyridin-7-carbonitril;
4-[(2*R*,6*S*)-2-Methyl-6-[[2-(4-methylpiperazin-1-yl)-6,7-dihydro-4*H*-thiazolo[5,4-c]pyridin-5-yl]methyl]morpholin-4-yl]pyrazolo[1,5-a]pyridin-7-carbonitril;
4-[(2*S*,6*R*)-2-[[5-[2-(Dimethylamino)ethoxy]isoindolin-2-yl]methyl]-6-methylmorpholin-4-yl]pyrazolo[1,5-a]pyridin-7-carbonitril;
3-Fluor-4-[(2*R*,6*S*)-2-methyl-6-[(3-piperazin-1-yl-5,7-dihydropyrrolo[3,4-b]pyridin-6-yl)methyl]morpholin-4-yl]pyrazolo[1,5-a]pyridin-7-carbonitril;
8-[(2*R*,6*S*)-2-Methyl-6-[(3-piperazin-1-yl-5,7-dihydropyrrolo[3,4-b]pyridin-6-yl)methyl]morpholin-4-yl]chinoxalin-5-carbonitril;
(2*R*,6*S*)-4-(8-Chlor-5-chinolyl)-2-methyl-6-[(5-piperazin-1-ylisoindolin-2-yl)methyl]morpholin;
(2*R*,6*S*)-4-(8-Chlor-5-chinolyl)-2-methyl-6-[(6-piperazin-1-yl-3,4-dihydro-1*H-*isochinolin-2-yl)methyl]morpholin;
(2*R*,6*S*)-4-(8-Chlorchinoxalin-5-yl)-2-methyl-6-[(5-piperazin-1-ylisoindolin-2-yl)methyl]morpholin;
(2*R*,6*S*)-4-(8-Chlorchinoxalin-5-yl)-2-methyl-6-[(6-piperazin-1-yl-3,4-dihydro-1*H-*isochinolin-2-yl)methyl]morpholin;
8-[(2*S*,6*R*)-2-[[5-(4-Cyclopropylpiperazin-1-yl)isoindolin-2-yl]methyl]-6-methylmorpholin-4-yl] chinoxalin-5 -carbonitril;
8-[(2*R*,6*S*)-2-Methyl-6-[[5-(3-methylpiperazin-1-yl)isoindolin-2-yl]methyl]morpholin-4-yl]chinoxalin-5-carbonitril;
8-[(2*S*,6*R*)-2-[[5-[4-(2-Methoxyethyl)piperazin-1-yl]isoindolin-2-yl]methyl]-6-methylmorpholin-4-yl] chinoxalin-5 -carbonitril;
8-[(2*S*,6*R*)-2-[[5-(4,7-Diazaspiro[2.5]octan-7-yl)isoindolin-2-yl]methyl]-6-methylmorpholin-4-yl] chinoxalin-5 -carbonitril;
8-[(2*R*,6*S*)-2-Methyl-6-[[5-(2-oxopiperazin-1-yl)isoindolin-2-yl]methyl]morpholin-4-yl]chinoxalin-5-carbonitril;
8-[(2*S*,6*R*)-2-[[5-(1,3,4,6,7,8,9,9a-Octahydropyrido[1,2-a]pyrazin-2-yl)isoindolin-2-yl]methyl]-6-methylmorpholin-4-yl]chinoxalin-5-carbonitril;
8-[(2*S*,6*R*)-2-[[5-(3,8-Diazabicyclo[3.2.1]octan-8-yl)isoindolin-2-yl]methyl]-6-methylmorpholin-4-yl]chinoxalin-5-carbonitril;
5-[(2*S*,6*R*)-2-[[5-(3,8-Diazabicyclo[3.2.1]octan-3-yl)isoindolin-2-yl]methyl]-6-methylmorpholin-4-yl]chinolin-8-carbonitril;
5-[(2*S*,6*R*)-2-[[5-(3,8-Diazabicyclo[3.2.1]octan-8-yl)isoindolin-2-yl]methyl]-6-methylmorpholin-4-yl]chinolin-8-carbonitril;
8-[(2*S*,6*R*)-2-[[5-(3,8-Diazabicyclo[3.2.1]octan-3-yl)isoindolin-2-yl]methyl]-6-methylmorpholin-4-yl]chinoxalin-5-carbonitril;
4-[(2*R*,6*S*)-2-Methyl-6-[[5-(1-methylimidazol-4-yl)isoindolin-2-yl]methyl]morpholin-4-yl]pyrazolo[1,5-a]pyridin-7-carbonitril;
5-[(2*R*,6*S*)-2-Methyl-6-[[5-(3-oxa-7,9-diazabicyclo[3.3.1]nonan-9-yl)isoindolin-2-yl]methyl]morpholin-4-yl]chinolin-8-carbonitril;
5-[(2*S*,6*R*)-2-[[5-(2,6-Diazaspiro[3.3]heptan-2-yl)isoindolin-2-yl]methyl]-6-methylmorpholin-4-yl]chinolin-8-carbonitril;
5-[(2*R*,6*S*)-2-Methyl-6-[(1-methyl-5-piperazin-1-yl-isoindolin-2-yl)methyl]morpholin-4-yl]chinolin-8-carbonitril;
8-[(2*R*,6*S*)-2-Methyl-6-[[5-(3-oxa-7,9-diazabicyclo[3.3.1]nonan-7-yl)isoindolin-2-yl]methyl]morpholin-4-yl]chinoxalin-5-carbonitril;
Ethyl-1-[2-[[(2*S*,6*R*)-4-(8-cyano-5-chinolyl)-6-methylmorpholin-2-yl]methyl]isoindolin-5-yl]piperazin-2-carboxylat;
4-[(2*R*,6*S*)-2-Methyl-6-[[2-(4-methylpiperazin-1-yl)-5,7-dihydropyrrolo[3,4-d]pyrimidin-6-yl]methyl]morpholin-4-yl]pyrazolo[1,5-a]pyridin-7-carbonitril;
4-[(2*R*,6*S*)-2-Methyl-6-[(5-pyrrolidin-3-yloxyisoindolin-2-yl)methyl]morpholin-4-yl]pyrazolo[1,5-a]pyridin-7-carbonitril;
4-[(2*S*,6*R*)-2-[[5-(2,6-Dimethyl-4-pyridyl)isoindolin-2-yl]methyl]-6-methylmorpholin-4-yl]pyrazolo[1,5-a]pyridin-7-carbonitril;
4-[(2*S*,6*R*)-2-[[5-(2,6-Dimethyl-4-pyridyl)isoindolin-2-yl]methyl]-6-methylmorpholin-4-yl]-3-fluorpyrazolo[1,5-a]pyridin-7-carbonitril;
5-[(2*S*,6*R*)-2-[[5-(1,6-Diazaspiro[3.3]heptan-6-yl)isoindolin-2-yl]methyl]-6-methylmorpholin-4-yl]chinolin-8-carbonitril;
1-[2-[[(2*S*,6*R*)-4-(8-Cyano-5-chinolyl)-6-methylmorpholin-2-yl]methyl]isoindolin-5-yl]piperazin-2-carbonsäure;
*trans*-5-[(2*R*,6*S*)-2-Methyl-6-[[5-[3,5-dimethylpiperazin-1-yl]isoindolin-2-yl]methyl]morpholin-4-yl]chinolin-8-carbonitril;
5-[(2*S*,6*R*)-2-[[5-(3,4,6,7,9,9a-Hexahydro-1*H*-pyrazino[2,1-c][1,4]oxazin-8-yl)isoindolin-2-yl]methyl]-6-methylmorpholin-4-yl]chinolin-8-carbonitril;
5-[(2*S*,6*R*)-2-[(4-Fluor-6-piperazin-1-yl-isoindolin-2-yl)methyl]-6-methylmorpholin-4-yl]chinolin-8-carbonitril;
8-[(2*R*,6*S*)-2-Methyl-6-[(6-piperazin-1-yl-1,3-dihydropyrrolo[3,4-c]pyridin-2-yl)methyl]morpholin-4-yl]chinoxalin-5-carbonitril;
5-[(2*R*,6*S*)-2-Methyl-6-[(6-piperazin-1-yl-1,3-dihydropyrrolo[3,4-c]pyridin-2-yl)methyl]morpholin-4-yl]chinolin-8-carbonitril;
5-[(2*R*,6*S*)-2-Methyl-6-[[5-[(3*R*)-3-methylpiperazin-1-yl]isoindolin-2-yl]methyl]morpholin-4-yl]chinolin-8-carbonitril;
5-[(2*R*,6*S*)-2-Methyl-6-[(2-piperazin-1-yl-5,7-dihydropyrrolo[3,4-d]pyrimidin-6-yl)methyl]morpholin-4-yl]chinolin-8-carbonitril;
5-[(2*R*,6*S*)-2-Methyl-6-[[5-[(3*S*)-3-methylpiperazin-1-yl]isoindolin-2-yl]methyl]morpholin-4-yl]chinolin-8-carbonitril;
8-[(2*R*,6*S*)-2-Methyl-6-[[5-[(3*R*)-3-methylpiperazin-1-yl]isoindolin-2-yl]methyl]morpholin-4-yl]chinoxalin-5-carbonitril;
8-[(2*R*,6*S*)-2-Methyl-6-[[5-[(3*S*)-3-methylpiperazin-1-yl]isoindolin-2-yl]methyl]morpholin-4-yl]chinoxalin-5-carbonitril;
Methyl-4-[2-[[(2*S*,6*R*)-4-(8-cyano-5-chinolyl)-6-methylmorpholin-2-yl]methyl]isoindolin-5-yl]piperazin-2-carboxylat;
4-[2-[[(2*S*,6*R*)-4-(8-Cyano-5-chinolyl)-6-methylmorpholin-2-yl]methyl]isoindolin-5-yl]piperazin-2-carbonsäure;
5-[(2*S*,6*R*)-2-[[4-Fluor-6-(4-methylpiperazin-1-yl)isoindolin-2-yl]methyl]-6-methylmorpholin-4-yl]chinolin-8-carbonitril;
5-[(2*R*,6*S*)-2-Methyl-6-[(6-piperazin-1-yl-3,4-dihydro-1*H*-2,7-naphthyridin-2-yl)methyl]morpholin-4-yl]chinolin-8-carbonitril;
5-[(2*R*,6*S*)-2-Methyl-6-[[5-[(3*R*,5*S*)-3,5-dimethylpiperazin-1-yl]isoindolin-2-yl]methyl]morpholin-4-yl]chinolin-8-carbonitril;
5-[(2*R*,6*S*)-2-Methyl-6-[[5-(3-oxa-7,9-diazabicyclo[3.3.1]nonan-7-yl)isoindolin-2-yl]methyl]morpholin-4-yl]chinolin-8-carbonitril;
5-[(2*R*,6*S*)-2-[[5-[2-(Hydroxymethyl)piperazin-1-yl]isoindolin-2-yl]methyl]-6-methylmorpholin-4-yl]chinolin-8-carbonitril;
5-[(2*R*,6*S*)-2-Methyl-6-[[5-(1-piperidylmethyl)isoindolin-2-yl]methyl]morpholin-4-yl]chinolin-8-carbonitril;
5-[(2*S*,6*R*)-2-[(4-Hydroxy-6-piperazin-1-yl-3,4-dihydro-1*H*-isochinolin-2-yl)methyl]-6-methylmorpholin-4-yl]chinolin-8-carbonitril;
5-[(2*S*,6*R*)-2-[[5-[2-(Difluormethyl)piperazin-1-yl]isoindolin-2-yl]methyl]-6-methylmorpholin-4-yl]chinolin-8-carbonitril;
5-[(2*S*,6*R*)-2-[[5-(3-Aminoazetidin-1-yl)isoindolin-2-yl]methyl]-6-methylmorpholin-4-yl]chinolin-8-carbonitril;
5-[(2*R*,6*S*)-2-Methyl-6-[[5-(2-methylpiperazin-1-yl)isoindolin-2-yl]methyl]morpholin-4-yl]chinolin-8-carbonitril;
5-[(2*R*,6*S*)-2-Methyl-6-[(2-piperazin-1-yl-7,8-dihydro-5*H*-1,6-naphthyridin-6-yl)methyl]morpholin-4-yl]chinolin-8-carbonitril;
5-[(2*R*,6*S*)-2-Methyl-6-[(2-piperazin-1-yl-5,7-dihydropyrrolo[3,4-b]pyridin-6-yl)methyl]morpholin-4-yl]chinolin-8-carbonitril;
5-[(2*S*,6*R*)-2-[[5-[(2*R*)-2-(Hydroxymethyl)piperazin-1-yl]isoindolin-2-yl]methyl]-6-methylmorpholin-4-yl]chinolin-8-carbonitril;
5-[(2*S*,6*R*)-2-[[5-[(2*S*)-2-(Hydroxymethyl)piperazin-1-yl]isoindolin-2-yl]methyl]-6-methylmorpholin-4-yl]chinolin-8-carbonitril;
5-[(2*S*,6*R*)-2-[[5-[3-(Hydroxymethyl)piperazin-1-yl]isoindolin-2-yl]methyl]-6-methylmorpholin-4-yl]chinolin-8-carbonitril;
5-[(2*R*,6*S*)-2-Methyl-6-[[5-(morpholinomethyl)isoindolin-2-yl]methyl]morpholin-4-yl]chinolin-8-carbonitril;
oder einem pharmazeutisch unbedenklichen Salz, Enantiomer oder Diastereomer davon.

11. Verfahren zur Herstellung einer Verbindung nach einem der Ansprüche 1 bis 10, umfassend einen der folgenden Schritte:
a) Koppeln einer Verbindung der Formel (VI) 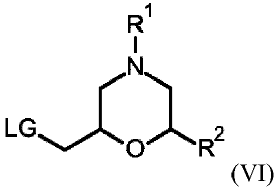 mit einem Amin (VII) in der Gegenwart einer Base;
b) Koppeln einer Verbindung der Formel (VI) 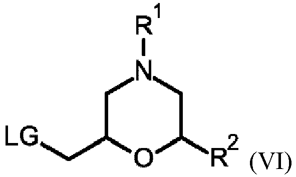 mit einem Amin (VIII) in der Gegenwart einer Base;
c) Koppeln einer Verbindung der Formel (IX) 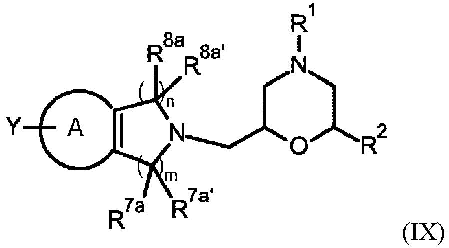 mit einem Amin (X) unter Buchwald-Hartwig-Aminierungsbedingungen;
wobei die Base in Schritt a) und b) K₂CO₃, DIPEA oder Cs₂CO₃ ist; die Buchwald-Hartwig-Aminierungsbedingung in Schritt c) einen Katalysator, der Ruphos Pd G2 ist, und eine Base, die Cs₂CO₃ ist, einschließt; LG OTf, OTs oder OMs ist; Y Halogen, ((C₁₋₆-Alkyl)₂-amino)-C₁₋₆-alkoxy, -NR⁹R¹⁰, Heterocyclyl oder Heterocyclyl-C₁₋₆-alkyl ist; R^{7a}, R^{7b}, R^{8a}, R^{8b} unabhängig voneinander aus H und C₁₋₆-Alkyl ausgewählt sind; R⁹ und R¹⁰ unabhängig voneinander aus H, Amino-C₁₋₆-alkylamino und Aminoazetidinyl ausgewählt sind; oder R⁹ und R¹⁰ zusammen mit dem Stickstoff, an den sie gebunden sind, ein Heterocyclyl bilden; R¹ und R² wie in einem der Ansprüche 1 bis 9 definiert sind.

12. Verbindung oder pharmazeutisch unbedenkliches Salz, Enantiomer oder Diastereomer nach einem der Ansprüche 1 bis 10 zur Verwendung als therapeutisch wirksame Substanz.

13. Pharmazeutische Zusammensetzung, umfassend eine Verbindung nach einem der Ansprüche 1 bis 10 und einen therapeutisch inerten Träger.

14. Verbindung oder pharmazeutisch unbedenkliches Salz, Enantiomer oder Diastereomer nach einem der Ansprüche 1 bis 10 zur Verwendung bei der Behandlung oder Prophylaxe von systemischem Lupus erythematodes oder Lupusnephritis.

## Revendications

1. Composé de formule (I), 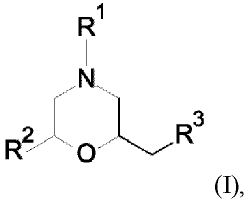 dans lequel
R¹ représente 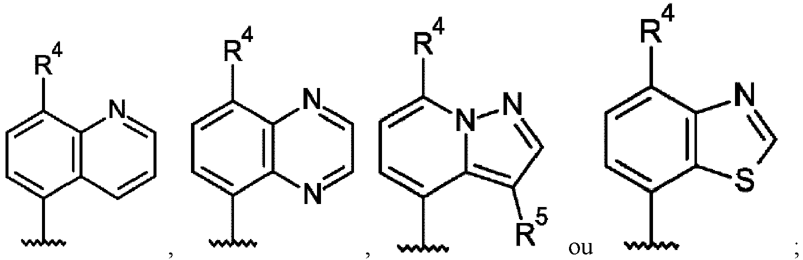 dans lequel R⁴ représente un cyano ou un halogène ; R⁵ représente H ou un halogène ;
R² représente un alkyle en C₁₋₆ ;
R³ représente un 1,3-dihydropyrrolo[3,4-c]pyridinyle substitué par un pipérazinyle ;
un 3,4-dihydro-1H-2,7-naphtyridinyle substitué par un pipérazinyle ;
un 3,4-dihydro-1H-isoquinoléinyle substitué par un ou deux substituants indépendamment choisis parmi un hydroxy, un pipérazinyle et un alkyle en C₁₋₆-pipérazinyle ;
un 3,4-dihydro-2H-quinoléinyle substitué par un pipérazinyle ;
un 5,7-dihydropyrrolo[3,4-b]pyridinyle substitué par un pipérazinyle ;
un 5,7-dihydropyrrolo[3,4-d]pyrimidinyle substitué par un pipérazinyle ou un alkyle en C₁₋₆-pipérazinyle ;
un 6,7-dihydro-4H-thiazolo[5,4-c]pyridinyle substitué par un alkyle en C₁₋₆-pipérazinyle ;
un 7,8-dihydro-5H-1,6-naphtyridinyle substitué par un pipérazinyle ;
un isoindolinyle substitué par un ou deux substituants indépendamment choisis parmi un ((alkyle en C₁₋₆)₂amino)alcoxy en C₁₋₆ ; un (alcoxy en C₁₋₆-alkyle en C₁₋₆)pipérazinyle ; un (alkyle en C₁₋₆)₂pipérazinyle ; un (alkyle en C₁₋₆)₂pyridinyle ; un (hydroxyalkyle en C₁₋₆)pipérazinyle ; un 1,3,4,6,7,8,9,9a-octahydropyrido[1,2-a]pyrazinyle ; un 1,6-diazaspiro[3.3]heptanyle ; un 2,6-diazaspiro[3.3]heptanyle ; un 3,4,6,7,9,9a-hexahydro-1H-pyrazino[2,1-c][1,4]oxazinyle ; un 3,8-diazabicyclo[3.2.1]octanyle ; un 3-oxa-7,9-diazabicyclo[3.3.1]nonanyle ; un 4,7-diazaspiro[2.5]octanyle ; un aminoazétidinyle ; un aminoalkyle en C₁₋₆-amino ; un alcoxy en C₁₋₆-carbonylpipérazinyle ; un alkyle en C₁₋₆ ; un alkyle en C₁₋₆-imidazolyle ; un alkyle en C₁₋₆-pipérazinyle ; un cycloalkyle en C₃₋₇-pipérazinyle ; un carboxypipérazinyle ; un halogénoalkyle en C₁₋₆-pipérazinyle ; un halogène ; un morpholinylalkyle en C₁₋₆ ; un oxopipérazinyle ; un pipérazinyle ; un pipéridinyle ; un pipéridinylalkyle en C₁₋₆ ; un pyrrolidinyle et un pyrrolidinyloxy ; ou
-NR^{6a}R^{6b} ; dans lequel
R^{6a} représente H ou un alkyle en C₁₋₆ ;
R^{6b} représente un phénylalkyle en C₁₋₆ substitué par un pipérazinyle ;
ou un sel pharmaceutiquement acceptable, un énantiomère ou un diastéréoisomère de celui-ci.

2. Composé selon la revendication 1 de formule (Ia), 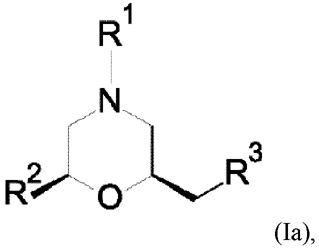 dans lequel R¹, R² et R³ sont tels que décrits dans la revendication 1.

3. Composé selon la revendication 1 ou 2, dans lequel
R¹ représente 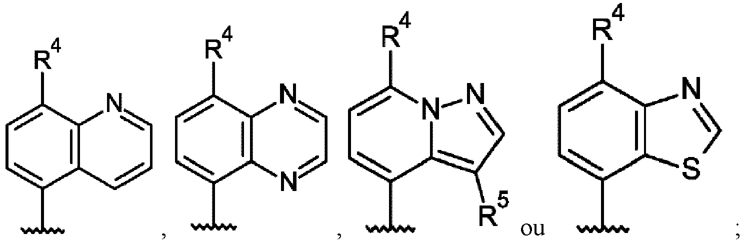 dans lequel R⁴ représente un cyano ou un chlore ; R⁵ représente H ou un fluor ;
R² représente un méthyle ;
R³ représente un pipérazinyl-1,3-dihydropyrrolo[3,4-c]pyridinyle ; un pipérazinyl-3,4-dihydro-1H-2,7-naphtyridinyle ; un pipérazinyl-3,4-dihydro-1H-isoquinoléinyle ; un méthylpipérazinyl-3,4-dihydro-1H-isoquinoléinyle ; un hydroxy(pipérazinyl)-3,4-dihydro-1H-isoquinoléinyle ; un pipérazinyl-3,4-dihydro-2H-quinoléinyle ; un pipérazinyl-5,7-dihydropyrrolo[3,4-b]pyridinyle ; un pipérazinyl-5,7-dihydropyrrolo[3,4-d]pyrimidinyle ; un méthylpipérazinyl-5,7-dihydropyrrolo[3,4-d]pyrimidinyle ; un méthylpipérazinyl-6,7-dihydro-4H-thiazolo[5,4-c]pyridinyle ; un pipérazinyl-7,8-dihydro-5H-1,6-naphtyridinyle ; un (difluorométhyl)pipérazinylisoindolinyle ; un (diméthylamino)éthoxyisoindolinyle ; un (hydroxyméthyl)pipérazinylisoindolinyle ; un (méthoxyéthyl)pipérazinylisoindolinyle ; un 1,3,4,6,7,8,9,9a-octahydropyrido[1,2-a]pyrazinylisoindolinyle ; un 1,6-diazaspiro[3.3]heptanylisoindolinyle ; un 2,6-diazaspiro[3.3]heptanylisoindolinyle ; un 3,4,6,7,9,9a-hexahydro-1H-pyrazino[2,1-c][1,4]oxazinylisoindolinyle ; un 3,8-diazabicyclo[3.2.1]octanylisoindolinyle ; un 3-oxa-7,9-diazabicyclo[3.3.1]nonanylisoindolinyle ; un 4,7-diazaspiro[2.5]octanylisoindolinyle ; un aminoazétidinylisoindolinyle ; un aminoéthylaminoisoindolinyle ; un carboxypipérazinylisoindolinyle ; un cyclopropylpipérazinylisoindolinyle ; un diméthylpipérazinylisoindolinyle ; un diméthylpyridinylisoindolinyle ; un éthoxycarbonylpipérazinylisoindolinyle ; un méthoxycarbonylpipérazinylisoindolinyle ; un méthylimidazolylisoindolinyle ; un méthylpipérazinyl(fluoro)isoindolinyle ; un méthylpipérazinylisoindolinyle ; un morpholinylméthylisoindolinyle ; un oxopipérazinylisoindolinyle ; un pipérazinyl(fluoro)isoindolinyle ; un pipérazinyl(méthyl)isoindolinyle ; un pipérazinylisoindolinyle ; un pipéridinylisoindolinyle ; un pipéridinylméthylisoindolinyle ; un pyrrolidinylisoindolinyle ; un pyrrolidinyloxyisoindolinyle ; un (pipérazinylphényl)méthylamino ou un (pipérazinylphényl)méthyl(méthyl)amino ;
ou un sel pharmaceutiquement acceptable, un énantiomère ou un diastéréoisomère de celui-ci.

4. Composé selon la revendication 1 ou 2, dans lequel R¹ représente 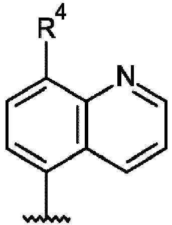 ou

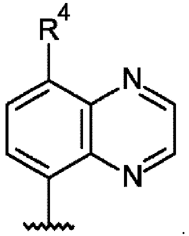

5. Composé selon la revendication 4, dans lequel R⁴ représente un cyano ou un chlore.

6. Composé selon la revendication 4, dans lequel
R³ représente un 1,3-dihydropyrrolo[3,4-c]pyridinyle substitué par un pipérazinyle ;
un 3,4-dihydro-1H-2,7-naphtyridinyle substitué par un pipérazinyle ;
un 3,4-dihydro-1H-isoquinoléinyle substitué par un ou deux substituants indépendamment choisis parmi un hydroxy, un pipérazinyle et un alkyle en C₁₋₆-pipérazinyle ;
un 3,4-dihydro-2H-quinoléinyle substitué par un pipérazinyle ;
un 5,7-dihydropyrrolo[3,4-b]pyridinyle substitué par un pipérazinyle ;
un 5,7-dihydropyrrolo[3,4-d]pyrimidinyle substitué par un pipérazinyle ou un alkyle en C₁₋₆-pipérazinyle ;
un 7,8-dihydro-5H-1,6-naphtyridinyle substitué par un pipérazinyle ;
un isoindolinyle substitué par un (alcoxy en C₁₋₆-alkyle en C₁₋₆)pipérazinyle, un (alkyle en C₁₋₆)₂pipérazinyle, un (hydroxyalkyle en C₁₋₆)pipérazinyle, un alkyle en C₁₋₆-pipérazinyle, un cycloalkyle en C₃₋₇-pipérazinyle, un halogénoalkyle en C₁₋₆-pipérazinyle, un morpholinylalkyle en C₁₋₆, un pipérazinyle, un pipéridinyle, un pipéridinylalkyle en C₁₋₆ ou un pyrrolidinyle ; ou
-NR^{6a}R^{6b} ; dans lequel
R^{6a} représente H ;
R^{6b} représente un phénylalkyle en C₁₋₆ substitué par un pipérazinyle.

7. Composé selon la revendication 6, dans lequel R³ représente un pipérazinyl-1,3-dihydropyrrolo[3,4-c]pyridinyle ; un pipérazinyl-3,4-dihydro-1H-2,7-naphtyridinyle ; un pipérazinyl-3,4-dihydro-1H-isoquinoléinyle ; un méthylpipérazinyl-3,4-dihydro-1H-isoquinoléinyle ; un hydroxy(pipérazinyl)-3,4-dihydro-1H-isoquinoléinyle ; un pipérazinyl-3,4-dihydro-2H-quinoléinyle ; un pipérazinyl-5,7-dihydropyrrolo[3,4-b]pyridinyle ; un pipérazinyl-5,7-dihydropyrrolo[3,4-d]pyrimidinyle ; un méthylpipérazinyl-5,7-dihydropyrrolo[3,4-d]pyrimidinyle ; un pipérazinyl-7,8-dihydro-5H-1,6-naphtyridinyle ; un (difluorométhyl)pipérazinylisoindolinyle ; un (hydroxyméthyl)pipérazinylisoindolinyle ; un (méthoxyéthyl)pipérazinylisoindolinyle ; un cyclopropylpipérazinylisoindolinyle ; un diméthylpipérazinylisoindolinyle ; un méthylpipérazinylisoindolinyle ; un morpholinylméthylisoindolinyle ; un pipérazinylisoindolinyle ; un pipéridinylisoindolinyle ; un pipéridinylméthylisoindolinyle ; un pyrrolidinylisoindolinyle ; ou un (pipérazinylphényl)méthylamino.

8. Composé selon la revendication 1 ou 2, dans lequel
R¹ représente 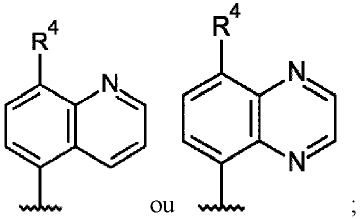 dans lequel R⁴ représente un cyano ou un halogène ;
R² représente un alkyle en C₁₋₆ ;
R³ représente un 1,3-dihydropyrrolo[3,4-c]pyridinyle substitué par un pipérazinyle ;
un 3,4-dihydro-1H-2,7-naphtyridinyle substitué par un pipérazinyle ;
un 3,4-dihydro-1H-isoquinoléinyle substitué par un ou deux substituants indépendamment choisis parmi un hydroxy, un pipérazinyle et un alkyle en C₁₋₆-pipérazinyle ;
un 3,4-dihydro-2H-quinoléinyle substitué par un pipérazinyle ;
un 5,7-dihydropyrrolo[3,4-b]pyridinyle substitué par un pipérazinyle ;
un 5,7-dihydropyrrolo[3,4-d]pyrimidinyle substitué par un pipérazinyle ou un alkyle en C₁₋₆-pipérazinyle ;
un 7,8-dihydro-5H-1,6-naphtyridinyle substitué par un pipérazinyle ;
un isoindolinyle substitué par un (alcoxy en C₁₋₆-alkyle en C₁₋₆)pipérazinyle, un (alkyle en C₁₋₆)₂pipérazinyle, un (hydroxyalkyle en C₁₋₆)pipérazinyle, un alkyle en C₁₋₆-pipérazinyle, un cycloalkyle en C₃₋₇-pipérazinyle, un halogénoalkyle en C₁₋₆-pipérazinyle, un morpholinylalkyle en C₁₋₆, un pipérazinyle, un pipéridinyle, un pipéridinylalkyle en C₁₋₆ ou un pyrrolidinyle ; ou
-NR^{6a}R^{6b} ; dans lequel
R^{6a} représente H ;
R^{6b} représente un phénylalkyle en C₁₋₆ substitué par un pipérazinyle ;
ou un sel pharmaceutiquement acceptable, un énantiomère ou un diastéréoisomère de celui-ci.

9. Composé selon la revendication 8, dans lequel
R¹ représente 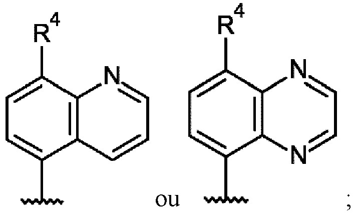 dans lequel R⁴ représente un cyano ou un chlore ;
R² représente un méthyle ;
R³ représente un pipérazinyl-1,3-dihydropyrrolo[3,4-c]pyridinyle ; un pipérazinyl-3,4-dihydro-1H-2,7-naphtyridinyle ; un pipérazinyl-3,4-dihydro-1H-isoquinoléinyle ; un méthylpipérazinyl-3,4-dihydro-1H-isoquinoléinyle ; un hydroxy(pipérazinyl)-3,4-dihydro-1H-isoquinoléinyle ; un pipérazinyl-3,4-dihydro-2H-quinoléinyle ; un pipérazinyl-5,7-dihydropyrrolo[3,4-b]pyridinyle ; un pipérazinyl-5,7-dihydropyrrolo[3,4-d]pyrimidinyle ; un méthylpipérazinyl-5,7-dihydropyrrolo[3,4-d]pyrimidinyle ; un pipérazinyl-7,8-dihydro-5H-1,6-naphtyridinyle ; un (difluorométhyl)pipérazinylisoindolinyle ; un (hydroxyméthyl)pipérazinylisoindolinyle ; un (méthoxyéthyl)pipérazinylisoindolinyle ; un cyclopropylpipérazinylisoindolinyle ; un diméthylpipérazinylisoindolinyle ; un méthylpipérazinylisoindolinyle ; un morpholinylméthylisoindolinyle ; un pipérazinylisoindolinyle ; un pipéridinylisoindolinyle ; un pipéridinylméthylisoindolinyle ; un pyrrolidinylisoindolinyle ; ou un (pipérazinylphényl)méthylamino ;
ou un sel pharmaceutiquement acceptable, un énantiomère ou un diastéréoisomère de celui-ci.

10. Composé selon la revendication 1 ou 2, choisi parmi :
le 4-[(2*R*,6*S*)-2-méthyl-6-[(4-pipérazin-1-ylisoindolin-2-yl)méthyl]morpholin-4-yl]pyrazolo[1,5-a]pyridine-7-carbonitrile ;
le 4-[(2*R*,6*S*)-2-méthyl-6-[(5-pipérazin-1-ylisoindolin-2-yl)méthyl]morpholin-4-yl]pyrazolo[1,5-a]pyridine-7-carbonitrile ;
le 4-[(2*R*,6*S*)-2-méthyl-6-[(8-pipérazin-1-yl-3,4-dihydro-1*H*-isoquinoléin-2-yl)méthyl]morpholin-4-yl]pyrazolo[1,5-a]pyridine-7-carbonitrile ;
le 4-[(2*R*,6*S*)-2-méthyl-6-[(7-pipérazin-1-yl-3,4-dihydro-2*H*-quinoléin-1-yl)méthyl]morpholin-4-yl]pyrazolo[1,5-a]pyridine-7-carbonitrile ;
le 3-fluoro-4-[(2*R*,6*S*)-2-méthyl-6-[(5-pipérazin-1-ylisoindolin-2-yl)méthyl]morpholin-4-yl]pyrazolo[1,5-a]pyridine-7-carbonitrile ;
le 4-[(2*R*,6*S*)-2-méthyl-6-[[5-(4-pipéridyl)isoindolin-2-yl]méthyl]morpholin-4-yl]pyrazolo[1,5-a]pyridine-7-carbonitrile ;
le 4-[(2*R*,6*S*)-2-méthyl-6-[(5-pyrrolidin-3-ylisoindolin-2-yl)méthyl]morpholin-4-yl]pyrazolo[1,5-a]pyridine-7-carbonitrile ;
le 4-[(2*R*,6*S*)-2-méthyl-6-[(6-pipérazin-1-yl-3,4-dihydro-1*H*-isoquinoléin-2-yl)méthyl]morpholin-4-yl]pyrazolo[1,5-a]pyridine-7-carbonitrile ;
le 4-[(2*R*,6*S*)-2-méthyl-6-[(7-pipérazin-1-yl-3,4-dihydro-1*H*-isoquinoléin-2-yl)méthyl]morpholin-4-yl]pyrazolo[1,5-a]pyridine-7-carbonitrile ;
le 8-[(2*R*,6*S*)-2-méthyl-6-[(5-pipérazin-1-ylisoindolin-2-yl)méthyl]morpholin-4-yl]quinoxaline-5-carbonitrile ;
le 8-[(2*R*,6*S*)-2-méthyl-6-[(6-pipérazin-1-yl-3,4-dihydro-1*H*-isoquinoléin-2-yl)méthyl]morpholin-4-yl]quinoxaline-5-carbonitrile ;
le 3-fluoro-4-[(2*R*,6*S*)-2-méthyl-6-[(6-pipérazin-1-yl-3,4-dihydro-1*H*-isoquinoléin-2-yl)méthyl]morpholin-4-yl]pyrazolo[1,5-a]pyridine-7-carbonitrile ;
le 4-[(2*R*,6*S*)-2-méthyl-6-[[6-(4-méthylpipérazin-1-yl)-3,4-dihydro-1*H*-isoquinoléin-2-yl]méthyl]morpholin-4-yl]pyrazolo[1,5-a]pyridine-7-carbonitrile
le 4-[(2*R*,6*S*)-2-méthyl-6-[[5-(4-méthylpipérazin-1-yl)isoindolin-2-yl]méthyl]morpholin-4-yl]pyrazolo[1,5-a]pyridine-7-carbonitrile ;
le 4-[(2*R*,6*S*)-2-méthyl-6-[(3-pipérazin-1-yl-5,7-dihydropyrrolo[3,4-b]pyridin-6-yl)méthyl]morpholin-4-yl]pyrazolo[1,5-a]pyridine-7-carbonitrile ;
le 5-[(2*R*,6*S*)-2-méthyl-6-[(6-pipérazin-1-yl-3,4-dihydro-1*H*-isoquinoléin-2-yl)méthyl]morpholin-4-yl]quinoléine-8-carbonitrile ;
le 5-[(2*R*,6*S*)-2-méthyl-6-[(3-pipérazin-1-yl-5,7-dihydropyrrolo[3,4-b]pyridin-6-yl)méthyl]morpholin-4-yl]quinoléine-8-carbonitrile ;
le 5-[(2*R*,6*S*)-2-méthyl-6-[(5-pipérazin-1-ylisoindolin-2-yl)méthyl]morpholin-4-yl]quinoléine-8-carbonitrile ;
le 7-[(2*R*,6*S*)-2-méthyl-6-[(5-pipérazin-1-ylisoindolin-2-yl)méthyl]morpholin-4-yl]-1,3-benzothiazole-4-carbonitrile ;
le 7-[(2*R*,6*S*)-2-méthyl-6-[(6-pipérazin-1-yl-3,4-dihydro-1*H*-isoquinoléin-2-yl)méthyl]morpholin-4-yl]-1,3-benzothiazole-4-carbonitrile ;
le 7-[(2*R*,6*S*)-2-méthyl-6-[(3-pipérazin-1-yl-5,7-dihydropyrrolo[3,4-b]pyridin-6-yl)méthyl]morpholin-4-yl]-1,3-benzothiazole-4-carbonitrile ;
le 4-[(2*R*,6*S*)-2-[[5-(2-aminoéthylamino)isoindolin-2-yl]méthyl]-6-méthyl-morpholin-4-yl]pyrazolo[1,5-a]pyridine-7-carbonitrile ;
le 4-[(2*R*,6*S*)-2-méthy1-6-[[2-(4-méthylpipérazin-1-yl)-6,7-dihydro-4*H*-thiazolo[5,4-c]pyridin-5-yl]méthyl]morpholin-4-yl]pyrazolo[1,5-a]pyridine-7-carbonitrile ;
le 4-[(2*S*,6*R*)-2-[[5-[2-(diméthylamino)éthoxy]isoindolin-2-yl]méthyl]-6-méthyl-morpholin-4-yl]pyrazolo[1,5-a]pyridine-7-carbonitrile ;
le 3-fluoro-4-[(2*R*,6*S*)-2-méthyl-6-[(3-pipérazin-1-yl-5,7-dihydropyrrolo[3,4-b]pyridin-6-yl)méthyl]morpholin-4-yl]pyrazolo[1,5-a]pyridine-7-carbonitrile ;
le 8-[(2*R*,6*S*)-2-méthyl-6-[(3-pipérazin-1-yl-5,7-dihydropyrrolo[3,4-b]pyridin-6-yl)méthyl]morpholin-4-yl]quinoxaline-5-carbonitrile ;
la (2*R*,6*S*)-4-(8-chloro-5-quinoléyl)-2-méthyl-6-[(5-pipérazin-1-ylisoindolin-2-yl)méthyl]morpholine ;
la (2*R*,6*S*)-4-(8-chloro-5-quinoléyl)-2-méthyl-6-[(6-pipérazin-1-yl-3,4-dihydro-1*H-*isoquinoléin-2-yl)méthyl]morpholine ;
la (2*R*,6*S*)-4-(8-chloroquinoxalin-5-yl)-2-méthyl-6-[(5-pipérazin-1-ylisoindolin-2-yl)méthyl]morpholine ;
la (2*R*,6*S*)-4-(8-chloroquinoxalin-5-yl)-2-méthyl-6-[(6-pipérazin-1-yl-3,4-dihydro-1*H-*isoquinoléin-2-yl)méthyl]morpholine ;
le 8-[(2*S*,6*R*)-2-[[5-(4-cyclopropylpipérazin-1-yl)isoindolin-2-yl]méthyl]-6-méthyl-morpholin-4-yl]quinoxaline-5-carbonitrile ;
le 8-[(2*R*,6*S*)-2-méthyl-6-[[5-(3-méthylpipérazin-1-yl)isoindolin-2-yl]méthyl]morpholin-4-yl]quinoxaline-5-carbonitrile ;
le 8-[(2*S*,6*R*)-2-[[5-[4-(2-méthoxyéthyl)pipérazin-1-yl]isoindolin-2-yl]méthyl]-6-méthyl-morpholin-4-yl]quinoxaline-5-carbonitrile ;
le 8-[(2*S*,6*R*)-2-[[5-(4,7-diazaspiro[2.5]octan-7-yl)isoindolin-2-yl]méthyl]-6-méthyl-morpholin-4-yl]quinoxaline-5-carbonitrile ;
le 8-[(2*R*,6*S*)-2-méthyl-6-[[5-(2-oxopipérazin-1-yl)isoindolin-2-yl]méthyl]morpholin-4-yl]quinoxaline-5-carbonitrile ;
le 8-[(2*S*,6*R*)-2-[[5-(1,3,4,6,7,8,9,9a-octahydropyrido[1,2-a]pyrazin-2-yl)isoindolin-2-yl] méthyl] -6-méthyl-morpholin-4-yl] quinoxaline- 5 -carbonitrile ;
le 8-[(2*S*,6*R*)-2-[[5-(3,8-diazabicyclo[3.2.1]octan-8-yl)isoindolin-2-yl]méthyl]-6-méthyl-morpholin-4-yl]quinoxaline-5-carbonitrile ;
le 5-[(2*S*,6*R*)-2-[[5-(3,8-diazabicyclo[3.2.1]octan-3-yl)isoindolin-2-yl]méthyl]-6-méthyl-morpholin-4-yl]quinoléine-8-carbonitrile ;
le 5-[(2*S*,6*R*)-2-[[5-(3,8-diazabicyclo[3.2.1]octan-8-yl)isoindolin-2-yl]méthyl]-6-méthyl-morpholin-4-yl]quinoléine-8-carbonitrile ;
le 8-[(2*S*,6*R*)-2-[[5-(3,8-diazabicyclo[3.2.1]octan-3-yl)isoindolin-2-yl]méthyl]-6-méthyl-morpholin-4-yl]quinoxaline-5-carbonitrile ;
le 4-[(2*R*,6*S*)-2-méthyl-6-[[5-(1-méthylimidazol-4-yl)isoindolin-2-yl]méthyl]morpholin-4-yl]pyrazolo[1,5-a]pyridine-7-carbonitrile ;
le 5-[(2*R*,6*S*)-2-méthyl-6-[[5-(3-oxa-7,9-diazabicyclo[3.3.1]nonan-9-yl)isoindolin-2-yl]méthyl]morpholin-4-yl]quinoléine-8-carbonitrile ;
le 5-[(2*S*,6*R*)-2-[[5-(2,6-diazaspiro[3.3]heptan-2-yl)isoindolin-2-yl]méthyl]-6-méthyl-morpholin-4-yl]quinoléine-8-carbonitrile ;
le 5-[(2R,6S)-2-méthyl-6-[(1-méthyl-5-pipérazin-1-yl-isoindolin-2-yl)méthyl]morpholin-4-yl]quinoléine-8-carbonitrile ;
le 8-[(2*R*,6*S*)-2-méthyl-6-[[5-(3-oxa-7,9-diazabicyclo[3.3.1]nonan-7-yl)isoindolin-2-yl]méthyl]morpholin-4-yl]quinoxaline-5-carbonitrile ;
le 1-[2-[[(2*S*,6*R*)-4-(8-cyano-5-quinoléyl)-6-méthyl-morpholin-2-yl]méthyl]isoindolin-5-yl]pipérazine-2-carboxylate d'éthyle ;
le 4-[(2*R*,6*S*)-2-méthyl-6-[[2-(4-méthylpipérazin-1-yl)-5,7-dihydropyrrolo[3,4-d]pyrimidin-6-yl]méthyl]morpholin-4-yl]pyrazolo[1,5-a]pyridine-7-carbonitrile ;
le 4-[(2*R*,6*S*)-2-méthyl-6-[(5-pyrrolidin-3-yloxyisoindolin-2-yl)méthyl]morpholin-4-yl]pyrazolo[1,5-a]pyridine-7-carbonitrile ;
le 4-[(2*S*,6*R*)-2-[[5-(2,6-diméthyl-4-pyridyl)isoindolin-2-yl]méthyl]-6-méthyl-morpholin-4-yl]pyrazolo[1,5-a]pyridine-7-carbonitrile ;
le 4-[(2*S*,6*R*)-2-[[5-(2,6-diméthyl-4-pyridyl)isoindolin-2-yl]méthyl]-6-méthyl-morpholin-4-yl]-3-fluoro-pyrazolo[1,5-a]pyridine-7-carbonitrile ;
le 5-[(2*S*,6*R*)-2-[[5-(1,6-diazaspiro[3.3]heptan-6-yl)isoindolin-2-yl]méthyl]-6-méthyl-morpholin-4-yl]quinoléine-8-carbonitrile ;
l'acide 1-[2-[[(2*S*,6*R*)-4-(8-cyano-5-quinoléyl)-6-méthyl-morpholin-2-yl]méthyl]isoindolin-5-yl]pipérazine-2-carboxylique ;
le *trans*-5-[(2*R*,6*S*)-2-méthyl-6-[[5-[3,5-diméthylpipérazin-1-yl]isoindolin-2-yl]méthyl]morpholin-4-yl]quinoléine-8-carbonitrile ;
le 5-[(2*S*,6*R*)-2-[[5-(3,4,6,7,9,9a-hexahydro-1*H*-pyrazino[2,1-c][1,4]oxazin-8-yl)isoindolin-2-yl]méthyl]-6-méthyl-morpholin-4-yl]quinoléine-8-carbonitrile ;
le 5-[(2*S*,6*R*)-2-[(4-fluoro-6-pipérazin-1-yl-isoindolin-2-yl)méthyl]-6-méthyl-morpholin-4-yl]quinoléine-8-carbonitrile ;
le 8-[(2*R*,6*S*)-2-méthyl-6-[(6-pipérazin-1-yl-1,3-dihydropyrrolo[3,4-c]pyridin-2-yl)méthyl]morpholin-4-yl]quinoxaline-5-carbonitrile ;
le 5-[(2*R*,6*S*)-2-méthyl-6-[(6-pipérazin-1-yl-1,3-dihydropyrrolo[3,4-c]pyridin-2-yl)méthyl]morpholin-4-yl]quinoléine-8-carbonitrile ;
le 5-[(2*R*,6*S*)-2-méthyl-6-[[5-[(3*R*)-3-méthylpipérazin-1-yl]isoindolin-2-yl]méthyl]morpholin-4-yl]quinoléine-8-carbonitrile ;
le 5-[(2*R*,6*S*)-2-méthyl-6-[(2-pipérazin-1-yl-5,7-dihydropyrrolo[3,4-d]pyrimidin-6-yl)méthyl]morpholin-4-yl]quinoléine-8-carbonitrile ;
le 5-[(2*R*,6*S*)-2-méthyl-6-[[5-[(3*S*)-3-méthylpipérazin-1-yl]isoindolin-2-yl]méthyl]morpholin-4-yl]quinoléine-8-carbonitrile ;
le 8-[(2*R*,6*S*)-2-méthyl-6-[[5-[(3*R*)-3-méthylpipérazin-1-yl]isoindolin-2-yl]méthyl]morpholin-4-yl]quinoxaline-5-carbonitrile ;
le 8-[(2*R*,6*S*)-2-méthyl-6-[[5-[(3*S*)-3-méthylpipérazin-1-yl]isoindolin-2-yl]méthyl]morpholin-4-yl]quinoxaline-5-carbonitrile ;
le 4-[2-[[(2*S*,6*R*)-4-(8-cyano-5-quinoléyl)-6-méthyl-morpholin-2-yl]méthyl]isoindolin-5-yl]pipérazine-2-carboxylate de méthyle ;
l'acide 4-[2-[[(2*S*,6*R*)-4-(8-cyano-5-quinoléyl)-6-méthyl-morpholin-2-yl]méthyl]isoindolin-5-yl]pipérazine-2-carboxylique ;
le 5-[(2*S*,6*R*)-2-[[4-fluoro-6-(4-méthylpipérazin-1-yl)isoindolin-2-yl]méthyl]-6-méthyl-morpholin-4-yl]quinoléine-8-carbonitrile ;
le 5-[(2*R*,6*S*)-2-méthyl-6-[(6-pipérazin-1-yl-3,4-dihydro-1*H*-2,7-naphtyridin-2-yl)méthyl]morpholin-4-yl]quinoléine-8-carbonitrile ;
le 5-[(2*R*,6*S*)-2-méthyl-6-[[5-[(3*R*,5*S*)-3,5-diméthylpipérazin1-yl]isoindolin-2-yl]méthyl]morpholin-4-yl]quinoléine-8-carbonitrile ;
le 5-[(2*R*,6*S*)-2-méthyl-6-[[5-(3-oxa-7,9-diazabicyclo[3.3.1]nonan-7-yl)isoindolin-2-yl]méthyl]morpholin-4-yl]quinoléine-8-carbonitrile ;
le 5-[(2*S*,6*R*)-2-[[5-[2-(hydroxyméthyl)pipérazin-1-yl]isoindolin-2-yl]méthyl]-6-méthyl-morpholin-4-yl]quinoléine-8-carbonitrile ;
le 5-[(2*R*,6*S*)-2-méthyl-6-[[5-(1-pipéridylméthyl)isoindolin-2-yl]méthyl]morpholin-4-yl]quinoléine-8-carbonitrile ;
le 5-[(2S,6R)-2-[(4-hydroxy-6-pipérazin-1-yl-3,4-dihydro-1H-isoquinoléin-2-yl)méthyl]-6-méthyl-morpholin-4-yl]quinoléine-8-carbonitrile ;
le 5-[(2*S*,6*R*)-2-[[5-[2-(difluorométhyl)pipérazin-1-yl]isoindolin-2-yl]méthyl]-6-méthyl-morpholin-4-yl]quinoléine-8-carbonitrile ;
le 5-[(2*S*,6*R*)-2-[[5-(3-aminoazétidin-1-yl)isoindolin-2-yl]méthyl]-6-méthyl-morpholin-4-yl]quinoléine-8-carbonitrile ;
le 5-[(2*R*,6*S*)-2-méthyl-6-[[5-(2-méthylpipérazin-1-yl)isoindolin-2-yl]méthyl]morpholin-4-yl]quinoléine-8-carbonitrile ;
le 5-[(2*R*,6*S*)-2-méthyl-6-[(2-pipérazin-1-yl-7,8-dihydro-5*H*-1,6-naphtyridin-6-yl)méthyl]morpholin-4-yl]quinoléine-8-carbonitrile ;
le 5-[(2*R*,6*S*)-2-méthyl-6-[(2-pipérazin-1-yl-5,7-dihydropyrrolo[3,4-b]pyridin-6-yl)méthyl]morpholin-4-yl]quinoléine-8-carbonitrile ;
le 5-[(2S,6R)-2-[[5-[(2R)-2-(hydroxyméthyl)pipérazin-1-yl]isoindolin-2-yl]méthyl]-6-méthyl-morpholin-4-yl]quinoléine-8-carbonitrile ;
le 5-[(2S,6R)-2-[[5-[(2S)-2-(hydroxyméthyl)pipérazin-1-yl]isoindolin-2-yl]méthyl]-6-méthyl-morpholin-4-yl]quinoléine-8-carbonitrile ;
le 5-[(2*S*,6*R*)-2-[[5-[3-(hydroxyméthyl)pipérazin-1-yl]isoindolin-2-yl]méthyl]-6-méthyl-morpholin-4-yl]quinoléine-8-carbonitrile ;
le 5-[(2*R*,6*S*)-2-méthyl-6-[[5-(morpholinométhyl)isoindolin-2-yl]méthyl]morpholin-4-yl]quinoléine-8-carbonitrile ;
ou un sel pharmaceutiquement acceptable, un énantiomère ou un diastéréoisomère de ceux-ci.

11. Procédé de préparation d'un composé selon l'une quelconque des revendications 1 à 10 comprenant l'une quelconque des étapes suivantes :
a) le couplage d'un composé de formule (VI), 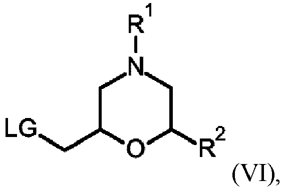 avec une amine (VII) en présence d'une base ;
b) le couplage d'un composé de formule (VI), 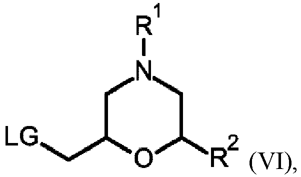 avec une amine (VIII) en présence d'une base ;
c) le couplage d'un composé de formule (IX), 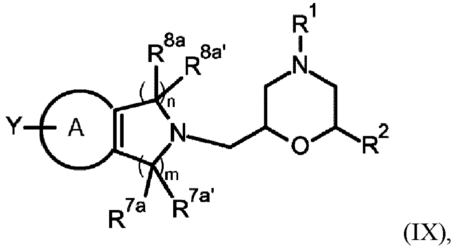 avec une amine (X) dans des conditions d'amination de Buchwald-Hartwig ;
dans lequel la base de l'étape a) et b) est le K₂CO₃, la DIPEA ou le Cs₂CO₃ ; la condition d'amination de Buchwald-Hartwig de l'étape c) comprend un catalyseur qui est le Ruphos Pd G2, et une base qui est le Cs₂CO₃ ; LG représente OTf, OTs ou OMs ; Y représente un halogène, un ((alkyle en C₁₋₆)₂amino)alcoxy en C₁₋₆, -NR⁹R¹⁰, un hétérocyclyle ou un hétérocyclylalkyle en C₁₋₆ ; R^{7a}, R^{7b}, R^{8a}, R^{8b} sont indépendamment choisis parmi H et un alkyle en C₁₋₆ ; R⁹ et R¹⁰ sont indépendamment choisis parmi H, un aminoalkyle en C₁₋₆-amino et un aminoazétidinyle ; ou R⁹ et R¹⁰ conjointement avec l'azote auquel ils sont liés forment un hétérocyclyle ; R¹ et R² sont tels que définis dans l'une quelconque des revendications 1 à 9.

12. Composé ou sel pharmaceutiquement acceptable, énantiomère ou diastéréoisomère selon l'une quelconque des revendications 1 à 10 pour une utilisation comme substance thérapeutiquement active.

13. Composition pharmaceutique comprenant un composé selon l'une quelconque des revendications 1 à 10 et un véhicule thérapeutiquement inerte.

14. Composé ou sel pharmaceutiquement acceptable, énantiomère ou diastéréoisomère selon l'une quelconque des revendications 1 à 10 pour une utilisation dans le traitement ou la prophylaxie du lupus érythémateux disséminé ou de la néphropathie lupique.
